(19) Europäisches Patentamt
European Patent Office
Office européen des brevets

(11) EP 3 842 537 A1

(12) EUROPEAN PATENT APPLICATION

(43) Date of publication:
30.06.2021 Bulletin 2021/26

(51) Int Cl.:
*C12N 15/67* (2006.01) *C12N 15/85* (2006.01)

(21) Application number: 20202943.5

(22) Date of filing: 30.12.2014

(84) Designated Contracting States:
AL AT BE BG CH CY CZ DE DK EE ES FI FR GB GR HR HU IE IS IT LI LT LU LV MC MK MT NL NO PL PT RO RS SE SI SK SM TR

(30) Priority: 30.12.2013 PCT/EP2013/003946

(62) Document number(s) of the earlier application(s) in accordance with Art. 76 EPC:
18199987.1 / 3 495 486
14854873.8 / 3 090 053

(71) Applicant: CureVac AG
72076 Tübingen (DE)

(72) Inventor: THEß, Andreas
72706 Tübingen (DE)

(74) Representative: Graf von Stosch Patentanwaltsgesellschaft mbH
Prinzregentenstraße 22
80538 München (DE)

Remarks:
•This application was filed on 21-10-2020 as a divisional application to the application mentioned under INID code 62.
•Claims filed after the date of filing of the application / after the date of receipt of the divisional application (Rule 68(4) EPC).

# (54) ARTIFICIAL NUCLEIC ACID MOLECULES

(57) The invention relates to an artificial nucleic acid molecule comprising at least one open reading frame and at least one 3'-untranslated region element (3'-UTR) element comprising a nucleic acid sequence which is derived from the 3'-UTR of a ribosomal protein gene. The invention further relates to the use of such an artificial nucleic acid molecule in gene therapy and/or genetic vaccination. Furthermore, the invention relates to the use of a 3'-UTR element comprising a nucleic acid sequence which is derived from the 3'-UTR of a ribosomal protein gene for enhancing, stabilizing and/or prolonging protein expression from a nucleic acid sequence comprising such 3'-UTR element.

EP 3 842 537 A1

SEQUENCE LISTING

<110> CureVac GmbH

<120> Artificial nucleic acid molecules

<130> CU01P155WO1

<160> 206

<170> PatentIn version 3.5

<210> 1
<211> 70
<212> DNA
<213> Artificial Sequence

<220>
<223> 3-UTR element of Homo sapiens ribosomal protein S9 (RPS9)

<400> 1
gtccacctgt ccctcctggg ctgctggatt gtctcgtttt cctgccaaat aaacaggatc 60

agcgctttac 70

<210> 2
<211> 70
<212> RNA
<213> Artificial Sequence

<220>
<223> 3-UTR element of Homo sapiens ribosomal protein S9 (RPS9)

<400> 2
guccaccugu cccuccuggg cugcuggauu gucucguuuu ccugccaaau aaacaggauc 60

agcgcuuuac 70

<210> 3
<211> 140
<212> DNA
<213> Artificial Sequence

<220>
<223> 3-UTR element of Homo sapiens ribosomal protein S9 (RPS9) + poly A

<400> 3
gtccacctgt ccctcctggg ctgctggatt gtctcgtttt cctgccaaat aaacaggatc 60

agcgctttac agatctaaaa aaaaaaaaaa aaaaaaaaaa aaaaaaaaaa aaaaaaaaaa 120

aaaaaaaaaa aaaaaaaaaa 140

<210> 4
<211> 140
<212> RNA
<213> Artificial Sequence

<220>

<223> 3-UTR element of Homo sapiens ribosomal protein S9 (RPS9) + poly A

<400> 4
guccaccugu cccuccuggg cugcuggauu gucucguuuu ccugccaaau aaacaggauc 60

agcgcuuuac agaucuaaaa aaaaaaaaaa aaaaaaaaaa aaaaaaaaaa aaaaaaaaaa 120

aaaaaaaaaa aaaaaaaaaa 140


<210> 5
<211> 24
<212> DNA
<213> Artificial Sequence

<220>
<223> A particular preferred histone stem-loop sequence

<400> 5
caaaggctct tttcagagcc acca 24


<210> 6
<211> 42
<212> DNA
<213> Artificial Sequence

<220>
<223> 5-UTR of human ribosomal protein Large 32 lacking the 5 terminal oligopyrimidine tract

<400> 6
ggcgctgcct acggaggtgg cagccatctc cttctcggca tc 42


<210> 7
<211> 1918
<212> DNA
<213> Artificial Sequence

<220>
<223> rpl32 PpLuc(GC) rps9 A64 C30 histoneSL

<400> 7
ggggcgctgc ctacggaggt ggcagccatc tccttctcgg catcaagctt gaggatggag 60

gacgccaaga acatcaagaa gggcccggcg cccttctacc cgctggagga cgggaccgcc 120

ggcgagcagc tccacaaggc catgaagcgg tacgccctgg tgccgggcac gatcgccttc 180

accgacgccc acatcgaggt cgacatcacc tacgcggagt acttcgagat gagcgtgcgc 240

ctggccgagg ccatgaagcg gtacggcctg aacaccaacc accggatcgt ggtgtgctcg 300

gagaacagcc tgcagttctt catgccggtg ctgggcgccc tcttcatcgg cgtggccgtc 360

gccccggcga acgacatcta caacgagcgg gagctgctga acagcatggg gatcagccag 420

ccgaccgtgg tgttcgtgag caagaagggc ctgcagaaga tcctgaacgt gcagaagaag 480

ctgcccatca tccagaagat catcatcatg gacagcaaga ccgactacca gggcttccag 540

tcgatgtaca cgttcgtgac cagccacctc ccgccgggct tcaacgagta cgacttcgtc 600

ccggagagct tcgaccggga caagaccatc gccctgatca tgaacagcag cggcagcacc 660

ggcctgccga agggggtggc cctgccgcac cggaccgcct gcgtgcgctt ctcgcacgcc 720

cgggacccca tcttcggcaa ccagatcatc ccggacaccg ccatcctgag cgtggtgccg 780

ttccaccacg gcttcggcat gttcacgacc ctgggctacc tcatctgcgg cttccgggtg 840

gtcctgatgt accggttcga ggaggagctg ttcctgcgga gcctgcagga ctacaagatc 900

cagagcgcgc tgctcgtgcc gaccctgttc agcttcttcg ccaagagcac cctgatcgac 960

aagtacgacc tgtcgaacct gcacgagatc gccagcgggg gcgccccgct gagcaaggag 1020

gtgggcgagg ccgtggccaa gcggttccac ctcccgggca tccgccaggg ctacggcctg 1080

accgagacca cgagcgcgat cctgatcacc cccgaggggg acgacaagcc gggcgccgtg 1140

ggcaaggtgg tcccgttctt cgaggccaag gtggtggacc tggacaccgg caagaccctg 1200

ggcgtgaacc agcggggcga gctgtgcgtg cgggggccga tgatcatgag cggctacgtg 1260

aacaacccgg aggccaccaa cgccctcatc gacaaggacg gctggctgca cagcggcgac 1320

atcgcctact gggacgagga cgagcacttc ttcatcgtcg accggctgaa gtcgctgatc 1380

aagtacaagg gctaccaggt ggcgccggcc gagctggaga gcatcctgct ccagcacccc 1440

aacatcttcg acgccggcgt ggccgggctg ccggacgacg acgccggcga gctgccggcc 1500

gcggtggtgg tgctggagca cggcaagacc atgacggaga aggagatcgt cgactacgtg 1560

gccagccagg tgaccaccgc caagaagctg cggggcggcg tggtgttcgt ggacgaggtc 1620

ccgaagggcc tgaccgggaa gctcgacgcc cggaagatcc gcgagatcct gatcaaggcc 1680

aagaagggcg gcaagatcgc cgtgtaagac tagtgtccac ctgtccctcc tgggctgctg 1740

gattgtctcg ttttcctgcc aaataaacag gatcagcgct ttacagatct aaaaaaaaaa 1800

aaaaaaaaaa aaaaaaaaaa aaaaaaaaaa aaaaaaaaaa aaaaaaaaaa aaaatgcatc 1860

cccccccccc cccccccccc cccccccccc aaaggctctt ttcagagcca ccagaatt 1918

<210> 8
<211> 1848
<212> DNA
<213> Artificial Sequence

<220>
<223> rpl32 PpLuc(GC) A64 C30 histoneSL

<400> 8
ggggcgctgc ctacggaggt ggcagccatc tccttctcgg catcaagctt gaggatggag 60

gacgccaaga acatcaagaa gggcccggcg cccttctacc cgctggagga cgggaccgcc 120

ggcgagcagc tccacaaggc catgaagcgg tacgccctgg tgccgggcac gatcgccttc 180

accgacgccc acatcgaggt cgacatcacc tacgcggagt acttcgagat gagcgtgcgc 240

```
ctggccgagg ccatgaagcg gtacggcctg aacaccaacc accggatcgt ggtgtgctcg      300
gagaacagcc tgcagttctt catgccggtg ctgggcgccc tcttcatcgg cgtggccgtc      360
gccccggcga acgacatcta caacgagcgg gagctgctga acagcatggg gatcagccag      420
ccgaccgtgg tgttcgtgag caagaagggc ctgcagaaga tcctgaacgt gcagaagaag      480
ctgcccatca tccagaagat catcatcatg gacagcaaga ccgactacca gggcttccag      540
tcgatgtaca cgttcgtgac cagccacctc ccgccgggct tcaacgagta cgacttcgtc      600
ccggagagct tcgaccggga caagaccatc gccctgatca tgaacagcag cggcagcacc      660
ggcctgccga agggggtggc cctgccgcac cggaccgcct gcgtgcgctt ctcgcacgcc      720
cgggacccca tcttcggcaa ccagatcatc ccggacaccg ccatcctgag cgtggtgccg      780
ttccaccacg gcttcggcat gttcacgacc ctgggctacc tcatctgcgg cttccgggtg      840
gtcctgatgt accggttcga ggaggagctg ttcctgcgga gcctgcagga ctacaagatc      900
cagagcgcgc tgctcgtgcc gaccctgttc agcttcttcg ccaagagcac cctgatcgac      960
aagtacgacc tgtcgaacct gcacgagatc gccagcgggg gcgccccgct gagcaaggag     1020
gtgggcgagg ccgtggccaa gcggttccac ctcccgggca tccgccaggg ctacggcctg     1080
accgagacca cgagcgcgat cctgatcacc cccgaggggg acgacaagcc gggcgccgtg     1140
ggcaaggtgg tcccgttctt cgaggccaag gtggtggacc tggacaccgg caagaccctg     1200
ggcgtgaacc agcggggcga gctgtgcgtg cgggggccga tgatcatgag cggctacgtg     1260
aacaacccgg aggccaccaa cgccctcatc gacaaggacg gctggctgca cagcggcgac     1320
atcgcctact gggacgagga cgagcacttc ttcatcgtcg accggctgaa gtcgctgatc     1380
aagtacaagg gctaccaggt ggcgccggcc gagctggaga gcatcctgct ccagcacccc     1440
aacatcttcg acgccggcgt ggccgggctg ccggacgacg acgccggcga gctgccggcc     1500
gcggtggtgg tgctggagca cggcaagacc atgacggaga aggagatcgt cgactacgtg     1560
gccagccagg tgaccaccgc caagaagctg cggggcggcg tggtgttcgt ggacgaggtc     1620
ccgaagggcc tgaccgggaa gctcgacgcc cggaagatcc gcgagatcct gatcaaggcc     1680
aagaagggcg gcaagatcgc cgtgtaagac tagtagatct aaaaaaaaaa aaaaaaaaaa     1740
aaaaaaaaaa aaaaaaaaaa aaaaaaaaaa aaaaaaaaaa aaaatgcatc cccccccccc     1800
cccccccccc cccccccccc aaaggctctt ttcagagcca ccagaatt                  1848
```

<210> 9
<211> 1914
<212> DNA
<213> Artificial Sequence

<220>
<223> rpl32 PpLuc(GC) ag A64 C30 histoneSL

<400> 9
gggcgctgc ctacggaggt ggcagccatc tccttctcgg catcaagctt gaggatggag 60

gacgccaaga acatcaagaa gggcccggcg cccttctacc cgctggagga cgggaccgcc 120

ggcgagcagc tccacaaggc catgaagcgg tacgccctgg tgccgggcac gatcgccttc 180

accgacgccc acatcgaggt cgacatcacc tacgcggagt acttcgagat gagcgtgcgc 240

ctggccgagg ccatgaagcg gtacggcctg aacaccaacc accggatcgt ggtgtgctcg 300

gagaacagcc tgcagttctt catgccggtg ctgggcgccc tcttcatcgg cgtggccgtc 360

gccccggcga acgacatcta caacgagcgg gagctgctga acagcatggg gatcagccag 420

ccgaccgtgg tgttcgtgag caagaagggc ctgcagaaga tcctgaacgt gcagaagaag 480

ctgcccatca tccagaagat catcatcatg gacagcaaga ccgactacca gggcttccag 540

tcgatgtaca cgttcgtgac cagccacctc ccgccgggct tcaacgagta cgacttcgtc 600

ccggagagct tcgaccggga caagaccatc gccctgatca tgaacagcag cggcagcacc 660

ggcctgccga agggggtggc cctgccgcac cggaccgcct gcgtgcgctt ctcgcacgcc 720

cgggaccccca tcttcggcaa ccagatcatc ccggacaccg ccatcctgag cgtggtgccg 780

ttccaccacg gcttcggcat gttcacgacc ctgggctacc tcatctgcgg cttccgggtg 840

gtcctgatgt accggttcga ggaggagctg ttcctgcgga gcctgcagga ctacaagatc 900

cagagcgcgc tgctcgtgcc gaccctgttc agcttcttcg ccaagagcac cctgatcgac 960

aagtacgacc tgtcgaacct gcacgagatc gccagcgggg gcgccccgct gagcaaggag 1020

gtgggcgagg ccgtggccaa gcggttccac ctcccgggca tccgccaggg ctacggcctg 1080

accgagacca cgagcgcgat cctgatcacc cccgaggggg acgacaagcc gggcgccgtg 1140

ggcaaggtgg tcccgttctt cgaggccaag gtggtggacc tggacaccgg caagaccctg 1200

ggcgtgaacc agcggggcga gctgtgcgtg cgggggccga tgatcatgag cggctacgtg 1260

aacaacccgg aggccaccaa cgccctcatc gacaaggacg gctggctgca cagcggcgac 1320

atcgcctact gggacgagga cgagcacttc ttcatcgtcg accggctgaa gtcgctgatc 1380

aagtacaagg gctaccaggt ggcgccggcc gagctggaga gcatcctgct ccagcacccc 1440

aacatcttcg acgccggcgt ggccgggctg ccggacgacg acgccggcga gctgccggcc 1500

gcggtggtgg tgctggagca cggcaagacc atgacggaga aggagatcgt cgactacgtg 1560

gccagccagg tgaccaccgc caagaagctg cggggcggcg tggtgttcgt ggacgaggtc 1620

ccgaagggcc tgaccgggaa gctcgacgcc cggaagatcc gcgagatcct gatcaaggcc 1680

aagaagggcg gcaagatcgc cgtgtaagac tagttataag actgactagc ccgatgggcc 1740

tcccaacggg ccctcctccc ctccttgcac cgagattaat agatctaaaa aaaaaaaaaa 1800

aaaaaaaaaa aaaaaaaaaa aaaaaaaaaa aaaaaaaaaa aaaaaaaaaa tgcatccccc 1860

cccccccccc cccccccccc ccccccaaag gctcttttca gagccaccag aatt 1914

<210> 10
<211> 109
<212> DNA
<213> Homo sapiens

<400> 10
gatctaagag ttacctggct acagaaagaa gatgccagat gacacttaag acctacttgt 60

gatatttaaa tgatgcaata aaagacctat tgatttggac cttcttctt 109

<210> 11
<211> 57
<212> DNA
<213> Homo sapiens

<400> 11
tgccaggaac agattttgca gttggtgggg tctcaataaa agttattttc cactgac 57

<210> 12
<211> 91
<212> DNA
<213> Homo sapiens

<400> 12
actcttaaat ttgattattc cataaaggtc aaatcatttt ggacagcttc ttttgaataa 60

agacctgatt atacaggcag tgagaaacat g 91

<210> 13
<211> 58
<212> DNA
<213> Homo sapiens

<400> 13
acccagcaat tttctatgat tttttcagat atagataata aacttatgaa cagcaact 58

<210> 14
<211> 33
<212> DNA
<213> Homo sapiens

<400> 14
atgtcttaag aacctaatta aatagctgac tac 33

<210> 15
<211> 67
<212> DNA
<213> Homo sapiens

<400> 15
ggtgtctacc atgattattt ttctaagctg gttggttaat aaacagtacc tgctctcaaa 60

ttgaaat 67

<210> 16
<211> 59
<212> DNA
<213> Homo sapiens

<400> 16
atgtacactg ttgagttttc tgtacataaa aataattgaa ataatacaaa ttttccttc 59

<210> 17
<211> 50
<212> DNA
<213> Homo sapiens

<400> 17
attcccgttt ctatccaaaa gagcaataaa aagttttcag tgaaatgtgc 50

<210> 18
<211> 46
<212> DNA
<213> Homo sapiens

<400> 18
gcacaaagga aaacatttca ataaaggatc atttgacaac tggtgg 46

<210> 19
<211> 3787
<212> DNA
<213> Homo sapiens

<400> 19
agccctcctg gggacttgga atcagtcggc agtcatgctg ggtctccacg tggtgtgttt 60

cgtgggaaca actgggcctg ggatggggct tcactgctgt gacttcctcc tgccagggga 120

tttggggctt tcttgaaaga cagtccaagc cctggataat gctttacttt ctgtgttgaa 180

gcactgttgg ttgtttggtt agtgactgat gtaaaacggt tttcttgtgg ggaggttaca 240

gaggctgact tcagagtgga cttgtgtttt ttctttttaa agaggcaagg ttgggctggt 300

gctcacagct gtaatcccag cactttgagg ttggctggga gttcaagacc agcctggcca 360

acatgtcaga actactaaaa ataaagaaat cagccatgct tggtgctgca cacttgtagt 420

tgcagctcct gggaggcaga ggtgagggat cacttaaccc aggaggcaga ggctgcactg 480

agccaggatc acgccactgc actctagcct gggcaacagt gagactgtct caaaaaaaaa 540

aaaagagaca gggtcttcgg cacccaggct ggagtacagt gccacaatca tggctcactg 600

cagtcttgaa ctcatggcct caagcagtcc tccctcagcc tcccaagtag aggggtttat 660

aggcacgaga ccctgcaccc aacctagagt tgcctttttt aagcaaagca gtttctagtt 720

aatgtagcat cttggacttt ggggcgtcat tcttaagctt gttgtgcccg gtaaccatgg 780

tcctcttgct ctgattaacc cttccttcaa tgggcttctt cacccagaca ccaaggtatg 840

agatggccct gccaagtgtc ggcctctcct gttaaacaaa aacattctaa agccattgtt 900

cttgcttcat ggacaagagg cagccagaga gagtgccagg gtgccctggt ctgagctggc 960
atccccatgt cttctgtgtc cgagggcagc atggtttctc gtgcagtgct cagacacagc 1020
ctgccctagt cctaccagct cacagcagca cctgctctcc ttggcagcta tggccatgac 1080
aaccccagag aagcagcttc agggaccgag tcagattctg ttttgtctac atgcctctgc 1140
cgggtgccgg tattgaggca cccagggagc tgttactggc gtggaaatag gtgatgctgc 1200
tacctctgct gctgcactca cagccacact tgatacacga tgacaccttg cttgtttgga 1260
aacatctaaa catctagtag atgacttgca ggctgttggc taccagtttc ctgtctgagg 1320
tgtatatgtt aacttcgtga tcagtttgta tgtttgggac tcttgtccta tgtaaagtta 1380
aggtgggccg ggtgcagtgg ctcacgcctg taatcctaac actgggaggc cgaggcgggt 1440
ggatcacctg atggtgaaac ctcatctcta ctgaaaatac aaaaattagc tgagtggtga 1500
cacacgcctg taatcccagc tacttggtag gcttgaaccc aggaggcaga gattgcagtg 1560
agccgagctg caccactgtg ctccagcctg ggtgacagcg agactcagtc tcaaaaaaag 1620
ttgtacaagg tggatggttg gaagcttgag cctaggctcg aatccctctc acgtgagagg 1680
gcctgaagat ttctggtgga ttccaacctg gctgaagact ggccgtgggg ggtgcagggg 1740
tctccagcgc tctgccctcc agcctgcttc ctccctgccc acaccgcact aggggaaggg 1800
cctttcctgc tgcctgcggg gccgcacctg gagtaggtaa tgccatgtgg tgacgtgaat 1860
ggagcagagg tctgtgcccc atcacaccgc cttgctgttt ttactgtggg acaaaagcac 1920
tctgatctgc gtgttccggg ggccctccta ccagccgact tgacgggaag tcagggttca 1980
ggtatcatct gtgcacctgg ggcggggtag tctgcactga acctgccaga gtcccctcct 2040
catttcactg aaagtcacag tctccagggc tgtgttgcta accttacgtt ctctccgttt 2100
gcttaatcta ttaagagccc taacaggaga ggatgggctt tctctgttgt ctggggccct 2160
gctgttggcc ggtgctctta gcaagaggtc atttttctag gttgcgctgg gacattgtga 2220
gtttggtgag ggtcatggat gtgggctggg ctgggctggg ctgggccggg ctgcctgctg 2280
cctgctgctc ccctacctga aatgcagcta gtgcggctct gcccttcctg gggctgagga 2340
aggcttctgc aggatagctg gggggctggg caggtgggtg aggcagcctc cctgctgaca 2400
ctcagtcctt gtagctggag caagatctcc tgatccaggt acgggcctgt ctgctccaag 2460
aaagactctg ccaccagatg caaagggggcc ctttgtttta acttagtccc tggggaccgc 2520
ctgattcagc acctgtcggc ccaggatacc ccgctggtgg ggacaagtgc ctgagtgtgg 2580
gccgtgcccg agtgtggcca tccctgagtg gggccgtcct gactaggaag tggcttttca 2640
gttgtgatgt gtgggcctga cctaggggggc gctgtggaac ccgggctgga accagccctc 2700
tgtgccaggc cgcagacagg ttccgccggc cctgaggggc agctgccatg gcgtgggtca 2760

ctgggagctg agaggaaggg cccccaccgc acctcaggca aagcggctct gggaacacct 2820

tgatttcgtc catgtgagcc gtcccaggga gggcagccaa gctgtgaagc ctgagaaact 2880

gacctgtgtg ccacgagctt gtggtctgct gcccggtgga ggaagtgcag gtgcgcccag 2940

gctcctcatt ccgttttgca ggattccttc ggggtgtgag catttcctat tcagcctgtc 3000

gcccccgggg agcacgggct ggctctgtgg tgcccgtggc cttttgtaga agcgttggtt 3060

ttacggcagg ttcatctctg gggcagcctc ccacagtggg tggggctttg ccagcagtgc 3120

ccacgggggt catggggcca ggcgcgctcc ggcgcctgca gaactgatcg gggatagtct 3180

caggaggcgc tagtcacgtg ccccggtgat cggggatagt ctcagaaggc gctagtctcc 3240

tgccccggtg atcggggata gtctcaggag gcacgagtcg cctgcctcgg tgatgcaccg 3300

tttctcacac cggctgctct ggcccgagct aaaggggaag acgtgtgcgg ataggagctg 3360

cacacaattt tcctccatgt attgtttatt ttgctttttc ttttggctag acattaggaa 3420

tttcagtttt cccaagttgt atttttcctt ttctatttta aaattatcat gcagggctgg 3480

gtgaggtcgc tcacgcctat agtctcaaaa ctttgggagg ctgagggggg aggatggcat 3540

gagcccagga gtttaaggct gcagtgagcc gagatcgctc cactgtcctc cagcctgcat 3600

gacagagcga gaccctatct caggaaaaaa aaaaacaaaa ctattatgca gtagtttcga 3660

ccctggaaga cgagtgtgca tctttgagtt gtaacacgtg tacctcgccc atccaggcgt 3720

agtttcattt ggaatctggt tatcctgtag ttgctttgtt aaaaatatat gtaattgcaa 3780

atcattt 3787

<210> 20
<211> 63
<212> DNA
<213> Homo sapiens

<400> 20
ttctccagta tatttgtaaa aaataaaaaa aaaaactaaa cccattaaaa agtatttgtt 60

tgc 63

<210> 21
<211> 45
<212> DNA
<213> Homo sapiens

<400> 21
ccctggatcc tactctctta ttaaaaagat ttttgctgac agtgc 45

<210> 22
<211> 61
<212> DNA
<213> Homo sapiens

<400> 22

gtgcagggcc ctcgtccggg tgtgccccaa ataaactcag gaacgccccg gtgctcgccg 60

c 61

<210> 23
<211> 79
<212> DNA
<213> Homo sapiens

<400> 23
aacctcccac tttgtctgta catactggcc tctgtgatta catagatcag ccattaaaat 60

aaaacaagcc ttaatctgc 79

<210> 24
<211> 38
<212> DNA
<213> Homo sapiens

<400> 24
taggtgttaa aaaaaaaat aaaggacctc tgggctac 38

<210> 25
<211> 1651
<212> DNA
<213> Homo sapiens

<400> 25
atttcattta tctggaaaat tttgtatgag ttcttgaata aaacttggga accaaaatgg 60

tggtttatcc ttgtatctct gcagtgtgga ttgaacagaa aattggaaat catagtcaaa 120

gggcttccct tggttcgcca ctcatttatt tgtaacttga cttctttttt tttctgctta 180

aaaatttcaa ttctcgtggt aataccagag tagaaggaga gggtgacttt accgaactga 240

cagccattgg ggaggcagat gcgggtgtgg aggtgtgggc tgaaggtagt gactgtttga 300

ttttaaaaag tgtgactgtc agttgtatct gttgcttttc tcaatgattc agggatacaa 360

atgggcttct ctcattcatt aaaagaaaac gcgacatctt tctaagattc tctgtgggaa 420

aatgactgtc aataaaatgc gggtttctgg gccattcgtc ttactttcat tttttgatta 480

caaatttctc ttgacgcaca caattatgtc tgctaatcct cttcttccta gagagagaaa 540

ctgtgctcct tcagtgttgc tgccataaag gggtttgggg aatcgattgt aaaagtccca 600

ggttctaaat taactaaatg tgtacagaaa tgaacgtgta agtaatgttt ctacaggtct 660

ttgcaacaaa ctgtcacttt cgtctccagc agagggagct gtaggaatag tgcttccaga 720

tgtggtctcc cgtgtggggc ccagcaatgg gggcccctga tgccaagagc tctggaggtt 780

cttgaaagag gggacacgaa ggaggagtga ctgggaagcc tcccatgcca aggaggtggg 840

aggtgccctg gaaatagctg cctcatgcca cttaggccat gactggattt aatgtcagtg 900

gtgtgccaca gtgcagaggc tagacaactg aaaggggcta ccaaggctgg gaaaaaaatg 960

caattgttgc tgtgagtgac tttgaaagac tctggtgcct tgtggtgccc ttctgaaatt 1020

caaacagtaa tgcaaaagtg tctgcattag aatttacggt gtctaaaatt catgtttttta 1080

aaagagcttg cctacagatg gtttccacac ttgaaattgt gccctgcgag ttgcatagct 1140

ggaagttcaa tgctcagtcc taccttggct cccattaaac atttggtgct ctgtggattg 1200

agttgaacgt gttgaggctt tgcaatttca cttgtgttaa aggctctggc atttttccat 1260

ttctatgcaa atttctttga agcagaattg cttgcatatt tcttctctgc cgtcacagaa 1320

agcagagttt ctttcaaact tcactgaggc atcagttgct ctttggcaat gtcccttaac 1380

catgattatt aactaagttt gtggcttgag tttacaaatt ctacttgttg cattgatgtt 1440

cccatgtagt aagtcatttt tagtttggtt gtgaaaaaac cctgggctga agttggcatt 1500

tcagttaaaa gaaaaaaaga aactagtccc agatttgaaa acttgtaata aaattgaaac 1560

tcactggttt tctatgtctt tttgaactct tgtaatcgag ttttgatcat attttctatt 1620

aaagtggcta acacctggct actcttactg t 1651

<210> 26
<211> 474
<212> DNA
<213> Homo sapiens

<400> 26
actgagtcca gctgcctaat tctgaatata tatatatata tatcttttca ccatatacat 60

gcctgtctgt caatttctgg ttgggctggg aggccacaca cacacactga catgacaggg 120

cttgggcaag actcctgttc tacttatcct tttgaaatac ctcaccctgc cactccacca 180

tgtatgatca ttccagagat ctttgtgact agagttagtg tcctaggaaa accagaactc 240

agaacttgcc tccatggttg agtaacaagc tgtacaagaa ccccttttat ccctggaaga 300

ggctgtgtat gaaaccaatg cccagggttt gaagggtgtt agcatccatt tcaggggagt 360

gtggattggc tggctctctg gtagcatttt gtcctcacac acccatctac tatgtccaac 420

cggtctgtct gcttccctca ccccttgccc aataaaggac aaggacttca gagg 474

<210> 27
<211> 36
<212> DNA
<213> Homo sapiens

<400> 27
attcagcatt aaaataaatg taattaaaag gaaaag 36

<210> 28
<211> 45
<212> DNA
<213> Homo sapiens

<400> 28

actggcagat tagattttta aataaagatt ggattataac tctag 45

<210> 29
<211> 48
<212> DNA
<213> Homo sapiens

<400> 29
agtaatctta tatacaagct ttgattaaaa cttgaaacaa agagcctg 48

<210> 30
<211> 32
<212> DNA
<213> Homo sapiens

<400> 30
atgctttgtt ttgatcatta aaaattataa ag 32

<210> 31
<211> 90
<212> DNA
<213> Homo sapiens

<400> 31
accttttcac ctacaaaatt tcacctgcaa accttaaacc tgcaaaattt tcctttaata 60

aaatttgctt gttttaaaaa cattgtatct 90

<210> 32
<211> 4065
<212> DNA
<213> Homo sapiens

<400> 32
agccacatgg agggagtttc attaaatgct aactactttt tccttgtggt gtgagtgtag 60

gttcttcagt ggcacctcta catcctgtgt gcattgggag cccaggttct agtacttagg 120

gtatgaagac atggggtcct ctcctgactt ccctcaaata tatggtaaac gtaagaccaa 180

cacagacgtt ggccagttaa acatttctgt ttataaagtc agaataatac ctgttgatca 240

ctgaaaggcc tgcatgtatt gtactctgaa ttttacagtg aatgagagaa tgtaccctaa 300

ttgttcaaca gggctcaaaa ggaaagattc cattttgatg ggtcacattc taaagagggg 360

cagtgtgata ggaatgagat ggtcctttag gacttaagtt ctcagcccaa ggtttttcca 420

cgtggccccc tcatcttttt ttttttttta aacggagtct ctcttgccag gctggagtgc 480

agtggcacga tctcggctca ctgcagcctc cgcctcccag gttaagcgat tctcctgcct 540

cagcttcctg actaactggg attacaggcg cccaccacca tgcccagcta atttttgtat 600

tttcagtaga gatggggttt caccatgttg gccatgctgg tctctaactc ctaacctcaa 660

gtgatctgcc cacatcggcc tccaaaagtt ctgggattat agtgtgagcc actgcgcccg 720

gccatggctc cttaatcttg atccaaatta ttgttacatc cagaatgtga tgaatcaaaa 780

tctcgagatg ggggtccagc aatctgaaat ttcagtatgc cagggctttt ctgtatgtca 840
aagtgggttt gaaatagtta atttttcttc tagtctgaaa tgtatcggga aaatttggaa 900
atcctgaagg ctggaaattg aaataagttt ttctaggatt tgtgtctctt gctattggaa 960
aactgatggt gaccaattca tgtttacaaa taagatcctc atagatctcg gtaaattata 1020
atttgctaca gttttatggt tcttcctgtg attttgagct ttttttgacc caaaataata 1080
cagtctaaaa ctatagacaa ataagatggc acttagactc ctgggtttta gttagtggag 1140
gtttccttag tgcactgtgg ggtcataata agccgagaac catggctgtc tatgggacac 1200
atctgtcagg acaaccttta gaggatgttg gggatcaaat agaaggcaca gagaagcact 1260
gaattggctt acataagaat aggctagaat tacaagtagt gaaacctcga ttcagctgga 1320
caattttaaa caaatgtatc atttggcttg tatcttctgt tgtgctggag aagttagaaa 1380
taagggctct ccagaccagc ctgaccaacc tggagaaacc ttgtctctac taaatacaca 1440
aaattagcca ggcgtggtgg cacatgcctg taatcccagc tactttggag gctgagccag 1500
gagaatctcc aggaggcgga ggttgctgtg agccgagatc gtgccattgc actccagctt 1560
gggcaacaag agtgaaactc tgtccacccc ccccaaaaaa agtaagggct ctccattagg 1620
gcccatagag gacttgtaat atggaacctg aatccaagga tcccacaata agtggtcagt 1680
agttcatgat gaattaaaag actcaatatt tggtcttcac ccaatacctg tgtgactttt 1740
agtcctaatt tcctcatctt taaaatttca gtgaaagtgc ctacctgagg attgtgtaga 1800
ttaaaatgga aaccgtgcac ttaatttttt gttttgtttt gagacggagt ctcgctctgt 1860
cgcccaggct ggagtgcagt ggtgcgatct cagatcactg caagctccgc ctcctaggtt 1920
cagaccattc tcctgcctca gcttcccaag tagctgggac tacaggcgcc cgccactgcg 1980
cccggctaat ttttgcatt tttagtagag acagggtttc accgtgttag ccaggatggt 2040
ctcgatctcc tgatctgccc gcctcagcct cccaaagtgc tgggattaca ggcatgagcc 2100
accgcgcccg gcccaggcac ttaatttttg tgtttgactt agtaacttaa gtgcaaacta 2160
ttacgggagc agatggagtc aattggcctt catgtgattg tcagtgggaa attggtccaa 2220
gcagagggaa tactggttca ggaaactggt ttgggaaggt taggcaaacg ggaagtgcta 2280
tggtggagag aaagattact ctggccgggc tgtaaaggac ggctacaatg ggaggctgaa 2340
ggcagaacca agaaaatggg agtgagtatg gaaaaggtac gattcagacg gcataatgga 2400
cgggacttgg agactgaatt gtagtgggcc gaccacaaaa tgataaggca tggaaggaag 2460
tagagtttgg ggggaaggat ccctagtccc ttaatggcta ccttcttccc caggagttgt 2520
taggccatcc gatcccctgg cctgggaaag aaacactgat ttcgttgctg gcttgttcac 2580
tcaccagaag ctacagctac taacagttct aaaaactgtt tcatgtgatg aggaacagac 2640

gaaaatagtt ttgagcccta agtccgccga ttccagtgct ttcttgaacc cgcatttact 2700

aaaatatttt catgactgcc aagctttgaa tagcctgctg tgttcatgga ggctcatact 2760

ggcgatctct agtggctggc taaagcttga attgcaaaag atctaatttc tggtctaatg 2820

tatatatgcc ttaaatatag ttgcgttcaa acgtgggagc tgcaggtgca acttgatttt 2880

atgacaaatg gctgccacat aatttgcaca agcagtgctc gtcaagggca gctaaatcag 2940

gcgagctttc aatcaaaata aatgtactac taaaccctac ttagcggcta actagcccaa 3000

gagcagacag cccacggacg gactgcaagt cggaagcgcg ggcggaagct gtgcagcgcc 3060

cacctggtgg ctccatcggc cgcgttcatc agtcagcacg acccgacctc agtggcgtcc 3120

tcacaacaca gaccggacct tgggtcttac cccggcacct gagaaccact tccggtgagt 3180

agcttctact tccggagacg atgactcccc cgcgtcccag accggaagaa gcccggcgga 3240

gaccggcctc gctcggccac ttccggcaag ggcggagccg gccagtggtg cgcgagcgca 3300

gataactccc ctggagaggc gggatgttca actccacccc tggtccttgg gcggccgtgg 3360

gtccccttcg aagcggagga atggccaacc tcgccgcact tcgagcccct ttagggtgcg 3420

tttaagaaca gtgggcgtgg cctttacgta aatcttcgag atgggaacct ccagaatttg 3480

tctcaattgt ctaaaaggta atgagcgtca gcgacattca agggcacttt gggctaaaaa 3540

agaaagtgct tgtacacgga tggaaatatt ctagaagaac ataaaaggaa tttcctctta 3600

ggaggttagg gaaatgagca cgaagtatgt tttggtgcag ttttttgttc aacccaatgc 3660

gtattttcat attgagaggc aatataaatg gagcgaaagt atcttgagaa aaaaaaaaaa 3720

actaccagaa cttgccgttg ctgaaaagta atatttctc tttcgagagt tttcatggcc 3780

ttttaaatta cacccccacc tccacaggca aataaatttg ttttggaatg cataccacat 3840

catctggctc tagaaacgta ttttgtgtag ctcccctagc aagaatatag gttaaagcgt 3900

aaatttaatt cctggctcta ttttacatcc caatttttat tttcctctca ttcccacttt 3960

acgttgtttc aaataaccta gtttgtgtat ccctgtaagt cattttggta taaagtaggt 4020

tataagtgta catgcgaaaa gatgttttta acaaaaatgt aactg 4065

<210> 33
<211> 3865
<212> DNA
<213> Homo sapiens

<400> 33
gccttgctct gctcccccgc ccccaggcag ccatccgcag ggccagcgcc atcctgcgca 60

gccagaagcc tgtgatggtg aagaggaagc ggacccgccc caccaagagc tcctgagccc 120

cctgccccca gagcaataaa gtcagctggc tttctcacct gcctcgactg ggcctccctt 180

tttgaaacgc tctggggagc tctggccctg tgtgttgtca ttcaggccat gtcatcaaaa 240

ctctgcatgt caccttgtcc atctggaggt gatgtcaatg gctggccatg caggaggggt 300
ggggtagctg ccttgtccct ggtgagggca agggtcactg tcttcacaga aaaagtttgc 360
tgacttgtga ttgagaccta ctgtcccatt gtgaggtggc ctgaagaatc ccagctgggg 420
cagtggcttc cattcagaag aagaaaggcc ttttctagcc cagaagggtg caggctgagg 480
gctgggccct gggccctggt gctgtagcac ggtttgggga cttggggtgt tcccaagacc 540
tgggggacga cagacatcac gggaggaaga tgagatgact tttgcatcca gggagtgggt 600
gcagccacat ttggagggga tgggctttac ttgatgcaac ctcatctctg agatgggcaa 660
cttggtgggt ggtggcttat aactgtaagg gagatggcag ccccagggta cagccagcag 720
gcattgagca gccttagcat tgtcccccta ctcccgtcct ccaggtgtcc ccatccctcc 780
cctgtctctt tgagctggct cttgtcactt aggtctcatc tcagtggccg ctcctgggcc 840
accctgtcac ccaagctttc ctgattgccc agccctcttg tttcctttgg cctgtttgct 900
ccctagtgtt tattacagct tgtgaggcca ggagtttgag accatcctag gcaacataat 960
gagacaccgt ctctaaaata aaattagctg ggtgtggtgg tgcaccgcct gtggtcccag 1020
ctcctcagag gttgagtaga ggctgaggtg agcggagcac ttgagccaag agtatgaggc 1080
tgcagtgagc ccatgagccc caccactaca ctccagcctg gaagacacca tgacacacag 1140
tgaggcctgg atggggaaag agtcctgctg ttgatcctca catgtttcct gggcacctaa 1200
ctctgtcagc cactgccagg gaccaaggat ccagcatcca tggcacccct ggttcctgcc 1260
atcctggggt acccgattca aagaaggact ctgctccctg tctgagacca cccccggctc 1320
tgactgagag taaggggact gtcagggcct cgacttgcca ttggttgggg tcgtacgggg 1380
ctgggagccc tgcgttttga ggcagaccac tgcccttccg acctcagtcc tgtctgctcc 1440
agtcttgccc agctcgaagg agagcagatc tgaccacttg ccagcccctg tctgctgtga 1500
attaccattt cctttgtcct tcccttagtt gggtctatta gctcagattg agaggtgttg 1560
ccttaaaact gagttgggtg acttggtacc tgctcaggac cccccgcact gtcccaatcc 1620
cactcaggcc cacctccagc tggcctcact ccgctggtga cttcgtacct gctcaggagc 1680
ccccactgtc ccagtcccac tcaggcccat ctctggctgg cctcactgcg ctgggactcc 1740
gccttcataa ggagagctca ctgctcacgt tagtagatgg ccccttctcg tgaggcctct 1800
cccctggcac ctgcttcagt tgtcctccac agcactgatt tgcagcccac aagctggcag 1860
gtttatctgt ctcatgtttg tcttgtgctg gtgggcaagg ggtttgtcta gcacaccagc 1920
atataatgag atgcttgatg aatggtgcat attgaatgta taaagcccac cggtcctgag 1980
agtttgctca ctggagactt tctggagatg gagtctcgct ctgttgccca ggctggcgag 2040
tgcaatggcg cgatcttggc tcactgcagc ctccacctcc tgggttcaag cgattctcct 2100
gcctcagcct cccgagtagc tgggattaca ggtgggtgtc accacaccca gctcagtatt 2160

gtatttttag cagagatggg gtttcaccat tttgcccagg ctggtttgga actcctgact 2220

tcaaattacc cacctgcctc agcctcccaa agtgctggca ttacaggcgc tcgaggcttt 2280

ctgatgtggc tgctgctgct cagaaggcct tgtccttaac cacctccttg cctgccctgg 2340

aggcttgtgc ctctaggccc cacccccctgt ggagtcctgc tggctttctc catccctatc 2400

tgaatcctcc ctgctgtgtg gcctcccctg gtctcatccg taacacagcc cagcttagtg 2460

ggcctctgtt cctgcgggtg gccagcctgt ctgtgtggct gggctgggga ggccacgtct 2520

ggtatctgaa tgctatcggt gggttggggt ggaggaacca ggagagggct ggagggaggg 2580

agatggtctc agccccacag agtttggagt cctcagtgtg ctgagcaaac gtggagacac 2640

catttccctc ctctagacct catcttggag agagagatgt tggatggggc catctattcc 2700

agctttattc acacaaatca tgtctgttgg cctggaaatt ggaaaaccag ttaaaccaaa 2760

aacatgatat taagaaaaca ggcaggctca ccatagtaaa aatgctgaaa gccaaagaca 2820

aaattgggag aacaaaagaa aagcgtcttg tcacatacag aaggtccctg ataaagttag 2880

tagctgccct catcagaaac caggcccagg cagtggggac acatccagag tgctgaaaga 2940

acctccccca ggtcatccta tccccaagag tgatgcccgg cagcattccc agctcagggc 3000

taatggttca cggaagccag gaatcaaact gcctgggttc cagtcccagc tctgccagtt 3060

atgcccagct gtggggactt gggcagctcg tttagtagca ccgtgcctca gtttcccata 3120

tgtaaaaggc cattttgagt gcctttcaca gccctgcata aggcaggtgt ctcagtgttc 3180

actgctgtct ctccagctct tagtccagta gctgcatggt gagtgagcgt agggcgcacc 3240

ctggaaggct gccaagccca aagttgtgca gagcgctggg gactccagac tccccacagc 3300

agcagagact cgggactgag gcatcctctg ttcacaggac atgctggcat ctactgggtc 3360

agggctctgc tgctcggtgg ctgtgcaacc ttgggcaagt tcctcaacct ctctgtgtct 3420

tcgtaccctc atctgtaaca tgcgtgtcga tagaccctac tactcagggt tgatgagaag 3480

attaaatgtg caaaacctgc ttgactgtgc ccacaaatcc tgattgtagg aataaattaa 3540

tgacttttta taaatatttt gatcagatgg actcatgatc acagatgtct tcacatgcct 3600

atgactaatt tgtacacaaa ctaatgctcg tgtttcccaa gcacctggaa gacatgccag 3660

atccatgtgc agtaatgcct ggtggctcca ggtctgcccc gccgtcctgt ggggctgtga 3720

gctttcccag cctcctgccc gtgtttgtga atatcattct gtcctcagct gcatttccag 3780

cccaggctgt ttggcgctgc ccaggaatgg tatcaattcc cctgtttctc ttgtagccag 3840

ttactagaat aaaatcatct acttt 3865

<210> 34
<211> 288
<212> DNA

<213> Homo sapiens

<400> 34
tttttttactg tcaggcagga agagcggtaa ctgccatcgc ggcgggcatc cctggcgcca 60

gggtgttggt ctgggtaccg gcttccctct cggccgactt gtcagctctg tgagccgcgc 120

gcgtctgagc ccgtgtcctc acctgtaaag tggagaaatg aaaaaggacc tgaacttcct 180

cggtggttgt tgagagttaa ggcacggggt tgatgttttc agatgaaatt ctcaaagcaa 240

gtcagggtgg ggatggatgg tttcatccca caggtgggaa gattgagg 288

<210> 35
<211> 430
<212> DNA
<213> Homo sapiens

<400> 35
gtttttctca ggtccttgat tggaactgcc tcagagccaa gggtcctttt actcagtggc 60

agcaacaaac gcagtctgtt ggctagtgat cctcctgtct cagggacacg tagtccaggg 120

agcagccaat tgcttggcac ttggggaccc cgttctgggg agtcctgaaa gctttcacct 180

cttggattgc cgaatacatg ggtggccctt cctagactaa gggactggcc tgagtgaggc 240

tgggcctctc agccaagctg atgttgaacc actgctgtgg ggatgggcct ggggttcctg 300

ggaagctgtt catacccatt gccaggagcg tgggctctgg ctggacctgg atcagatcct 360

aactgaagcg gcagctttct ggcatgagaa aggagtgttt tcatggtgga cagaattggg 420

ctatgagtgt 430

<210> 36
<211> 120
<212> DNA
<213> Homo sapiens

<400> 36
atatctctgc caacatgagg acagaaggac tggtgcgacc ccccaccccc gcccctgggc 60

taccatctgc atggggctgg ggtcctcctg tgctatttgt acaaataaac ctgaggcagg 120

<210> 37
<211> 655
<212> DNA
<213> Homo sapiens

<400> 37
agggagccct cctggaagtg gatgaggcct tgggtctcgg ctcttcattg cttcctgagc 60

tgcagcagat gcctttacaa ccaagctcac cgaggacgtc tgtctcccat attaccctgg 120

cagagggcca ggcctgttct acacggccgg ggtttcaaca aggtactgat gtcttctgcc 180

cttgcctctt cgacaggcaa gtaataagac ttaagtgaag agaattcttt aggcacacaa 240

attcacattt gatgtaatct cattatactt cctgatctgt gattgaaaac tttcatttcg 300

taactagtat gtctgtccca cctttaaaaa gtttttcatt atgaaagtaa gtatttgtta 360

gaattaagtc tatttaaatg aaaaaaactt agatatgagt ctgcatggcc tcaggaaaat 420

gatgttttaa aatagagatt ttaggttgtc tgcactctag ctttttgtc gttttcttaa 480

ggcttttta actgcatcaa aaattcagat acgaaacata cactaaaaaa taatacatca 540

tatcttaatt tccactgaac ttgatttaaa ttcagagtta cacagtatga atatcacaat 600

cagatatgtt caaaaaggtc tgaacaattg attttctgaa accatgaagg actac 655

<210> 38
<211> 331
<212> DNA
<213> Homo sapiens

<400> 38
agccatttaa attcattaga aaaatgtcct tacctcttaa aatgtgaatt catctgttaa 60

gctaggggtg acacacgtca ttgtaccctt tttaaattgt tggtgtggga agatgctaaa 120

gaatgcaaaa ctgatccata tctgggatgt aaaaaggttg tggaaaatag aatgcccaga 180

cccgtctaca aaaggttttt agagttgaaa tatgaaatgt gatgtgggta tggaaattga 240

ctgttacttc ctttacagat ctacagacag tcaatgtgga tgagaactaa tcgctgatcg 300

tcagatcaaa taaagttata aaattgcctt c 331

<210> 39
<211> 1152
<212> DNA
<213> Homo sapiens

<400> 39
gcagctcatg tgcacgtttt ctgtttaaat aaatgtaaaa actgccatct ggcatcttcc 60

ttccttgatt ttaagtcttc agcttcttgg ccaacttagt ttgccacaga gattgttctt 120

ttgcttaagc ccctttggaa tctcccattt ggaggggatt tgtaaaggac actcagtcct 180

tgaacagggg aatgtggcct caagtgcaca gactagcctt agtcatctcc agttgaggct 240

gggtatgagg ggtacagact tggccctcac accaggtagg ttctgagaca cttgaagaag 300

cttgtggctc ccaagccaca agtagtcatt cttagccttg cttttgtaaa gttaggtgac 360

aagttattcc atgtgatgct tgtgagaatt gagaaaatat gcatggaaat atccagatga 420

atttcttaca cagattctta cgggatgcct aaattgcatc ctgtaacttc tgtccaaaaa 480

gaacaggatg atgtacaaat tgctcttcca ggtaatccac cacggttaac tggaaaagca 540

ctttcagtct cctataaccc tcccaccagc tgctgcttca ggtataatgt tacagcagtt 600

tgccaaggcg gggacctaac tggtgacaat tgagcctctt gactggtact cagaatttag 660

tgacacgtgg tcctgatttt ttttggagac ggggtcttgc tctcacccag gctgggagtg 720

cagtggcaca ctgactacag ccttgacctc cccaggctca ggtgatcttc ccacctcagc 780

cttccaagta gctgggacta cagatgcaca cctccaaacc tgggtagttt ttgaagtttt 840

tttgtagagg tggtctagcc atgttgccta ggctcccgaa ctcctgagct caagcaatcc 900

tgcttcagcc tcccaaagta ctgggattac aggcatcttc tgtagtatat aggtcatgag 960

ggatatggga tgtggtactt atgagacaga aatgcttaca ggatgttttt ctgtaaccat 1020

cctggtcaac ttagcagaaa tgctgcgctg ggtataataa agcttttcta cttctagtct 1080

agacaggaat cttacagatt gtctcctgtt caaaacctag tcataaatat ttataatgca 1140

aactggtcct tc 1152

<210> 40
<211> 75
<212> DNA
<213> Homo sapiens

<400> 40
actaacgaaa aatcaataaa taaatgtgga tttgtgctct tgtattttta agtggattaa 60

aaaacttact acctt 75

<210> 41
<211> 1176
<212> DNA
<213> Homo sapiens

<400> 41
gaatgtcaac gattagtcat gcaataaatg ttctggtttt aaaaaataca tatctggttt 60

tggtaaggta tttttaatca attaggcttg tagtatcagt gaaatactgt aggtttaggg 120

actgggctag cttcatatca gatttacttg ttaagtgact gttttggaat gtttactttt 180

ggactgggtt tgtaacacgg ttaaaggcaa tgagaaacaa gcagaattcc aggagtcctt 240

gaagcagagg gcactggaag acaatatagc agattaaaat agcacagctc atgtggcata 300

ggtgggtatt ttagatgttt gagtaaattt gaaagagtat gatgtttaaa ttacctttag 360

caacatgttc atctgctatg ctgtcatgac tagggggatg attattagtc acatagagct 420

tgggagtacc actggaaacg tatgggtagg agtttaggtg gcttctgttt ttcaaaagat 480

gatcttatcc tagtatctgt aatgctcact tggcacacct gacttgtggg ctgtgtgtaa 540

ggtggctagc taagtgaaaa aagcctgcta ggtgtgagtc aacttaagaa tatgtaaata 600

ggtttgagaa aaagtagggc ttgggtgcaa gtaaagattg agcaggaaat aaaggaaaat 660

caagtataat ccctgagatt tgtagactaa aggcaatgat gtgggactac ttggtcgaat 720

ttttttagcc ctcaacttgg taattgggtg tttctgtgtt aaagcactga aacttgctgt 780

cgtgccttcc tagttttcgt ggtttattga cagggttggg ggtttttttt gtttttttaa 840

aatgaaggga caaagtcaac tggactgctg agtgagaggg caggggcagt tgaagggaac 900

atgaattgct ggaacagcta cataaaatag tgatgtagcc aagtcatgct atttaaatta 960

taattctcca ctgtgtttag aataacatct gaggttctta acctggcctt ggaagggtat 1020

cacttttact tgtaacctgg aatggcttta taatgtgcta gctaattgct actctcatct 1080

tgtattttaa ctcctaattt acccttcagg tctcagcttc agaacattca cttataaaga 1140

aaccctgctg attaaatctc tcttgggctt cctccc 1176

<210> 42
<211> 59
<212> DNA
<213> Homo sapiens

<400> 42
acgctcctct actctttgag acatcactgg cctataataa atgggttaat ttatgtaac 59

<210> 43
<211> 49
<212> DNA
<213> Homo sapiens

<400> 43
accagacaca ctgattggaa ctgtattata ttaaaatact aaaaatcct 49

<210> 44
<211> 178
<212> DNA
<213> Homo sapiens

<400> 44
ggaattgcac atgagatggc acacatattt atgctgtctg aaggtcacga tcatgttacc 60

atatcaagct gaaaatgtca ccactatctg gagatttcga cgtgttttcc tctctgaatc 120

tgttatgaac acgttggttg gctggattca gtaataaata tgtaaggcct ttcttttt 178

<210> 45
<211> 73
<212> DNA
<213> Homo sapiens

<400> 45
tcaccaaaaa gcaaccaact tagccagttt tatttgcaaa acaaggaaat aaaggcttac 60

ttctttaaaa agt 73

<210> 46
<211> 38
<212> DNA
<213> Homo sapiens

<400> 46
acctctttta taacatgttc aataaaaagc tgaacttt 38

<210> 47
<211> 40
<212> DNA
<213> Homo sapiens

<400> 47
attcctgctc ccctgcaaat aaagcctttt tacacatctc 40

<210> 48
<211> 1301
<212> DNA
<213> Homo sapiens

<400> 48
cagggtctcc ttggcagctg tattctggag tctggatgtt gctctctaaa gacctttaat 60

aaaattttgt acaaagacac aaggtctgac tagactgttc agtattcaga ctgaggggca 120

tgttggcctc tggagcatta catatcttct tggttttaac catacttgtg gtatttgcaa 180

gggccagaac agtaagaccc aagcagagcc aaccagagaa ataatatttg tgtgatagag 240

aaggctgata gcaagcaagg cagcaccttg attcgttgtc ctgtagttca ggattgtagg 300

tttagaagag ggatatgttt gagtttttcc tatgcataag gcgatccacg ttgcacatag 360

aaagtgaata taaatggcca ttatattttg tgtcatgctg tgctctaagt gttctttaca 420

tatgtactcg ttaatcaacc tctctaaagt gtaaaggaaa tttgcttgca ccactgaagg 480

cacataaggc tcagaagtaa atttgcctaa gcagtataaa gctatcatta gaatccacat 540

tcctaagttg tgttctctta ggggatcatg gaaccagtca ttggtactac aggctattat 600

gttctggaga actgtgaaga acatttaaat tgtctctgat tttatctatc aatgttttga 660

agtattttct accagtgtct gtacttcaca agaaattcgg cactattttt tcaggcaaaa 720

ctagtgaggg acaggttggc ttgaaaatca tgagactgtt gttaaatcag atgctggttg 780

atcacagagg ggacttccag ggaaagctgt tatcaggtgg ctgcttcctg gtgatgcagc 840

ctggctgatg agataaccct ggctccacag atggcttagc aggtgctgtg atgatttggt 900

tttcttctca attagactga gctgcacatg gtgtttatat tgcttggcac atggtaaggg 960

cttaatattt gaggtaatta tgtagggcgt acactgacaa gtatctgacc ccccttcct 1020

ttttgactca taaattggtc atcttaacca tttaagtgta cacttctata gtgacagagt 1080

tagccctctg tccaagggat ttgcatctgt ggattcaacc aactttgggt caaaaataat 1140

caaaaaggat ggttgtgtgt gtattgaaca tgtagactta tttttcttat tttcaaaata 1200

ctatattttc ttgtcactta ttttcttgta cactgcagtt gtaacagcta tgtagcatgt 1260

acattaggta ttaaaagtaa tccagtgaag attgaaagtc t 1301

<210> 49
<211> 576
<212> DNA

<213> Homo sapiens

<400> 49
cagcctcttc catgagtggg gagcccgctg cttgtctcca gctcctagca gtgagtcctg 60

ataatctcaa atttaaggac agtaactttg tctgggatga gtgtgggaaa ggatgtgttt 120

gggaacagac gcgagcctgc agaggtgttt gtaaccatct ctttctaagt ggtgggaagc 180

agacatttta ttctttaact gttaatatat atagtgtgtg tttttatgc atgaaatatt 240

ttatagtttt taaaaatgcc cacactacta ttttgaaagt aaatgaggta atgtatgtgt 300

cagaacccaa tacccaaagc gatcgtagta agaggtgggg cctttgggaa ggcattaaat 360

tgcttaggga atgagggtgg aaccctcatg aatgagatta gagccttata ggagaggttg 420

gagggagttg cctggcctcc ctctcccatg tgaagactca gcaagaaaac attatttagg 480

aagcagagag ccctcatcaa acaccagatc tgctggccac ctgatctggc actttccagc 540

cttcagaact gtgagaaata aatttctgtt gtctat 576

<210> 50
<211> 57
<212> DNA
<213> Homo sapiens

<400> 50
agttcagact tcaaatagtg gcaaataaaa agtgctattt gtgatggttt gcttctg 57

<210> 51
<211> 1324
<212> DNA
<213> Homo sapiens

<400> 51
agctcacgtt gatgtcaaga ctaccgatgg ttacttgctt cgtctgttct gtgttggttt 60

tactaaaaaa cgcaacaatc agatacggaa gacctcttat gctcagcacc aacaggtccg 120

ccaaatccgg aagaagatga tggaaatcat gacccgagag gtgcagacaa atgacttgaa 180

agaagtggtc aataaattgt aagtgtttct ttgcttcctc acacaacaca accttgagta 240

ttggattatt cctgagatga gagaacgcat atgagacaag gtaaaggtct gttgaaatcc 300

tgtctgtgaa tccttctagc tatatctctt taagtgaaag agtgttaagt actcagtaaa 360

tatgattatt attactatta ttatttgagt cagagtcttg ctctgttgcc caggctcgag 420

tgcagtattg tgatcctcct tggctcactg taaccactgc ttcctgggtt caagcagttc 480

ttgagcctca gcctcctgag tatctgggaa tacaggggac tgccaccata cccagctaat 540

ttttttaaat ttttagtaga gatggggttt catcatgttg gccaggctgg tcttgaactc 600

ctgacttcag gtgatctgcc agtactctaa atgataacag ttttttcgtg tttatttatt 660

ttgaatgaag ctgtctcaca gtagatggag ttgaaggaca ggaaatgttt ttccccctact 720

tggaaaatac actgaataag ttgagtgggg tgggatgtgc ctggagtccc agctactcag 780

gaggctgagg tggtaggatt gtttgagccc aggagtttga ggccagcctg ggcaatatag 840

ggagaccctg tcccaaaaaa taaaaaatat acgtatatat atatacacac acaaagaaaa 900

aatacactga atagacaaaa cctttcatga ttaatgatgc acgggaataa gtgatgaaaa 960

aagtttcggt cccagatgat ggccagtgat aacaacattt ttctgatgtt cccatgcaat 1020

atacagttag ctaagagggt gtaatggaaa aagcataagg cttggactca gaagactcta 1080

ctaactttgc cactagctag ctatgtaatt cagatcatct atcctttaca tgtgaaaggt 1140

aaataatggc ttatcttaac aggaggattt atgcaggtta aatgaggtag gtgttatgtg 1200

taggtttatt ccaaggcttc tctactttta aaggaaatgg cttatatctg agaactagga 1260

cttttagaaa aaaatttact gttactggtt tgcaggattc cagacagcat tggaaaagac 1320

atag 1324

<210> 52
<211> 68
<212> DNA
<213> Homo sapiens

<400> 52
aatgggtccc tgggtgacat gtcagatctt tgtacgtaat taaaaatatt gtggcaggat 60

taatagca 68

<210> 53
<211> 60
<212> DNA
<213> Homo sapiens

<400> 53
attgcagtag cagcatatct ttttttcttt gcacaaataa acagtgaatt ctcgtttctt 60

<210> 54
<211> 53
<212> DNA
<213> Homo sapiens

<400> 54
ttttcccagc tgctgcccaa taaacctgtc tgccctttgg ggcagtccca gcc 53

<210> 55
<211> 37
<212> DNA
<213> Homo sapiens

<400> 55
gatttttga gtaacaaata aataagatca gactctg 37

<210> 56
<211> 36

<212> DNA
<213> Homo sapiens

<400> 56
acaaaaatga ctaaataaaa agtatatatt cacagt 36

<210> 57
<211> 55
<212> DNA
<213> Homo sapiens

<400> 57
atccttgttt tgtcttcacc catgtaataa aggtgtttat tgttttgttc ccaca 55

<210> 58
<211> 70
<212> DNA
<213> Homo sapiens

<400> 58
gtccacctgt ccctcctggg ctgctggatt gtctcgtttt cctgccaaat aaacaggatc 60

agcgctttac 70

<210> 59
<211> 50
<212> DNA
<213> Homo sapiens

<400> 59
aattggagag gattcttttg cattgaataa acttacagcc aaaaaacctt 50

<210> 60
<211> 77
<212> DNA
<213> Homo sapiens

<400> 60
ggctggacat cggcccgctc cccacaatga aataaagtta ttttctcatt cccaggccag 60

acttgggatc ttccgcg 77

<210> 61
<211> 21
<212> DNA
<213> Homo sapiens

<400> 61
agaaataaat ctttggctca c 21

<210> 62
<211> 41
<212> DNA
<213> Homo sapiens

<400> 62
atttgtctgt gtactcaagc aataaaatga ttgtttaact a 41

<210> 63
<211> 31
<212> DNA
<213> Homo sapiens

<400> 63
tggctcagct aataaaggcg cacatgactc c 31

<210> 64
<211> 53
<212> DNA
<213> Homo sapiens

<400> 64
ggatgtaata catatattta caaataaaat gcctcatgga ctctggtgct tcc 53

<210> 65
<211> 64
<212> DNA
<213> Homo sapiens

<400> 65
gcccatcgtg actcaaaact cacttgtata ataaacagtt tttgagggat tttaaagttt 60

caag 64

<210> 66
<211> 37
<212> DNA
<213> Homo sapiens

<400> 66
aacaaaccat gctgggttaa taaattgcct cattcgt 37

<210> 67
<211> 386
<212> DNA
<213> Homo sapiens

<400> 67
ctgcattctc ctccgccaaa aaagtgacca agcagagtct ttctctgtca cccaggctgg 60

agtgcaatgg cgtgatctca gctcactgca acctctgcct cctgggttca agtgattctc 120

gtgtctcagc ctcctgagta gctgagacta caggtgtgca ccagtgttcc cagctgattt 180

ttgtatttta tgtagagatg gggttatgcc attttggcca ggctagtctc gaactcctga 240

gctcaggtga tacacacacc tcagcaaatc ttttaaatta tacattctgt gatatttcct 300

tgactttctt atccagcact tgtattgatt atttttcatt ttgataatgt tgggttttta 360

aaaactcctt tatgatggaa aatttc 386

<210> 68
<211> 281

<212> DNA
<213> Homo sapiens

<400> 68
gtcaactatt ttaataaatt gatgaccagt tgttaacttc tgttggtttt tattcagaat 60

actggcagat tttaggaata taaaggtgta ctatgagact tccacttttc aggtggaata 120

tatgggtatc ttagagtggt ctatcctgtt ttcgttgtcg tttgagtcat ttgaaaactg 180

gattccgtta actacataat atgtgagacc tgactggttt tattggacac tggcagttta 240

taactttggc atactctaga taaattctga ttggtatggg g 281

<210> 69
<211> 53
<212> DNA
<213> Homo sapiens

<400> 69
ctggagagaa tcacagatgt ggaatatttg tcataaataa ataatgaaaa cct 53

<210> 70
<211> 2785
<212> DNA
<213> Homo sapiens

<400> 70
atattaatgg tgaaaacact gtagtaataa attttcatat gccaaaaaat gtttgtatct 60

tactgtcccc tgttctcacc acgaagatca tgttcattac caccaccacc ccccttatt 120

ttttttatcc taaaccagca aacgcaggac ctgtaccaat tttaggagac aataagacag 180

ggttgtttca ggattctcta gagttaataa catttgtaac ctggcacagt ttccctcatc 240

ctgtggaata agaaaatggg atagatctgg aataaatgtg cagtattgta gtattacttt 300

aagaacttta agggaacttc aaaaactcac tgaaattcta gtgagatact ttctttttta 360

ttcttggtat tttccatatc gggtgcaaca cttcagttac caaatttcat tgcacataga 420

ttatcttagg tacccttgga aatgcacatt cttgtatcca tcttacaggg gcccaagatg 480

ataaatagta aactcaaaat tgctccccac tctgtttatt atttaaaggt gtcaggatct 540

gtgttgtaat gtgtctacat taatgtgttt aggagaatac aggcattgga tcatttagtt 600

gatggaagta tatgccaggc aagggagata aggtatacga caagactgat gttttcagta 660

tcttctcatg aggttgtcag agaccttcat gtcttcaaag actagtcagc aaatgaagtg 720

gtttagtgta gagacaagat tggttgtgtt ttgataattt aagctaggta ttgagtacat 780

gtggattttg ctgtccacaa atacttgttt cagagttttc atggatacag tggcatggtt 840

gaaatgaagc tgtgagcctt ctgctttaaa tctgatgtaa gaaactcctg ttaacaaata 900

gtaagtatgg gttaattagc cctttgatca aagcctagct ttacattgtt taggatcttt 960

ggaaaacaat tggtttggtt gcccactttc cgtaggatca agagcagaac ctttcacatg 1020

gcacagaaga acccaggttg cgcttcatac ctgcatattc cagccttagc ctgccatttc 1080

tctccttggc actttgtgct ccagcaacac tggtctcagt tggtcatcct caaacttggg 1140

ttccatatcc agcctcagga cctctgttcc tgttactatg gttccttgca tgtcgcctgc 1200

tcttactaaa gagctcgtgt gttttccagc acacttcggt ttatctcttg atgatgatgc 1260

tagtctctcc ctccgcaagg gcggaaaggc tgcctgttgg tttgtaccag tgtttcctaa 1320

cgtgtagctg cagtcagtat ttggctaagc tgttcccagg ggctcaacag atgctttcgg 1380

atgagcctta actgacccaa tcctttgtga tgcgggagag attgctaggc ctcgctcacc 1440

tggccagaac cagggaaaga ggccgcggtt gcagcgcgat tccaggccct gggcgtcagg 1500

cgcggggtgg gcagctctcc ccgggcggtg gggcccttgt gaccgcgagg cggggcgcac 1560

caggaaggga gtgggacagc gcgggcgccc agggatgtgg cctggttacc tgccttctct 1620

gatacgtcaa gacaccttca acaatggctt gcagctgtac cctgttggct gcacccagga 1680

cgcccttttc actgctaagc agtcctacct gaggcccagg ggctgccaga ttgacccata 1740

aataatctcc ggcgcctcag atccagaagc tgctgagcct gatcttagtg ccttctcctt 1800

tctctgtgtg gccccccagc ccctttcccc actgccttgt gtccaaggcc ctttccttca 1860

tgtatccatg gaggagagac aaaaatacac atcaataaaa taagataggg aatccataaa 1920

tagacattca gaagtatggc caacggattt atcttaaaac caatggagga agaagagttt 1980

caataaatgt tgtggacttc catttgtcaa agaccaaaac aaaggaaccc caaccttaca 2040

tgtaatacaa acttaactca aaatggatca tatatctaaa tgtaaaatgg aaagctataa 2100

aactgaaaac agactatctt tacaacctag gcgtaggtat agtttttaga cattacacca 2160

aaagcacatg ccgtaaaaga aaaaatagat aaattggtgg atttcattaa aattaaaaaa 2220

ctttttctct ctgaaaaatc ctgttaagct gggcgctgtg gttcatgcct gtaatcccag 2280

cactttggga ggctgagttg ggaagaaatt aatagcttga ggccaggagt tcaagatcat 2340

cctgggcagc aaagtcatac actcttgagg gaagagagag accttctcat attgttttat 2400

attgttttat actcagtacc tgttttaaga aaaaaacaag gaagtgaaat caaagacagg 2460

cagcccggca ccaggcctga aaccagccct gggcctgcct ggcctaaacc tagtagttaa 2520

aaatcaactt acgacttaga acctgatgtt atccgtagat tccaagcatt gtataaaaaa 2580

attgtgaaac tccctgttgt gttctgtacc agtgcatgaa accccctgtca catatcccct 2640

agattgctca atcaatcacg accctttcat gtgaaatctt tagtgttgtg agcccttaaa 2700

agggacagaa attgtgcact tgaggagctc agattttaag gctgtagctt gccgatgctc 2760

ccagctgaat aaagcccttc cttct 2785

<210> 71

<211> 48
<212> DNA
<213> Homo sapiens

<400> 71
ataggtccaa ccagctgtac atttggaaaa ataaaacttt attaaatc 48

<210> 72
<211> 67
<212> DNA
<213> Homo sapiens

<400> 72
ggagctgagt tcttaaagac tgaagacagg ctattctctg gagaaaaata aaatggaaat 60

tgtactt 67

<210> 73
<211> 62
<212> DNA
<213> Homo sapiens

<400> 73
aagcactctg agtcaagatg agtgggaaac catctcaata aacacatttt ggataaatcc 60

tg 62

<210> 74
<211> 276
<212> DNA
<213> Homo sapiens

<400> 74
ctgtatgagt taataaaaga catgaactaa catttattgt tgggttttat tgcagtaaaa 60

agaatggttt ttaagcacca aattgatggt cacaccattt ccttttagta gtgctactgc 120

tatcgctgtg tgaatgttgc ctctggggat tatgtgaccc agtggttctg tatacctgcc 180

aggtgccaac cacttgtaaa ggtcttgata ttttcaattc ttagactacc tatactttgg 240

cagaagttat atttaatgta agttgtctaa atataa 276

<210> 75
<211> 139
<212> DNA
<213> Homo sapiens

<400> 75
gcttggctgc tcgctgggtc ttggatgtcg ggttcgacca cttggccgat gggaatggtc 60

tgtcacagtc tgctcctttt ttttgtccgc cacacgtaac tgagatgctc ctttaaataa 120

agcgtttgtg tttcaagtt 139

<210> 76
<211> 94
<212> DNA

<213> Homo sapiens

<400> 76
atgctcttcc ttcagaggat tatccggggc atctactcaa tgaaaaacca tgataattct 60

ttgtatataa aataaacatt tgaaaaaacc cttc 94

<210> 77
<211> 1355
<212> DNA
<213> Homo sapiens

<400> 77
tataagtaaa gtttgtaaaa ttcatactta ataaacaatt taggacagtc atgtctgctt 60

acaggtgtta tttgtctgtt aaaactagtc tgcagatgtt tcttgaatgc tttgtcaaat 120

taagaaagtt aaagtgcaat aatgtttgaa gacaataagt ggtggtgtat cttgtttcta 180

ataagataaa ctttttgtc tttgctttat cttattaggg agttgtatgt cagtgtataa 240

aacatactgt gtggtataac aggcttaata aattctttaa aaggagagaa ctgaaactag 300

ccctgtagat ttgtctggtg catgtgatga aacctgcagc tttatcggag tgatggcaat 360

gctctgctgg tttattttca agtggctgcg tttttttag tttggcaggt gtagactttt 420

taagttgggc tttagaaaat ctgggttagc ctgaagaaaa ttgcctcagc ctccacagta 480

ccattttaaa ttcacataaa aggtgaaagc tcctggttca gtgccatggc ttcatggcat 540

tcagtgatta gtggtaatgg taaacactgg tgtgttttga agttgaatgt gcgataaaat 600

tattagcctt aagattggta agctagcaat gaatgctagg gtgggaagct ggtgagccag 660

tggccattag ataaatacct ttcaagtgtg agcttagacg tcaaccctaa aatacttaac 720

cgtaatgcta attgtgatca ttatgaatcc cttcagtcac attaggggga aagtagttgg 780

ctataagtac gtcattctta gtccagtcag tcttaaaaac atcttgggtt acccactctg 840

tccactccca taggctacag aaaaagtcac aagcgcatgg tttccaacca tatgtgtttt 900

ctgcagttat ttctcttgtt ctggccaaac aaccctaaaa atccttacca ttccacaaag 960

ttggaccatc acttgtgcac ccactttgac tatgagtata ccaccacatt gcatttctgt 1020

ttgcaccatg tcttccagga gactagacta ctgttgtcca gggtcaattt gagtgtaaag 1080

aaaatgtaga caaggaattg cccaatttta aattctgact ttgctgactt aatttaaatg 1140

ctcgttctga accaattttc tcctatcttc tctaggggtt tcaaaagact cagttaattg 1200

atttccagga agtactcata gcaagttcat aaaagttctt gagacctaaa tttcttcaca 1260

aaaaaagaaa agatcttaag tcatacattt taattgtgta gaggttgttc aactgaagga 1320

ataaatgtct attaaactaa aacaaatgga ccttc 1355

<210> 78
<211> 2017

<212> DNA
<213> Homo sapiens

<400> 78
gcaattcttc tgcctcggcc tcccaaatag ccaggactac aggcgcacac tgccatgccc 60

agctaagttt tgtattttta gtagagactg ggtttcacta tgttggccag gctggtctcg 120

aactcctgac ctcaagtgat ccacctgcct tggcctccca aagtgctggg attacaggcg 180

tgagccacca cccccagccc aatttttatt ttttgtacag acaggatctc actatgttgc 240

ccaggttggt ctcaaactac tggcctcaag caatcctgcc ttggcctccc aaagtgctgg 300

aattatagga atgagccacc acaccgggcc caaatttact ttagtaataa caacaattgg 360

ctgggtgcgg tggctcacgc ctgcaatccc aacactttcg gtaaccaagg tgggcttgag 420

ctcatgagtt agagagcagc ctgagcaacg tggtgagagc ccatctcaca aaaaataaca 480

aatcagctgg gcatggtgtt gcacgcctgt agtctccgaa atcacaccac tgcactccca 540

tcttgggtga tagagccaga acttgtctca aaaataacaa ttggtttctt acaatcccaa 600

aaggtgcagt tactagtatt aatccttttt tgccaatgag gaaacacaaa gatgaagcaa 660

cttgctcaaa gtcatacagt gacagtctga attcaaatcc tatacactta aagtttattt 720

gttttgtttt ggtttttttt gagatggagt ctcactgtgt cgcaaggctg gagtgcagtg 780

gcacgatctc agctcactgc aacccgggtt caagcgattc tcctgcctca gcctcccgag 840

tagctgggac tacaggcacg caccaccaca cccagctaat tttgtatttt ttagtagaga 900

cggtttcacc atgttggcca ggatggtctc gagctcctga cctcaggtga tcctcccgcc 960

ttggcctccc aaagtgccgg gattacaggt gtcagccact gcacgtggcc aacttaaagt 1020

ttttgataga taatacatta acgttaaaaa ttcaaaagat aagtataggc tctacagtac 1080

aaacccttct gcctcctagt tcctctccct ggaggcaagg tgatcagttt aacaatattt 1140

ttttattttg agacagggtc tcactgttgc ccaggctgga gtgtagtggc gcgttcacaa 1200

cttactgtag cctcaacctc ctggctcaag caatcctccc acctcagcct gtcgagtagc 1260

tggaaccaca ggtgcacacc accatgccag gctaattttt gtattttttg tagagacagg 1320

gtttcaccat gttgttcagg ctggtctcaa agtcctgggc tcaagcaatc ttcctgtctc 1380

tgcttcccaa agtgctggga ttacagatgt gggccacggt gcctggccta catatgtatt 1440

ttttcctttt cttccccaag tggtaggata tgatacacat tgttgatttt tttgtttagt 1500

tatgtatctc agagcttatt ctttatcagc tcatgaggaa cttcattttt tttttttttt 1560

ttgagatgta gttttgctct tatagcccag gttggagtac agtaacacaa tcttggctcg 1620

cagcaacttc tgcctcccag gttcaagcga ttctcctgcc tcagcctccg agtagctagg 1680

attacaggtg cctgccacta catccagcta tttttgtatt ttcagtagag acggggtttc 1740

accattttgg ccaagctggt ctcgaactcc tgacctcagg tgatccgccc atctcagcct 1800

cccaaagtag tgggattaca ggcatgagca accgtgcccg gctggaactt cattcttttg 1860

gtataactgc atggtatccc atcatgtgga tgtaccatga ttcattggat gtggaccctc 1920

ctgatggaca tttaaatttc ttccaatctg ttgctattac aaaaagaaaa atgtgtgcat 1980

acatctttat tcatctgtag aataaattct tagaagt 2017

<210> 79
<211> 37
<212> DNA
<213> Homo sapiens

<400> 79
ggtttttata caagaaaaat aaagtgaatt aagcgtg 37

<210> 80
<211> 140
<212> DNA
<213> Homo sapiens

<400> 80
gtggcaatca taaaaagtaa taaaggttct ttttgacctg ttgacaaatg tatttaagcc 60

tttggattta aagcctgttg aggctggagt taggaggcag attgatagta ggattataat 120

aaacattaaa taatcagttc 140

<210> 81
<211> 55
<212> DNA
<213> Homo sapiens

<400> 81
acaagattcc tcaaaatatt ttctgttaat aaattgcctt catgtaaact gtttc 55

<210> 82
<211> 1485
<212> DNA
<213> Homo sapiens

<400> 82
gggcttccaa tgtgctgccc ccctcttaat actcaccaat aaattctact tcctgtccac 60

ctatgtcttt gtatctacat tcttgacggg gaaggaactt cctctgggaa cctttgggtc 120

attgcccttt cacttcagaa acaggttgac aactcagccc tgctcatgag gcagcaaacc 180

ctgcaaaggg ctgggactgg tggccttatg tcagttgtct actctggagc ttgacttgga 240

cctccccagg tcctaggcag taggttgaaa aacactgaag tgcttttcat gaagcacagc 300

tgcagcaaag ccttgcaatc ccaggctggg gtcagcctac agttgtgttg cttattacaa 360

cacatgcgga ccaagagggg cttgtgggct agaggctgac cagcagcgtt tatttagcaa 420

gggtaggtgt gcatcacatt gggcttgttc tcacccatct ggtttggcca ttcctccttg 480

gtgggaatca tccaggtact gctgaggtca cctgcgattt gccccatttc ctatctctag 540

caacctcctg ggccccatgc ccccacccct tctagaacct gcattcccag ggccttcacc 600

acctgaccaa aggtctaggc taacctttgg tcatttgtaa caagacctcg gaacagacac 660

gtgtgtggca tggtttggcc tggggatctt agatgtctga cctgaactat tgtagaacag 720

cgctggcttt tgggggagca gcaaaaatga gaggagtgct aggtgggtgg cctgagcatc 780

tgtatccagg gacaggactc caaaggcttt tggtcccaga gctggggtat gttggcccca 840

gcccccagcc tgtggctccc aaaaggcctc tggttttttg taatctcagt ttacagccat 900

ttcttaggtt tttaattacc tttattttat tttgccaaac atacctggga ataccttttta 960

tttttttttt accttggggt gatggttcca aaccataaat gtgattatag ttaacacatg 1020

acccttctag cgtcccagcc agtgtttttc ctgacctctg ttctttggag aggaggatgg 1080

aagggagggg tccggcacgc tgctggcatt ttgctgtgtc ctgcagcccc tttccgggac 1140

acctgggttc acacagcttt ttagcttaca taactggtgc agattttctg tgtggagatg 1200

ttgccttgac cagccttggc tggactttac caggcatgca gaagcctgta ccaacacaga 1260

ctacagcacc caggaggtgc gagtgtggct gctcagcggt tataacaggc ctgactgcat 1320

tgttcaccgg attataatga gccaaaatgt ttcccggtgt ttgctggttt cagggaagga 1380

gtttgatata gcagattaac caccctcctt gtagctattg gggcttaatg gtttcctggt 1440

gattcttacc aatccacaat aaacatggcc cattggcata tctgc 1485

<210> 83
<211> 31
<212> DNA
<213> Homo sapiens

<400> 83
ggcacatttg aataaattct attaccagtt c 31

<210> 84
<211> 39
<212> DNA
<213> Homo sapiens

<400> 84
ggggcgcatt gtcaataaag cacagctggc tgagactgc 39

<210> 85
<211> 486
<212> DNA
<213> Homo sapiens

<400> 85
gcccaataaa gactgttaat tcctcatgcg ttgcctgccc ttcctccatt gttgccctgg 60

aatgtacggg acccaggggc agcagcagtc caggtgccac aggcagccct gggacatagg 120

aagctgggag caaggaaagg gtcttagtca ctgcctcccg aagttgcttg aaagcactcg 180

gagaattgtg caggtgtcat ttatctatga ccaataggaa gagcaaccag ttactatgag 240

tgaaagggag ccagaagact gattggaggg ccctatcttg tgagtggggc atctgttgga 300

ctttccacct ggtcatatac tctgcagctg ttagaatgtg caagcacttg ggacagcat 360

gagcttgctg ttgtacacag ggtatttcta gaagcagaaa tagactggga agatgcacaa 420

ccaaggggtt acaggcatcg cccatgctcc tcacctgtat tttgtaatca gaaataaatt 480

gctttt 486

<210> 86
<211> 44
<212> DNA
<213> Homo sapiens

<400> 86
gcccctcccc tgccctctcc ctgaaataaa gaacagcttg acag 44

<210> 87
<211> 406
<212> DNA
<213> Homo sapiens

<400> 87
gtgtcatctt ttattatgaa gacaataaaa tcttgagttt atgttcactt catttgtttg 60

ctgttcatct tttgggaggg aataagctag agccatcaat acaattccgc ttgtggggaa 120

atttatgcct cttactggta ctacttgttt tgcattgaag ctgactggtt gagttcacat 180

catatgttgc aattttctaa tttggcactt caatcactag gggccttatg aggcagtttg 240

tcattatgca atggttattg gttatcatgt gagtagacac atttcaggct aatagggaga 300

agtcagtaac acattcatag tgaatatgag atgtctttgc taagagttaa gtgtcagatc 360

tttgttataa cagttaattt aataaagaat tttggcattg ttcttc 406

<210> 88
<211> 1994
<212> DNA
<213> Homo sapiens

<400> 88
ttgccgtaag gatatgcact tgtctctagt ccacacactt catgatatag gtatagcgtt 60

agtttagcga agttttcact gcactgatat atctagtagg tgatggagct gggaatgcaa 120

ctcatgtctg actagtccac aatactgcac tatttcagtg tttacgattt tttatccttt 180

cccttctgaa gaggcaaaaa attgaggaat gtgccctgct ttcctaagaa ctgaagtgtg 240

agtacactgg taaatccttt catttgcctt gttccttatc tgtcaatatg tctgaatcct 300

cgcttgttgg ttgcactaag aattgttctg ttgtttctca tcacagaaat ctgcagtcaa 360

ctacctgttc tcgtgaagtc ttaaaactct tatagaatag ccatttaggc ctttctgcta 420

gcctcctgaa ttctgtattc tcaggctgag cgagtttctg tttactctca aaccttaggt 480

gatttggcta actcttaaag taattagcac gatgattgga acggagcatt ctctccaaca 540

cagcatttct tttggcactt tgcttcttgt gcagtttagc tccagaaagt attaaggaat 600

gactttagtg ctcatttgga tgcagtaagt ggtttgatct cagggtggca aaaagaatgc 660

tttttttata ccttttcaca ttcggataac ttgtttagaa gacagaggtt ctaactaggt 720

tttggcctat taagaactgc aaactagcag cagcagaact ctggctaaag gggcaagctt 780

attaggaaat tgagtattta aaagttgagc taccatatga tccaacaatc ccactgctgg 840

gtatataccc agaagaaaat cggtatatca aagagatatc tgcactccta tgtttgttgt 900

agcactgttt ataatagcta agatttagaa gcaaccttag tgtccatcgg gatgaatgga 960

taaagaaaat gtacctatac gcggccaggc acggtggctt gtgcctagca ctttggaaag 1020

ccgaggcggg tggatcacct gaggtcagga gttcgagacc agcctggcca agatagtgaa 1080

accccgtctc tagtaaaaat acaaaaatta gccgggcttg tggtgtgggc ctgtaatctc 1140

agccacccgg gaggctgagg caggagaatc gctggaacct gggaggcaga ggctgcagtg 1200

agccgagatc acgccactgt actccagcct gggcgacaga gcaagactcc atctcaaaaa 1260

aaaaaaaaaa aaaaagggaa aaagaaaatg cacctataca cagtggtact attcagccat 1320

aaaaagaatg agatccagtc atttacaaca acatgggtgg aactggagat cgttatgtta 1380

agtgaaatag gcacacaaag acaagcatca catgttcttg tttgtgggat ctaaaaatca 1440

aaacaagtgg acttgtcata tagagagtag aaggatggtt accagaagct gagaacttct 1500

ggtggcggga ggtggggatg gttaatgggt acaaaaagaa aaaagaatga attagaccaa 1560

ctatttgata gcacgacagc gtgactaaag tcaataactt agttacatat tttaaaataa 1620

cttagagtgt aattggattg tttgtacctc aaagaaaaaa tgcaataaaa ctttacagtg 1680

gagaaaccta acaagcacta cctcagccag gtaatcaagg ttaacatcaa cagtcacgag 1740

tcatgttgat atataccctt gataaggtgt gatgaaaatg acacttaaac ctaaaaatcc 1800

ataaccctat ctaatgagaa aaataacaaa tcccaagagg ggcattttac aaaatacttg 1860

accagtagtg cggaaattgt caaggtcatc aaaaaagtct gagaaattgc cacagccaaa 1920

ggagtctaga gacatgatga ctaaatgtta ggtggtgtcc tgcgtggggt cctagaacag 1980

aaaaaggaca ttag 1994

<210> 89
<211> 307
<212> DNA
<213> Homo sapiens

<400> 89

accgctagct tgttgcaccg tggaggccac aggagcagaa acatggaatg ccagacgctg 60

gggatgctgg tacaagttgt gggactgcat gctactgtct agagcttgtc tcaatggatc 120

tagaacttca tcgccctctg atcgccgatc acctctgaga cccaccttgc tcataaacaa 180

aatgcccatg ttggtcctct gccctggacc tgtgacattc tggactattt ctgtgtttat 240

ttgtggccga gtgtaacaac catataataa atcacctctt ccgctgtttt agctgaagaa 300

ttaaatc 307

<210> 90
<211> 38
<212> DNA
<213> Homo sapiens

<400> 90
gtctgtaggc cttgtctgtt aataaatagt ttatatac 38

<210> 91
<211> 133
<212> DNA
<213> Homo sapiens

<400> 91
agtgtctagc agtgagctgg agattggatc acagccgaag gagtaaaggt gctgcaatga 60

tgttagctgt ggccactgtg gatttttcgc aagaacatta ataaactaaa aacttcatgt 120

gtctggttgt ttg 133

<210> 92
<211> 1802
<212> DNA
<213> Homo sapiens

<400> 92
tgtcactgcc atggccgcct tgctgcattt ctgaggatgc ttcatctctc caccttcttc 60

tccactcagc agccagcagg gcactgtgga aatcggagtc acatgagctg gcacctctgt 120

tcagaaccct ccagggctcc acatctctct cacccaaatg ccaaagacct ccccacgccc 180

ccacaatccc ccacgacctg gccactggcc tcccaccacc ttccagctcc agcggctcct 240

accacattta aggctttcct tcctagtttt aatttttcct cgtcagcagt tgattttatt 300

attttcttgt ttattggtat tttcccacta gaaatgaagc tgcgtgaagt tagagatttt 360

ttttttggt ctgtgttcct aattagctca ttgctatacc cctggcgccc agaacaatgc 420

cttggacaca gtacgcagta gactaaataa atacttgttg aatgactgac tgacggaatg 480

acggctgtgt ggggagtgga ttgggtcgtg aggcagaggc tgcggtggaa actcaggcag 540

gaggtgatgg tggttcttgg ggctgcggaa tgccaagttt agaagctctt cctctgctgt 600

ggcacatgaa ccggtcactc gagaaggctt ttagatttac tttgcctaat cccctcttag 660

tgcatgtggg gaaactgagg tacacaaaag gaattcccca ccaagttagg ggcagaacct 720

agccccccttg tctcccagat ggatatcttc tttttttttt gagacggagt cttgctctgt 780

tgcccaggct ggagtgcagt ggtaccatct tggctcactg caacctctgc ttcccaggtt 840

caagcgattc tcctgcctca gcctcctgag tgtctgcgat tacaggtgca cacaaccacg 900

cctggctaat ttttgtattt ttagtagaga cggggtttca ccgtgttggt cagggtgacc 960

tcaaactcct gacctcatga tccacccagc tcagcctccc aacgtgctgg gattacaggc 1020

atgagccacc gtgcctggct ggacatcttg ttattaaagc ttcttctctc tttgtagggg 1080

agggggagat gcctctggtg gagaagacca gtgtggcagt gactgtgtct gttagtgaac 1140

ctggtggctg gttgagggtc tgtcgtggtg actgaggaca catacaaagt gcttttctca 1200

gtggtcacct tggtgttggt gaataagggt cagaagatgg ctcctgtcct agggcactgc 1260

cagtcggttt ggaagctgaa atgcctgctt agcagtttga ggaaacacag accttggagg 1320

atcttctggt tgcctcttca agaattcatt ctattcccct tctgctcccc aaatttgctt 1380

ttcttggggt gggtcttggt tggcctaagc caagaaagta tggcatctac tccttccata 1440

gcaatagctc aggaataggc agtgacccag acctgaacca atcagtgcat ggaattaccc 1500

ctggccaaag tggttgattg aggctgggtg caagcagagt tgtgagaagg ctcccatttg 1560

gtggttggag agatcgcact tgctccagag gtcataatgt gcagatctga ggcttggaac 1620

tgctgcagac attttgctac cacaagtgaa gccaccctga cgacacagtt gacaatttgg 1680

agcagggcag agctgagaga acagcaggga aacagccaga gtcttgctca agcctccctg 1740

aagtatctat acccctggac tctagttatg gggctaata aatgttatat actgtttaag 1800

gt 1802

<210> 93
<211> 34
<212> DNA
<213> Homo sapiens

<400> 93
tgctgagggc ctcaataaag tttgtgttta tgcc 34

<210> 94
<211> 502
<212> DNA
<213> Homo sapiens

<400> 94
aaaaatgaaa cttttttgag taataaaaat gaaaagacgc tgtccaatag aaaaagttgg 60

tgtgctggag ctacctcacc tcagcttgag agagccagtt gtgtgcatct ctttccagtt 120

ttgcatccag tgacgtctgc ttggcatctt gagattgtta tggtgagagt atttacacct 180

cagcaaatgc tgcaaaatcc tgttttcccc cagagagctg gaggttaaat actaccagca 240

catccctaga tactactcaa gttacagtat atgatcacta atatagtatg ctcttggtac 300

caggagctct gatatatatc tggtacatgt ttgataatga cttgattgtt attataagta 360

cttattaata cttcgattct gtaaagagtt tagggtttga ttttataaaa tccaaaatga 420

gccttttatt gaatccagtt ctctatgtga ccagttctct gtatgaatgg aagggaaaag 480

aattaaaaat cttgcaaagg gg 502

<210> 95
<211> 502
<212> DNA
<213> Homo sapiens

<400> 95
aaaaatgaaa ctttttgag taataaaaat gaaaagacgc tgtccaatag aaaaagttgg 60

tgtgctggag ctacctcacc tcagcttgag agagccagtt gtgtgcatct ctttccagtt 120

ttgcatccag tgacgtctgc ttggcatctt gagattgtta tggtgagagt atttacacct 180

cagcaaatgc tgcaaaatcc tgttttcccc cagagagctg gaggttaaat actaccagca 240

catccctaga tactactcaa gttacagtat atgatcacta atatagtatg ctcttggtac 300

caggagctct gatatatatc tggtacatgt ttgataatga cttgattgtt attataagta 360

cttattaata cttcgattct gtaaagagtt tagggtttga ttttataaaa tccaaaatga 420

gccttttatt gaatccagtt ctctatgtga ccagttctct gtatgaatgg aagggaaaag 480

aattaaaaat cttgcaaagg gg 502

<210> 96
<211> 48
<212> DNA
<213> Homo sapiens

<400> 96
atttttctg tagtgctgta ttattttcaa taaatctggg acaacagc 48

<210> 97
<211> 171
<212> DNA
<213> Homo sapiens

<400> 97
gctgttcttg cataggctct taagcagcat ggaaaaatgg ttgatggaaa ataaacatca 60

gtttctaaaa gttgtcttca tttagtttgc tttttactcc agatcagaat acctgggatt 120

gcatatcaaa gcataataat aaatacatgt ctcgacatga gttgtacttc t 171

<210> 98
<211> 2309
<212> DNA
<213> Homo sapiens

<400> 98
ggtggttctt tccttgaagg gcagcctcct gcccaggccc cgtggccctg gagcctcaat 60

aaagtgtccc tttcattgac tggagcagca attggtgtcc tcatggctga tctgtccagg 120

gaggtggctg aagagtgggc atctccctta gggactctac tcagcactcc attctgtgcc 180

acctgtgggg tcttctgtcc tagattctgt cacatcggca ttggtccctg ccctatgccc 240

ctgactctgg atttgtcatc tgtaaaactg gagtaaaaac ctcagtcgtg taattggtgg 300

gactgaggat cagttttgtc attgctggga tcctgtcagg cactttgagg tgtccctcag 360

gccttggccc tgaagtgtct aggtgtgtgg agatgggtag aaaattaggt acacccaatg 420

gtgtagaacg ttgattctca aattttttta ttttatacaa atggggtctc actatgttgt 480

ccaggctggt cttgaactcc tgggctcaag ccatccgccc atctcagccc ctcaaagtgt 540

tgggattaca agcaagaact gccatgcctg acccagttct cagtttttttg tttgtttgtt 600

tgtttgtttg ttttgagacg gagtcttgct ctgtcgccca ggctggagtg cagtggcgca 660

gtctcggctt actacaacct ctgcctccgg ggttcacatc cttctcctgc ctcagcctcc 720

cgagtagctg ggactacagg tgcccgccac aactcctggc taattttttg tatttttagt 780

agagacgggg tttcactggg ttagccaggt tggtctcgat ctcctgacct tgtgatccat 840

tcgccttggc ctcccagaat gctggtatta caggcgtgag ccagcacgcc tggcccagtt 900

actcagtttt gaatctgagg ccgtgacatc actcatggtc tgcagtcagt gctctgcccc 960

tgagctgtac cctctcctat gataatcact cttaagaagg gcaacccttg gtgttttccc 1020

cttaaggtca cccaggctgg aatgcagtgg tgtggtcatg gctccctgta ccctggaact 1080

caggcttggg tgatcctctc tcctttgcct ccgaagtagc caggactaca ggtgtgcacc 1140

caccaccaca ctcagataat tgctttggtg tttttaaagc ttgtaatgat cagtaggctg 1200

aggtgggcaa atcataaggt caagagtttt ttagatgggg tgagcacaga ccaattcctg 1260

ttttatttac tgatttaaaa ttttgagaca gtctcactgt cacccaggtt ggggtgcagt 1320

ggtaggatca tagcttgctg cagccttgat ctcccaggat cttgcctcag cctcccgagt 1380

agctgggact gcatgcttgt gccaccacac tcggttaata ttttgtagag atggggtctt 1440

gctatgttgc ccaggctggc ttcaaactcc tgaacttaaa agcctcctgt ttagttttgg 1500

tttttatca cttttttttt ttttttttga gatggagcct tgctcccatc gtgcaggctg 1560

gagtgcggtg gcgcagtctc ggctcactgc agcttctgcc tctcgggttc aagcgattct 1620

cctttctcag cctcttgagt agctggaatt accagtgtgc gccaccacca ccacgcctgg 1680

ctagtttttc tgtttttagt agagacaggg ttttgctatg ttggccaggc tggtcttgaa 1740

ctactgacct cttgtgatct acctgtcttg gccttccaaa gtgctaggat tacaagcgta 1800

agccacagcg cctggccttg ctacattttt tttttttttt tttttttac agacatggtc 1860

tcgctatgtt gcccagaatg gttttgcact gggtccaagc agttctgccg cagcctccca 1920

aagtgctggg attacagggg tgaggcacct tgctggcccc tgttttgatt agggtgcagt 1980

gctggtgaag ccggtgcacg aggccagtga tgcatcctaa tgaggggtgg agttggcggg 2040

acttcctggg ccagtttggg gactttcaca aaagaccccc atgactcagg gttttgagtt 2100

cttaactgat cgaatgaagg attcaaaatt aaccactcca aggggggatt gaaggaagaa 2160

ccactcttaa tggacaaaaa gaaagaaagg ggagggagta acagggatat gagctctagc 2220

cgcccaagct agcaatggca acccttctgg gtccccttcc agcatgtgga agctttcctt 2280

tcgcttcatt caataaacag ctgctgctc 2309

<210> 99
<211> 58
<212> DNA
<213> Homo sapiens

<400> 99
gtcttttgta attctggctt tctctaataa aaaagccact tagttcagtc atcgaaaa 58

<210> 100
<211> 1502
<212> DNA
<213> Homo sapiens

<400> 100
gcaaaggctc cccttacagg gctttgctta ttaataaaat aaatgaagta tacatgagaa 60

ataccaagaa attggctttt agtttatcag tgaataaaaa atattatact cttgaacttt 120

tgtctcattt ttttgagtat gctgtttata tgattttgat ttccctctga taactatcaa 180

cagtatttaa atagcttata gctggtataa ttttttccca cgatttccaa aatcttttat 240

gtactcaggt aaaagtagcg ttatatagga aatctttttt ttagacactc tcgttctgtc 300

acccaggctg gagtgcagtg actcagcttc ctaaatagct ggaattacag gtgtgagcca 360

ccatgcccgg ctaatttttt gtacttttag tagagtaggg tttggccatg ttggccaggc 420

tggtttcaaa ctcctgacct caagtgatct acccacctcg gcttcccaaa gtgctgatta 480

tagctgtgaa ccaccatgcc cggccaggaa atcttactgt agaacaattt tttatatagc 540

tgtataaaat gtatatgatt gtcttgacag tctcaaatac tgtttttaat agcttgtaaa 600

tgtaatctca agtgcttaga acagttctta catataagtt gctctgtagt ttgctcttat 660

agttagccca aagactctgg gtgtgaggcc tgctgtaaac caatgttaaa ctgcttatta 720

gaaagcccta accacctgct ttgtaggcac cagaaactca aaaccaaatc tcaactcagc 780

tacagaatct actgtggtcc ttgtctgaaa aaattagttc actcggttgg aatcttgtct 840

cagagcatcc tcatctcttt ctcaaaagcc cctaccccaa caccggcgtg ttggttgtct 900

attgaaactt acaagtggat ggaccctttc tcccgaataa actggccttt gaaagctcta 960

atcgaaatgg tttggcaaaa tccatactgc aggagattag ggaggacaag aatgatgtgc 1020

ctttttgtac tgctgagcct gatggtggtg ccactacttc aggtacttag atgagtcttg 1080

atgctaatag aattgtgtcg ccaaacatat ctggacagtt acaacctaat ctatgcatta 1140

attggtttgg gaattgcttg aaattattgt ttaattcaat gttttaattc gttttcctaa 1200

aaatttaagt gcccccatca tcgtgcaata cctcagtgca gcaactcctt gattcttgga 1260

tgactgaact tcctaacttg gctctgcccc attgttccca tttttcatgt tttcacaaa 1320

tagttaacca ggtacctact actgtgcacc gctgcagagc attgaggatg tatgtgatga 1380

gtaaaaacac ccagcctgct ctgctgtgtt agtattatga cggaaactga tcaaatcaca 1440

tgtgaacaaa tttactgcta caaaagggag ggcttaataa aaggaatttc atctgggaag 1500

gc 1502

<210> 101
<211> 48
<212> DNA
<213> Homo sapiens

<400> 101
atttttctg tagtgctgta ttattttcaa taaatctggg acaacagc 48

<210> 102
<211> 193
<212> DNA
<213> Homo sapiens

<400> 102
ggaattgcac atgagatggc acacatattt atgctgtatc aagttcacga tcatcttacg 60

atatcaagct gaaaatgtca ccactacctg gacagttgca catgttttac tgggaatatt 120

tttttctgtt tttctgtatg ctctgtgcta gtagggtgga ttcagtaata aatatgtgaa 180

agcttttgtt tcc 193

<210> 103
<211> 73
<212> DNA
<213> Homo sapiens

<400> 103
aggttttggc agtactgtct ccttgggcca tgctggtctg acttatgctt actaataaat 60

tctgtttact ggc 73

<210> 104
<211> 93
<212> DNA
<213> Homo sapiens

<400> 104
actttgggat atttttcttc aattttgaag agaaaatggt gaagccatag aaaagttacc 60

cgagggaaaa taaatacagt gatattctta cgc 93

<210> 105
<211> 257
<212> DNA
<213> Homo sapiens

<400> 105
gctgtgtggg gtggatgaac cctgaagcgc accgcactgt ctgccccaat gtctaacaaa 60

ggccggaggc gactcttcct gcgaggtctc agagcgctgt gtaaccgccc aaggggttca 120

ccttgcctgc tgcctagaca aagccgattc attaagacag gggaattgca atagagaaag 180

agtaattcac acagagctgg ctgtgcggga gaccggagtt ttatgtttta ttattactca 240

aatcgatctc tttgagc 257

<210> 106
<211> 692
<212> DNA
<213> Homo sapiens

<400> 106
tgattcaaac agcttcctga attttaattt tgtgttgtct cacagaaagc cttatcataa 60

attccataat tctaattaat ttaccaagat aatgtaatta catttggttt tgtaaggtat 120

acagcagtaa tctcctattt tggtgtcagt ttttcaataa agttttgatt atgggcaaat 180

cccctctttt tcttttttta aaatatattt gagtatgcca tacatttata tatatggtgt 240

atatgaattt ggtttaaaca ttttaaaatt tattctgatt agtttgtgtc tttttttttt 300

tttttgagag agagagtcct gctctgtcac tcaagctgga gtgcagtggt gcgatctcgg 360

ctcactgcaa cctccgcctc ccaggtccaa gcaattctct tgccttgtcc tcccaagtag 420

ctgggattat aggcacacac caccatgcct ggctaatttg tgtctcattt tcaagagtag 480

aaaccctaaa tattttattt tcattccttt tccaaattgc tatgaatggg attaaaggat 540

tacagatgta aagtctatta tttgtgaatt ctaaatgtag ttctgctgtt gtacctgtgg 600

aaacatctta aagaagtaca tattttgcac gtcctgcacg tgtaccccag aacttaaact 660

ataattaaaa agaatagttt caaaaaaata ca 692

<210> 107
<211> 228
<212> DNA
<213> Homo sapiens

<400> 107
atagaacctg ttgtgcaacc acggtttaac cggagatttt gaggctaggg tgtgtttctt 60

tcgaactttt cggaatgtct ggaacatttc atttcctgtt ttgttacctg tgcctctgta 120

aatctacttt tgcaatttta agtaataatt ttatgaataa aaatgggaaa tgcttcctaa 180

ttccacatag tatttgcatt gttttataaa taaattccac ttactatc 228

<210> 108
<211> 529
<212> DNA
<213> Homo sapiens

<400> 108
acccaggtga ggcagggctg aaaactgccc ttgggctgac ttttgatagg ccatgccttg 60

ccactttaca agttcttttt gcatttacta gtatttaaga gtaaccttga gattgggagg 120

aatagaggag gctggtacaa atagatggag acctgctggg atcagtgaat gcctgattag 180

gacatggggc tatgcatagc ctaagagtta taggcttaaa gatgtcgagt aactaaaaac 240

tgtattgctg gccgggcgcg gtggctcacg cctgtaatcc cagcactttg ggaggccaag 300

gcgggcagac catgaggtca ggagattgag accatcctgg ccaacatggt gaaaccctgt 360

ctctactaaa aatacaaaaa tgagctgggt gtggtggcac gtgcctgtag tcccagctac 420

tcgagaggct aaggcaggaa aatcgcttga acccaggagg cagagattgc agtgagccaa 480

gattgcacca gtgcactcca gctgggcgac agagcgagac tccatctcg 529

<210> 109
<211> 174
<212> DNA
<213> Homo sapiens

<400> 109
gtggaaaaga acatgaaaaa gaaaactgac aaatacacac aggtctcctc aagatccatg 60

gacttctggt ctgagcctaa taaagactgt ttgtttattc ctcaaaaaca aacaaacaaa 120

aaaaaaccct ctgtattata aattattctg tgtaatggtg tgttaccata catt 174

<210> 110
<211> 156
<212> DNA
<213> Homo sapiens

<400> 110
atgctcctct actctttgag acatctctgg cctataacaa atgggttaat ttatgttaaa 60

aaaaaaaaaa gagagagaga gtgaaacaac aatctacaca atcagagaaa atatttgcaa 120

atcttatatc tgattagaaa ttagtatctg gaacat 156

<210> 111
<211> 46
<212> DNA
<213> Homo sapiens

<400> 111

aattggagag gattatttca cattgaataa acttacagcc aaaaaa 46

<210> 112
<211> 67
<212> DNA
<213> Homo sapiens

<400> 112
ggagctgagt tcttaaagac tgaagacagg ctattctctg gagaaaaata aaatggaaat 60

tgtactt 67

<210> 113
<211> 294
<212> DNA
<213> Homo sapiens

<400> 113
ggaattgaac atgagatggc acacatattt atgctgtcta aaggtcacaa tcatgttacc 60

atatcaagct gaaaatgtca ccactatctg gacagttgga catgtttttt tgggaatata 120

ctttttctct ctgaatctgt taggaacttt ctggttggct gggttccgta ataaatacat 180

gagacctttc atttcaaaaa aaagaaaaat aggcctcctt cccaggggct ccggatttca 240

tcagccttct gtgcatgccc agccatacaa accacgcagg gatggctcca agtg 294

<210> 114
<211> 171
<212> DNA
<213> Homo sapiens

<400> 114
tcaccaaaaa gcaaccaact tagccagctt tatttgcaaa acaaggaaat aaaggcttac 60

ttctttaaaa aataaataaa taaataaata aataaataat aaataaataa ataaataaat 120

aaatagataa ataaataaaa agttttctac tcacactgaa gtgacgaagt c 171

<210> 115
<211> 49
<212> DNA
<213> Homo sapiens

<400> 115
gccccctcc cctgccctct ccctgaaata aagaatagct tgacagaaa 49

<210> 116
<211> 88
<212> DNA
<213> Mus musculus

<400> 116
ggaggcctca gttcctggcc ccagaaacga gatcctgacc acatgaacaa tttgggctct 60

tttgggagaa taaaagactt atatattg 88

<210> 117
<211> 69
<212> DNA
<213> Mus musculus

<400> 117
ttccaggacc actttgtgca gatggtgggg tctcaccaat aaaatatttc tactcacact 60

ggttttccc 69

<210> 118
<211> 118
<212> DNA
<213> Mus musculus

<400> 118
actattaaaa attgttaaat tccagagagc aagtagagac cgcatatttc aataaatcaa 60

acatgtggtg acaaaccctt gtgtgactct taaattgtgg atgtttccaa gccccttg 118

<210> 119
<211> 1008
<212> DNA
<213> Mus musculus

<400> 119
agcagttttc tatgaagatt ttttcataaa gacaataaac atattgatca agcagctttt 60

tctgtgttaa gctgttatta atgagactat aggaaatagt gtgaaattac aaaagcaaag 120

aagtagatag ttatttaatt aattaaatta attttacctt ttgtgttgca ccataaccta 180

ccactggtgg gattaagggc aagtattacc atgcctagct gagagtcttt ctccaggaaa 240

aaccagctta catgggttcc tgcaaatctc atgagtgttt cttgggtttc tagtcttcct 300

gggaggtgtc cttatctttc agattttcag atctggtaat tagcatgatc atcaggacat 360

ttattacaaa caaattgatt agtgggaaga aagtatctca aggtcaatct tggaagtgaa 420

caactggtgc taatccatgg ctttaaagat ttgagaacaa cggtgaaatt tggtttgagg 480

agaagggggt gtctaggacg tttcattttt atggtacatg ccagacatga atgtacatag 540

gaaaataact tgaaagggtc aaatattaaa ccttgaatat caggttcact tgggaaagca 600

ttaggtgctt atgcctctta gtaaatagcc cttcatccca gaaggagcaa gaattgtctt 660

cctgacttaa tccagtctta gctgaggtgc tgtgcatctt tatcatcttt gccttgcctc 720

acagtgtcag gctctgtggt actggggcta cacaggtcag gtaaacagtt aactgcttac 780

ctacatcccc agcaaagata atgtgacgat actaagatga acctatcaga gcttaaagat 840

aatgagtttc agtcacagtg ataactgcat gctaacttca gcatgtagaa tatatgccga 900

agctaaaagc cattccacag ttgactccat ctgaagttaa agtgtgtaag tacacagtaa 960

atcatgctat attaactgaa ctttttaata aatgagtcat ttgaattt 1008

<210> 120
<211> 306
<212> DNA
<213> Mus musculus

<400> 120
attgttaacc taattaaaca gcttcatagg ttcttttggt gtccttttg tgtgttgtgt 60

gtgcacatgt ttgttgggtg ggtgttttgc tggtgtcttt tcctctgtgt cttcctctgg 120

ccctttctgg aaagacctgc ttaatctgaa gcatgtgagc taggctagtc cactgggtcc 180

tgctctctgc ccatccccag ctggctttgg attagaggca catacactgc catggctgcc 240

ttttactgtg gctgtggttt tgcccttttt ttttaagcaa atagaaaatg ctgctgacta 300

tactgg 306

<210> 121
<211> 63
<212> DNA
<213> Mus musculus

<400> 121
ggtgtcaccc attgtatttt tgtaatctgg tcagttaata aacagtcaca gcttggcaaa 60

ttg 63

<210> 122
<211> 49
<212> DNA
<213> Mus musculus

<400> 122
atgtacacta aattttctgt acctaaatat aattacaaaa ttatcttga 49

<210> 123
<211> 39
<212> DNA
<213> Mus musculus

<400> 123
acttgatcca aaaagctaat aaaattttct cagaaatgc 39

<210> 124
<211> 47
<212> DNA
<213> Mus musculus

<400> 124
gaagcaacaa gaaaatattc caataaaaga ctatctgata accagtg 47

<210> 125
<211> 42
<212> DNA
<213> Mus musculus

<400> 125
ttctgtgttg gagagctgca ataaattttc cataaagcaa aa 42

<210> 126
<211> 556
<212> DNA
<213> Mus musculus

<400> 126
ttctccagtg tatttgtaaa atatattcat taaagtctct gctctgagag ctggtcttct 60

tgacaccttt tccaatatca gctttgcaga aggaaactta aatttcagtt cagggcatga 120

ccttcatgac cttgcagaac ttcttcactt tccaggttaa gtaaaggcga tctttagggg 180

ctgtccagat ggatcagcta taaagattca attgtagaag gttcacgtct caatgcccac 240

gtggtagctg taacttcaat taaaaaacaa aaacagccgg gcgtggtggt gcacgccttt 300

aatcccagca cttgggaggc agaggcaggc ggatttctga ggccagcctg atctacagag 360

tgagttccag gacagccagg aatacacaga gaaacccttg tctccaaaaa ccaaaaaaaa 420

caaaacacgc attcttttca ggtctttgct gggaccaggt acacataaca cagataaata 480

ttagagcaaa ccatgcacat atggtaaatt atctttgggt tttgggtccc taaaataaag 540

tggtgtgttc attgtg 556

<210> 127
<211> 47
<212> DNA
<213> Mus musculus

<400> 127
ccctggatct taactgttaa taaaaaaaac attggatgat gatggta 47

<210> 128
<211> 693
<212> DNA
<213> Mus musculus

<400> 128
acacagagac ccactgaata aaaacttgag actgtccttg cttgtttgct tctatgtccc 60

tggagaggtc ccagttggtc ccgtccctaa caacatgcta gccctgctca cctgcctgtc 120

agccttgctc agtggcatct ttccataggt gtgtatcccc ttagattagc ttcagcccca 180

ctacgatttg tctaggacat agcctgagcc ctgcctgtga cactgagggg tagcagtctg 240

tttctggact ccagggtgct gctgtctcag gcctaagaat tccagacatg actataatcc 300

aagcctgggg acctggttga gctttttatc ctgctggctc taagcttcag ctaggtggaa 360

atgaggccag ccaagcccca cagtgagctt gcaagcttta gatggggaca gggttacgct 420

ttggtgaatg atggaggaaa catgggggtt ccttttgttg ggtgcagcca gcacggcatc 480

atcatggtgc ccaatcttga aagggcacag gcctgaagct tcctgggact gttctgtcac 540

agggaggaac ctactgcagt tgcctacaat tgctacctct gagggacttg cctctggccc 600

cttgtagaca tttccatgtc tacacatggc ccagagtact ttcagggata gcaatgtgtg 660

aatggcactt agaagataac atgtgaaagc cat 693

<210> 129
<211> 98
<212> DNA
<213> Mus musculus

<400> 129
agcttccctc gtgtctgtac atagcggcct ggctgtggcc tcatgtggat cagtctttaa 60

aataaaacaa gcctttgtct gttgccctct tgtttagc 98

<210> 130
<211> 1077
<212> DNA
<213> Mus musculus

<400> 130
tgtacacaaa gaaataaaat accagcacca ggactgtgaa gtgttttcct tagactgtag 60

tgtggggttt gctcattggc tttcttgttc agattttact aattgttcta aatgatacag 120

cttagtgtgc agaaaatatc ctcttgattg gaaaatagcc aaatatttac aaaacaggta 180

tactagtttg aagaggctct atatgggggg agggggtgct ggaaaacatt agtgggtgac 240

cagtaatggt ggtacagctc taactcctag caccaggagt ccgaggcagg gggatcttca 300

ggctgcatga tgtgcatagt agcatgctgg tattagggag tgggtatctg tggttcccac 360

tttgaaaata accaaaattc tccaaagtgg gcagacctaa gccaggagag ggctggccac 420

agacatttgg tactgcttgc tgagaaagca ctgattgttt tcctaaccta aagattatat 480

atggcccacc ataccatctt tgaaacaatg tgtactggcc tttggttcac ctttcttgtc 540

tttgaagttg tacttggtgg gtgcatttaa ccttgccaca gagtggggag gatagagtct 600

aatggacctt aagtggtctc tggtggccat gtcggcagtg cttaggttgt agcccagggt 660

tggagtcggc agtgacaagc aaataactat attcttgctt gctgtggcag ctatacagaa 720

atttacggta taggtaagag ggttctctag aagtactacc tgtcttagtg aaagagattg 780

cttggttaac atcctgttat gtagtggggc tacttttaaa ctgtgtgaag tccccattag 840

ccacctccat aggcaatgga gctaacattc ttgctacagt ggccgcagct cattaacacc 900

taatgatgtg tttaacatgt gtccacatgg tgtgaatgtg ggtacgcatg tgcccagtat 960

tcagttcaca gaattgtcat catcttccat catgtcttca gtgagggact ctgcagatgc 1020

ccaccccagt ccttggttgt ggtgattctg ttagcattaa atgcactgga gagcttc 1077

<210> 131

<211> 1658
<212> DNA
<213> Mus musculus

<400> 131
gacacattgg tctgcaatgt tttgtattaa ttcataaata aaatttagga acaaaaccgg 60

tggtttatcc ttgcatctct gcagtgtgga ttggacagga agttggaaat gacagggact 120

ttaactgggc tgctgctcct ttgtatatag acactttttt cctgctcaga aacttgagtt 180

ctccagtagc aatggccaaa cagaagaacc aggctagggg gctgcatctg acagagcaag 240

tagacgagag gctgggtggt gggctccggc cagcccgagt cttagagctg gtggttggtt 300

atatctggtg cctgtctcga ggagggcttg agacacagtg tggtgctcct cagaagcaga 360

caggtgattt ctttgtgtga tttttctttt cccctgggac aatgacagtc agtaagacag 420

gtttcaggga cttttgtgtc caggtctgag cactagtcgc tcacagttgt gtgtactaac 480

cttcttcctt cctattgaaa tggcaggggt ctttgagtct cactgctgca tgttctgcct 540

tcatagggat ctgtaagtat gctgggcatc tgggctttta gggggctctc tatagggtgt 600

ctgagataga ggtcaacaag ggcttataga caactcaaac aaagcccatg gcttgagcaa 660

gtctgcaaca agctgtttgt ctagcctcca gcagagggcg agggagacag cttccagatg 720

ttcccagtag gtggagcccc tccaagccca gggctcagga ggcttacagg gtgggaactc 780

caatactggt ggagggagga gggcgtttga tgggaagata gggaagttgc tgcttcctaa 840

actgtcacaa ctgggcttgg ataggagtca tagtctggga ccacagccct gtggtagaat 900

gctagcctgg tgtgctccag gtttaatctc catcactgca gaaatgagtc caagctgtgt 960

gtacctccag ggcactgggc atggggttcc cttgccattg tgtgtgcccg gagaactggc 1020

aggcgggaaa tgtctttatc aagggttacc ttggaagagg tcccaacact gtagggtgct 1080

cctgttgtca aaacctatgc agaggcatct gcttgctctc taataacagt atgcaatgct 1140

aaagggctcg cttacagccg gtggccacac tggaggcctg cacatcaggt ggccacaagt 1200

tctgctgctg cgcctccgag gaaacacttg gtcctccgat cgattttaac ctgttgaggc 1260

tttgcaatcc ccctgtggca aaggctccag tgttttctat ttctatgcaa atttcttgaa 1320

gcagaactgt tactgtcttt ctcctctgcc ctgggaggag gcgctagcgt ttccttccaa 1380

cttcaggtgc agccccccctc gtggttagcg gtcttaagtt cgtgacttgg gtttgcagat 1440

ctttttgtt acatcgccgg accatgtggt ggtctttagc tgtaaacaac attaaccctg 1500

ggttgattag catatgcttc taaaagatgg tcccagattc tgcgacttgt aataaaatgg 1560

aaacttgctg gtttttatgc ctttctaact cttgtatttg aatgaatgtt gatcactttt 1620

tgtattaaag tggctgacac atggctactg tcactgtg 1658

<210> 132

<211> 1795
<212> DNA
<213> Mus musculus

<400> 132
aatatctcac caaaaaatat ttgaagaaga acaacctccg agactggctg cgtgttgtcg 60

ccaacagcaa agagagttac gagctgcgtt acttccagat taaccaggat gaagaggagg 120

aggaagacga ggattaggac acattggtct gcaatgtttt gtattaattc ataaataaaa 180

tttaggaaca aaaccggtgg tttatccttg catctctgca gtgtggattg gacaggaagt 240

tggaaatgac agggacttta actgggctgc tgctcctttg tatatagaca ctttttttcct 300

gctcagaaac ttgagttctc cagtagcaat ggccaaacag aagaaccagg ctagggggct 360

gcatctgaca gagcaagtag acgagaggct gggtggtggg ctccggccag cccgagtctt 420

agagctggtg gttggttata tctggtgcct gtctcgagga gggcttgaga cacagtgtgg 480

tgctcctcag aagcagacag gtgatttctt tgtgtgattt ttcttttccc ctgggacaat 540

gacagtcagt aagacaggtt tcagggactt ttgtgtccag gtctgagcac tagtcgctca 600

cagttgtgtg tactaacctt cttccttcct attgaaatgg caggggtctt tgagtctcac 660

tgctgcatgt tctgccttca tagggatctg taagtatgct gggcatctgg gcttttaggg 720

ggctctctat agggtgtctg agatagaggt caacaagggc ttatagacaa ctcaaacaaa 780

gcccatggct tgagcaagtc tgcaacaagc tgtttgtcta gcctccagca gagggcgagg 840

gagacagctt ccagatgttc ccagtaggtg gagcccctcc aagcccaggg ctcaggaggc 900

ttacagggtg ggaactccaa tactggtgga gggaggaggg cgtttgatgg gaagataggg 960

aagttgctgc ttcctaaact gtcacaactg ggcttggata ggagtcatag tctgggacca 1020

cagccctgtg gtagaatgct agcctggtgt gctccaggtt taatctccat cactgcagaa 1080

atgagtccaa gctgtgtgta cctccagggc actgggcatg gggttccctt gccattgtgt 1140

gtgcccggag aactggcagg cgggaaatgt ctttatcaag ggttaccttg gaagaggtcc 1200

caacactgta gggtgctcct gttgtcaaaa cctatgcaga ggcatctgct tgctctctaa 1260

taacagtatg caatgctaaa gggctcgctt acagccggtg gccacactgg aggcctgcac 1320

atcaggtggc cacaagttct gctgctgcgc ctccgaggaa acacttggtc ctccgatcga 1380

ttttaacctg ttgaggcttt gcaatccccc tgtggcaaag gctccagtgt tttctatttc 1440

tatgcaaatt tcttgaagca gaactgttac tgtctttctc ctctgccctg ggaggaggcg 1500

ctagcgtttc cttccaactt caggtgcagc ccccctcgtg gttagcggtc ttaagttcgt 1560

gacttgggtt tgcagatctt ttttgttaca tcgccggacc atgtggtggt ctttagctgt 1620

aaacaacatt aaccctgggt tgattagcat atgcttctaa aagatggtcc cagattctgc 1680

gacttgtaat aaaatggaaa cttgctggtt tttatgcctt tctaactctt gtatttgaat 1740

gaatgttgat cactttttgt attaaagtgg ctgacacatg gctactgtca ctgtg 1795

<210> 133
<211> 48
<212> DNA
<213> Mus musculus

<400> 133
actgagtcca gatggctaat tctaaatata tactttttc accataaa 48

<210> 134
<211> 140
<212> DNA
<213> Mus musculus

<400> 134
attcagcata aaataaaggc agataaagtt aaaggtcttc tggtggtctt taatgagccc 60

tgttgggagt gaggtgcttt aacatggaga agcatgttat taaacagtga aatagatggt 120

tcaaaaccac gtgaccatgt 140

<210> 135
<211> 46
<212> DNA
<213> Mus musculus

<400> 135
tgtggtagag cagagttgga aataaagctc tatctttaac tctagg 46

<210> 136
<211> 40
<212> DNA
<213> Mus musculus

<400> 136
agacatctcg tgcacggctt tcattaaaga ctgcttaagt 40

<210> 137
<211> 109
<212> DNA
<213> Mus musculus

<400> 137
gtatattttt gttttggtca ttaaaaatta aaaaaaaaa aatacaagtg tctgcctatt 60

gcatttgtgt gggaagagac tggggaaata aaacaggtgt gctgttgtg 109

<210> 138
<211> 45
<212> DNA
<213> Mus musculus

<400> 138
acaagaaagt tttcctttaa taaaactttg ccagagctcc ttttg 45

<210> 139
<211> 640
<212> DNA
<213> Mus musculus

<400> 139
aagccacacc ggaggttaat taaatgctaa cattttccat gtggtctttg catccttcct 60

tgtctgcatg ttggaaatct gcctaacatt ctaggaagag gtgaggtgtg ggcccttgag 120

agtcagtctg tgggaataag tgtagcccaa ctatgcacag ttgtaaattc ctacatcccc 180

gtgtgtattg gtcttgatat tcaaagaatt gatgaatgcc attactttca gtccaaagtg 240

aagaaacctg gtctcaaaaa atcccgagga ccagaaatga gatgggtttt cctgaaaatc 300

taaagttctt gaaaaacctt gccatccaga ttgctagcaa ctgcctagct ttgtaagctt 360

actgtgatgg acaggtagct caggacgact ggtcacttaa tactggacag attagcatgg 420

aaaacttaag gggaggagga ggtagtaggt tccatccagc ttcgctttgt tggtggcatc 480

taggtgttgt tccaagggag catgcctacc tgcaacagga catcactggt tgggaatact 540

gtagaaccag agctgtgacc tttgaactac tagaaagatg aaattttatg taaagagtac 600

cttggagtaa ataaataaag cccaagatcc tgattgtcta 640

<210> 140
<211> 40
<212> DNA
<213> Mus musculus

<400> 140
gccccacacg cccgaagcaa taaagagtcc actgacttcc 40

<210> 141
<211> 124
<212> DNA
<213> Mus musculus

<400> 141
aaaaggctcc tgccagtgtg aagacagacg gactgctgtg acacacctcc ccacacacta 60

tttgcagatg accagtgtcc tatgctgttc ttacaaataa actcaggcaa gatctgttag 120

cttg 124

<210> 142
<211> 575
<212> DNA
<213> Mus musculus

<400> 142
cctgctcgtg tcaaataaag ttgcagaact gccttcaggg tttggttttc ctttctgttg 60

tctgcctcat gggtggaatt tttgggtcta cagggtgttg gaaattaatc tgagaatctc 120

tgttctgggt acatgggaaa ttagaaatac gtgaaacatt cttttcacag aagtcacttt 180

attaggattg tggatttggg ttggttttga aacagggttt cttgtggcac tgcttgttct 240

atagaatagg gtggccttga actcagaaat ccacctgcct tttcctccct agtattggca 300

attaaatgcc cagcttgttt gtaagctctc attttcagtt ccaggtttat gtgtgagcct 360

aagattaggt aaagattgag gttataactt aaacgtactg aattaactta tgttgtgtgg 420

gtcccaggaa ttggacctgg gacatcaact ctgcctttcc agccatcttt gccaaccagt 480

agctcatctc tgggatgtgt ctgccctcaa aatgacattt taaaaaagtc agtacaaaag 540

aacgattttt attaaaaacc ttgaggacaa acatt 575

<210> 143
<211> 54
<212> DNA
<213> Mus musculus

<400> 143
atggcttgtg tgcatgtttt atgtttaaat aaaatcacaa aacctgccgt cgta 54

<210> 144
<211> 68
<212> DNA
<213> Mus musculus

<400> 144
actaatggag agtaaataaa taaaagtaga tttgtgctct tgtatttttt tttcacatct 60

gtcctaaa 68

<210> 145
<211> 504
<212> DNA
<213> Mus musculus

<400> 145
ggatttcaat cagtcataaa ataaatgttc tgctttcaaa aattctgtgg tgatctaagg 60

tactttaaca tcggttcaga gttcggttat atgattgctc tgggatccta cgcttcttcc 120

ttcatagttc ctgtgggtcc gaagctggga ggggctgggt ggactctcgg gaaagatact 180

ctgagcctgt ctcggtcccc atcgtgtttg cttggccctg ggcatggaag tgggtgagtg 240

atgagctgaa cgagcaggct tgctagagat gaggacagtt actggtgtgg ttatatcact 300

accatgccta cagtgtctta agacgcttac agtctgtaag ggacttaaat gatttgagct 360

cttacttatc ctgtagtttc tgatttttaa catttacttg aataaagcca agcaagataa 420

gcctttattc ccagcacttg gtgacaggtg gatctatgag ttggggatca gagctacaca 480

ttaaaactct taattcatct tact 504

<210> 146
<211> 306
<212> DNA

<213> Mus musculus

<400> 146
ggatttcaat cagtcataaa ataaatgttc tgctttcaaa aaaaaaaaaa attaatcctc 60

tgtgatggcc agcagttaac attcaacagt ttctctctag gctcttgatt ctctgactat 120

tgtagggatt cgatcagcac tcgcatacca gaagtgtgag atggtccgtc ctttttcaag 180

acaagatttc tctgtgtagc cctggctgtc ctggaaccca ctctgtagac caggctggcc 240

ttgaatttac agagatcccc ttgcctccgc ttgctgagtg ctaggattaa aggcatgcgc 300

actatg 306

<210> 147
<211> 246
<212> DNA
<213> Mus musculus

<400> 147
aagccctgct gtctgagact tgcctagcct gcaataaacg ggttatttac gtaacttttt 60

tttttttgcc ttgtttgtgg ttaattaaaa catttggtgt gtgttctatt ttttattttc 120

gaaagatgct tgttttgaga catactgtgt gaccctggct ggccttgagt gcctggttct 180

ccttacaagt gtagatacat ctggcttaag attttagtct ttcagaaata aaaatgttgc 240

taagac 246

<210> 148
<211> 41
<212> DNA
<213> Mus musculus

<400> 148
accagccctc tgcgtgtgac tattaaaaac cctgaaaagt g 41

<210> 149
<211> 181
<212> DNA
<213> Mus musculus

<400> 149
ggattcacac aatggcaaga ctgaggattt atactgaatt gtcatcaatc agtcctacca 60

gatggatttc aacatttaaa cctggagact cttcgtgtct tgaattagga tgtttgtcca 120

gtaataaaat atagaacctt tcaaaatgct tttctggttt ataaagtact gaattgccct 180

t 181

<210> 150
<211> 285
<212> DNA
<213> Mus musculus

<400> 150

tcccgccaaa gcaaccaagt cagcctgctt aatttgagaa agatggaaat aaaggcttac 60

ttctcttaaa actccggtct ggatttattt agtttgttca cttaagcagg atgaaaaagc 120

aaaaccgcta ctgtttactt tgtgttggca tctttgtttc taaaattaaa gctcctagtg 180

tttttgtggg ctttgtttgt tttttgagac agtctcttga cttggtgcca tagctagtct 240

gggacaaaga ttttccaggt gtgaattaaa ggtgtatgtc atcaa 285

<210> 151
<211> 40
<212> DNA
<213> Mus musculus

<400> 151
actgcttttg ttaagttggc taataaagag ctgaacctgt 40

<210> 152
<211> 42
<212> DNA
<213> Mus musculus

<400> 152
attcctgctc ccctgcaaat aaagtctttt tatgtatctt ga 42

<210> 153
<211> 1059
<212> DNA
<213> Mus musculus

<400> 153
cagggtcttg gcagctgcat ctggaggcat ttaataaaat aaagacattt aataaaatct 60

tgaacaaaga caaggcctga ctggattgtg tccagtattc aactgagtta tgttgtctat 120

ggagccatgc ttattctgtt ggtttaagct ggagggcatg agcagagctg accagagaag 180

tcatgaagtt ggtgaccctg tgttgaacaa ttgagggtta gaagagcagt ttggttttgg 240

tgctcttgat ggaacccagg tgcttggaca tagtaagcac acataagaca gagtaagact 300

gctgtgtctc tggcctggag tagtctttct tgcttttttt tttttttttt tttttctcta 360

gaatgaaagc agatggccca gcgagttagg tgcttcctat gaaagcatgt gtgctggttt 420

gtcatgcaca cagccctgca ggagagagta tggcaacaca gccgctcagc atcccaagat 480

aaaaagggag tttctactgc cattttgagc ttgggagttt gaaatgtaaa gcctgtccat 540

atgttttaag gatccatgta tttctgtttt gtttgttttt caaaacaggg tttctctgta 600

gccctggctg tcctggaacc cactctgtat gtagaccagg ctggccttga actcagaaat 660

ccacctgcct ctggcgatcc atatatttct aagtcctgta cttagacgct gttttggaaa 720

attcattttg gaagcattta ctgttggtgt gttttgtggg gaatgaatga tagcttggga 780

attcttttct gtttggtgag agtgaagctg tcagcccggt tgtagcctgg ctggtgctca 840

aaggctttct ctcattgtct tcacctacgt agctttacgt ggggtaagga cttaagttac 900

ttaagttggg tgcacactga ccatgtccac aacctgttaa ccaactctac atgatgagta 960

cagatgtacc tttttagaaa gtgttaatgt gtagccctgg ctggcctctg cctcagggta 1020

ttatgaataa agtgtgcaac cttcatctgg ttgattaaa 1059

<210> 154
<211> 49
<212> DNA
<213> Mus musculus

<400> 154
aatccagatt tttaatagtg acaaataaaa agtcctattt gtgatcgtt 49

<210> 155
<211> 87
<212> DNA
<213> Mus musculus

<400> 155
aatggtctct aggagacatg ctggaaaagt gtttgtacaa gcctttctag gcaacataca 60

tgctagatta aacagcatgg tgaaact 87

<210> 156
<211> 100
<212> DNA
<213> Mus musculus

<400> 156
agtggactct gagggacatt gcggggaagg ggcgtttacg tttgtttata cttaaaagtt 60

ttttaagcag catgttgaat taaaaaagaa agcaagcttc 100

<210> 157
<211> 45
<212> DNA
<213> Mus musculus

<400> 157
tttcccagct gctgcctaat aaactgtgtc ctttggaaca actat 45

<210> 158
<211> 603
<212> DNA
<213> Mus musculus

<400> 158
gtctttaaga gcaacaaata aataatgacc ttgaatcttt cattggcttt cattaatagt 60

gtaactagat aaatgatggg aaagatgaga cagaagaagg aatacatcta taggactgct 120

agaatatggg gagagtgatt attttcaaat taatatgtat cgagcttcta ccccaaggta 180

aataaataac atttggagac cattaaaatg taggatggca tagaagaggc ctttactaag 240

attaataatt aaagaaacac agcctttaaa gtaaaaaaca cactgtgcct ttgaaacttg 300

ctaaaaagat taacttctgt cccaaaaggt atcagccatg cgctaccagc ctccctgccc 360

ctacagtggc agtggctgca ttcttggtga atggtagtgg aagggattaa acctaggcct 420

cagtcatgct tcccagtcac tggtactgat ttgtatgcac ccgcttaggt gtgaaggtag 480

ttttggtgtg tatcacaagt tagcctgtgt agcgaagaca ggttttctcc accgtgtttt 540

ttgttacaca tgactattca caaatgtgct gcagacagta aaatgagaaa tacccttcca 600

agg 603

<210> 159
<211> 269
<212> DNA
<213> Mus musculus

<400> 159
agaaaatgac tgaataaagt gtcattcata gtatttggtt gtagtaactt gtcaaaatct 60

cagggccatg ggtgcacgac agcagtagct tcttgaatga actgaagttt tcaagaggtg 120

cctggaaggt gaaaaacaca ctgaagccag tcatgttgat atgggggcat tctgctgctg 180

tgaaacagac tggggttcac acccaccttg cgggattaga acttcactgc cctccaactt 240

ctttctttgt aaacaactgt ccacatttt 269

<210> 160
<211> 42
<212> DNA
<213> Mus musculus

<400> 160
gcctcatgtg tagtgtaata aaggtgtctg ctgttctatc tg 42

<210> 161
<211> 69
<212> DNA
<213> Mus musculus

<400> 161
ttaatacttg gctgaactgg aggattgtct agttttccag ctgaaaaata aaaaagaatt 60

gatacttgg 69

<210> 162
<211> 36
<212> DNA
<213> Mus musculus

<400> 162
agttggagtt tatgttgtat tgaataaact ttaaag 36

<210> 163
<211> 47

<212> DNA
<213> Mus musculus

<400> 163
ggggactctg gccaatgccc tagaacaaat aaagttattt tccaacg 47

<210> 164
<211> 50
<212> DNA
<213> Mus musculus

<400> 164
ataaattttg gctgattttt ctcttgtatt tcttgtttgc tggtataaaa 50

<210> 165
<211> 43
<212> DNA
<213> Mus musculus

<400> 165
atgctgttgt gtgcacaagc aataaaatca ctttgagtaa ctt 43

<210> 166
<211> 34
<212> DNA
<213> Mus musculus

<400> 166
ccgaggccaa taaagactgg ttttggtccc tgga 34

<210> 167
<211> 3272
<212> DNA
<213> Mus musculus

<400> 167
acgtaaagca taaataaaaa gcctttgtgg actgtgctca gggtcagtcc ttttgaatct 60

ctgcagcaga gtagctggct gtgctgactg gtgacacttc tggtgatgct cagctgtgag 120

gttttatgta gatattgaaa gcatgaccat tgtcttcact tcacctccag cttgggttgt 180

atgccagtaa catcagcata aggtggttaa tgacaggatg gtcccttgag tgtgcagtga 240

gtctggttta tttgccaatg agaagcacag gcctcctgta tgggtctttg cctacagccc 300

cctttcatca cccagacttg gtagacttac attctgtcac actgttggct cttaatctca 360

gccctgaaaa atgccatttc ttgggtatca aggctagtct agattcagaa accatataaa 420

ggttgacagc tggtttaaaa aaaaaaaggc ttggagcttg agttgggttc gcaggttatt 480

ccagggtatc tgtctgcact ttgtctccca gatttaaagg taagtgccac catgcctagc 540

atggtgactt attagctttg ttgctgtgga acatacatca aatgaaacat tggtatggct 600

ctggtttcac tgtccatggt tagtatctgg tgggtggaca cctggtggaa ccaagctgct 660

catcccagaa ctaaggagcc attccccaga gacttgtctt cctactaagt tccatcccta 720

cagcttctag tagtagcttc agaggttgtg aattgtggac ttagtctgcc ataacattta 780

aaataggtat taaattcaag tcatttggtc actcagcacc cacgtggctc ttcagaacaa 840

cagaagcccc tcggacttgt ctgttggaaa aaccagtttg aaataatgta cctgctttag 900

ttgagaaaac gctacaactg gtgctgtgtc ctgccatgct gatgagctct gctctgcgac 960

ctgccgaact tgggggatct ctacccccag actttgctca gatctgttga tgatttgtcc 1020

atgcaggaaa gtttacaagg tctctgtgtg tctactactt actagttgct gtgacaaaaa 1080

tactgaaagt gtttactgtg tgtgaggcac aaagtttgtg ggaagctgta ccctccctca 1140

ttttggtgct gctcctgcct tgactgacag gatgagctgc cccaaccatc gttgccatct 1200

tccataagaa gcaggtggct ctattgagtt ccctggaggt gatcccaagg gaaggaggag 1260

cctgggaaag tggatctcaa gtcccctagt ctggcagttg gctgtttagg aaagtccagt 1320

gtcagtgttt gatatgttgt aaggaaacaa attcagtttt atttagctta ttggctctgg 1380

ggaaatggca gttcccatta attggtgctg tctttctctt tgaggatcaa aattagcttc 1440

ctgttcagtt gttaagcata tttcatagtc aaataatcct cttatcttta caagtgaggt 1500

tttccttcga gtggatatca gagtccctcc cacggcttct atccctccta tccttgtgta 1560

ggaagttaag cttgctcatt tgtagattag tggtcggtta cactgcaatt tagagtatca 1620

tgtgtactct acacatgatt gattctaagc cccccttccct ttccatgtcc ttcaaaaatt 1680

tttttattct gagacagtgt taggattttt cctgggctag ccaaatgcaa gaagtgttgg 1740

tcccagactt gtaatctttc tgccttagcc tcccaaattc taggattata gatttatgtc 1800

atgtgatgag tggtctttta aagattatct tttattttt ttgagatggg gtttttctgt 1860

gtagttttgg ctttcttggg acttgctctg tagacccagg atggtcttta ctcagatctg 1920

cttgcctctg cctcccaact gctaggatta aagcatgagc ctgagccttc atgcctggct 1980

gacaggtgaa ttctcaatac ctacctagcc atagggaaag tgattgtgtc cccttcctca 2040

tagaggggca tagtcaccca ggccatacct ttagcttggg cttttggtca gtgaagaagt 2100

atgaagggac aagaacacta tcaagagcct aatgtgctct ggcctggatg gtcagcacaa 2160

aatgaataga cttaccaaat tctgctgtct ccttggtaga tgtgaagttg ttggaagagt 2220

cctaaattta gcagactata ctgtcagcct atcagactat aggctgccgg agggcaagtc 2280

tgctacctat ttccatctca tgcctgcatt gttcatcccc ctatgtaacc cacctacctg 2340

tcactcattc ctccatccaa aaactattgt aggttcagtg gaaatttcaa gcttgcctgt 2400

ctcagcatct ttcttacctt acccctaagg atggcatctc tcttggctac atctttggtt 2460

tatctggaga tccttgatta atttgaacaa gagctacctt gggttatgca gtttatgcct 2520

ccagtgtccc agagaccggc atttgagaga tccctgatag caaacccata gggtggcctt 2580

tttttcatcc accccattct ccctcccacc tccctctttt gaccttgagt cctcaccaga 2640

gagaaaccag gcccacttaa ttagttctac atgtgtacac tacatgggtg cagtgcccaa 2700

gcaggagagg tgttagattc ccttttaact atagttaata gacagttttt aagccccagt 2760

ggatgctggg aagcaaacct acatcctcta gaagagcagc tatttctctg agccatttct 2820

ccagcaccat tttcccctct tttaaaagca ggtcttgcag tgtggcctag tctggccccc 2880

tgaggtgttt gcattgcatg gcaggcatgt ccacaggaac accatagttc tcaccactcg 2940

tacagcacag caagtggggt gccgcagggg attatcactt gagtataaaa taagggttgc 3000

tttagattga ataggataac cacgcgttct cagaacaatc aaggaaggct ggggtgagcc 3060

agcaccgacc ttaattgttt acttagtaaa ctactaaatg tatgcacgtg taagcttttg 3120

ccttgattga ggtcaagctg tcgagaaatg gttctcttta cagtggatcc agtcaggatt 3180

ggcagcaagc acctttgcct gctgagccta catgttgtat agaatggcaa cgttgtgtag 3240

aatggcagta cattaaatgg gtttttcatt ta 3272

<210> 168
<211> 59
<212> DNA
<213> Mus musculus

<400> 168
gcccatctca aggatcgggg tttacctttg taataaacat cctaggattt taacgttcc 59

<210> 169
<211> 35
<212> DNA
<213> Mus musculus

<400> 169
aacaaaggat gctgggttaa taaattgcct cattc 35

<210> 170
<211> 3167
<212> DNA
<213> Mus musculus

<400> 170
gacaactgaa taaatcgtct taatggtcaa attttgctgg cttttgttca ggtttttttt 60

ttttaattca tgtttatgag tgtaaatgtg tgtgtcacag gggtgtcaga tgttctttga 120

accaccatgt aggtgctgga aacccaacct gcatcctcgg agagaacagg ttccttaacc 180

actgagcaag tactgaagca ttaaactgct tttaaaaatg aaggtgtgct aacagattgg 240

tcaggtgaaa aagagacgtt aggtttcctg caggggcgc taagccaatt taaagactaa 300

gttgggttag aaaagagcag attgcatcct tgatctttta agcctgggga ttttgttttg 360

ttttgggata gggtcccaac acagaacagg ctgacctcat taaatatcaa tcttatttga 420

```
ttgcctctgc tcccagagta ctggaattaa aggcagggac cactgtatta gccattctga      480
gttatttgaa atggactctg caggccatac ttggtcaaaa ttctgccttc tcaattacag      540
gcatgagcca ctatgcctgg tttacttact aatagatgtc caaagactag tgtatgaaaa      600
ttttgctttt ccaggtgatt tgtgaaaggc agggtggcct ctcccatgtc acactacttg      660
ggttactcat gttgcaacat atctgcaact ttaggttgag gggatttgag cctgcatgtg      720
ccactttggc caactgaact aatctttaat tccatctaaa acttttaaat ctcagtcatg      780
tgttcaactg gaaaataacc tagagtgtgc tatgttgact tcaggtacac atcaaagcag      840
gttttagtga tgtagaagct gtgtttgagt tgaactagtg ttgaggctag gcttaggtac      900
catagaactt tggtttttca agacagggtt tctctgtgta gccctggctg tcctggaact      960
cactgtagac caggctggcc ttgaactcag aaatctgcct gcctgtgcct cccaacacac     1020
ccagctctag ctttaaattc cttgcaccaa gagatgcttt atccctccgc tgagaataca     1080
ggtgcatgtc agcatgctaa actctagata aaatttcatc ttgtttgaaa ggacaataat     1140
ataagaaaag tgtatttgca ctgtatacca tgcccttttg tgtttaaagt taaactggca     1200
acagtgtccc atagaggttc cagaagaaac tgcttctaag gggaggacca taaagggaaa     1260
tgcttaccat agaacttttt aaatgttcct acaggttgta ctctggatag gtatatgaac     1320
acctttctat tagaacagtt ttatagtagt acttagtgaa tatgtaaata atactatttg     1380
tgaaatagtt tgaggtttct ctatagtcct ggctggcttg agctctatac cagattggct     1440
tctaaatcag aaatctgcct ctatctcctg aatggttatg atagagatgc aagttactta     1500
atttcttaca tgaattgcac tttgtacatg cttttggata tgggcctagg cttttgcttt     1560
tggattagac tgtctttatt acatttactg gcttgatact ttacagtctt aagcccactt     1620
gcctgggtgt gatgcacacc tttaatccca gcactttggg atttctgagt tccaggacag     1680
ccagggctac gcagagaaac cctgtcttgg aggggaaaaa aagaaacttt ggaaatcaaa     1740
acttcttgga aagccacttt tagagacttg aatctaagga taactaacca ggtagtaacc     1800
acgagtcatt gattctgtga atcttgtatg agtgggttac aggcagaaat taacttcctc     1860
tgagagcact gctgttttag aaatgcgacc tagtaattac caaaggcatt gaagccactt     1920
gactacagtc tcaggtttct gcatctgaca ttgctgggac tgtgtggggt ttatgggtgt     1980
aaaataaaac aggcgaaagg atgttgagtg gaaagctttg gcttcagaat caaattccaa     2040
atcaattacc agatcaaatc aaatgccaga tcaaattaca agacttcctt atgactaaat     2100
tcttcagtga cttaggatac taatagtatc tgcctcaaaa gtgtatgtgg ttatttttgt     2160
cccagtgaag ccaagattca catgctatgg gcatggattt cctgaggaca tgctagccta     2220
tggtatgagt tacttgaaag gactctgaga aatcttagtc cccagctgtg aggtttttaa     2280
agatcatgct caatggaaag tggggcagta atttgagagc atggcacaaa tgaggtttta     2340
```

tcttttgaaa ctaagtgaat ctatgtcctt ggactagcat attttaaatc acacatcaaa 2400

tgaaatttct gttcaattcc tataaacagt ttatttcata ttttgtagtt accattttca 2460

ttaacagcat acacccttca attgtgttgc taaaactgag tcacattatt ctgtaagaac 2520

ttactccagt atcacaactt ggtgctcatc caatattttt attttcattc tattttccct 2580

gtctagccac gtatggccct attctctctt cttggattga ccctagcttc aacacaagaa 2640

agcttgcagt attttttttt tgcgcctggt tcattgtttt gtcctcaagt tctatccatg 2700

tgtccagaaa ttcgaggatt ttttaaaatt tacttttctg cctaaatact ggtatgtgga 2760

tagtctgtta tttttactgt tggttgcata tctgtagcat atttctgtat ttgcaatgac 2820

tacattgaat gtcccttagt taacctcatt cttcttaact attttgtagg cgtttgctat 2880

tcaaagcaca atctcaatta aaatgttttt aatagcatct ttccacttgg atgtgtaaag 2940

aaagtatttc tagaagtctg aatttttgtt gcattgtaga tgtgtacaca atagggctgg 3000

gaaagtagct cacagtgggt aaaagccatt tgttgccaat cctaacagcc tgagttcaat 3060

cccagaatct ataaggtaga agaaaaaaac cccagctccc acaagttatc tggccctctg 3120

cacacatata aatagtgcaa taaaaattaa ccatttaaaa atataaa 3167

<210> 171
<211> 58
<212> DNA
<213> Mus musculus

<400> 171
gcagaagaaa tcgggaattt gttacaaata aaagttttaa gtacctgtga cagttaag 58

<210> 172
<211> 49
<212> DNA
<213> Mus musculus

<400> 172
aatttgacaa tggaaacaca gtaataaatt ttcatattct gaaaaaata 49

<210> 173
<211> 264
<212> DNA
<213> Mus musculus

<400> 173
acaggttcaa tcagctgtac atttggaaaa ataaaacttt attgaatcaa atgaatgggt 60

gcatctgttt cctaaggcag ccggggagga tttggtctta ggaataatag ctggaattgg 120

tttgttggcc atgaagtcag atgcaattgc gcctgggaac cttcagcttt tccctttacg 180

tggttgcttg cttcttgttg cagcttcggt tttgaattga tgcctgaaag aaaataaaaa 240

cttagcaaga ctaatggtaa atct 264

<210> 174
<211> 71
<212> DNA
<213> Mus musculus

<400> 174
agaggccgtt ttgtaaggac ggaaggaaaa ttaccctgga aaaataaaat ggaagttgta 60

ctttacatgg c 71

<210> 175
<211> 56
<212> DNA
<213> Mus musculus

<400> 175
aagcccctga ttgaagatga gtgggaacct tcccaataaa cacgttttgg atatat 56

<210> 176
<211> 38
<212> DNA
<213> Mus musculus

<400> 176
ttgtgtatgc gttaataaaa agaaggaact cgtactta 38

<210> 177
<211> 92
<212> DNA
<213> Mus musculus

<400> 177
tcttgcagct gggttctgga tatccactac ttagcccacg gaatgatctg caactgttaa 60

ataaagcatt tatattaatt cttgtctaga aa 92

<210> 178
<211> 91
<212> DNA
<213> Mus musculus

<400> 178
gcgaccttga atggattcga ctgactacta ccaagtggaa ccgatcatgc tagtctttgt 60

acacaaagaa taaaaatgtg aagaacttta a 91

<210> 179
<211> 1187
<212> DNA
<213> Mus musculus

<400> 179
tacacgtgat atttgtaaaa ttcatatcca ataaacaatt taggacagtc atttctgctt 60

aaaggtgtta tttgttaaaa ctagtctaca gattgtcatg agtgttctgt gaaaatgtag 120

aagttaagtg caataattga aaactgcagg tgatggcata tcttgtttct gatgtacttt 180

gcattatctg cttatgagat taagtgtata tagtgtttgt gccaagtggt gttctgtgtg 240

taagaccctg taagaggtaa aaagtcctga aactgaccct ggatgtgttg gtgcatgaga 300

tagaatctac agctttacga tggcatcttt tggttcactg aagtggctgc ttgggagttt 360

gatgagtaca aactttatag agttggattt tgcttagaaa tgtgtaggaa gagagggttt 420

caaaacctgt tttgtgcata gaaaagtgag atcaactata acccacattt tgagaattga 480

atccagtgtt attttcagaa gaccaggtaa atttgttgga agaggtttca ctttctgttg 540

gatctagagt atggatttgg agatgaaggt tgaacattgg atgtagtagg gcttatttag 600

ggcagattat ttccattaga tttgtaaact tggttgtggt tgcaagttaa tatcaaccaa 660

gcaaatataa gtttgattaa gcttgcagac attaagcttt tctagcagct ggtgtgtgca 720

gaggcagatg gctctctggt tccaggacta cctacaatat agagaaattt tctctattcc 780

agtcaattca tcatgggtaa gaatagtcca ttttggtagt gttatttcat cgtttacaat 840

ctacctatgg gtagtggctg gtaactgcct ggatgttgac atttcacaaa ggccatactt 900

aaccacactt ttatttctat ttgtgcgatg ccttggagta gtttcccaaa gtgattttga 960

gtgtggaaga aatggtattg tccccgaaca gctggcttgg tctcaaaatc taattgatgc 1020

ttttattaaa ttggttttcc tttggagatt ttaaaaggat gatttgattt ccagaaaata 1080

ctagactcaa aatcttgata gctaaaattc ttttctattc agcaaaacaa gtcactgtat 1140

agaggttgtt caaatcaact aaagtaataa atgtcttaaa caagtgg 1187

<210> 180
<211> 43
<212> DNA
<213> Mus musculus

<400> 180
gggtttttat atgagaaaaa taaaagaatt aagtctgctg att 43

<210> 181
<211> 259
<212> DNA
<213> Mus musculus

<400> 181
ggctacacag aataataaag gttctttttg acggtggtaa atctcatgtg tggactctaa 60

gcttgtcgcc aagtgggaaa tagactggtg ggattgtaga taggatgggc tacttaaact 120

cattctaccc aggccttagt acttagcata cagccagagt caaactgatc ctttatacag 180

ggggtaccat gacagtacaa cagtgtcgtt aaccctaaca aataaatttc ccaccaacgg 240

gtggaattcc ttcattttg 259

<210> 182
<211> 48
<212> DNA
<213> Mus musculus

<400> 182
acaggacttc tcattatttt ctgttaataa attgctttgt gtaagcta 48

<210> 183
<211> 67
<212> DNA
<213> Mus musculus

<400> 183
aggcttcaat agttctccta taccctacca aatcgttcaa taataaaatc tcgcatcaag 60

ttcgctt 67

<210> 184
<211> 33
<212> DNA
<213> Mus musculus

<400> 184
gatgctccaa taaacctcac tgctgccact cag 33

<210> 185
<211> 36
<212> DNA
<213> Mus musculus

<400> 185
gatgacaacg acaataaagt gcgagactga ctggct 36

<210> 186
<211> 388
<212> DNA
<213> Mus musculus

<400> 186
acccaataaa gactgtttgc ctcatgcctg cctggcctgc ccttcctccg ccgccaacta 60

gggaagtggg gaccaaaggt tccttaggca ctgctcctgt gggtagaggg gacattagag 120

agctgacagc gcaccacctg catgagtttt tattaaagtg caaaccatgg gatgaatcag 180

ttgagcttca gtgttgaaaa tgagtagcag ggctgcccca cccacctgac caagtaccct 240

attctgcagc tatgaaaatg agatctgcac atgagctggg gttcacaagt gcacacttgg 300

agcactgcct tgctccttcc cagcagacca caaagcagta tttttctgga ggattttatg 360

tgctaataaa ttatttgact taagtgtg 388

<210> 187
<211> 32
<212> DNA
<213> Mus musculus

<400> 187
tgaaccctcc cccaataaaa gatggttcct ac 32

<210> 188
<211> 103
<212> DNA
<213> Mus musculus

<400> 188
gcagattttg ttatgaagac aataaaatct tgacctttca accccttga ttgcagttgt 60

tcgtttggga gggaatacat taaaagcttt cagaaattac ctg 103

<210> 189
<211> 277
<212> DNA
<213> Mus musculus

<400> 189
gccagcccgt gcacctacga cgcctgcagg agcagaagtg agggatgctg agggccggga 60

caagctatcg gactgtgtgc tgccatcggt aatgagtctc agtagacctg gaacgtcacc 120

tcgccgcgat cgcctggaga aatgaccgcc tttcttacaa ccaaaacagt ccctctgccc 180

tggacccccg gcactctgga ctagctctgt tctcttgtgg ccaagtgtag ctcgtgtaca 240

ataaaccctc ttgcagtcag ctgaagaatc aaactgc 277

<210> 190
<211> 44
<212> DNA
<213> Mus musculus

<400> 190
gtctctgggc ctttgctgtt aataaatagt ttatatacct atga 44

<210> 191
<211> 404
<212> DNA
<213> Mus musculus

<400> 191
aatacctagc agtgagtgga gattggatac agccaaagga gtagatctgc ggtgacttga 60

tgttttgctg tgatgtgcag atttctgaga ggacaaataa actaaaaagc tcctacacgt 120

ctgctctgct gcttattggg cattagaaga atcaggtggc tgcttgggtg ttgatgcagt 180

caagtgcact gggcttggtg aaaagcccag tgtaagaggc cggtacagat ccttcctggc 240

agagggtggt gatggagaga acataaataa ctacatgggc aaagtgtagg accaattacc 300

ctgttagcat cgtctttgct caacaccttt ctgtgtccct agactctgag tttttttcta 360

attgattttt attgaacact gagtgttttg aggttttatt tttt 404

<210> 192
<211> 39
<212> DNA
<213> Mus musculus

<400> 192
agttcaggag ctaataaagt acgtcctttg gctaatccg 39

<210> 193
<211> 48
<212> DNA
<213> Mus musculus

<400> 193
atatgcacat tttttaagta ataaaaatca agacttgatc tacgcttc 48

<210> 194
<211> 36
<212> DNA
<213> Mus musculus

<400> 194
tctgttatgc catattttca ataaacctga aaacaa 36

<210> 195
<211> 875
<212> DNA
<213> Mus musculus

<400> 195
gctgctgtgc aggtgcctga gcaaagggaa aaaagatgga aggaaaataa agttgctaaa 60

agctgtctta tggtcctcac tgcagactgt acctggattg gcatttggct atacaacaga 120

ggcatggtcc tactgacatg ttttgtgttg aatacttaag catgtgaaca catgggtttt 180

tttttttttt aatgtaaaat gtagtaacac aatgtttagg tggctttggt gttagctctg 240

gagacttcat gtgtcatcta ggtgaggtgt tctttaacac agggtctctg ttctgtcatg 300

cctcatagat ccttctacct ccagattgga gagggaaaag gcttatgtca ctgaacctgg 360

ccagattggg attttgtgtc ccaggaacaa agttaatgct aaaaagttaa tgccttggtg 420

agactgatag tctgatggtg tgaattcaca gtaagtggtt gggattgcca gatggaattc 480

cctgagctgc cgtgacaggt ggcattgcag aagtgaagga ttcaggaatt tgagtgttgg 540

gtgggggcct gtgaatagca cttgggctgg gaggggagac tgctgcccct gaatgtcctg 600

gaattcaagg acagtacctg gttaaatgtt tttctagctt ttctaaaaag tttgttaggc 660

ctggcattgg cggcgcacac ctttaatccc agcacttggg agtcaggcag gtggatttct 720

ggcctggtct acagagagtt ccaggatagc cagggataca cagaaatcct gcctcaggaa 780

aaaaccaaaa agaagtttgc taaaaataag catttttgct tgatggtatt gaagattgta 840

agacattaaa ttgtgtcatt acttctccag gtact 875

<210> 196
<211> 67
<212> DNA
<213> Mus musculus

<400> 196
agctgtggtc atacctggca tgtgaccccg ggaccaaata aagtcccctt ccatccactg 60

gagcagc 67

<210> 197
<211> 50
<212> DNA
<213> Mus musculus

<400> 197
gtcctattgc caccctgcca tgctaataaa gccactgtgt ccagacttct 50

<210> 198
<211> 84
<212> DNA
<213> Mus musculus

<400> 198
ttggcctctg cttgtaatac aatgaaagta ttctaaagaa atataaaatt ggactttatg 60

agaaaataaa agtcatttca ctct 84

<210> 199
<211> 36
<212> DNA
<213> Mus musculus

<400> 199
tctgttatgc catattttca ataaacctga aaacaa 36

<210> 200
<211> 446
<212> DNA
<213> Mus musculus

<400> 200
ggaaccacac gtacttatgc tgtaacttac tgtagcgttt acagcgttac cgctgtctgg 60

acagctgagt gtgtttctta ggaatataag ttttctttct gtgctttagt gagttcattc 120

agcagttcag taataaatat gtgaaacctt ttgtttcgaa aaaaattgct tctgtgttac 180

aacttacttt gttttatggt tcaggatcat ctgcataata gacaagtatt ctatcagtga 240

gcgatatcct ggatcttgtt tgtgtagttt ggggttgaga caagtccaga ctgccctaaa 300

actcacgatc ttccttcctc ggccttctaa atgattggag gactccaaac ctaagtgatg 360

gaagtaaaaa gaaacttata tgtcaagact cattttctct atcatttcat gtgacaattg 420

aaattagatt atttcttttt tcaatc 446

<210> 201
<211> 332
<212> DNA
<213> Mus musculus

<400> 201
ggactttgct ggcagaaaag aagtacagat gaggttatag tttgaaaaac gttaattccg 60

tttattgact ttagaatgtt actttgcata gtatagacca ttacaatgga aagtacctgc 120

cttaagaaac aagaaaactg cagtttatag agaaagaatt ttcaattttg acccatgtac 180

ttaaaatttt tggtgtatac tgcagtgtag caaatgtttt gtggtgacgg tataaatggt 240

actgtttgtt atcttggatt aagagtggct agagaagttg gaagacgtgt gagaagttct 300

ttatagagaa ttaaacatga aaattacatc tc 332

<210> 202
<211> 80
<212> DNA
<213> Mus musculus

<400> 202
aacttgtgtt ctctgagagg aaaatactga agcagtagag aaatgacctg ctagagaata 60

aagttactgt taatgatacc 80

<210> 203
<211> 93
<212> DNA
<213> Mus musculus

<400> 203
gatgtctgga atggagcaca cctgggatgt agcactcttg ctatctgtcc ggtccttttt 60

gttcaataaa gtcctgaggc aactctctct gtc 93

<210> 204
<211> 152
<212> DNA
<213> Mus musculus

<400> 204
tgagctatga agttccggaa tttgtgtttt tcacagaaag ccttaccaac ttcagttact 60

ttaccaagac aatgtaatta ttgtttgatt ttataaagtc tacaacaatg atctcctatt 120

ttggtgtcag tttttcaata aagttttaat ta 152

<210> 205
<211> 1841
<212> DNA
<213> Mus musculus

<400> 205
agcaggagca ggttttccaa aagcacccct cggaagtgtt tttgtcgtcg tttaaaatta 60

tcaagtatct tcagagaaga ttattttctg ccttcagaaa ctgaaggaag gcttgggcct 120

agagaacgac agtaaggtgc gagcaccgga gacacttaac acagctcagt ccatggaagg 180

acgagttccc tcattggctg cctgtctcga aatccacgca agctgtggag gaaagaatta 240

ccctgctcat cctgccttct atcttggtgt ttaatgttgg gtgggcaaca agcacaaacc 300

tccctcccac cccctccaag actgttagag cagtgggcca gaccaagcgg cgcacttgaa 360

catggatcaa gagggtcccg gttttacttt ttatttttgt cagggtaggc agtcttgtgt 420

ttgctttgtt caaagcaggg tctccctgtt ggccctggct ggccttatac tccacagcag 480

tcctgcctcc tcctcctagg tgctgggatt aaaggcgtgc gccaccacgc ccggctacag 540

cctgcatttt tatgcacatt ggtctgttaa gctagttgca ttctgtgcta ccggagggga 600

ctgaagttta atcacttgtc ttctattaaa aactagtgtt tgcctgggcc tggtgtgtat 660

acctttagtt gcagcgcttg ggaggcagag gcaggcagac ttcatgagtt cagggacagg 720

caagcctgct ctacagattt ccaggatacc cagggctaca catagagaaa atgttaaaga 780

taaacaaaaa gctggacagt gggggagcac acctttaatc ccagcactcg ggaggcagag 840

gcaggcggat ttctgagttc gaggccagcc tggtctacag agtgagttcc aggacagcca 900

ggactacaca gagaaaccct gtctcagaaa aaaaacaaaa caaaaaccaa tgcagtgata 960

gattgttgtt tcctaaacca catgtaccca ggaaatgccc actaaatttc acctggatca 1020

gtgttaactg atcattggga aatgaggtga ccaaaaatgc atcgcaacct tggacaaaca 1080

gcatggctat ttaacattct gggatcctgc agaatcctgc atcttcctaa gtagggaagc 1140

actgtagcat tggagagagg cctgggcgag cagagctaag gcttccattt ctggcttgct 1200

tggaatttaa aacaagcttt tttctatata gtaaaagatt gtttttaaga tttttgcgtg 1260

tgagtacatg ccaaagtagc caggaagtgt cacttgccct ggagctagaa ttactggcaa 1320

atgaaggctc agaggtggat gctgggacca attctaggcc ctctgagaaa gcaggtgcac 1380

ttggcttgtg cctccagccc caaaggcgat ggcttattgt gagcctgagg ccagccaggg 1440

ttacagagac tcaagaaaca agtggggttg tccatgttgc tggagatgac ccaggtctat 1500

taggaccttg actacatgga tagacattct ggcagctagt atactgccat tggggcctat 1560

ggagaagtag ccaccgagcc tatttagaaa gaaggactgc tggcaagctt ggtgtcacta 1620

tgaaggcaga caaagatcga tgtattaacg accccgactc caaaaacact cgaggggggcc 1680

caaggtgggc tcagtggtta agagccgttc gcccaggggc tggagagttg gctcagtggt 1740

accacatggt ggctcacaac catctgtaat gagatctgac gccctcttct ggtgtatctg 1800

aagacagcta cagtgtactt acataaaata aataaatctt t 1841

<210> 206
<211> 2035

<212> DNA
<213> Artificial Sequence

<220>
<223> sequence encoding the mRNA rpl32 - PpLuc(GC) - albumin - A64 - C30 - histoneSL

<400> 206
```
ggggcgctgc ctacggaggt ggcagccatc tccttctcgg catcaagctt gaggatggag      60

gacgccaaga acatcaagaa gggcccggcg cccttctacc cgctggagga cgggaccgcc     120

ggcgagcagc tccacaaggc catgaagcgg tacgccctgg tgccgggcac gatcgccttc     180

accgacgccc acatcgaggt cgacatcacc tacgcggagt acttcgagat gagcgtgcgc     240

ctggccgagg ccatgaagcg gtacggcctg aacaccaacc accggatcgt ggtgtgctcg     300

gagaacagcc tgcagttctt catgccggtg ctgggcgccc tcttcatcgg cgtggccgtc     360

gccccggcga acgacatcta caacgagcgg gagctgctga acagcatggg gatcagccag     420

ccgaccgtgg tgttcgtgag caagaagggc ctgcagaaga tcctgaacgt gcagaagaag     480

ctgcccatca tccagaagat catcatcatg gacagcaaga ccgactacca gggcttccag     540

tcgatgtaca cgttcgtgac cagccacctc ccgccgggct tcaacgagta cgacttcgtc     600

ccggagagct tcgaccggga caagaccatc gccctgatca tgaacagcag cggcagcacc     660

ggcctgccga agggggtggc cctgccgcac cggaccgcct gcgtgcgctt ctcgcacgcc     720

cgggacccca tcttcggcaa ccagatcatc ccggacaccg ccatcctgag cgtggtgccg     780

ttccaccacg gcttcggcat gttcacgacc ctgggctacc tcatctgcgg cttccgggtg     840

gtcctgatgt accggttcga ggaggagctg ttcctgcgga gcctgcagga ctacaagatc     900

cagagcgcgc tgctcgtgcc gaccctgttc agcttcttcg ccaagagcac cctgatcgac     960

aagtacgacc tgtcgaacct gcacgagatc gccagcgggg gcgccccgct gagcaaggag    1020

gtgggcgagg ccgtggccaa gcggttccac ctcccgggca tccgccaggg ctacggcctg    1080

accgagacca cgagcgcgat cctgatcacc cccgaggggg acgacaagcc gggcgccgtg    1140

ggcaaggtgg tcccgttctt cgaggccaag gtggtggacc tggacaccgg caagaccctg    1200

ggcgtgaacc agcggggcga gctgtgcgtg cgggggccga tgatcatgag cggctacgtg    1260

aacaacccgg aggccaccaa cgccctcatc gacaaggacg gctggctgca cagcggcgac    1320

atcgcctact gggacgagga cgagcacttc ttcatcgtcg accggctgaa gtcgctgatc    1380

aagtacaagg gctaccaggt ggcgccggcc gagctggaga gcatcctgct ccagcacccc    1440

aacatcttcg acgccggcgt ggccgggctg ccggacgacg acgccggcga gctgccggcc    1500

gcggtggtgg tgctggagca cggcaagacc atgacggaga aggagatcgt cgactacgtg    1560

gccagccagg tgaccaccgc caagaagctg cggggcggcg tggtgttcgt ggacgaggtc    1620

ccgaagggcc tgaccgggaa gctcgacgcc cggaagatcc gcgagatcct gatcaaggcc    1680
```

```
aagaagggcg gcaagatcgc cgtgtaagac tagtgcatca catttaaaag catctcagcc     1740
taccatgaga ataagagaaa gaaaatgaag atcaatagct tattcatctc tttttctttt     1800
tcgttggtgt aaagccaaca ccctgtctaa aaaacataaa tttctttaat cattttgcct     1860
cttttctctg tgcttcaatt aataaaaaat ggaaagaacc tagatctaaa aaaaaaaaaa     1920
aaaaaaaaaa aaaaaaaaaa aaaaaaaaaa aaaaaaaaaa aaaaaaaaaa atgcatcccc     1980
cccccccccc cccccccccc cccccccaaa ggctcttttc agagccacca gaatt          2035
```

## Description

The invention relates to artificial nucleic acid molecules comprising an open reading frame, a 3'-untranslated region element (3'-UTR element) and optionally a poly(A) sequence and/or a polyadenylation-signal. The invention relates further to a vector comprising a 3'-UTR element, to a cell comprising the artificial nucleic acid molecule or the vector, to a pharmaceutical composition comprising the artificial nucleic acid molecule or the vector and to a kit comprising the artificial nucleic acid molecule, the vector and/or the pharmaceutical composition, preferably for use in the field of gene therapy and/or genetic vaccination.

Gene therapy and genetic vaccination belong to the most promising and quickly developing methods of modern medicine. They may provide highly specific and individual options for therapy of a large variety of diseases. Particularly, inherited genetic diseases but also autoimmune diseases, cancerous or tumour-related diseases as well as inflammatory diseases may be the subject of such treatment approaches. Also, it is envisaged to prevent early onset of such diseases by these approaches.

The main conceptual rational behind gene therapy is appropriate modulation of impaired gene expression associated with pathological conditions of specific diseases. Pathologically altered gene expression may result in lack or overproduction of essential gene products, for example, signalling factors such as hormones, housekeeping factors, metabolic enzymes, structural proteins or the like. Altered gene expression may not only be due to misregulation of transcription and/or translation, but also due to mutations within the ORF coding for a particular protein. Pathological mutations may be caused by e.g. chromosomal aberration, or by more specific mutations, such as point or frame-shift-mutations, all of them resulting in limited functionality and, potentially, total loss of function of the gene product. However, misregulation of transcription or translation may also occur, if mutations affect genes encoding proteins which are involved in the transcriptional or translational machinery of the cell. Such mutations may lead to pathological up- or down-regulation of genes which are - as such - functional. Genes encoding gene products which exert such regulating functions, may be, e.g., transcription factors, signal receptors, messenger proteins or the like. However, loss of function of such genes encoding regulatory proteins may, under certain circumstances, be reversed by artificial introduction of other factors acting further downstream of the impaired gene product. Such gene defects may also be compensated by gene therapy via substitution of the affected gene itself.

Genetic vaccination allows evoking a desired immune response to selected antigens, such as characteristic components of bacterial surfaces, viral particles, tumour antigens or the like. Generally, vaccination is one of the pivotal achievements of modern medicine. However, effective vaccines are currently available only for a limited number of diseases. Accordingly, infections that are not preventable by vaccination still affect millions of people every year.

Commonly, vaccines may be subdivided into "first", "second" and "third" generation vaccines. "First generation" vaccines are, typically, whole-organism vaccines. They are based on either live and attenuated or killed pathogens, e.g. viruses, bacteria or the like. The major drawback of live and attenuated vaccines is the risk for a reversion to life-threatening variants. Thus, although attenuated, such pathogens may still intrinsically bear unpredictable risks. Killed pathogens may not be as effective as desired for generating a specific immune response. In order to minimize these risks, "second generation" vaccines were developed. These are, typically, subunit vaccines, consisting of defined antigens or recombinant protein components which are derived from pathogens.

Genetic vaccines, i.e. vaccines for genetic vaccination, are usually understood as "third generation" vaccines. They are typically composed of genetically engineered nucleic acid molecules which allow expression of peptide or protein (antigen) fragments characteristic for a pathogen or a tumor antigen *in vivo.* Genetic vaccines are expressed upon administration to a patient after uptake by target cells. Expression of the administered nucleic acids results in production of the encoded proteins. In the event these proteins are recognized as foreign by the patient's immune system, an immune response is triggered.

As can be seen from the above, both methods, gene therapy and genetic vaccination, are essentially based on the administration of nucleic acid molecules to a patient and subsequent transcription and/or translation of the encoded genetic information. Alternatively, genetic vaccination or gene therapy may also comprise methods which include isolation of specific body cells from a patient to be treated, subsequent *ex vivo* transfection of such cells, and re-administration of the treated cells to the patient.

DNA as well as RNA may be used as nucleic acid molecules for administration in the context of gene therapy or genetic vaccination. DNA is known to be relatively stable and easy to handle. However, the use of DNA bears the risk of undesired insertion of the administered DNA-fragments into the patient's genome potentially resulting mutagenic events such as in loss of function of the impaired genes. As a further risk, the undesired generation of anti-DNA antibodies has emerged. Another drawback is the limited expression level of the encoded peptide or protein that is achievable upon DNA administration because the DNA must enter the nucleus in order to be transcribed before the resulting mRNA can be translated. Among other reasons, the expression level of the administered DNA will be dependent on the presence of specific transcription factors which regulate DNA transcription. In the absence of such factors, DNA transcription will not yield satisfying amounts of RNA. As a result, the level of translated peptide or protein obtained is limited.

By using RNA instead of DNA for gene therapy or genetic vaccination, the risk of undesired genomic integration and generation of anti-DNA antibodies is minimized or avoided. However, RNA is considered to be a rather unstable molecular species which may readily be degraded by ubiquitous RNAses.

*In vivo,* RNA degradation contributes to the regulation of the RNA half-life time. That effect was considered and proven to fine tune the regulation of eukaryotic gene expression (Friedel et al., 2009. Conserved principles of mammalian transcriptional regulation revealed by RNA half-life, Nucleic Acid Research 37(17): 1-12). Accordingly, each naturally occurring mRNA has its individual half-life depending on the gene from which the mRNA is derived and in which cell type it is expressed. It contributes to the regulation of the expression level of this gene. Unstable RNAs are important to realize transient gene expression at distinct points in time. However, long-lived RNAs may be associated with accumulation of distinct proteins or continuous expression of genes. *In vivo,* the half-life of mRNAs may also be dependent on environmental factors, such as hormonal treatment, as has been shown, e.g., for insulin-like growth factor I, actin, and albumin mRNA (Johnson et al., Newly synthesized RNA: Simultaneous measurement in intact cells of transcription rates and RNA stability of insulin-like growth factor I, actin, and albumin in growth hormone-stimulated hepatocytes, Proc. Natl. Acad. Sci., Vol. 88, pp. 5287-5291, 1991).

For gene therapy and genetic vaccination, usually stable RNA is desired. This is, on the one hand, due to the fact that it is usually desired that the product encoded by the RNA sequence accumulates *in vivo.* On the other hand, the RNA has to maintain its structural and functional integrity when prepared for a suitable dosage form, in the course of its storage, and when administered. Thus, efforts were made to provide stable RNA molecules for gene therapy or genetic vaccination in order to prevent them from being subject to early degradation or decay.

It has been reported that the G/C-content of nucleic acid molecules may influence their stability. Thus, nucleic acids comprising an increased amount of guanine (G) and/or cytosine (C) residues may be functionally more stable than nucleic acids containing a large amount of adenine (A) and thymine (T) or uracil (U) nucleotides. In this context, WO02/098443 provides a pharmaceutical composition containing an mRNA that is stabilised by sequence modifications in the coding region. Such a sequence modification takes advantage of the degeneracy of the genetic code. Accordingly, codons which contain a less favourable combination of nucleotides (less favourable in terms of RNA stability) may be substituted by alternative codons without altering the encoded amino acid sequence. This method of RNA stabilization is limited by the provisions of the specific nucleotide sequence of each single RNA molecule which is not allowed to leave the space of the desired amino acid sequence. Also, that approach is restricted to coding regions of the RNA.

As an alternative option for mRNA stabilisation, it has been found that naturally occurring eukaryotic mRNA molecules contain characteristic stabilising elements. For example, they may comprise so-called untranslated regions (UTR) at their 5'-end (5'-UTR) and/or at their 3'-end (3'-UTR) as well as other structural features, such as a 5'-cap structure or a 3'-poly(A) tail. Both, 5'-UTR and 3'-UTR are typically transcribed from the genomic DNA and are, thus, an element of the premature mRNA. Characteristic structural features of mature mRNA, such as the 5'-cap and the 3'-poly(A) tail (also called poly(A) tail or poly(A) sequence) are usually added to the transcribed (premature) mRNA during mRNA processing.

A 3'-poly(A) tail is typically a monotonous sequence stretch of adenosine nucleotides added to the 3'-end of the transcribed mRNA. It may comprise up to about 400 adenosine nucleotides. It was found that the length of such a 3'-poly(A) tail is a potentially critical element for the stability of the individual mRNA.

Also, it was shown that the 3'-UTR of $\alpha$-globin mRNA may be an important factor for the well-known stability of $\alpha$-globin mRNA (Rodgers et al., Regulated $\alpha$-globin mRNA decay is a cytoplasmic event proceeding through 3'-to-5' exosome-dependent decapping, RNA, 8, pp. 1526-1537, 2002). The 3'-UTR of $\alpha$-globin mRNA is apparently involved in the formation of a specific ribonucleoprotein-complex, the $\alpha$-complex, whose presence correlates with mRNA stability *in vitro* (Wang et al., An mRNA stability complex functions with poly(A)-binding protein to stabilize mRNA in vitro, Molecular and Cellular biology, Vol 19, No. 7, July 1999, p. 4552-4560).

An interesting regulatory function has further been demonstrated for the UTRs in ribosomal protein mRNAs: while the 5'-UTR of ribosomal protein mRNAs controls the growth-associated translation of the mRNA, the stringency of that regulation is conferred by the respective 3'-UTR in ribosomal protein mRNAs (Ledda et al., Effect of the 3'-UTR length on the translational regulation of 5'-terminal oligopyrimidine mRNAs, Gene, Vol. 344, 2005, p. 213-220). This mechanism contributes to the specific expression pattern of ribosomal proteins, which are typically transcribed in a constant manner so that some ribosomal protein mRNAs such as ribosomal protein S9 or ribosomal protein L32 are referred to as housekeeping genes (Janovick-Guretzky et al., Housekeeping Gene Expression in Bovine Liver is Affected by Physiological State, Feed Intake, and Dietary Treatment, J. Dairy Sci., Vol. 90, 2007, p. 2246-2252). The growth-associated expression pattern of ribosomal proteins is thus mainly due to regulation on the level of translation.

Irrespective of factors influencing mRNA stability, effective translation of the administered nucleic acid molecules by the target cells or tissue is crucial for any approach using nucleic acid molecules for gene therapy or genetic vaccination. As can be seen from the examples cited above, along with the regulation of stability, also translation of the majority of mRNAs is regulated by structural features like UTRs, 5'-cap and 3'-poly(A) tail. In this context, it has been reported that the length of the poly(A) tail may play an important role for translational efficiency as well. Stabilizing 3'-elements,

however, may also have an attenuating effect on translation.

It is the object of the invention to provide nucleic acid molecules, which may be suitable for application in gene therapy and/or genetic vaccination. Particularly, it is the object of the invention to provide an mRNA species, which is stabilized against preterm degradation or decay without exhibiting significant functional loss in translational efficiency. It is also an object of the invention to provide an artificial nucleic acid molecule, preferably an mRNA, which is characterized by enhanced expression of the respective protein encoded by said nucleic acid molecule. One particular object of the invention is the provision of an mRNA, wherein the efficiency of translation of the respective encoded protein is enhanced. Another object of the present invention is to provide nucleic acid molecules coding for such a superior mRNA species, which may be amenable for use in gene therapy and/or genetic vaccination. It is a further object of the present invention to provide a pharmaceutical composition for use in gene therapy and/or genetic vaccination. In summary, it is the object of the present invention to provide improved nucleic acid species which overcome the above discussed disadvantages of the prior art by a cost-effective and straight-forward approach.

The object underlying the present invention is solved by the claimed subject matter.

The present invention was made with support from the Government under Agreement No. HR0011-11-3-0001 awarded by DARPA. The Government has certain rights in the invention.

For the sake of clarity and readability the following definitions are provided. Any technical feature mentioned for these definitions may be read on each and every embodiment of the invention. Additional definitions and explanations may be specifically provided in the context of these embodiments.

Adaptive immune response: The adaptive immune response is typically understood to be an antigen-specific response of the immune system. Antigen specificity allows for the generation of responses that are tailored to specific pathogens or pathogen-infected cells. The ability to mount these tailored responses is usually maintained in the body by "memory cells". Should a pathogen infect the body more than once, these specific memory cells are used to quickly eliminate it. In this context, the first step of an adaptive immune response is the activation of naïve antigen-specific T cells or different immune cells able to induce an antigen-specific immune response by antigen-presenting cells. This occurs in the lymphoid tissues and organs through which naïve T cells are constantly passing. The three cell types that may serve as antigen-presenting cells are dendritic cells, macrophages, and B cells. Each of these cells has a distinct function in eliciting immune responses. Dendritic cells may take up antigens by phagocytosis and macropinocytosis and may become stimulated by contact with e.g. a foreign antigen to migrate to the local lymphoid tissue, where they differentiate into mature dendritic cells. Macrophages ingest particulate antigens such as bacteria and are induced by infectious agents or other appropriate stimuli to express MHC molecules. The unique ability of B cells to bind and internalize soluble protein antigens via their receptors may also be important to induce T cells. MHC-molecules are, typically, responsible for presentation of an antigen to T-cells. Therein, presenting the antigen on MHC molecules leads to activation of T cells, which induces their proliferation and differentiation into armed effector T cells. The most important function of effector T cells is the killing of infected cells by CD8+ cytotoxic T cells and the activation of macrophages by Th1 cells, which together make up cell-mediated immunity, and the activation of B cells by both Th2 and Th1 cells to produce different classes of antibody, thus driving the humoral immune response. T cells recognize an antigen by their T cell receptors which do not recognize and bind the antigen directly, but instead recognize short peptide fragments e.g. of pathogen-derived protein antigens, e.g. so-called epitopes, which are bound to MHC molecules on the surfaces of other cells.

Adaptive immune system: The adaptive immune system is essentially dedicated to eliminate or prevent pathogenic growth. It typically regulates the adaptive immune response by providing the vertebrate immune system with the ability to recognize and remember specific pathogens (to generate immunity), and to mount stronger attacks each time the pathogen is encountered. The system is highly adaptable because of somatic hypermutation (a process of accelerated somatic mutations), and V(D)J recombination (an irreversible genetic recombination of antigen receptor gene segments). This mechanism allows a small number of genes to generate a vast number of different antigen receptors, which are then uniquely expressed on each individual lymphocyte. Because the gene rearrangement leads to an irreversible change in the DNA of each cell, all of the progeny (offspring) of such a cell will then inherit genes encoding the same receptor specificity, including the Memory B cells and Memory T cells that are the keys to long-lived specific immunity.

Adjuvant/adjuvant component: An adjuvant or an adjuvant component in the broadest sense is typically a pharmacological and/or immunological agent that may modify, e.g. enhance, the effect of other agents, such as a drug or vaccine. It is to be interpreted in a broad sense and refers to a broad spectrum of substances. Typically, these substances are able to increase the immunogenicity of antigens. For example, adjuvants may be recognized by the innate immune systems and, e.g., may elicit an innate immune response. "Adjuvants" typically do not elicit an adaptive immune response. Insofar, "adjuvants" do not qualify as antigens. Their mode of action is distinct from the effects triggered by antigens resulting in an adaptive immune response.

Antigen: In the context of the present invention "antigen" refers typically to a substance which may be recognized by the immune system, preferably by the adaptive immune system, and is capable of triggering an antigen-specific immune response, e.g. by formation of antibodies and/or antigen-specific T cells as part of an adaptive immune response.

Typically, an antigen may be or may comprise a peptide or protein, which may be presented by the MHC to T-cells. In the sense of the present invention an antigen may be the product of translation of a provided nucleic acid molecule, preferably an mRNA as defined herein. In this context, also fragments, variants and derivatives of peptides and proteins comprising at least one epitope are understood as antigens. In the context of the present invention, tumour antigens and pathogenic antigens as defined herein are particularly preferred.

Artificial nucleic acid molecule: _An artificial nucleic acid molecule may typically be understood to be a nucleic acid molecule, e.g. a DNA or an RNA, that does not occur naturally. In other words, an artificial nucleic acid molecule may be understood as a non-natural nucleic acid molecule. Such nucleic acid molecule may be non-natural due to its individual sequence (which does not occur naturally) and/or due to other modifications, e.g. structural modifications of nucleotides, which do not occur naturally. An artificial nucleic acid molecule may be a DNA molecule, an RNA molecule or a hybrid-molecule comprising DNA and RNA portions. Typically, artificial nucleic acid molecules may be designed and/or generated by genetic engineering methods to correspond to a desired artificial sequence of nucleotides (heterologous sequence). In this context an artificial sequence is usually a sequence that may not occur naturally, i.e. it differs from the wild type sequence by at least one nucleotide. The term "wild type" may be understood as a sequence occurring in nature. Further, the term "artificial nucleic acid molecule" is not restricted to mean "one single molecule" but is, typically, understood to comprise an ensemble of identical molecules. Accordingly, it may relate to a plurality of identical molecules contained in an aliquot.

Bicistronic RNA, multicistronic RNA: A bicistronic or multicistronic RNA is typically an RNA, preferably an mRNA, that typically may have two (bicistronic) or more (multicistronic) open reading frames (ORF). An open reading frame in this context is a sequence of codons that is translatable into a peptide or protein.

Carrier /polymeric carrier: A carrier in the context of the invention may typically be a compound that facilitates transport and/or complexation of another compound (cargo). A polymeric carrier is typically a carrier that is formed of a polymer. A carrier may be associated to its cargo by covalent or non-covalent interaction. A carrier may transport nucleic acids, e.g. RNA or DNA, to the target cells. The carrier may - for some embodiments - be a cationic component.

Cationic component: The term "cationic component" typically refers to a charged molecule, which is positively charged (cation) at a pH value typically from 1 to 9, preferably at a pH value of or below 9 (e.g. from 5 to 9), of or below 8 (e.g. from 5 to 8), of or below 7 (e.g. from 5 to 7), most preferably at a physiological pH, e.g. from 7.3 to 7.4. Accordingly, a cationic component may be any positively charged compound or polymer, preferably a cationic peptide or protein, which is positively charged under physiological conditions, particularly under physiological conditions *in vivo.* A "cationic peptide or protein" may contain at least one positively charged amino acid, or more than one positively charged amino acid, e.g. selected from Arg, His, Lys or Orn. Accordingly, "polycationic" components are also within the scope exhibiting more than one positive charge under the conditions given.

5'-cap: A 5'-cap is an entity, typically a modified nucleotide entity, which generally "caps" the 5'-end of a mature mRNA. A 5'-cap may typically be formed by a modified nucleotide, particularly by a derivative of a guanine nucleotide. Preferably, the 5'-cap is linked to the 5'-terminus via a 5'-5'-triphosphate linkage. A 5'-cap may be methylated, e.g. m7GpppN, wherein N is the terminal 5' nucleotide of the nucleic acid carrying the 5'-cap, typically the 5'-end of an RNA. Further examples of 5'cap structures include glyceryl, inverted deoxy abasic residue (moiety), 4',5' methylene nucleotide, 1-(beta-D-erythrofuranosyl) nucleotide, 4'-thio nucleotide, carbocyclic nucleotide, 1,5-anhydrohexitol nucleotide, L-nucleotides, alpha-nucleotide, modified base nucleotide, threo-pentofuranosyl nucleotide, acyclic 3',4'-seco nucleotide, acyclic 3,4-dihydroxybutyl nucleotide, acyclic 3,5 dihydroxypentyl nucleotide, 3'-3'-inverted nucleotide moiety, 3'-3'-inverted abasic moiety, 3'-2'-inverted nucleotide moiety, 3'-2'-inverted abasic moiety, 1,4-butanediol phosphate, 3'-phosphoramidate, hexylphosphate, aminohexyl phosphate, 3'-phosphate, 3'phosphorothioate, phosphorodithioate, or bridging or non-bridging methylphosphonate moiety.

Cellular immunity/cellular immune response: Cellular immunity relates typically to the activation of macrophages, natural killer cells (NK), antigen-specific cytotoxic T-lymphocytes, and the release of various cytokines in response to an antigen. In more general terms, cellular immunity is not based on antibodies, but on the activation of cells of the immune system. Typically, a cellular immune response may be characterized e.g. by activating antigen-specific cytotoxic T-lymphocytes that are able to induce apoptosis in cells, e.g. specific immune cells like dendritic cells or other cells, displaying epitopes of foreign antigens on their surface. Such cells may be virus-infected or infected with intracellular bacteria, or cancer cells displaying tumor antigens. Further characteristics may be activation of macrophages and natural killer cells, enabling them to destroy pathogens and stimulation of cells to secrete a variety of cytokines that influence the function of other cells involved in adaptive immune responses and innate immune responses.

DNA: DNA is the usual abbreviation for deoxy-ribonucleic acid. It is a nucleic acid molecule, i.e. a polymer consisting of nucleotides. These nucleotides are usually deoxy-adenosine-monophosphate, deoxy-thymidine-monophosphate, deoxy-guanosine-monophosphate and deoxy-cytidine-monophosphate monomers which are - by themselves - composed of a sugar moiety (deoxyribose), a base moiety and a phosphate moiety, and polymerise by a characteristic backbone structure. The backbone structure is, typically, formed by phosphodiester bonds between the sugar moiety of the nucleotide, i.e. deoxyribose, of a first and a phosphate moiety of a second, adjacent monomer. The

specific order of the monomers, i.e. the order of the bases linked to the sugar/phosphate-backbone, is called the DNA sequence. DNA may be single stranded or double stranded. In the double stranded form, the nucleotides of the first strand typically hybridize with the nucleotides of the second strand, e.g. by A/T-base-pairing and G/C-base-pairing.

Epitope: (also called "antigen determinant") can be distinguished in T cell epitopes and B cell epitopes. T cell epitopes or parts of the proteins in the context of the present invention may comprise fragments preferably having a length of about 6 to about 20 or even more amino acids, e.g. fragments as processed and presented by MHC class I molecules, preferably having a length of about 8 to about 10 amino acids, e.g. 8, 9, or 10, (or even 11, or 12 amino acids), or fragments as processed and presented by MHC class II molecules, preferably having a length of about 13 or more amino acids, e.g. 13, 14, 15, 16, 17, 18, 19, 20 or even more amino acids, wherein these fragments may be selected from any part of the amino acid sequence. These fragments are typically recognized by T cells in form of a complex consisting of the peptide fragment and an MHC molecule, i.e. the fragments are typically not recognized in their native form. B cell epitopes are typically fragments located on the outer surface of (native) protein or peptide antigens as defined herein, preferably having 5 to 15 amino acids, more preferably having 5 to 12 amino acids, even more preferably having 6 to 9 amino acids, which may be recognized by antibodies, i.e. in their native form.

Such epitopes of proteins or peptides may furthermore be selected from any of the herein mentioned variants of such proteins or peptides. In this context antigenic determinants can be conformational or discontinuous epitopes which are composed of segments of the proteins or peptides as defined herein that are discontinuous in the amino acid sequence of the proteins or peptides as defined herein but are brought together in the three-dimensional structure or continuous or linear epitopes which are composed of a single polypeptide chain.

Fragment of a sequence: A fragment of a sequence may typically be a shorter portion of a full-length sequence of e.g. a nucleic acid molecule or an amino acid sequence.

Accordingly, a fragment, typically, consists of a sequence that is identical to the corresponding stretch within the full-length sequence. A preferred fragment of a sequence in the context of the present invention, consists of a continuous stretch of entities, such as nucleotides or amino acids corresponding to a continuous stretch of entities in the molecule the fragment is derived from, which represents at least 5%, 10%, 20%, preferably at least 30%, more preferably at least 40%, more preferably at least 50%, even more preferably at least 60%, even more preferably at least 70%, and most preferably at least 80% of the total (i.e. full-length) molecule from which the fragment is derived.

G/C modified: A G/C-modified nucleic acid may typically be a nucleic acid, preferably an artificial nucleic acid molecule as defined herein, based on a modified wild-type sequence comprising a preferably increased number of guanosine and/or cytosine nucleotides as compared to the wild-type sequence. Such an increased number may be generated by substitution of codons containing adenosine or thymidine nucleotides by codons containing guanosine or cytosine nucleotides. If the enriched G/C content occurs in a coding region of DNA or RNA, it makes use of the degeneracy of the genetic code. Accordingly, the codon substitutions preferably do not alter the encoded amino acid residues, but exclusively increase the G/C content of the nucleic acid molecule.

Gene therapy: Gene therapy may typically be understood to mean a treatment of a patient's body or isolated elements of a patient's body, for example isolated tissues/cells, by nucleic acids encoding a peptide or protein. It typically may comprise at least one of the steps of a) administration of a nucleic acid, preferably an artificial nucleic acid molecule as defined herein, directly to the patient - by whatever administration route - or *in vitro* to isolated cells/tissues of the patient, which results in transfection of the patient's cells either *in vivo*/*ex vivo* or *in vitro,* b) transcription and/or translation of the introduced nucleic acid molecule; and optionally c) re-administration of isolated, transfected cells to the patient, if the nucleic acid has not been administered directly to the patient.

Genetic vaccination: Genetic vaccination may typically be understood to be vaccination by administration of a nucleic acid molecule encoding an antigen or an immunogen or fragments thereof. The nucleic acid molecule may be administered to a subject's body or to isolated cells of a subject. Upon transfection of certain cells of the body or upon transfection of the isolated cells, the antigen or immunogen may be expressed by those cells and subsequently presented to the immune system, eliciting an adaptive, i.e. antigen-specific immune response. Accordingly, genetic vaccination typically comprises at least one of the steps of a) administration of a nucleic acid, preferably an artificial nucleic acid molecule as defined herein, to a subject, preferably a patient, or to isolated cells of a subject, preferably a patient, which usually results in transfection of the subject's cells either *in vivo* or *in vitro;* b) transcription and/or translation of the introduced nucleic acid molecule; and optionally c) re-administration of isolated, transfected cells to the subject, preferably the patient, if the nucleic acid has not been administered directly to the patient.

Heterologous sequence: Two sequences are typically understood to be 'heterologous' if they are not derivable from the same gene. I.e., although heterologous sequences may be derivable from the same organism, they naturally (in nature) do not occur in the same nucleic acid molecule, such as in the same mRNA.

Humoral immunity/humoral immune response: Humoral immunity refers typically to antibody production and optionally to accessory processes accompanying antibody production. A humoral immune response may be typically characterized, e.g., by Th2 activation and cytokine production, germinal center formation and isotype switching, affinity maturation and memory cell generation. Humoral immunity also typically may refer to the effector functions of antibodies,

which include pathogen and toxin neutralization, classical complement activation, and opsonin promotion of phagocytosis and pathogen elimination.

Immunogen: In the context of the present invention, an immunogen may be typically understood to be a compound that is able to stimulate an immune response. Preferably, an immunogen is a peptide, polypeptide, or protein. In a particularly preferred embodiment, an immunogen in the sense of the present invention is the product of translation of a provided nucleic acid molecule, preferably an artificial nucleic acid molecule as defined herein. Typically, an immunogen elicits at least an adaptive immune response.

Immunostimulatory composition: In the context of the invention, an immunostimulatory composition may be typically understood to be a composition containing at least one component which is able to induce an immune response or from which a component, which is able to induce an immune response, is derivable. Such immune response may be preferably an innate immune response or a combination of an adaptive and an innate immune response. Preferably, an immunostimulatory composition in the context of the invention contains at least one artificial nucleic acid molecule, more preferably an RNA, for example an mRNA molecule. The immunostimulatory component, such as the mRNA may be complexed with a suitable carrier. Thus, the immunostimulatory composition may comprise an mRNA/carrier-complex. Furthermore, the immunostimulatory composition may comprise an adjuvant and/or a suitable vehicle for the immunostimulatory component, such as the mRNA.

Immune response: An immune response may typically be a specific reaction of the adaptive immune system to a particular antigen (so called specific or adaptive immune response) or an unspecific reaction of the innate immune system (so called unspecific or innate immune response), or a combination thereof.

Immune system: The immune system may protect organisms from infection. If a pathogen succeeds in passing a physical barrier of an organism and enters this organism, the innate immune system provides an immediate, but non-specific response. If pathogens evade this innate response, vertebrates possess a second layer of protection, the adaptive immune system. Here, the immune system adapts its response during an infection to improve its recognition of the pathogen. This improved response is then retained after the pathogen has been eliminated, in the form of an immunological memory, and allows the adaptive immune system to mount faster and stronger attacks each time this pathogen is encountered. According to this, the immune system comprises the innate and the adaptive immune system. Each of these two parts typically contains so called humoral and cellular components.

Immunostimulatory RNA: An immunostimulatory RNA (isRNA) in the context of the invention may typically be an RNA that is able to induce an innate immune response. It usually does not have an open reading frame and thus does not provide a peptide-antigen or immunogen but elicits an immune response e.g. by binding to a specific kind of Toll-like-receptor (TLR) or other suitable receptors. However, of course also mRNAs having an open reading frame and coding for a peptide/protein may induce an innate immune response and, thus, may be immunostimulatory RNAs.

Innate immune system: The innate immune system, also known as non-specific (or unspecific) immune system, typically comprises the cells and mechanisms that defend the host from infection by other organisms in a non-specific manner. This means that the cells of the innate system may recognize and respond to pathogens in a generic way, but unlike the adaptive immune system, it does not confer long-lasting or protective immunity to the host. The innate immune system may be, e.g., activated by ligands of Toll-like receptors (TLRs) or other auxiliary substances such as lipopolysaccharides, TNF-alpha, CD40 ligand, or cytokines, monokines, lymphokines, interleukins or chemokines, IL-1, IL-2, IL-3, IL-4, IL-5, IL-6, IL-7, IL-8, IL-9, IL-10, IL-11, IL-12, IL-13, IL-14, IL-15, IL-16, IL-17, IL-18, IL-19, IL-20, IL-21, IL-22, IL-23, IL-24, IL-25, IL-26, IL-27, IL-28, IL-29, IL-30, IL-31, IL-32, IL-33, IFN-alpha, IFN-beta, IFN-gamma, GM-CSF, G-CSF, M-CSF, LT-beta, TNF-alpha, growth factors, and hGH, a ligand of human Toll-like receptor TLR1, TLR2, TLR3, TLR4, TLR5, TLR6, TLR7, TLR8, TLR9, TLR10, a ligand of murine Toll-like receptor TLR1, TLR2, TLR3, TLR4, TLR5, TLR6, TLR7, TLR8, TLR9, TLR10, TLR11, TLR12 or TLR13, a ligand of a NOD-like receptor, a ligand of a RIG-I like receptor, an immunostimulatory nucleic acid, an immunostimulatory RNA (isRNA), a CpG-DNA, an antibacterial agent, or an anti-viral agent. The pharmaceutical composition according to the present invention may comprise one or more such substances. Typically, a response of the innate immune system includes recruiting immune cells to sites of infection, through the production of chemical factors, including specialized chemical mediators, called cytokines; activation of the complement cascade; identification and removal of foreign substances present in organs, tissues, the blood and lymph, by specialized white blood cells; activation of the adaptive immune system; and/or acting as a physical and chemical barrier to infectious agents.

Cloning site: A cloning site is typically understood to be a segment of a nucleic acid molecule, which is suitable for insertion of a nucleic acid sequence, e.g., a nucleic acid sequence comprising an open reading frame. Insertion may be performed by any molecular biological method known to the one skilled in the art, e.g. by restriction and ligation. A cloning site typically comprises one or more restriction enzyme recognition sites (restriction sites). These one or more restrictions sites may be recognized by restriction enzymes which cleave the DNA at these sites. A cloning site which comprises more than one restriction site may also be termed a multiple cloning site (MCS) or a polylinker.

Nucleic acid molecule: A nucleic acid molecule is a molecule comprising, preferably consisting of nucleic acid components. The term nucleic acid molecule preferably refers to DNA or RNA molecules. It is preferably used synonymous

with the term "polynucleotide".

Preferably, a nucleic acid molecule is a polymer comprising or consisting of nucleotide monomers, which are covalently linked to each other by phosphodiester-bonds of a sugar/phosphate-backbone. The term "nucleic acid molecule" also encompasses modified nucleic acid molecules, such as base-modified, sugar-modified or backbone-modified etc. DNA or RNA molecules.

Open reading frame: An open reading frame (ORF) in the context of the invention may typically be a sequence of several nucleotide triplets, which may be translated into a peptide or protein. An open reading frame preferably contains a start codon, i.e. a combination of three subsequent nucleotides coding usually for the amino acid methionine (ATG), at its 5'-end and a subsequent region, which usually exhibits a length which is a multiple of 3 nucleotides. An ORF is preferably terminated by a stop-codon (e.g., TAA, TAG, TGA). Typically, this is the only stop-codon of the open reading frame. Thus, an open reading frame in the context of the present invention is preferably a nucleotide sequence, consisting of a number of nucleotides that may be divided by three, which starts with a start codon (e.g. ATG) and which preferably terminates with a stop codon (e.g., TAA, TGA, or TAG). The open reading frame may be isolated or it may be incorporated in a longer nucleic acid sequence, for example in a vector or an mRNA. An open reading frame may also be termed "protein coding region".

Peptide: A peptide or polypeptide is typically a polymer of amino acid monomers, linked by peptide bonds. It typically contains less than 50 monomer units. Nevertheless, the term peptide is not a disclaimer for molecules having more than 50 monomer units. Long peptides are also called polypeptides, typically having between 50 and 600 monomeric units.

Pharmaceutically effective amount: A pharmaceutically effective amount in the context of the invention is typically understood to be an amount that is sufficient to induce a pharmaceutical effect, such as an immune response, altering a pathological level of an expressed peptide or protein, or substituting a lacking gene product, e.g., in case of a pathological situation.

Protein A protein typically comprises one or more peptides or polypeptides. A protein is typically folded into 3-dimensional form, which may be required for to protein to exert its biological function.

Poly(A) sequence: A poly(A) sequence, also called poly(A) tail or 3'-poly(A) tail, is typically understood to be a sequence of adenosine nucleotides, e.g., of up to about 400 adenosine nucleotides, e.g. from about 20 to about 400, preferably from about 50 to about 400, more preferably from about 50 to about 300, even more preferably from about 50 to about 250, most preferably from about 60 to about 250 adenosine nucleotides. A poly(A) sequence is typically located at the 3'end of an mRNA. In the context of the present invention, a poly(A) sequence may be located within an mRNA or any other nucleic acid molecule, such as, e.g., in a vector, for example, in a vector serving as template for the generation of an RNA, preferably an mRNA, e.g., by transcription of the vector.

Polyadenylation: Polyadenylation is typically understood to be the addition of a poly(A) sequence to a nucleic acid molecule, such as an RNA molecule, e.g. to a premature mRNA. Polyadenylation may be induced by a so-called polyadenylation signal. This signal is preferably located within a stretch of nucleotides at the 3'-end of a nucleic acid molecule, such as an RNA molecule, to be polyadenylated. A polyadenylation signal typically comprises a hexamer consisting of adenine and uracil/thymine nucleotides, preferably the hexamer sequence AAUAAA. Other sequences, preferably hexamer sequences, are also conceivable. Polyadenylation typically occurs during processing of a pre-mRNA (also called premature-mRNA). Typically, RNA maturation (from pre-mRNA to mature mRNA) comprises the step of polyadenylation.

Restriction site: A restriction site, also termed restriction enzyme recognition site, is a nucleotide sequence recognized by a restriction enzyme. A restriction site is typically a short, preferably palindromic nucleotide sequence, e.g. a sequence comprising 4 to 8 nucleotides. A restriction site is preferably specifically recognized by a restriction enzyme. The restriction enzyme typically cleaves a nucleotide sequence comprising a restriction site at this site. In a double-stranded nucleotide sequence, such as a double-stranded DNA sequence, the restriction enzyme typically cuts both strands of the nucleotide sequence.

RNA, mRNA: RNA is the usual abbreviation for ribonucleic-acid. It is a nucleic acid molecule, i.e. a polymer consisting of nucleotides. These nucleotides are usually adenosine-monophosphate, uridine-monophosphate, guanosine-monophosphate and cytidine-monophosphate monomers which are connected to each other along a so-called backbone. The backbone is formed by phosphodiester bonds between the sugar, i.e. ribose, of a first and a phosphate moiety of a second, adjacent monomer. The specific succession of the monomers is called the RNA-sequence. Usually RNA may be obtainable by transcription of a DNA-sequence, e.g., inside a cell. In eukaryotic cells, transcription is typically performed inside the nucleus or the mitochondria. In vivo, transcription of DNA usually results in the so-called premature RNA which has to be processed into so-called messenger-RNA, usually abbreviated as mRNA. Processing of the premature RNA, e.g. in eukaryotic organisms, comprises a variety of different posttranscriptional-modifications such as splicing, 5'-capping, polyadenylation, export from the nucleus or the mitochondria and the like. The sum of these processes is also called maturation of RNA. The mature messenger RNA usually provides the nucleotide sequence that may be translated into an amino-acid sequence of a particular peptide or protein. Typically, a mature mRNA comprises

a 5'-cap, a 5'-UTR, an open reading frame, a 3'-UTR and a poly(A) sequence. Aside from messenger RNA, several non-coding types of RNA exist which may be involved in regulation of transcription and/or translation.

Sequence of a nucleic acid molecule: The sequence of a nucleic acid molecule is typically understood to be the particular and individual order, i.e. the succession of its nucleotides. The sequence of a protein or peptide is typically understood to be the order, i.e. the succession of its amino acids.

Sequence identity: Two or more sequences are identical if they exhibit the same length and order of nucleotides or amino acids. The percentage of identity typically describes the extent to which two sequences are identical, i.e. it typically describes the percentage of nucleotides that correspond in their sequence position with identical nucleotides of a reference-sequence. For determination of the degree of identity, the sequences to be compared are considered to exhibit the same length, i.e. the length of the longest sequence of the sequences to be compared. This means that a first sequence consisting of 8 nucleotides is 80% identical to a second sequence consisting of 10 nucleotides comprising the first sequence. In other words, in the context of the present invention, identity of sequences preferably relates to the percentage of nucleotides of a sequence which have the same position in two or more sequences having the same length. Gaps are usually regarded as non-identical positions, irrespective of their actual position in an alignment.

Stabilized nucleic acid molecule: A stabilized nucleic acid molecule is a nucleic acid molecule, preferably a DNA or RNA molecule that is modified such, that it is more stable to disintegration or degradation, e.g., by environmental factors or enzymatic digest, such as by an exo- or endonuclease degradation, than the nucleic acid molecule without the modification. Preferably, a stabilized nucleic acid molecule in the context of the present invention is stabilized in a cell, such as a prokaryotic or eukaryotic cell, preferably in a mammalian cell, such as a human cell. The stabilization effect may also be exerted outside of cells, e.g. in a buffer solution etc., for example, in a manufacturing process for a pharmaceutical composition comprising the stabilized nucleic acid molecule.

Transfection: The term "transfection" refers to the introduction of nucleic acid molecules, such as DNA or RNA (e.g. mRNA) molecules, into cells, preferably into eukaryotic cells. In the context of the present invention, the term "transfection" encompasses any method known to the skilled person for introducing nucleic acid molecules into cells, preferably into eukaryotic cells, such as into mammalian cells. Such methods encompass, for example, electroporation, lipofection, e.g. based on cationic lipids and/or liposomes, calcium phosphate precipitation, nanoparticle based transfection, virus based transfection, or transfection based on cationic polymers, such as DEAE-dextran or polyethylenimine etc. Preferably, the introduction is non-viral.

Vaccine: A vaccine is typically understood to be a prophylactic or therapeutic material providing at least one antigen, preferably an immunogen. The antigen or immunogen may be derived from any material that is suitable for vaccination. For example, the antigen or immunogen may be derived from a pathogen, such as from bacteria or virus particles etc., or from a tumor or cancerous tissue. The antigen or immunogen stimulates the body's adaptive immune system to provide an adaptive immune response.

Vector: The term "vector" refers to a nucleic acid molecule, preferably to an artificial nucleic acid molecule. A vector in the context of the present invention is suitable for incorporating or harboring a desired nucleic acid sequence, such as a nucleic acid sequence comprising an open reading frame. Such vectors may be storage vectors, expression vectors, cloning vectors, transfer vectors etc. A storage vector is a vector, which allows the convenient storage of a nucleic acid molecule, for example, of an mRNA molecule. Thus, the vector may comprise a sequence corresponding, e.g., to a desired mRNA sequence or a part thereof, such as a sequence corresponding to the open reading frame and the 3'-UTR of an mRNA. An expression vector may be used for production of expression products such as RNA, e.g. mRNA, or peptides, polypeptides or proteins. For example, an expression vector may comprise sequences needed for transcription of a sequence stretch of the vector, such as a promoter sequence, e.g. an RNA polymerase promoter sequence. A cloning vector is typically a vector that contains a cloning site, which may be used to incorporate nucleic acid sequences into the vector. A cloning vector may be, e.g., a plasmid vector or a bacteriophage vector. A transfer vector may be a vector, which is suitable for transferring nucleic acid molecules into cells or organisms, for example, viral vectors. A vector in the context of the present invention may be, e.g., an RNA vector or a DNA vector. Preferably, a vector is a DNA molecule. Preferably, a vector in the sense of the present application comprises a cloning site, a selection marker, such as an antibiotic resistance factor, and a sequence suitable for multiplication of the vector, such as an origin of replication. Preferably, a vector in the context of the present application is a plasmid vector.

Vehicle: A vehicle is typically understood to be a material that is suitable for storing, transporting, and/or administering a compound, such as a pharmaceutically active compound. For example, it may be a physiologically acceptable liquid, which is suitable for storing, transporting, and/or administering a pharmaceutically active compound.

3'-untranslated region (3'-UTR): Generally, the term "3'-UTR" refers to a part of the artificial nucleic acid molecule, which is located 3' (i.e. "downstream") of an open reading frame and which is not translated into protein. Typically, a 3'-UTR is the part of an mRNA which is located between the protein coding region (open reading frame (ORF) or coding sequence (CDS)) and the poly(A) sequence of the mRNA. In the context of the invention, the term 3'-UTR may also comprise elements, which are not encoded in the template, from which an RNA is transcribed, but which are added after transcription during maturation, e.g. a poly(A) sequence. A 3'-UTR of the mRNA is not translated into an amino

acid sequence. The 3'-UTR sequence is generally encoded by the gene, which is transcribed into the respective mRNA during the gene expression process. The genomic sequence is first transcribed into pre-mature mRNA, which comprises optional introns. The pre-mature mRNA is then further processed into mature mRNA in a maturation process. This maturation process comprises the steps of 5'capping, splicing the pre-mature mRNA to excise optional introns and modifications of the 3'-end, such as polyadenylation of the 3'-end of the pre-mature mRNA and optional endo-/ or exonuclease cleavages etc.. In the context of the present invention, a 3'-UTR corresponds to the sequence of a mature mRNA, which is located between the stop codon of the protein coding region, preferably immediately 3' to the stop codon of the protein coding region, and the poly(A) sequence of the mRNA. The term "corresponds to" means that the 3'-UTR sequence may be an RNA sequence, such as in the mRNA sequence used for defining the 3'-UTR sequence, or a DNA sequence, which corresponds to such RNA sequence. In the context of the present invention, the term "a 3'-UTR of a gene", such as "a 3'-UTR of a ribosomal protein gene", is the sequence, which corresponds to the 3'-UTR of the mature mRNA derived from this gene, i.e. the mRNA obtained by transcription of the gene and maturation of the pre-mature mRNA. The term "3'-UTR of a gene" encompasses the DNA sequence and the RNA sequence (both sense and antisense strand and both mature and immature) of the 3'-UTR.

5'-untranslated region (5'-UTR): A 5'-UTR is typically understood to be a particular section of messenger RNA (mRNA). It is located 5' of the open reading frame of the mRNA. Typically, the 5'-UTR starts with the transcriptional start site and ends one nucleotide before the start codon of the open reading frame. The 5'-UTR may comprise elements for controlling gene expression, also called regulatory elements. Such regulatory elements may be, for example, ribosomal binding sites. The 5'-UTR may be post-transcriptionally modified, for example by addition of a 5'-CAP. In the context of the present invention, a 5'-UTR corresponds to the sequence of a mature mRNA, which is located between the 5'-CAP and the start codon. Preferably, the 5'-UTR corresponds to the sequence, which extends from a nucleotide located 3' to the 5'-CAP, preferably from the nucleotide located immediately 3' to the 5'-CAP, to a nucleotide located 5' to the start codon of the protein coding region, preferably to the nucleotide located immediately 5' to the start codon of the protein coding region. The nucleotide located immediately 3' to the 5'-CAP of a mature mRNA typically corresponds to the transcriptional start site. The term "corresponds to" means that the 5'-UTR sequence may be an RNA sequence, such as in the mRNA sequence used for defining the 5'-UTR sequence, or a DNA sequence, which corresponds to such RNA sequence. In the context of the present invention, the term "a 5'-UTR of a gene" is the sequence, which corresponds to the 5'-UTR of the mature mRNA derived from this gene, i.e. the mRNA obtained by transcription of the gene and maturation of the pre-mature mRNA. The term "5'-UTR of a gene" encompasses the DNA sequence and the RNA sequence of the 5'-UTR. By the inventive embodiments such a 5'-UTR may be provided 5'-terminal to the ORF. Its length is typically less than 500, 400, 300, 250 or less than 200 nucleotides. In other embodiments its length may be in the range of at least 10, 20, 30 or 40, preferably up to 100 or 150, nucleotides.

5'Terminal Oligopyrimidine Tract (TOP): The 5'terminal oligopyrimidine tract (TOP) is typically a stretch of pyrimidine nucleotides located in the 5' terminal region of a nucleic acid molecule, such as the 5' terminal region of certain mRNA molecules or the 5' terminal region of a functional entity, e.g. the transcribed region, of certain genes. The sequence starts with a cytidine, which usually corresponds to the transcriptional start site, and is followed by a stretch of usually about 3 to 30 pyrimidine nucleotides. For example, the TOP may comprise 3, 4, 5, 6, 7, 8, 9, 10, 11, 12, 13, 14, 15, 16, 17, 18, 19, 20, 21, 22, 23, 24, 25, 26, 27, 28, 29, 30 or even more nucleotides. The pyrimidine stretch and thus the 5' TOP ends one nucleotide 5' to the first purine nucleotide located downstream of the TOP. Messenger RNA that contains a 5'terminal oligopyrimidine tract is often referred to as TOP mRNA. Accordingly, genes that provide such messenger RNAs are referred to as TOP genes. TOP sequences have, for example, been found in genes and mRNAs encoding peptide elongation factors and ribosomal proteins.

TOP motif: In the context of the present invention, a TOP motif is a nucleic acid sequence which corresponds to a 5'TOP as defined above. Thus, a TOP motif in the context of the present invention is preferably a stretch of pyrimidine nucleotides having a length of 3-30 nucleotides. Preferably, the TOP-motif consists of at least 3 pyrimidine nucleotides, preferably at least 4 pyrimidine nucleotides, preferably at least 5 pyrimidine nucleotides, more preferably at least 6 nucleotides, more preferably at least 7 nucleotides, most preferably at least 8 pyrimidine nucleotides, wherein the stretch of pyrimidine nucleotides preferably starts at its 5'end with a cytosine nucleotide. In TOP genes and TOP mRNAs, the TOP-motif preferably starts at its 5'end with the transcriptional start site and ends one nucleotide 5' to the first purin residue in said gene or mRNA. A TOP motif in the sense of the present invention is preferably located at the 5'end of a sequence, which represents a 5'UTR, or at the 5'end of a sequence, which codes for a 5'UTR. Thus, preferably, a stretch of 3 or more pyrimidine nucleotides is called "TOP motif" in the sense of the present invention if this stretch is located at the 5'end of a respective sequence, such as the artificial nucleic acid molecule, the 5'UTR element of the artificial nucleic acid molecule, or the nucleic acid sequence which is derived from the 5'UTR of a TOP gene as described herein. In other words, a stretch of 3 or more pyrimidine nucleotides, which is not located at the 5'-end of a 5'UTR or a 5'UTR element but anywhere within a 5'UTR or a 5'UTR element, is preferably not referred to as "TOP motif".

TOP gene: TOP genes are typically characterised by the presence of a 5' terminal oligopyrimidine tract. Furthermore, most TOP genes are characterized by a growth-associated translational regulation. However, also TOP genes

with a tissue specific translational regulation are known. As defined above, the 5'UTR of a TOP gene corresponds to the sequence of a 5'UTR of a mature mRNA derived from a TOP gene, which preferably extends from the nucleotide located 3' to the 5'-CAP to the nucleotide located 5' to the start codon. A 5'UTR of a TOP gene typically does not comprise any start codons, preferably no upstream AUGs (uAUGs) or upstream open reading frames (uORFs). Therein, upstream AUGs and upstream open reading frames are typically understood to be AUGs and open reading frames that occur 5' of the start codon (AUG) of the open reading frame that should be translated. The 5'UTRs of TOP genes are generally rather short. The lengths of 5'UTRs of TOP genes may vary between 20 nucleotides up to 500 nucleotides, and are typically less than about 200 nucleotides, preferably less than about 150 nucleotides, more preferably less than about 100 nucleotides. Exemplary 5'UTRs of TOP genes in the sense of the present invention are the nucleic acid sequences extending from the nucleotide at position 5 to the nucleotide located immediately 5' to the start codon (e.g. the ATG) in the sequences according to SEQ ID Nos. 1-1363 of the patent application WO2013/143700, whose disclosure is incorporated herewith by reference. In this context, a particularly preferred fragment of a 5'UTR of a TOP gene is a 5'UTR of a TOP gene lacking the 5'TOP motif. The terms "5'UTR of a TOP gene" or "5'-TOP UTR" preferably refer to the 5'UTR of a naturally occurring TOP gene.

In a first aspect, the present invention relates to an artificial nucleic acid molecule comprising

a. at least one open reading frame (ORF); and
b. at least one 3'-untranslated region element (3'-UTR element) comprising or consisting of a nucleic acid sequence which is derived from the 3'-UTR of a ribosomal protein gene.

The term "3'-UTR element" refers to a nucleic acid sequence, which comprises or consists of a nucleic acid sequence that is derived from a 3'-UTR or from a variant of a 3'-UTR. A "3'-UTR element" preferably refers to a nucleic acid sequence which represents a 3'-UTR of an artificial nucleic acid sequence, such as an artificial mRNA, or which codes for a 3'-UTR of an artificial nucleic acid molecule. Accordingly, in the sense of the present invention, preferably, a 3'-UTR element may be the 3'-UTR of an mRNA, preferably of an artificial mRNA, or it may be the transcription template for a 3'-UTR of an mRNA. Thus, a 3'-UTR element preferably is a nucleic acid sequence, which corresponds to the 3'-UTR of an mRNA, preferably to the 3'-UTR of an artificial mRNA, such as an mRNA obtained by transcription of a genetically engineered vector construct. Preferably, a 3'-UTR element in the sense of the present invention functions as a 3'-UTR or codes for a nucleotide sequence that fulfils the function of a 3'-UTR.

The term "ribosomal protein gene" typically refers to a gene encoding a ribosomal protein. As used herein, the term refers to any ribosomal protein gene, irrespective of the species, from which it is derived. Specifically, the term refers to an eukaryotic ribosomal protein gene. Furthermore, in the context of the invention, the term "ribosomal protein gene" may also refer to a gene, which is similar to a ribosomal protein gene, either structurally or functionally. In particular, the term also comprises "ribosomal protein-like" genes, pseudogenes and genes sharing sequence or structural features, particularly in their 3'-UTR region, with a ribosomal protein gene. Preferably, the term refers to a vertebrate ribosomal protein gene, more preferably to a mammalian ribosomal protein gene, even more preferably to a primate ribosomal protein gene, in particular to a human ribosomal protein gene. Further, the term "ribosomal protein gene" also encompasses a rodent ribosomal protein gene, in particular a murine ribosomal protein gene. Examples of ribosomal protein genes in the meaning of the invention include, but are not limited to, ribosomal protein L9 (RPL9), ribosomal protein L3 (RPL3), ribosomal protein L4 (RPL4), ribosomal protein L5 (RPL5), ribosomal protein L6 (RPL6), ribosomal protein L7 (RPL7), ribosomal protein L7a (RPL7A), ribosomal protein L11 (RPL11), ribosomal protein L12 (RPL12), ribosomal protein L13 (RPL13), ribosomal protein L23 (RPL23), ribosomal protein L18 (RPL18), ribosomal protein L18a (RPL18A), ribosomal protein L19 (RPL19), ribosomal protein L21 (RPL21), ribosomal protein L22 (RPL22), ribosomal protein L23a (RPL23A), ribosomal protein L17 (RPL17), ribosomal protein L24 (RPL24), ribosomal protein L26 (RPL26), ribosomal protein L27 (RPL27), ribosomal protein L30 (RPL30), ribosomal protein L27a (RPL27A), ribosomal protein L28 (RPL28), ribosomal protein L29 (RPL29), ribosomal protein L31 (RPL31), ribosomal protein L32 (RPL32), ribosomal protein L35a (RPL35A), ribosomal protein L37 (RPL37), ribosomal protein L37a (RPL37A), ribosomal protein L38 (RPL38), ribosomal protein L39 (RPL39), ribosomal protein, large, P0 (RPLP0), ribosomal protein, large, P1 (RPLP1), ribosomal protein, large, P2 (RPLP2), ribosomal protein S3 (RPS3), ribosomal protein S3A (RPS3A), ribosomal protein S4, X- linked (RPS4X), ribosomal protein S4, Y-linked 1 (RPS4Y1), ribosomal protein S5 (RPS5), ribosomal protein S6 (RPS6), ribosomal protein S7 (RPS7), ribosomal protein S8 (RPS8), ribosomal protein S9 (RPS9), ribosomal protein S10 (RPS10), ribosomal protein S11 (RPS11), ribosomal protein S12 (RPS12), ribosomal protein S13 (RPS13), ribosomal protein S15 (RPS15), ribosomal protein S15a (RPS15A), ribosomal protein S16 (RPS16), ribosomal protein S19 (RPS19), ribosomal protein S20 (RPS20), ribosomal protein S21 (RPS21), ribosomal protein S23 (RPS23), ribosomal protein S25 (RPS25), ribosomal protein S26 (RPS26), ribosomal protein S27 (RPS27), ribosomal protein S27a (RPS27a), ribosomal protein S28 (RPS28), ribosomal protein S29 (RPS29), ribosomal protein L15 (RPL15), ribosomal protein S2 (RPS2), ribosomal protein L14 (RPL14), ribosomal protein S14 (RPS14), ribosomal protein L10 (RPL10), ribosomal protein L10a (RPL10A), ribosomal protein L35 (RPL35), ribosomal protein L13a (RPL13A), ribosomal protein L36 (RPL36), ribosomal protein

L36a (RPL36A), ribosomal protein L41 (RPL41), ribosomal protein S18 (RPS18), ribosomal protein S24 (RPS24), ribosomal protein L8 (RPL8), ribosomal protein L34 (RPL34), ribosomal protein S17 (RPS17), ribosomal protein SA (RPSA), ubiquitin A-52 residue ribosomal protein fusion product 1 (UBA52), Finkel-Biskis-Reilly murine sarcoma virus (FBR-MuSV) ubiquitously expressed (FAU), ribosomal protein L22-like 1 (RPL22L1), ribosomal protein S17 (RPS17), ribosomal protein L39-like (RPL39L), ribosomal protein L10-like (RPL10L), ribosomal protein L36a-like (RPL36AL), ribosomal protein L3-like (RPL3L), ribosomal protein S27-like (RPS27L), ribosomal protein L26-like 1 (RPL26L1), ribosomal protein L7-like 1 (RPL7L1), ribosomal protein L13a pseudogene (RPL13AP), ribosomal protein L37a pseudogene 8 (RPL37AP8), ribosomal protein S10 pseudogene 5 (RPS10P5), ribosomal protein S26 pseudogene 11 (RPS26P11), ribosomal protein L39 pseudogene 5 (RPL39P5), ribosomal protein, large, P0 pseudogene 6 (RPLP0P6) and ribosomal protein L36 pseudogene 14 (RPL36P14). Preferably, the term "ribosomal protein gene" refers to one of the aforementioned genes, which is derived from a mammalian, preferably from *Homo sapiens* or *Mus musculus.*

Preferably, the at least one open reading frame and the at least one 3'-UTR element are heterologous. The term "heterologous" in this context means that two sequence elements comprised by the artificial nucleic acid molecule, such as the open reading frame and the 3'-UTR element, are not occurring naturally (in nature) in this combination. Preferably, the 3'-UTR element is derived from a different gene than the open reading frame. For example, the ORF may be derived from a different gene than the 3'-UTR element, e.g. encoding a different protein or the same protein but of a different species etc. Preferably, the open reading frame does not code for a ribosomal protein. In a preferred embodiment, the ORF does not encode a human ribosomal protein or a plant (in particular Arabidopsis) ribosomal protein, in particular human ribosomal protein S6 (RPS6), human ribosomal protein L36alike (RPL36AL) or Arabidopsis ribosomal protein S16 (RPS16). In a further preferred embodiment, the open reading frame (ORF) does not encode ribosomal protein S6 (RPS6), ribosomal protein L36a-like (RPL36AL) or ribosomal protein S16 (RPS16).

In specific embodiments it is preferred that the open reading frame does not code for a reporter protein, e.g., selected from the group consisting of globin proteins (particularly beta-globin), luciferase protein, GFP proteins or variants thereof, for example, variants exhibiting at least 70% sequence identity to a globin protein, a luciferase protein, or a GFP protein. In a particularly preferred embodiment, the open reading frame (ORF) does not encode a reporter gene or is not derived from a reporter gene, wherein the reporter gene is preferably not selected from group consisting of globin proteins (particularly beta-globin), luciferase protein, beta-glucuronidase (GUS) and GFP proteins or variants thereof, preferably not selected from EGFP, or variants of any of the above genes, typically exhibiting at least 70% sequence identity to any of these reporter genes, preferably to a globin protein, a luciferase protein, or a GFP protein.

Even more preferably, the 3'-UTR element is heterologous to any other element comprised in the artificial nucleic acid as defined herein. For example, if the artificial nucleic acid according to the invention comprises a 3'-UTR element from a given gene, it does preferably not comprise any other nucleic acid sequence, in particular no functional nucleic acid sequence (e.g. coding or regulatory sequence element) from the same gene, including its regulatory sequences at the 5' and 3' terminus of the gene's ORF. In a particularly preferred embodiment, the artificial nucleic acid molecule comprises an ORF, a 3'-UTR and a 5'-UTR, all of which are heterologous to each other, e.g. they are recombinant as each of them is derived from different genes (and their 5' and 3' UTR's). In another preferred embodiment, the 3'-UTR is not derived from a 3'-UTR of a viral gene or is of non-viral origin.

Preferably, the at least one 3'-UTR element is functionally linked to the ORF. This means preferably that the 3'-UTR element is associated with the ORF such that it may exert a function, such as an enhancing or stabilizing function on the expression of the encoded peptide or protein or a stabilizing function on the artificial nucleic acid molecule. Preferably, the ORF and the 3'-UTR element are associated in 5'→3' direction. Thus, preferably, the artificial nucleic acid molecule comprises the structure 5'-ORF-(optional)-linker-3'-UTR element-3', wherein the linker may be present or absent. For example, the linker may be one or more nucleotides, such as a stretch of 1-50 or 1-20 nucleotides, e.g., comprising or consisting of one or more restriction enzyme recognition sites (restriction sites).

Preferably, the at least one 3'-UTR element comprises a nucleic acid sequence which is derived from the 3'-UTR of a eukaryotic ribosomal protein gene, preferably from the 3'-UTR of a vertebrate ribosomal protein gene, more preferably from the 3'-UTR of a mammalian ribosomal protein gene, even more preferably from the 3'-UTR of a primate ribosomal protein gene, in particular of a human ribosomal protein gene, or from the 3'-UTR of a rodent ribosomal protein gene, in particular of a murine ribosomal protein gene.

In a preferred embodiment, the at least one 3'-UTR element comprises or corresponds to a nucleic acid sequence, which is derived from the 3'-UTR sequence of a transcript selected from the group consisting of NM_000661.4, NM_001024921.2, NM_000967.3, NM_001033853.1, NM_000968.3, NM_000969.3, NM_001024662.1, NM_000970.3, NM_000971.3, NM_000972.2, NM_000975.3, NM_001199802.1, NM_000976.3, NM_000977.3, NM_033251.2, NM_001243130.1, NM_001243131, NM_000978.3, NM_000979.3, NM_001270490.1, NM_000980.3, NM_000981.3, NM_000982.3, NM_000983.3, NM_000984.5, NM_000985.4, NM_001035006.2, NM_001199340.1, NM_001199341.1, NM_001199342.1, NM_001199343.1, NM_001199344.1, NM_001199345.1, NM_000986.3, NM_000987.3, NM_000988.3, NM_000989.3, NM_000990.4, NM_001136134.1, NM_000991.4, NM_001136135.1, NM_001136136.1, NM_001136137.1, NM_000992.2, NM_000993.4, NM_001098577.2, NM_001099693.1, NM_000994.3,

NM_001007073.1, NM_001007074.1, NM_000996.2, NM_000997.4, NM_000998.4, NM_000999.3, NM_001035258.1, NM_001000.3, NM_001002.3, NM_053275.3, NM_001003.2, NM_213725.1, NM_001004.3 , NM_001005.4, NM_001256802.1, NM_001260506.1, NM_001260507.1 , NM_001006.4, NM_001267699.1, NM_001007.4, NM_001008.3, NM_001009.3, NM_001010.2, NM_001011.3, NM_001012.1, NM_001013.3, NM_001203245.2, NM_001014.4, NM_001204091.1, NM_001015.4, NM_001016.3, NM_001017.2, NM_001018.3, NM_001030009.1, NM_001019.4, NM_001020.4, NM_001022.3, NM_001146227.1, NM_001023.3, NM_001024.3, NM_001025.4, NM_001028.2, NM_001029.3, NM_001030.4, NM_002954, NM_001135592.2, NM_001177413.1, NM_001031.4, NM_001032.4, NM_001030001.2, NM_002948.3, NM_001253379.1, NM_001253380.1, NM_001253382.1, NM_001253383.1, NM_001253384.1, NM_002952.3, NM_001034996.2, NM_001025071.1, NM_001025070.1, NM_005617.3, NM_006013.3, NM_001256577.1, NM_001256580.1, NM_007104.4, NM_007209.3, NM_012423.3, NM_001270491.1, NM_033643.2, NM_015414.3, NM_021029.5, NM_001199972.1, NM_021104.1, NM_022551.2, NM_033022.3, NM_001142284.1, NM_001026.4, NM_001142285.1, NM_001142283.1, NM_001142282.1, NM_000973.3, NM_033301.1, NM_000995.3, NM_033625.2, NM_001021.3, NM_002295.4, NM_001012321.1, NM_001033930.1, NM_003333.3, NM_001997.4, NM_001099645.1, NM_001021.3, NM_052969.1, NM_080746.2, NM_001001.4 , NM_005061.2 , NM_015920.3 , NM_016093.2 , NM_198486.2 , NG_011172.1, NG_011253.1, NG_000952.4, NR_002309.1, NG_010827.2, NG_009952.2, NG_009517.1, NM_052835.3, NM_011287.2, NM_001162933.1, NM_009076.3, NM_009438.5, NM_025974.2, NM_025586.3, NM_001002239.3, NM_009077.2, NM_029751.4, NM_009078.2, NM_019647.6, NM_009079.3, NM_022891.3, NM_024218.4, NM_011975.3, NM_009081.2, NM_009082.2, NM_009083.4, NM_053257.3, ENSMUST00000081840 (NM_172086.2), NM_026724.2, NM_025592.3, NM_025589.4, NM_026069.3, NM_009084, NM_026055.1, NM_026594.2, NM_001163945.1, NM_024212.4, NM_016980.2, NM_011290.5, NM_011291.5, ENSMUST00000102898 (NM 013721.3), NM_025433.3, NM_012053.2, NM_011292.2, NM_007475.5, NM_018853.3, NM_026020.6, NM_025963.3, NM_013725.4, NM_011295.6, NM_020600.4, NM_009091.2, NM_170669.2, NM_013647.2, NM_009092.3, NM_008503.5, NM_026147.5 / ENSMUST00000138502 , NM_207635.1, NM_024266.3, NM_013765.2, NM_024277.2, NM_026467.3, NM_012052.2, NM_016959.4, ENSMUST00000071745, NM_009095.2, NM_009096.3, NM_011300.3, NM_011029.4, NM_018860.4, NM_001277113.1, NM_001277114.1, NM_001271590.1, NM_007990, NM_025919, NM_016738, NM_026517, NM_207523, NM_009080, NM_011289, NM_013762, NM_021338 , NM_018730, NM_019865, NM_023372.2, NM_026533.3, NM_009092, NM_011296, NM_023133, ENSMUST00000059080 (NM_025587.2), NM_024175, NM_027015, NM_016844, NM_009093.2, NM_009094, NM_009098, NM_029767, and NM_019883.

The phrase "nucleic acid sequence which is derived from the 3'-UTR of a ribosomal protein gene" preferably refers to a nucleic acid sequence, which is based on the 3'-UTR sequence of a ribosomal protein gene or on a fragment or part thereof. This phrase includes sequences corresponding to the entire 3'-UTR sequence, i.e. the full length 3'-UTR sequence of a ribosomal protein gene, and sequences corresponding to a fragment of the 3'-UTR sequence of a ribosomal protein gene. Preferably, a fragment of a 3'-UTR of a ribosomal protein gene consists of a continuous stretch of nucleotides corresponding to a continuous stretch of nucleotides in the full-length 3'-UTR of a ribosomal protein gene, which represents at least 5%, 10%, 20%, preferably at least 30%, more preferably at least 40%, more preferably at least 50%, even more preferably at least 60%, even more preferably at least 70%, even more preferably at least 80%, and most preferably at least 90% of the full-length 3'-UTR of a ribosomal protein gene. Such a fragment, in the sense of the present invention, is preferably a functional fragment as described herein. Preferably, the fragment retains a regulatory function for the translation of the ORF linked to the 3'-UTR or fragment thereof.

The term "3'-UTR of a ribosomal protein gene" preferably refers to the 3'-UTR of a naturally occurring ribosomal protein gene.

The terms "variant of the 3'-UTR of a ribosomal protein gene" and "variant thereof" in the context of a 3'-UTR of a ribosomal protein gene refers to a variant of the 3'-UTR of a naturally occurring ribosomal protein gene, preferably to a variant of the 3'-UTR of a vertebrate ribosomal protein gene, more preferably to a variant of the 3'-UTR of a mammalian ribosomal protein gene, even more preferably to a variant of the 3'-UTR of a primate ribosomal protein gene, in particular a human ribosomal protein gene as described above. Such variant may be a modified 3'-UTR of a ribosomal protein gene. For example, a variant 3'-UTR may exhibit one or more nucleotide deletions, insertions, additions and/or substitutions compared to the naturally occurring 3'-UTR from which the variant is derived. Preferably, a variant of a 3'-UTR of a ribosomal protein gene is at least 40%, preferably at least 50%, more preferably at least 60%, more preferably at least 70%, even more preferably at least 80%, even more preferably at least 90%, most preferably at least 95% identical to the naturally occurring 3'-UTR the variant is derived from. Preferably, the variant is a functional variant as described herein.

The phrase "a nucleic acid sequence which is derived from a variant of the 3'-UTR of a ribosomal protein gene" preferably refers to a nucleic acid sequence which is based on a variant of the 3'-UTR sequence of a ribosomal protein gene or on a fragment or part thereof as described above. This phrase includes sequences corresponding to the entire sequence of the variant of the 3'-UTR of a ribosomal protein gene, i.e. the full length variant 3'-UTR sequence of a ribosomal protein gene, and sequences corresponding to a fragment of the variant 3'-UTR sequence of a ribosomal

protein gene. Preferably, a fragment of a variant of the 3'-UTR of a ribosomal protein gene consists of a continuous stretch of nucleotides corresponding to a continuous stretch of nucleotides in the full-length variant of the 3'-UTR of a ribosomal protein gene, which represents at least 20%, preferably at least 30%, more preferably at least 40%, more preferably at least 50%, even more preferably at least 60%, even more preferably at least 70%, even more preferably at least 80%, and most preferably at least 90% of the full-length variant of the 3'-UTR of a ribosomal protein gene. Such a fragment of a variant, in the sense of the present invention, is preferably a functional fragment of a variant as described herein.

The terms "functional variant", "functional fragment", and "functional fragment of a variant" (also termed "functional variant fragment") in the context of the present invention, mean that the fragment of the 3'-UTR, the variant of the 3'-UTR, or the fragment of a variant of the 3'-UTR of a ribosomal protein gene fulfils at least one, preferably more than one function of the naturally occurring 3'-UTR of a ribosomal protein gene of which the variant, the fragment, or the fragment of a variant is derived. Such function may be, for example, stabilizing mRNA and/or enhancing, stabilizing and/or prolonging protein production from an mRNA and/or increasing protein expression or total protein production from an mRNA, preferably in a mammalian cell, such as in a human cell. Preferably, the function of the 3'-UTR concerns the translation of the protein encoded by the ORF. More preferably, the function comprises enhancing translation efficiency of the ORF linked to the 3'-UTR or fragment or variant thereof. It is particularly preferred that the variant, the fragment, and the variant fragment in the context of the present invention fulfil the function of stabilizing an mRNA, preferably in a mammalian cell, such as a human cell, compared to an mRNA comprising a reference 3'-UTR or lacking a 3'-UTR, and/or the function of enhancing, stabilizing and/or prolonging protein production from an mRNA, preferably in a mammalian cell, such as in a human cell, compared to an mRNA comprising a reference 3'-UTR or lacking a 3'-UTR, and/or the function of increasing protein production from an mRNA, preferably in a mammalian cell, such as in a human cell, compared to an mRNA comprising a reference 3'-UTR or lacking a 3'-UTR. A reference 3'-UTR may be, for example, a 3'-UTR naturally occurring in combination with the ORF. Furthermore, a functional variant, a functional fragment, or a functional variant fragment of a 3'-UTR of a ribosomal protein gene preferably does not have a substantially diminishing effect on the efficiency of translation of the mRNA which comprises such variant, fragment, or variant fragment of a 3'-UTR compared to the wild type 3'-UTR from which the variant, the fragment, or the variant fragment is derived. A particularly preferred function of a "functional fragment", a "functional variant" or a "functional fragment of a variant" of the 3'-UTR of a ribosomal protein gene in the context of the present invention is the enhancement, stabilization and/or prolongation of protein production by expression of an mRNA carrying the functional fragment, functional variant or functional fragment of a variant as described above.

Preferably, the efficiency of the one or more functions exerted by the functional variant, the functional fragment, or the functional variant fragment, such as mRNA and/or protein production stabilizing efficiency and/or the protein production increasing efficiency, is increased by at least 5%, more preferably by at least 10%, more preferably by at least 20%, more preferably by at least 30%, more preferably by at least 40%, more preferably by at least 50%, more preferably by at least 60%, even more preferably by at least 70%, even more preferably by at least 80%, most preferably by at least 90% with respect to the mRNA and/or protein production stabilizing efficiency and/or the protein production increasing efficiency exhibited by the naturally occurring 3'-UTR of a ribosomal protein gene from which the variant, the fragment or the variant fragment is derived.

In the context of the present invention, a fragment of the 3'-UTR of a ribosomal protein gene or of a variant of the 3'-UTR of a ribosomal protein gene preferably exhibits a length of at least about 3 nucleotides, preferably of at least about 5 nucleotides, more preferably of at least about 10, 15, 20, 25 or 30 nucleotides, even more preferably of at least about 50 nucleotides, most preferably of at least about 70 nucleotides. Preferably, such fragment of the 3'-UTR of a ribosomal protein gene or of a variant of the 3'-UTR of a ribosomal protein gene is a functional fragment as described above. In a preferred embodiment, the 3'-UTR of a ribosomal protein gene or a fragment or variant thereof exhibits a length of between 3 and about 500 nucleotides, preferably of between 5 and about 150 nucleotides, more preferably of between 10 and 100 nucleotides, even more preferably of between 15 and 90, most preferably of between 20 and 70.

Preferably, the at least one 3'-UTR element of the artificial nucleic acid molecule according to the present invention comprises or consists of a "functional fragment", a "functional variant" or a "functional fragment of a variant" of the 3'-UTR of a ribosomal protein gene.

In a preferred embodiment, the at least one 3'-UTR element of the artificial nucleic acid molecule according to the present invention increases the stability of the artificial nucleic acid molecule, e.g. increases the stability of an mRNA according to the present invention, compared to a respective nucleic acid (reference nucleic acid) lacking a 3'-UTR or comprising a reference 3'-UTR, such as a 3'-UTR naturally occurring in combination with the ORF. Preferably, the at least one 3'-UTR element of the artificial nucleic acid molecule according to the present invention increases the stability of protein production from the artificial nucleic acid molecule according to the present invention, e.g. from an mRNA according to the present invention, compared to a respective nucleic acid lacking a 3'-UTR or comprising a reference 3'-UTR, such as a 3'-UTR naturally occurring in combination with the ORF. Preferably, the at least one 3'-UTR element of the artificial nucleic acid molecule according to the present invention prolongs protein production from the artificial

nucleic acid molecule according to the present invention, e.g. from an mRNA according to the present invention, compared to a respective nucleic acid lacking a 3'-UTR or comprising a reference 3'-UTR, such as a 3'-UTR naturally occurring in combination with the ORF. Preferably, the at least one 3'-UTR element of the artificial nucleic acid molecule according to the present invention increases the protein expression and/or total protein production from the artificial nucleic acid molecule according to the present invention, e.g. from an mRNA according to the present invention, compared to a respective nucleic acid lacking a 3'-UTR or comprising a reference 3'-UTR, such as a 3'-UTR naturally occurring in combination with the ORF. Preferably, the at least one 3'-UTR element of the artificial nucleic acid molecule according to the present invention does not negatively influence translational efficiency of an nucleic acid compared to the translational efficiency of a respective nucleic acid lacking a 3'-UTR or comprising a reference 3'-UTR, such as a 3'-UTR naturally occurring in combination with the ORF. Even more preferably, the translation efficiency is enhanced by the 3'-UTR in comparison to the translation efficiency of the protein encoded by the respective ORF in its natural context.

The term "respective nucleic acid molecule" or "reference nucleic acid molecule" in this context means that - apart from the different 3'-UTRs - the reference nucleic acid molecule is comparable, preferably identical, to the inventive artificial nucleic acid molecule comprising the 3'-UTR element.

The term "stabilizing and/or prolonging protein production" from an artificial nucleic acid molecule such as an artificial mRNA preferably means that the protein production from the artificial nucleic acid molecule such as the artificial mRNA is stabilized and/or prolonged compared to the protein production from a reference nucleic acid molecule such as a reference mRNA, e.g. comprising a reference 3'-UTR or lacking a 3'-UTR, preferably in a mammalian expression system, such as in HeLa or HDF cells. Thus, protein produced from the artificial nucleic acid molecule such as the artificial mRNA is observable for a longer period of time than what may be seen for a protein produced from a reference nucleic acid molecule. In other words, the amount of protein produced from the artificial nucleic acid molecule such as the artificial mRNA measured over time undercuts a threshold value at a later time point than the amount of protein produced from a reference nucleic acid molecule such as a reference mRNA measured over time. Such a threshold value may be, for example, the amount of protein measured in the initial phase of expression, such as 1, 2, 3, 4, 5 or 6 hours post initiation of expression, such as post transfection of the nucleic acid molecule.

For example, the protein production from the artificial nucleic acid molecule such as the artificial mRNA - in an amount which is at least the amount observed in the initial phase of expression, such as 1, 2, 3, 4, 5, or 6 hours post initiation of expression, such as post transfection of the nucleic acid molecule - is prolonged by at least about 5 hours, preferably by at least about 10 hours, more preferably by at least about 24 hours compared to the protein production from a reference nucleic acid molecule, such as a reference mRNA, in a mammalian expression system, such as in mammalian cells, e.g. in HeLa or HDF cells. Thus, the artificial nucleic acid molecule according to the present invention preferably allows for prolonged protein production in an amount which is at least the amount observed in the initial phase of expression, such as 1, 2, 3, 4, 5, or 6 hours post initiation of expression, such as post transfection, by at least about 5 hours, preferably by at least about 10 hours, more preferably by at least about 24 hours compared to a reference nucleic acid molecule lacking a 3'-UTR or comprising a reference 3'-UTR.

In preferred embodiments, the period of protein production from the artificial nucleic acid molecule according to the present invention is extended at least 1.5 fold, preferably at least 2 fold, more preferably at least 2.5 fold compared to the protein production from a reference nucleic acid molecule lacking a 3'-UTR or comprising a reference 3'-UTR.

This effect of prolonging protein production may be determined by (i) measuring protein amounts, e.g. obtained by expression of an encoded reporter protein such as luciferase, preferably in a mammalian expression system such as in HeLa or HDF cells, over time, (ii) determining the time point at which the protein amount undercuts the amount of protein observed, e.g., at 1, 2, 3, 4, 5, or 6 hours post initiation of expression, e.g. 1, 2, 3, 4, 5, or 6 hours post transfection of the artificial nucleic acid molecule, and (iii) comparing the time point at which the protein amount undercuts the protein amount observed at 1, 2, 3, 4, 5, or 6 hours post initiation of expression to said time point determined for a nucleic acid molecule lacking a 3'-UTR or comprising a reference 3'-UTR.

Preferably, this stabilizing and/or prolonging effect on protein production is achieved, while the total amount of protein produced from the artificial nucleic acid molecule according to the present invention, e.g. within a time span of 48 or 72 hours, is at least the amount of protein produced from a reference nucleic acid molecule lacking a 3'-UTR or comprising a reference 3'-UTR, such as a 3'-UTR naturally occurring with the ORF of the artificial nucleic acid molecule. Thus, the present invention provides an artificial nucleic acid molecule which allows for prolonged and/or stabilized protein production in a mammalian expression system, such as in mammalian cells, e.g. in HeLa or HDF cells, as specified above, wherein the total amount of protein produced from said artificial nucleic acid molecule, e.g. within a time span of 48 or 72 hours, is at least the total amount of protein produced, e.g. within said time span, from a reference nucleic acid molecule lacking a 3'-UTR or comprising a reference 3'-UTR, such as a 3'-UTR naturally occurring with the ORF of the artificial nucleic acid molecule.

Thus, "stabilized protein expression" preferably means that there is more uniform protein production from the artificial nucleic acid molecule according to the present invention over a predetermined period of time, such as over 24 hours, more preferably over 48 hours, even more preferably over 72 hours, when compared to a reference nucleic acid

molecule, for example, an mRNA comprising a reference 3'-UTR or lacking a 3'-UTR. Accordingly, the level of protein production, e.g. in a mammalian system, from the artificial nucleic acid molecule comprising a 3'-UTR element according to the present invention, e.g. from an mRNA according to the present invention, preferably does not drop to the extent observed for a reference nucleic acid molecule, such as a reference mRNA as described above. For example, the amount of a protein (encoded by the ORF) observed 6 hours after initiation of expression, e.g. 6 hours post transfection of the artificial nucleic acid molecule according to the present invention into a cell, such as a mammalian cell, may be comparable to the amount of protein observed 48 hours after initiation of expression, e.g. 48 hours post transfection. Thus, the ratio of the amount of protein encoded by the ORF, such as of a reporter protein, e.g., luciferase, observed at 48 hours post initiation of expression, e.g. 48 hours post transfection, to the amount of protein observed 6 hours after initiation of expression, e.g. 6 hours post transfection, is preferably at least about 0.4, more preferably at least about 0.5, more preferably at least about 0.6, even more preferably at least about 0.7. Preferably, the ratio is between about 0.4 and about 4, preferably between about 0.65 and about 3, more preferably between about 0.7 and 2 for a nucleic acid molecule according to the present invention. For a respective reference nucleic acid molecule, e.g. an mRNA comprising a reference 3'-UTR or lacking a 3'-UTR, said ratio may be, e.g. between about 0.05 and about 0.3.

Thus, the present invention provides an artificial nucleic acid molecule comprising an ORF and a 3'-UTR element as described above, wherein the ratio of the (reporter) protein amount, e.g. the amount of luciferase, observed 48 hours after initiation of expression to the (reporter) protein amount observed 6 hours after initiation of expression, preferably in a mammalian expression system, such as in mammalian cells, e.g. in HeLa cells, is preferably above about 0.4, more preferably above about 0.5, more preferably above about 0.6, even more preferably above about 0.7, e.g. between about 0.4 and about 4, preferably between about 0.65 and about 3, more preferably between about 0.7 and 2, wherein preferably the total amount of protein produced from said artificial nucleic acid molecule, e.g. within a time span of 48 hours, is at least the total amount of protein produced, e.g. within said time span, from a reference nucleic acid molecule lacking a 3'-UTR or comprising a reference 3'-UTR, such as a 3'-UTR naturally occurring with the ORF of the artificial nucleic acid molecule. In a preferred embodiment, the present invention provides an artificial nucleic acid molecule comprising an ORF and a 3'-UTR element as described above, wherein the ratio of the (reporter) protein amount, e.g. the amount of luciferase, observed 72 hours after initiation of expression to the (reporter) protein amount observed 6 hours after initiation of expression, preferably in a mammalian expression system, such as in mammalian cells, e.g. in HeLa cells, is preferably above about 0.4, more preferably above about 0.5, more preferably above about 0.6, even more preferably above about 0.7, e.g. between about 0.4 and 1.5, preferably between about 0.65 and about 1.15, more preferably between about 0.7 and 1.0, wherein preferably the total amount of protein produced from said artificial nucleic acid molecule, e.g. within a time span of 72 hours, is at least the total amount of protein produced, e.g. within said time span, from a reference nucleic acid molecule lacking a 3'-UTR or comprising a reference 3'-UTR, such as a 3'-UTR naturally occurring with the ORF of the artificial nucleic acid molecule.

"Increased protein expression" or "enhanced protein expression" in the context of the present invention preferably means an increased/enhanced protein expression at one time point after initiation of expression or an increased/enhanced total amount of expressed protein compared to the expression induced by a reference nucleic acid molecule. Thus, the protein level observed at a certain time point after initiation of expression, e.g. after transfection, of the artificial nucleic acid molecule according to the present invention, e.g. after transfection of an mRNA according to the present invention, for example, 6, 12, 24, 48 or 72 hours post transfection, is preferably higher than the protein level observed at the same time point after initiation of expression, e.g. after transfection, of a reference nucleic acid molecule, such as a reference mRNA comprising a reference 3'-UTR or lacking a 3'-UTR. In a preferred embodiment, the maximum amount of protein (as determined e.g. by protein activity or mass) expressed from the artificial nucleic acid molecule is increased with respect to the protein amount expressed from a reference nucleic acid comprising a reference 3'-UTR or lacking a 3'-UTR. Peak expression levels are preferably reached within 48 hours, more preferably within 24 hours and even more preferably within 12 hours after, for instance, transfection.

In one embodiment, "increased total protein production" or "enhanced total protein production" from an artificial nucleic acid molecule according to the invention refers to an increased/enhanced protein production over the time span, in which protein is produced from an artificial nucleic acid molecule, preferably in a mammalian expression system, such as in mammalian cells, e.g. in HeLa or HDF cells in comparison to a reference nucleic acid molecule lacking a 3'-UTR or comprising a reference 3'-UTR. According to a preferred embodiment, the cumulative amount of protein expressed over time is increased when using the artificial nucleic acid molecule according to the invention.

According to the invention, an artificial nucleic acid molecule is provided, which is characterized by increased expression of the encoded protein in comparison to a respective nucleic acid molecule lacking the at least one 3'-UTR element or comprising a reference 3'-UTR ("reference nucleic acid") comprising a nucleic acid sequence which is derived from the 3'-UTR of a ribosomal protein gene or from a variant of the 3'-UTR of a ribosomal protein gene In order to assess the *in vivo* protein production by the inventive artificial nucleic acid molecule, the expression of the encoded protein is determined following injection/transfection of the inventive artificial nucleic acid molecule into target cells/tissue and compared to the protein expression induced by the reference nucleic acid. Quantitative methods for determining

protein expression are known in the art (e.g. Western-Blot, FACS, ELISA, mass spectometry). Particularly useful in this context is the determination of the expression of reporter proteins like luciferase, Green fluorescent protein (GFP), or secreted alkaline phosphatase (SEAP). Thus, an artificial nucleic acid according to the invention or a reference nucleic acid is introduced into the target tissue or cell, e.g. via transfection or injection. Several hours or several days (e.g. 6, 12, 24, 48 or 72 hours) post initiation of expression or post introduction of the nucleic acid molecule, a target cell sample is collected and measured via FACS and/or lysed. Afterwards the lysates can be used to detect the expressed protein (and thus determine the efficiency of protein expression) using several methods, e.g. Western-Blot, FACS, ELISA, mass spectrometry or by fluorescence or luminescence measurement.

Therefore, if the protein expression from an artificial nucleic acid molecule according to the invention is compared to the protein expression from a reference nucleic acid molecule at a specific time point (e.g. 6, 12, 24, 48 or 72 hours post initiation of expression or post introduction of the nucleic acid molecule), both nucleic acid molecules are introduced separately into target tissue/cells, a sample from the tissue/cells is collected after a specific time point, protein lysates are prepared according to the particular protocol adjusted to the particular detection method (e.g. Western Blot, ELISA, etc. as known in the art) and the protein is detected by the chosen detection method. As an alternative to the measurement of expressed protein amounts in cell lysates - or, in addition to the measurement of protein amounts in cell lysates prior to lysis of the collected cells or using an aliquot in parallel - protein amounts may also be determined by using FACS analysis.

If the total amount of protein for a specific time period is to be measured, tissue or cells can be collected after several time points after introduction of the artificial nucleic acid molecule (e.g. 6, 12, 24, 48 and 72 hours post initiation of expression or post introduction of the nucleic acid molecule; usually from different test animals), and the protein amount per time point can be determined as explained above. In order to calculate the cumulative protein amount, a mathematical method of determining the total amount of protein can be used, e.g. the area under the curve (AUC) can be determined according to the following formula:

$$\mathrm{AUC} = \int_a^b f(x)\, d(x)$$

In order to calculate the area under the curve for total amount of protein, the integral of the equation of the expression curve from each end point (a and b) is calculated.

Thus, "total protein production" preferably refers to the area under the curve (AUC) representing protein production over time.

Said increase in stability of the artificial nucleic acid molecule, said increase in stability of protein production, said prolongation of protein production and/or said increase/enhancement in protein expression and/or total protein production is preferably determined by comparison with a respective reference nucleic acid molecule lacking a 3'-UTR, e.g. an mRNA lacking a 3'-UTR, or a reference nucleic acid molecule comprising a reference 3'-UTR, such as a 3'-UTR naturally occurring with the ORF as describe above.

The mRNA and/or protein production stabilizing effect and efficiency and/or the protein production increasing effect and efficiency of the variants, fragments and/or variant fragments of the 3'-UTR of a ribosomal protein gene as well as the mRNA and/or protein production stabilizing effect and efficiency and/or the protein production increasing effect and efficiency of the at least one 3'-UTR element of the artificial nucleic acid molecule according to the present invention may be determined by any method suitable for this purpose known to skilled person. For example, artificial mRNA molecules may be generated comprising a coding sequence/open reading frame (ORF) for a reporter protein, such as luciferase, and no 3'-UTR, a 3'-UTR derived from a naturally occurring ribosomal protein gene, a 3'-UTR derived from a reference gene (i.e., a reference 3'-UTR, such as a 3'-UTR naturally occurring with the ORF), as 3'-UTR a variant of a 3'-UTR of a ribosomal protein gene, as 3'-UTR a fragment of a naturally occurring ribosomal protein gene, or as 3'-UTR a fragment of a variant of a 3'-UTR of a ribosomal protein gene. Such mRNAs may be generated, for example, by *in vitro* transcription of respective vectors such as plasmid vectors, e.g. comprising a T7 promoter and a sequence encoding the respective mRNA sequences. The generated mRNA molecules may be transfected into cells by any transfection method suitable for transfecting mRNA, for example they may be electroporated into mammalian cells, such as HELA cells, and samples may be analyzed certain time points after transfection, for example, 6 hours, 24 hours, 48 hours, and 72 hours post transfection. Said samples may be analyzed for mRNA quantities and/or protein quantities by methods well known to the skilled person. For example, the quantities of reporter mRNA present in the cells at the sample time points may be determined by quantitative PCR methods. The quantities of reporter protein encoded by the respective mRNAs may be determined, e.g., by Western Blot, ELISA assays, FACS analysis or reporter assays, such

as luciferase assays, depending on the reporter protein used. The effect of stabilizing protein expression and/or prolonging protein expression may be, for example, analyzed by determining the ratio of the protein level observed 48 hours post transfection and the protein level observed 6 hours post transfection. Preferably, the value of that ratio is greater than 1, i.e. the protein expression at the later time point is greater than the protein expression at the earlier time point. If the value for that ratio is lower than 1, the protein is more stable the closer said value is to 1. Such measurements may, of course, also be performed at 72 or more hours and the ratio of the protein level observed 72 hours post transfection and the protein level observed 6 hours post transfection may be determined to determine stability of protein expression.

In a preferred embodiment, the 3'-UTR element of the artificial nucleic acid molecule according to the present invention is derived from the 3'-UTR region of a gene encoding a ribosomal protein, preferably from the 3'-UTR region of ribosomal protein L9 (RPL9), ribosomal protein L3 (RPL3), ribosomal protein L4 (RPL4), ribosomal protein L5 (RPL5), ribosomal protein L6 (RPL6), ribosomal protein L7 (RPL7), ribosomal protein L7a (RPL7A), ribosomal protein L11 (RPL11), ribosomal protein L12 (RPL12), ribosomal protein L13 (RPL13), ribosomal protein L23 (RPL23), ribosomal protein L18 (RPL18), ribosomal protein L18a (RPL18A), ribosomal protein L19 (RPL19), ribosomal protein L21 (RPL21), ribosomal protein L22 (RPL22), ribosomal protein L23a (RPL23A), ribosomal protein L17 (RPL1 7), ribosomal protein L24 (RPL24), ribosomal protein L26 (RPL26), ribosomal protein L27 (RPL27), ribosomal protein L30 (RPL30), ribosomal protein L27a (RPL27A), ribosomal protein L28 (RPL28), ribosomal protein L29 (RPL29), ribosomal protein L31 (RPL31), ribosomal protein L32 (RPL32), ribosomal protein L35a (RPL35A), ribosomal protein L37 (RPL37), ribosomal protein L37a (RPL37A), ribosomal protein L38 (RPL38), ribosomal protein L39 (RPL39), ribosomal protein, large, P0 (RPLP0), ribosomal protein, large, P1 (RPLP1), ribosomal protein, large, P2 (RPLP2), ribosomal protein S3 (RPS3), ribosomal protein S3A (RPS3A), ribosomal protein S4, X- linked (RPS4X), ribosomal protein S4, Y-linked 1 (RPS4Y1), ribosomal protein S5 (RPS5), ribosomal protein S6 (RPS6), ribosomal protein S7 (RPS7), ribosomal protein S8 (RPS8), ribosomal protein S9 (RPS9), ribosomal protein S10 (RPS10), ribosomal protein S11 (RPS11), ribosomal protein S12 (RPS12), ribosomal protein S13 (RPS13), ribosomal protein S15 (RPS15), ribosomal protein S15a (RPS15A), ribosomal protein S16 (RPS16), ribosomal protein S19 (RPS19), ribosomal protein S20 (RPS20), ribosomal protein S21 (RPS21), ribosomal protein S23 (RPS23), ribosomal protein S25 (RPS25), ribosomal protein S26 (RPS26), ribosomal protein S27 (RPS27), ribosomal protein S27a (RPS27a), ribosomal protein S28 (RPS28), ribosomal protein S29 (RPS29), ribosomal protein L15 (RPL15), ribosomal protein S2 (RPS2), ribosomal protein L14 (RPL14), ribosomal protein S14 (RPS14), ribosomal protein L10 (RPL10), ribosomal protein L10a (RPL10A), ribosomal protein L35 (RPL35), ribosomal protein L13a (RPL13A), ribosomal protein L36 (RPL36), ribosomal protein L36a (RPL36A), ribosomal protein L41 (RPL41), ribosomal protein S18 (RPS18), ribosomal protein S24 (RPS24), ribosomal protein L8 (RPL8), ribosomal protein L34 (RPL34), ribosomal protein S17 (RPS17), ribosomal protein SA (RPSA) or ribosomal protein S17 (RPS17). In an alternative embodiment, the 3'-UTR element may be derived from a gene encoding a ribosomal protein or from a gene selected from ubiquitin A-52 residue ribosomal protein fusion product 1 (UBA52), Finkel-Biskis-Reilly murine sarcoma virus (FBR-MuSV) ubiquitously expressed (FAU), ribosomal protein L22-like 1 (RPL22L1), ribosomal protein L39-like (RPL39L), ribosomal protein L10-like (RPL10L), ribosomal protein L36a-like (RPL36AL), ribosomal protein L3-like (RPL3L), ribosomal protein S27-like (RPS27L), ribosomal protein L26-like 1 (RPL26L1), ribosomal protein L7-like 1 (RPL7L1), ribosomal protein L13a pseudogene (RPL13AP), ribosomal protein L37a pseudogene 8 (RPL37AP8), ribosomal protein S10 pseudogene 5 (RPS10P5), ribosomal protein S26 pseudogene 11 (RPS26P11), ribosomal protein L39 pseudogene 5 (RPL39P5), ribosomal protein, large, P0 pseudogene 6 (RPLP0P6) and ribosomal protein L36 pseudogene 14 (RPL36P14). Furthermore, the 3'-UTR element of the artificial nucleic acid molecule according to the present invention is preferably derived from the 3'-UTR region of a gene selected from the group consisting of ribosomal protein S4-like (RPS4l), putative 60S ribosomal protein L13a, putative 60S ribosomal protein L37a-like protein, putative 40S ribosomal protein S10-like, putative 40S ribosomal protein S26-like 1, putative 60S ribosomal protein L39-like 5, or 60S acidic ribosomal protein P0-like.

In a particularly preferred embodiment, the 3'-UTR element of the artificial nucleic acid molecule according to the present invention is derived from the 3'-UTR region of a gene encoding a ribosomal protein, preferably from the 3'-UTR region of ribosomal protein L3 (RPL3), ribosomal protein L11 (RPL11), ribosomal protein L13 (RPL13), ribosomal protein L23 (RPL23), ribosomal protein L23a (RPL23A), ribosomal protein L26 (RPL26), ribosomal protein L27 (RPL27), ribosomal protein L35a (RPL35A), ribosomal protein L38 (RPL38), ribosomal protein S4, X- linked (RPS4X), ribosomal protein S8 (RPS8), ribosomal protein S9 (RPS9), ribosomal protein S13 (RPS13), ribosomal protein S19 (RPS19), ribosomal protein S21 (RPS21), ribosomal protein S23 (RPS23), ribosomal protein S27 (RPS27), ribosomal protein S28 (RPS28), ribosomal protein S29 (RPS29), ribosomal protein L36 (RPL36), ribosomal protein L36a (RPL36A), ribosomal protein S18 (RPS18) or ribosomal protein S17 (RPS17). In another preferred embodiment, the 3'-UTR element may be derived from a gene encoding a ribosomal protein or from a gene selected from ubiquitin A-52 residue ribosomal protein fusion product 1 (UBA52), Finkel-Biskis-Reilly murine sarcoma virus (FBR-MuSV) ubiquitously expressed (FAU) and ribosomal protein L22-like 1 (RPL22L1).

Preferably, the at least one 3'-UTR element of the artificial nucleic acid molecule according to the present invention comprises or consists of a nucleic acid sequence which has an identity of at least about 1, 2, 3, 4, 5, 10, 15,

20, 30 or 40%, preferably of at least about 50%, preferably of at least about 60%, preferably of at least about 70%, more preferably of at least about 80%, more preferably of at least about 90%, even more preferably of at least about 95%, even more preferably of at least about 99%, most preferably of 100% to the nucleic acid sequence of a 3'-UTR of a ribosomal protein gene, such as to the nucleic acid sequences according to SEQ ID NOs:10 to 115 or the corresponding RNA sequence:

Homo sapiens ribosomal protein L9 (RPL9)

gatctaagagttacctggctacagaaagaagatgccagatgacacttaagacctacttgtgatatt
taaatgatgcaataaaagacctattgatttggaccttcttctt
(SEQ ID NO:10)

Homo sapiens ribosomal protein L3 (RPL3)
tgccaggaacagattttgcagttggtggggtctcaataaaagttattttccactgac
(SEQ ID NO:11)

Homo sapiens ribosomal protein L4 (RPL4)

actcttaaatttgattattccataaaggtcaaatcattttggacagcttcttttgaataaagacct
gattatacaggcagtgagaaacatg
(SEQ ID NO:12)

Homo sapiens ribosomal protein L5 (RPL5)
acccagcaattttctatgatttttcagatatagataataaacttatgaacagcaact
(SEQ ID NO:13)

Homo sapiens ribosomal protein L6 (RPL6)
atgtcttaagaacctaattaaatagctgactac
(SEQ ID NO:14)

Homo sapiens ribosomal protein L7 (RPL7)

ggtgtctaccatgattattttctaagctggttggttaataaacagtacctgctctcaaattgaaa
t
(SEQ ID NO:15)

Homo sapiens ribosomal protein L7a (RPL7A)
atgtacactgttgagttttctgtacataaaaataattgaaataatacaaattttccttc
(SEQ ID NO:16)

Homo sapiens ribosomal protein L11 (RPL11)
attcccgtttctatccaaaagagcaataaaaagttttcagtgaaatgtgc
(SEQ ID NO:17)

Homo sapiens ribosomal protein L12 (RPL12)
gcacaaaggaaaacatttcaataaaggatcatttgacaactggtgg
(SEQ ID NO:18)

Homo sapiens ribosomal protein L13 (RPL13)

agccctcctggggacttggaatcagtcggcagtcatgctgggtctccacgtggtgtgtttcgtggg
aacaactgggcctgggatggggcttcactgctgtgacttcctcctgccaggggatttggggctttc
ttgaaagacagtccaagccctggataatgctttactttctgtgttgaagcactgttggttgtttgg
ttagtgactgatgtaaaacggttttcttgtggggaggttacagaggctgacttcagagtggacttg
tgtttttttctttttaaagaggcaaggttgggctggtgctcacagctgtaatcccagcactttgagg
ttggctgggagttcaagaccagcctggccaacatgtcagaactactaaaaataaagaaatcagcca

tgcttggtgctgcacacttgtagttgcagctcctgggaggcagaggtgagggatcacttaacccag
gaggcagaggctgcactgagccaggatcacgccactgcactctagcctgggcaacagtgagactgt
ctcaaaaaaaaaaaaagagacagggtcttcggcacccaggctggagtacagtgccacaatcatggc
tcactgcagtcttgaactcatggcctcaagcagtcctccctcagcctcccaagtagaggggtttat
aggcacgagaccctgcacccaacctagagttgcctttttaagcaaagcagtttctagttaatgta
gcatcttggactttggggcgtcattcttaagcttgttgtgcccggtaaccatggtcctcttgctct
gattaacccttccttcaatgggcttcttcacccagacaccaaggtatgagatggccctgccaagtg
tcggcctctcctgttaaacaaaaacattctaaagccattgttcttgcttcatggacaagaggcagc
cagagagagtgccagggtgccctggtctgagctggcatccccatgtcttctgtgtccgagggcagc
atggtttctcgtgcagtgctcagacacagcctgccctagtcctaccagctcacagcagcacctgct
ctccttggcagctatggccatgacaaccccagagaagcagcttcagggaccgagtcagattctgtt
ttgtctacatgcctctgccgggtgccggtattgaggcacccagggagctgttactggcgtggaaat
aggtgatgctgctacctctgctgctgcactcacagccacacttgatacacgatgacaccttgcttg
tttggaaacatctaaacatctagtagatgacttgcaggctgttggctaccagtttcctgtctgagg
tgtatatgttaacttcgtgatcagtttgtatgtttgggactcttgtcctatgtaaagttaaggtgg
gccgggtgcagtggctcacgcctgtaatcctaacactgggaggccgaggcgggtggatcacctgat
ggtgaaacctcatctctactgaaaatacaaaaattagctgagtggtgacacacgcctgtaatccca
gctacttggtaggcttgaacccaggaggcagagattgcagtgagccgagctgcaccactgtgctcc
agcctgggtgacagcgagactcagtctcaaaaaaagttgtacaaggtggatggttggaagcttgag
cctaggctcgaatccctctcacgtgagagggcctgaagatttctggtggattccaacctggctgaa
gactggccgtgggggtgcaggggtctccagcgctctgccctccagcctgcttcctccctgcccac
accgcactaggggaagggcctttcctgctgcctgcggggccgcacctggagtaggtaatgccatgt
ggtgacgtgaatggagcagaggtctgtgccccatcacaccgccttgctgtttttactgtgggacaa
aagcactctgatctgcgtgttccgggggccctcctaccagccgacttgacgggaagtcagggttca
ggtatcatctgtgcacctggggcggggtagtctgcactgaacctgccagagtcccctcctcatttc
actgaaagtcacagtctccagggctgtgttgctaaccttacgttctctccgtttgcttaatctatt
aagagccctaacaggagaggatgggctttctctgttgtctggggccctgctgttggccggtgctct
tagcaagaggtcatttttctaggttgcgctgggacattgtgagtttggtgagggtcatggatgtgg
gctgggctgggctgggctgggccgggctgcctgctgcctgctgctcccctacctgaaatgcagcta
gtgcggctctgcccttcctggggctgaggaaggcttctgcaggatagctgggggctgggcaggtg
ggtgaggcagcctccctgctgacactcagtccttgtagctggagcaagatctcctgatccaggtac
gggcctgtctgctccaagaaagactctgccaccagatgcaaaggggccctttgttttaacttagtc
cctggggaccgcctgattcagcacctgtcggcccaggataccccgctggtggggacaagtgcctga
gtgtgggccgtgcccgagtgtggccatccctgagtggggccgtcctgactaggaagtggcttttca
gttgtgatgtgtgggcctgacctagggggcgctgtggaacccgggctggaaccagccctctgtgcc
aggccgcagacaggttccgccggccctgaggggcagctgccatggcgtgggtcactgggagctgag
aggaagggcccccaccgcacctcaggcaaagcggctctgggaacaccttgatttcgtccatgtgag
ccgtcccagggagggcagccaagctgtgaagcctgagaaactgacctgtgtgccacgagcttgtgg
tctgctgcccggtggaggaagtgcaggtgcgcccaggctcctcattccgttttgcaggattccttc
ggggtgtgagcatttcctattcagcctgtcgcccccggggagcacgggctggctctgtggtgcccg
tggccttttgtagaagcgttggttttacggcaggttcatctctggggcagcctcccacagtgggtg
gggctttgccagcagtgcccacggggtcatggggccaggcgcgctccggcgcctgcagaactgat
cggggatagtctcaggaggcgctagtcacgtgccccggtgatcggggatagtctcagaaggcgcta
gtctcctgccccggtgatcggggatagtctcaggaggcacgagtcgcctgcctcggtgatgcaccg
tttctcacaccggctgctctggcccgagctaaaggggaagacgtgtgcggataggagctgcacaca
attttcctccatgtattgtttattttgctttttcttttggctagacattaggaatttcagttttcc
caagttgtatttttccttttctattttaaaattatcatgcagggctgggtgaggtcgctcacgcct
atagtctcaaaactttgggaggctgagggggaggatggcatgagcccaggagtttaaggctgcag
tgagccgagatcgctccactgtcctccagcctgcatgacagagcgagaccctatctcaggaaaaaa
aaaaacaaaactattatgcagtagtttcgaccctggaagacgagtgtgcatctttgagttgtaaca
cgtgtacctcgcccatccaggcgtagtttcatttggaatctggttatcctgtagttgctttgttaa
aaatatatgtaattgcaaatcattt

(SEQ ID NO:19)

Homo sapiens ribosomal protein L23 (RPL23)

ttctccagtatatttgtaaaaaataaaaaaaaaaactaaacccattaaaaagtatttgtttgc
(SEQ ID NO:20)

Homo sapiens ribosomal protein L18 (RPL18)
ccctggatcctactctcttattaaaaagatttttgctgacagtgc
(SEQ ID NO:21)

Homo sapiens ribosomal protein L18a (RPL18A)
gtgcagggccctcgtccgggtgtgccccaaataaactcaggaacgccccggtgctcgccgc
(SEQ ID NO:22)

Homo sapiens ribosomal protein L19 (RPL19)

aacctcccactttgtctgtacatactggcctctgtgattacatagatcagccattaaaataaaaca
agccttaatctgc
(SEQ ID NO:23)

Homo sapiens ribosomal protein L21 (RPL21)
taggtgttaaaaaaaaaaataaaggacctctgggctac
(SEQ ID NO:24)

Homo sapiens ribosomal protein L22 (RPL22)

atttcatttatctggaaaattttgtatgagttcttgaataaaacttgggaaccaaaatggtggttt
atccttgtatctctgcagtgtggattgaacagaaaattggaaatcatagtcaaagggcttcccttg
gttcgccactcatttatttgtaacttgacttctttttttttctgcttaaaatttcaattctcgtg
gtaataccagagtagaaggagagggtgactttaccgaactgacagccattgggaggcagatgcgg
gtgtggaggtgtgggctgaaggtagtgactgtttgattttaaaaagtgtgactgtcagttgtatct
gttgcttttctcaatgattcagggatacaaatgggcttctctcattcattaaaagaaaacgcgaca
tctttctaagattctctgtgggaaaatgactgtcaataaaatgcgggtttctgggccattcgtctt
actttcatttttgattacaaatttctcttgacgcacacaattatgtctgctaatcctcttcttcc
tagagagagaaactgtgctccttcagtgttgctgccataaaggggtttggggaatcgattgtaaaa
gtcccaggttctaaattaactaaatgtgtacagaaatgaacgtgtaagtaatgtttctacaggtct
ttgcaacaaactgtcactttcgtctccagcagagggagctgtaggaatagtgcttccagatgtggt
ctcccgtgtggggcccagcaatgggggcccctgatgccaagagctctggaggttcttgaaagaggg
gacacgaaggaggagtgactgggaagcctcccatgccaaggaggtgggaggtgccctggaaatagc
tgcctcatgccacttaggccatgactggatttaatgtcagtggtgtgccacagtgcagaggctaga
caactgaaaggggctaccaaggctgggaaaaaaatgcaattgttgctgtgagtgactttgaaagac
tctggtgccttgtggtgcccttctgaaattcaaacagtaatgcaaaagtgtctgcattagaattta
cggtgtctaaaattcatgtttttaaaagagcttgcctacagatggtttccacacttgaaattgtgc
cctgcgagttgcatagctggaagttcaatgctcagtcctaccttggctcccattaaacatttggtg
ctctgtggattgagttgaacgtgttgaggctttgcaatttcacttgtgttaaaggctctggcattt
ttccatttctatgcaaatttctttgaagcagaattgcttgcatatttcttctctgccgtcacagaa
agcagagtttctttcaacttcactgaggcatcagttgctctttggcaatgtcccttaaccatgat
tattaactaagtttgtggcttgagtttacaaattctacttgttgcattgatgttcccatgtagtaa
gtcatttttagtttggttgtgaaaaaaccctgggctgaagttggcatttcagttaaaagaaaaaaa
gaaactagtcccagatttgaaaacttgtaataaaattgaaactcactggttttctatgtcttttg
aactcttgtaatcgagttttgatcatattttctattaaagtggctaacacctggctactcttactg
t
(SEQ ID NO:25)

Homo sapiens ribosomal protein L23a (RPL23A)

actgagtccagctgcctaattctgaatatatatatatatatatcttttcaccatatacatgcctgt
ctgtcaatttctggttgggctgggaggccacacacacacactgacatgacagggcttgggcaagac
tcctgttctacttatccttttgaaatacctcaccctgccactccaccatgtatgatcattccagag
atctttgtgactagagttagtgtcctaggaaaaccagaactcagaacttgcctccatggttgagta
acaagctgtacaagaaccccttttatccctggaagaggctgtgtatgaaaccaatgcccagggttt
gaagggtgttagcatccatttcaggggagtgtggattggctggctctctggtagcattttgtcctc
acacacccatctactatgtccaaccggtctgtctgcttccctcaccccttgcccaataaaggacaa
ggacttcagagg
(SEQ ID NO:26)

Homo sapiens ribosomal protein L17 (RPL17)
attcagcattaaaataaatgtaattaaaaggaaaag
(SEQ ID NO:27)

Homo sapiens ribosomal protein L24 (RPL24)
actggcagattagatttttaaataaagattggattataactctag
(SEQ ID NO:28)

Homo sapiens ribosomal protein L26 (RPL26)
agtaatcttatatacaagctttgattaaaacttgaaacaaagagcctg
(SEQ ID NO:29)

Homo sapiens ribosomal protein L27 (RPL27)
atgctttgttttgatcattaaaaattataaag
(SEQ ID NO:30)

Homo sapiens ribosomal protein L30 (RPL30)

accttttcacctacaaaatttcacctgcaaaccttaaacctgcaaaattttcctttaataaattt
gcttgttttaaaaacattgtatct
(SEQ ID NO:31)

Homo sapiens ribosomal protein L27a (RPL27A)

agccacatggagggagtttcattaaatgctaactacttttttccttgtggtgtgagtgtaggttctt
cagtggcacctctacatcctgtgtgcattgggagcccaggttctagtacttagggtatgaagacat
ggggtcctctcctgacttccctcaaatatatggtaaacgtaagaccaacacagacgttggccagtt
aaacatttctgtttataaagtcagaataatacctgttgatcactgaaaggcctgcatgtattgtac
tctgaattttacagtgaatgagagaatgtaccctaattgttcaacagggctcaaaaggaaagattc
cattttgatgggtcacattctaaagaggggcagtgtgataggaatgagatggtcctttaggactta
agttctcagcccaaggtttttccacgtggccccctcatcttttttttttttttaaacggagtctct
cttgccaggctggagtgcagtggcacgatctcggctcactgcagcctccgcctcccaggttaagcg
attctcctgcctcagcttcctgactaactgggattacaggcgcccaccaccatgcccagctaattt
ttgtattttcagtagagatggggtttcaccatgttggccatgctggtctctaactcctaacctcaa
gtgatctgcccacatcggcctccaaaagttctgggattatagtgtgagccactgcgcccggccatg
gctccttaatcttgatccaaattattgttacatccagaatgtgatgaatcaaaatctcgagatggg
ggtccagcaatctgaaatttcagtatgccagggcttttctgtatgtcaaagtgggtttgaaatagt
taatttttcttctagtctgaaatgtatcgggaaaatttggaaatcctgaaggctggaaattgaaat
aagtttttctaggatttgtgtctcttgctattggaaaactgatggtgaccaattcatgtttacaaa
taagatcctcatagatctcggtaaattataatttgctacagttttatggttcttcctgtgatttttg
agctttttttgacccaaaataatacagtctaaaactatagacaaataagatggcacttagactcct
gggttttagttagtggaggtttccttagtgcactgtggggtcataataagccgagaaccatggctg
tctatgggacacatctgtcaggacaacctttagaggatgttggggatcaaatagaaggcacagaga

agcactgaattggcttacataagaataggctagaattacaagtagtgaaacctcgattcagctgga
caattttaaacaaatgtatcatttggcttgtatcttctgttgtgctggagaagttagaaataaggg
ctctccagaccagcctgaccaacctggagaaaccttgtctctactaaatacacaaaattagccagg
cgtggtggcacatgcctgtaatcccagctactttggaggctgagccaggagaatctccaggaggcg
gaggttgctgtgagccgagatcgtgccattgcactccagcttgggcaacaagagtgaaactctgtc
caccccccccaaaaaaagtaagggctctccattagggcccatagaggacttgtaatatggaacctg
aatccaaggatcccacaataagtggtcagtagttcatgatgaattaaaagactcaatatttggtct
tcacccaatacctgtgtgacttttagtcctaatttcctcatctttaaaatttcagtgaaagtgcct
acctgaggattgtgtagattaaaatggaaaccgtgcacttaatttttttgttttgttttgagacgga
gtctcgctctgtcgcccaggctggagtgcagtggtgcgatctcagatcactgcaagctccgcctcc
taggttcagaccattctcctgcctcagcttcccaagtagctgggactacaggcgcccgccactgcg
cccggctaatttttttgcatttttagtagagacagggtttcaccgtgttagccaggatggtctcgat
ctcctgatctgcccgcctcagcctcccaaagtgctgggattacaggcatgagccaccgcgcccggc
ccaggcacttaattttttgtgtttgacttagtaacttaagtgcaaactattacgggagcagatggag
tcaattggccttcatgtgattgtcagtgggaaattggtccaagcagagggaatactggttcaggaa
actggtttgggaaggttaggcaaacgggaagtgctatggtggagagaaagattactctggccgggc
tgtaaaggacggctacaatgggaggctgaaggcagaaccaagaaaatgggagtgagtatggaaaag
gtacgattcagacggcataatggacgggacttggagactgaattgtagtgggccgaccacaaaatg
ataaggcatggaaggaagtagagtttgggggggaaggatccctagtcccttaatggctaccttcttc
cccaggagttgttaggccatccgatcccctggcctgggaaagaaacactgatttcgttgctggctt
gttcactcaccagaagctacagctactaacagttctaaaaactgtttcatgtgatgaggaacagac
gaaaatagttttgagccctaagtccgccgattccagtgctttcttgaacccgcatttactaaaata
ttttcatgactgccaagctttgaatagcctgctgtgttcatggaggctcatactggcgatctctag
tggctggctaaagcttgaattgcaaaagatctaatttctggtctaatgtatatatgccttaaatat
agttgcgttcaaacgtgggagctgcaggtgcaacttgattttatgacaaatggctgccacataatt
tgcacaagcagtgctcgtcaagggcagctaaatcaggcgagctttcaatcaaaataaatgtactac
taaaccctacttagcggctaactagcccaagagcagacagcccacggacggactgcaagtcggaag
cgcgggcggaagctgtgcagcgcccacctggtggctccatcggccgcgttcatcagtcagcacgac
ccgacctcagtggcgtcctcacaacacagaccggaccttgggtcttaccccggcacctgagaacca
cttccggtgagtagcttctacttccggagacgatgactcccccgcgtcccagaccggaagaagccc
ggcggagaccggcctcgctcggccacttccggcaagggcggagccggccagtggtgcgcgagcgca
gataactcccctggagaggcgggatgttcaactccacccctggtccttgggcggccgtgggtcccc
ttcgaagcggaggaatggccaacctcgccgcacttcgagcccctttagggtgcgtttaagaacagt
gggcgtggcctttacgtaaatcttcgagatgggaacctccagaatttgtctcaattgtctaaaagg
taatgagcgtcagcgacattcaagggcactttggctaaaaaagaaagtgcttgtacacggatgga
aatattctagaagaacataaaggaatttcctcttaggaggttagggaaatgagcacgaagtatgt
tttggtgcagtttttttgttcaacccaatgcgtattttcatattgagaggcaatataaatggagcga
aagtatcttgagaaaaaaaaaaaaaactaccagaacttgccgttgctgaaaagtaatattttctctt
tcgagagttttcatggcctttttaaattacacccccacctccacaggcaaataaatttgttttggaa
tgcataccacatcatctggctctagaaacgtattttgtgtagctcccctagcaagaatataggtta
aagcgtaaatttaattcctggctctatttttacatcccaattttttattttcctctcattcccacttt
acgttgtttcaaataacctagtttgtgtatccctgtaagtcattttggtataaagtaggttataag
tgtacatgcgaaaagatgtttttaacaaaaatgtaactg
(SEQ ID NO:32)

Homo sapiens ribosomal protein L28 (RPL28)

gccttgctctgctcccccgcccccaggcagccatccgcagggccagcgccatcctgcgcagccaga
agcctgtgatggtgaagaggaagcggacccgccccaccaagagctcctgagccccctgcccccaga
gcaataaagtcagctggctttctcacctgcctcgactgggcctcccttttttgaaacgctctgggga
gctctggccctgtgtgttgtcattcaggccatgtcatcaaaactctgcatgtcaccttgtccatct
ggaggtgatgtcaatggctggccatgcaggaggggtggggtagctgccttgtccctggtgaggca
agggtcactgtcttcacagaaaaagtttgctgacttgtgattgagacctactgtcccattgtgagg

tggcctgaagaatcccagctggggcagtggcttccattcagaagaagaaaggccttttctagccca
gaagggtgcaggctgagggctgggccctgggccctggtgctgtagcacggtttggggacttggggt
gttcccaagacctgggggacgacagacatcacgggaggaagatgagatgacttttgcatccaggga
gtgggtgcagccacatttggaggggatggctttacttgatgcaacctcatctctgagatgggcaa
cttggtgggtggtggcttataactgtaagggagatggcagccccagggtacagccagcaggcattg
agcagccttagcattgtcccccctactcccgtcctccaggtgtccccatccctcccctgtctctttg
agctggctcttgtcacttaggtctcatctcagtggccgctcctgggccaccctgtcacccaagctt
tcctgattgcccagccctcttgtttcctttggcctgtttgctccctagtgtttattacagcttgtg
aggccaggagtttgagaccatcctaggcaacataatgagacaccgtctctaaaataaaattagctg
ggtgtggtggtgcaccgcctgtggtcccagctcctcagaggttgagtagaggctgaggtgagcgga
gcacttgagccaagagtatgaggctgcagtgagcccatgagccccaccactacactccagcctgga
agacaccatgacacacagtgaggcctggatgggaaagagtcctgctgttgatcctcacatgtttc
ctgggcacctaactctgtcagccactgccagggaccaaggatccagcatccatggcacccctggtt
cctgccatcctggggtacccgattcaaagaaggactctgctccctgtctgagaccacccccggctc
tgactgagagtaaggggactgtcagggcctcgacttgccattggttggggtcgtacggggctggga
gccctgcgttttgaggcagaccactgcccttccgacctcagtcctgtctgctccagtcttgcccag
ctcgaaggagagcagatctgaccacttgccagcccctgtctgctgtgaattaccatttcctttgtc
cttcccttagttgggtctattagctcagattgagaggtgttgccttaaaactgagttgggtgactt
ggtacctgctcaggacccccgcactgtcccaatcccactcaggcccacctccagctggcctcact
ccgctggtgacttcgtacctgctcaggagcccccactgtcccagtcccactcaggcccatctctgg
ctggcctcactgcgctgggactccgccttcataaggagagctcactgctcacgttagtagatggcc
ccttctcgtgaggcctctcccctggcacctgcttcagttgtcctccacagcactgatttgcagccc
acaagctggcaggtttatctgtctcatgtttgtcttgtgctggtgggcaaggggtttgtctagcac
accagcatataatgagatgcttgatgaatggtgcatattgaatgtataaagcccaccggtcctgag
agtttgctcactggagactttctggagatggagtctcgctctgttgcccaggctggcgagtgcaat
ggcgcgatcttggctcactgcagcctccacctcctgggttcaagcgattctcctgcctcagcctcc
cgagtagctgggattacaggtgggtgtcaccacacccagctcagtattgtattttttagcagagatg
gggtttcaccattttgcccaggctggtttggaactcctgacttcaaattacccacctgcctcagcc
tcccaaagtgctggcattacaggcgctcgaggctttctgatgtggctgctgctgctcagaaggcct
tgtccttaaccacctccttgcctgccctggaggcttgtgcctctaggccccacccccctgtggagtc
ctgctggctttctccatccctatctgaatcctccctgctgtgtggcctcccctggtctcatccgta
acacagcccagcttagtgggcctctgttcctgcgggtggccagcctgtctgtgtggctgggctggg
gaggccacgtctggtatctgaatgctatcggtgggttggggtggaggaaccaggagagggctggag
ggagggagatggtctcagccccacagagtttggagtcctcagtgtgctgagcaaacgtggagacac
catttccctcctctagacctcatcttggagagagagatgttggatggggccatctattccagcttt
attcacacaaatcatgtctgttggcctggaaattggaaaaccagttaaaccaaaaacatgatatta
agaaaacaggcaggctcaccatagtaaaaatgctgaaagccaaagacaaaattgggagaacaaaag
aaaagcgtcttgtcacatacagaaggtccctgataaagttagtagctgccctcatcagaaaccagg
cccaggcagtggggacacatccagagtgctgaaagaacctcccccaggtcatcctatccccaagag
tgatgcccggcagcattcccagctcagggctaatggttcacggaagccaggaatcaaactgcctgg
gttccagtcccagctctgccagttatgcccagctgtggggacttgggcagctcgtttagtagcacc
gtgcctcagtttcccatatgtaaaaggccattttgagtgcctttcacagccctgcataaggcaggt
gtctcagtgttcactgctgtctctccagctcttagtccagtagctgcatggtgagtgagcgtaggg
cgcaccctggaaggctgccaagcccaaagttgtgcagagcgctggggactccagactccccacagc
agcagagactcgggactgaggcatcctctgttcacaggacatgctggcatctactgggtcagggct
ctgctgctcggtggctgtgcaaccttggcaagttcctcaacctctctgtgtcttcgtaccctcat
ctgtaacatgcgtgtcgatagaccctactactcagggttgatgagaagattaaatgtgcaaaacct
gcttgactgtgcccacaaatcctgattgtaggaataaattaatgactttttataaatattttgatc
agatggactcatgatcacagatgtcttcacatgcctatgactaatttgtacacaaactaatgctcg
tgtttcccaagcacctggaagacatgccagatccatgtgcagtaatgcctggtggctccaggtctg
ccccgccgtcctgtggggctgtgagctttcccagcctcctgcccgtgtttgtgaatatcattctgt

cctcagctgcatttccagcccaggctgtttggcgctgcccaggaatggtatcaattcccctgtttc
tcttgtagccagttactagaataaaatcatctacttt
(SEQ ID NO:33)

Homo sapiens ribosomal protein L28 (RPL28)

tttttactgtcaggcaggaagagcggtaactgccatcgcggcgggcatccctggcgccagggtgt
tggtctgggtaccggcttccctctcggccgacttgtcagctctgtgagccgcgcgtctgagccc
gtgtcctcacctgtaaagtggagaaatgaaaaaggacctgaacttcctcggtggttgttgagagtt
aaggcacggggttgatgttttcagatgaaattctcaaagcaagtcagggtggggatggatggtttc
atcccacaggtgggaagattgagg
(SEQ ID NO:34)

Homo sapiens ribosomal protein L28 (RPL28)

gtttttctcaggtccttgattggaactgcctcagagccaagggtccttttactcagtggcagcaac
aaacgcagtctgttggctagtgatcctcctgtctcagggacacgtagtccagggagcagccaattg
cttggcacttggggaccccgttctggggagtcctgaaagctttcacctcttggattgccgaataca
tgggtggcccttcctagactaagggactggcctgagtgaggctgggcctctcagccaagctgatgt
tgaaccactgctgtggggatgggcctggggttcctgggaagctgttcatacccattgccaggagcg
tgggctctggctggacctggatcagatcctaactgaagcggcagctttctggcatgagaaaggagt
gttttcatggtggacagaattgggctatgagtgt
(SEQ ID NO:35)

Homo sapiens ribosomal protein L29 (RPL29)

atatctctgccaacatgaggacagaaggactggtgcgacccccacccccgcccctgggctaccat
ctgcatggggctggggtcctcctgtgctatttgtacaaataaacctgaggcagg
(SEQ ID NO:36)

Homo sapiens ribosomal protein L31 (RPL31)

agggagccctcctggaagtggatgaggccttgggtctcggctcttcattgcttcctgagctgcagc
agatgcctttacaaccaagctcaccgaggacgtctgtctcccatattaccctggcagagggccagg
cctgttctacacggccggggtttcaacaaggtactgatgtcttctgcccttgcctcttcgacaggc
aagtaataagacttaagtgaagagaattctttaggcacacaaattcacatttgatgtaatctcatt
atacttcctgatctgtgattgaaaactttcatttcgtaactagtatgtctgtcccacctttaaaaa
gtttttcattatgaaagtaagtatttgttagaattaagtctatttaaatgaaaaaaacttagatat
gagtctgcatggcctcaggaaaatgatgttttaaaatagagattttaggttgtctgcactctagct
tttttgtcgttttcttaaggctttttaactgcatcaaaaattcagatacgaaacatacactaaaa
aataatacatcatatcttaatttccactgaacttgatttaaattcagagttacacagtatgaatat
cacaatcagatatgttcaaaaaggtctgaacaattgattttctgaaaccatgaaggactac
(SEQ ID NO:37)

Homo sapiens ribosomal protein L31 (RPL31)

agccatttaaattcattagaaaaatgtccttacctcttaaaatgtgaattcatctgttaagctagg
ggtgacacacgtcattgtacccttttaaattgttggtgtgggaagatgctaaagaatgcaaaact
gatccatatctgggatgtaaaaaggttgtggaaaatagaatgcccagacccgtctacaaaaggttt
ttagagttgaaatatgaaatgtgatgtggtatggaaattgactgttacttcctttacagatctac
agacagtcaatgtggatgagaactaatcgctgatcgtcagatcaaataaagttataaaattgcctt
c
(SEQ ID NO:38)

Homo sapiens ribosomal protein L32 (RPL32)

gcagctcatgtgcacgttttctgtttaaataaatgtaaaaactgccatctggcatcttccttcctt
gattttaagtcttcagcttcttggccaacttagtttgccacagagattgttcttttgcttaagccc

ctttggaatctcccatttggaggggatttgtaaaggacactcagtccttgaacaggggaatgtggc
ctcaagtgcacagactagccttagtcatctccagttgaggctgggtatgaggggtacagacttggc
cctcacaccaggtaggttctgagacacttgaagaagcttgtggctcccaagccacaagtagtcatt
cttagccttgcttttgtaaagttaggtgacaagttattccatgtgatgcttgtgagaattgagaaa
atatgcatggaaatatccagatgaatttcttacacagattcttacgggatgcctaaattgcatcct
gtaacttctgtccaaaaagaacaggatgatgtacaaattgctcttccaggtaatccaccacggtta
actggaaaagcactttcagtctcctataaccctcccaccagctgctgcttcaggtataatgttaca
gcagtttgccaaggcggggacctaactggtgacaattgagcctcttgactggtactcagaatttag
tgacacgtggtcctgatttttttggagacggggtcttgctctcacccaggctgggagtgcagtgg
cacactgactacagccttgacctccccaggctcaggtgatcttcccacctcagccttccaagtagc
tgggactacagatgcacacctccaaacctgggtagtttttgaagttttttgtagaggtggtctag
ccatgttgcctaggctcccgaactcctgagctcaagcaatcctgcttcagcctcccaaagtactgg
gattacaggcatcttctgtagtatataggtcatgagggatatgggatgtggtacttatgagacaga
aatgcttacaggatgtttttctgtaaccatcctggtcaacttagcagaaatgctgcgctgggtata
ataaagcttttctacttctagtctagacaggaatcttacagattgtctcctgttcaaaacctagtc
ataaatatttataatgcaaactggtccttc
(SEQ ID NO:39)

Homo sapiens ribosomal protein L35a (RPL35A)

actaacgaaaaatcaataaataaatgtggatttgtgctcttgtatttttaagtggattaaaaaact
tactacctt
(SEQ ID NO:40)

Homo sapiens ribosomal protein L37 (RPL37)

gaatgtcaacgattagtcatgcaataaatgttctggttttaaaaaatacatatctggttttggtaa
ggtatttttaatcaattaggcttgtagtatcagtgaaatactgtaggtttagggactgggctagct
tcatatcagatttacttgttaagtgactgttttggaatgtttactttggactgggtttgtaacac
ggttaaaggcaatgagaaacaagcagaattccaggagtccttgaagcagagggcactggaagacaa
tatagcagattaaaatagcacagctcatgtggcataggtgggtattttagatgtttgagtaaattt
gaaagagtatgatgtttaaattacctttagcaacatgttcatctgctatgctgtcatgactagggg
gatgattattagtcacatagagcttgggagtaccactggaaacgtatgggtaggagtttaggtggc
ttctgtttttcaaaagatgatcttatcctagtatctgtaatgctcacttggcacacctgacttgtg
ggctgtgtgtaaggtggctagctaagtgaaaaaagcctgctaggtgtgagtcaacttaagaatatg
taaataggtttgagaaaaagtagggcttgggtgcaagtaaagattgagcaggaaataaaggaaaat
caagtataatccctgagatttgtagactaaaggcaatgatgtgggactacttggtcgaatttttt
agccctcaacttggtaattgggtgtttctgtgttaaagcactgaaacttgctgtcgtgccttccta
gttttcgtggtttattgacagggttggggg tttttttgtttttttaaaatgaagggacaaagtca
actggactgctgagtgagagggcaggggcagttgaagggaacatgaattgctggaacagctacata
aaatagtgatgtagccaagtcatgctatttaaattataattctccactgtgtttagaataacatct
gaggttcttaacctggccttggaagggtatcacttttacttgtaacctggaatggctttataatgt
gctagctaattgctactctcatcttgtatttaactcctaatttacccttcaggtctcagcttcag
aacattcacttataaagaaaccctgctgattaaatctctcttgggcttcctccc
(SEQ ID NO:41)

Homo sapiens ribosomal protein L37a (RPL37A)
acgctcctctactctttgagacatcactggcctataataaatgggttaatttatgtaac
(SEQ ID NO:42)

Homo sapiens ribosomal protein L38 (RPL38)

accagacacactgattggaactgtattatattaaaatactaaaaatcct
(SEQ ID NO:43)

Homo sapiens ribosomal protein L39 (RPL39)

ggaattgcacatgagatggcacacatatttatgctgtctgaaggtcacgatcatgttaccatatca
agctgaaaatgtcaccactatctggagatttcgacgtgttttcctctctgaatctgttatgaacac
gttggttggctggattcagtaataaatatgtaaggcctttcttttt
(SEQ ID NO:44)

Homo sapiens ribosomal protein, large, P0 (RPLP0)

tcaccaaaaagcaaccaacttagccagttttatttgcaaaacaaggaaataaaggcttacttcttt
aaaaagt
(SEQ ID NO:45)

Homo sapiens ribosomal protein, large, P1 (RPLP1)
acctcttttataacatgttcaataaaaagctgaacttt
(SEQ ID NO:46)

Homo sapiens ribosomal protein, large, P2 (RPLP2)
attcctgctcccctgcaaataaagcctttttacacatctc
(SEQ ID NO:47)

Homo sapiens ribosomal protein S3 (RPS3)

cagggtctccttggcagctgtattctggagtctggatgttgctctctaaagacctttaataaaatt
ttgtacaaagacacaaggtctgactagactgttcagtattcagactgaggggcatgttggcctctg
gagcattacatatcttcttggttttaaccatacttgtggtatttgcaagggccagaacagtaagac
ccaagcagagccaaccagagaaataatatttgtgtgatagagaaggctgatagcaagcaaggcagc
accttgattcgttgtcctgtagttcaggattgtaggtttagaagagggatatgtttgagtttttcc
tatgcataaggcgatccacgttgcacatagaaagtgaatataaatggccattatattttgtgtcat
gctgtgctctaagtgttctttacatatgtactcgttaatcaacctctctaaagtgtaaaggaaatt
tgcttgcaccactgaaggcacataaggctcagaagtaaatttgcctaagcagtataaagctatcat
tagaatccacattcctaagttgtgttctcttaggggatcatggaaccagtcattggtactacaggc
tattatgttctggagaactgtgaagaacatttaaattgtctctgattttatctatcaatgttttga
agtattttctaccagtgtctgtacttcacaagaaattcggcactatttttcaggcaaaactagtg
agggacaggttggcttgaaaatcatgagactgttgttaaatcagatgctggttgatcacagagggg
acttccagggaaagctgttatcaggtggctgcttcctggtgatgcagcctggctgatgagataacc
ctggctccacagatggcttagcaggtgctgtgatgatttggttttcttctcaattagactgagctg
cacatggtgtttatattgcttggcacatggtaagggcttaatatttgaggtaattatgtagggcgt
acactgacaagtatctgaccccccttccttttgactcataaattggtcatcttaaccatttaag
tgtacacttctatagtgacagagttagccctctgtccaaggatttgcatctgtggattcaaccaa
ctttgggtcaaaaataatcaaaaaggatggttgtgtgtgtattgaacatgtagacttatttttctt
attttcaaaatactatattttcttgtcacttattttcttgtacactgcagttgtaacagctatgta
gcatgtacattaggtattaaaagtaatccagtgaagattgaaagtct
(SEQ ID NO:48)

Homo sapiens ribosomal protein S3 (RPS3)

cagcctcttccatgagtggggagcccgctgcttgtctccagctcctagcagtgagtcctgataatc
tcaaatttaaggacagtaactttgtctgggatgagtgtgggaaaggatgtgtttgggaacagacgc
gagcctgcagaggtgtttgtaaccatctctttctaagtggtgggaagcagacattttattctttaa
ctgttaatatatatagtgtgtgtttttatgcatgaaatatttatagttttaaaaatgcccaca
ctactattttgaaagtaaatgaggtaatgtatgtgtcagaacccaatacccaaagcgatcgtagta
agaggtggggcctttgggaaggcattaaattgcttagggaatgagggtggaaccctcatgaatgag
attagagccttataggagaggttggagggagttgcctggcctccctctcccatgtgaagactcagc
aagaaaacattatttaggaagcagagagccctcatcaaacaccagatctgctggccacctgatctg
gcactttccagccttcagaactgtgagaaataaatttctgttgtctat

(SEQ ID NO:49)

Homo sapiens ribosomal protein S3A (RPS3A)
agttcagacttcaaatagtggcaaataaaaagtgctatttgtgatggtttgcttctg
(SEQ ID NO:50)

Homo sapiens ribosomal protein S3A (RPS3A)

agctcacgttgatgtcaagactaccgatggttacttgcttcgtctgttctgtgttggttttactaa
aaaacgcaacaatcagatacggaagacctcttatgctcagcaccaacaggtccgccaaatccggaa
gaagatgatggaaatcatgacccgagaggtgcagacaaatgacttgaaagaagtggtcaataaatt
gtaagtgtttctttgcttcctcacacaacacaaccttgagtattggattattcctgagatgagaga
acgcatatgagacaaggtaaaggtctgttgaaatcctgtctgtgaatccttctagctatatctctt
taagtgaaagagtgttaagtactcagtaaatatgattattattactattattatttgagtcagagt
cttgctctgttgcccaggctcgagtgcagtattgtgatcctccttggctcactgtaaccactgctt
cctgggttcaagcagttcttgagcctcagcctcctgagtatctgggaatacaggggactgccacca
tacccagctaatttttttaaatttttagtagagatggggtttcatcatgttggccaggctggtctt
gaactcctgacttcaggtgatctgccagtactctaaatgataacagtttttttcgtgtttatttatt
ttgaatgaagctgtctcacagtagatggagttgaaggacaggaaatgtttttcccctacttggaaa
atacactgaataagttgagtggggtgggatgtgcctggagtcccagctactcaggaggctgaggtg
gtaggattgtttgagcccaggagtttgaggccagcctgggcaatatagggagaccctgtcccaaaa
aataaaaaatatacgtatatatatatacacacacaaagaaaaaatacactgaatagacaaaacctt
tcatgattaatgatgcacgggaataagtgatgaaaaaagtttcggtcccagatgatggccagtgat
aacaacatttttctgatgttcccatgcaatatacagttagctaagagggtgtaatggaaaaagcat
aaggcttggactcagaagactctactaactttgccactagctagctatgtaattcagatcatctat
cctttacatgtgaaaggtaaataatggcttatcttaacaggaggatttatgcaggttaaatgaggt
aggtgttatgtgtaggtttattccaaggcttctctacttttaaaggaaatggcttatatctgagaa
ctaggacttttagaaaaaaatttactgttactggtttgcaggattccagacagcattggaaaagac
atag
(SEQ ID NO:51)

Homo sapiens ribosomal protein S4, X linked (RPS4X)

aatgggtccctgggtgacatgtcagatctttgtacgtaattaaaaatattgtggcaggattaatag
ca
(SEQ ID NO:52)

Homo sapiens ribosomal protein S4, Y linked 1 (RPS4Y1)
attgcagtagcagcatatctttttttctttgcacaaataaacagtgaattctcgtttctt
(SEQ ID NO:53)

Homo sapiens ribosomal protein S5 (RPS5)
ttttcccagctgctgcccaataaacctgtctgccctttggggcagtcccagcc
(SEQ ID NO:54)

Homo sapiens ribosomal protein S6 (RPS6)
gattttttgagtaacaaataaataagatcagactctg
(SEQ ID NO:55)

Homo sapiens ribosomal protein S7 (RPS7)
acaaaaatgactaaataaaaagtatatattcacagt
(SEQ ID NO:56)

Homo sapiens ribosomal protein S8 (RPS8)
atccttgttttgtcttcacccatgtaataaaggtgtttattgttttgttcccaca
(SEQ ID NO:57)

Homo sapiens ribosomal protein S9 (RPS9)

gtccacctgtccctcctgggctgctggattgtctcgttttcctgccaaataaacaggatcagcgct
ttac
(SEQ ID NO:58)

Homo sapiens ribosomal protein S10 (RPS10)
aattggagaggattcttttgcattgaataaacttacagccaaaaaacctt
(SEQ ID NO:59)

Homo sapiens ribosomal protein S11 (RPS11)

ggctggacatcggcccgctccccacaatgaaataaagttattttctcattcccaggccagacttgg
gatcttccgcg
(SEQ ID NO:60)

Homo sapiens ribosomal protein S12 (RPS12)
agaaataaatctttggctcac
(SEQ ID NO:61)

Homo sapiens ribosomal protein S13 (RPS13)
atttgtctgtgtactcaagcaataaaatgattgtttaacta
(SEQ ID NO:62)

Homo sapiens ribosomal protein S15 (RPS15)
tggctcagctaataaaggcgcacatgactcc
(SEQ ID NO:63)

Homo sapiens ribosomal protein S15a (RPS15A)
ggatgtaatacatatatttacaaataaaatgcctcatggactctggtgcttcc
(SEQ ID NO:64)

Homo sapiens ribosomal protein S16 (RPS16)
gcccatcgtgactcaaaactcacttgtataataaacagtttttgagggattttaaagtttcaag
(SEQ ID NO:65)

Homo sapiens ribosomal protein S19 (RPS19)
aacaaaccatgctgggttaataaattgcctcattcgt
(SEQ ID NO:66)

Homo sapiens ribosomal protein S20 (RPS20)

ctgcattctcctccgccaaaaaagtgaccaagcagagtctttctctgtcacccaggctggagtgca
atggcgtgatctcagctcactgcaacctctgcctcctgggttcaagtgattctcgtgtctcagcct
cctgagtagctgagactacaggtgtgcaccagtgttcccagctgatttttgtattttatgtagaga
tggggttatgccattttggccaggctagtctcgaactcctgagctcaggtgatacacacacctcag
caaatcttttaaattatacattctgtgatatttccttgactttcttatccagcacttgtattgatt
atttttcattttgataatgttgggtttttaaaaactcctttatgatggaaaatttc
(SEQ ID NO:67)

Homo sapiens ribosomal protein S20 (RPS20)

gtcaactattttaataaattgatgaccagttgttaacttctgttggtttttattcagaatactggc
agattttaggaatataaaggtgtactatgagacttccacttttcaggtggaatatatgggtatctt
agagtggtctatcctgttttcgttgtcgtttgagtcatttgaaaactggattccgttaactacata
atatgtgagacctgactggttttattggacactggcagtttataactttggcatactctagataaa
ttctgattggtatgggg
(SEQ ID NO:68)

Homo sapiens ribosomal protein S21 (RPS21)
ctggagagaatcacagatgtggaatatttgtcataaataaataatgaaaacct
(SEQ ID NO:69)

Homo sapiens ribosomal protein S23 (RPS23)

atattaatggtgaaaacactgtagtaataaattttcatatgccaaaaaatgtttgtatcttactgt
cccctgttctcaccacgaagatcatgttcattaccaccaccacccccccttattttttttatccta
aaccagcaaacgcaggacctgtaccaatttaggagacaataagacagggttgtttcaggattctc
tagagttaataacatttgtaacctggcacagtttccctcatcctgtggaataagaaaatgggatag
atctggaataaatgtgcagtattgtagtattactttaagaactttaagggaacttcaaaaactcac
tgaaattctagtgagatactttctttttattcttggtattttccatatcgggtgcaacacttcag
ttaccaaatttcattgcacatagattatcttaggtacccttggaaatgcacattcttgtatccatc
ttacaggggcccaagatgataaatagtaaactcaaaattgctccccactctgtttattatttaaag
gtgtcaggatctgtgttgtaatgtgtctacattaatgtgtttaggagaatacaggcattggatcat
ttagttgatggaagtatatgccaggcaagggagataaggtatacgacaagactgatgttttcagta
tcttctcatgaggttgtcagagaccttcatgtcttcaaagactagtcagcaaatgaagtggtttag
tgtagagacaagattggttgtgttttgataatttaagctaggtattgagtacatgtggattttgct
gtccacaaatacttgtttcagagttttcatggatacagtggcatggttgaaatgaagctgtgagcc
ttctgctttaaatctgatgtaagaaactcctgttaacaaatagtaagtatgggttaattagccctt
tgatcaaagcctagctttacattgtttaggatctttggaaaacaattggtttggttgcccactttc
cgtaggatcaagagcagaacctttcacatggcacagaagaacccaggttgcgcttcatacctgcat
attccagccttagcctgccatttctctccttggcactttgtgctccagcaacactggtctcagttg
gtcatcctcaaacttgggttccatatccagcctcaggacctctgttcctgttactatggttccttg
catgtcgcctgctcttactaaagagctcgtgtgttttccagcacacttcggtttatctcttgatga
tgatgctagtctctccctccgcaagggcggaaaggctgcctgttggtttgtaccagtgtttcctaa
cgtgtagctgcagtcagtatttggctaagctgttcccaggggctcaacagatgctttcggatgagc
cttaactgacccaatcctttgtgatgcgggagagattgctaggcctcgctcacctggccagaacca
gggaaagaggccgcggttgcagcgcgattccaggccctgggcgtcaggcgcggggtgggcagctct
ccccgggcggtggggcccttgtgaccgcgaggcggggcgcaccaggaagggagtgggacagcgcgg
gcgcccagggatgtggcctggttacctgccttctctgatacgtcaagacaccttcaacaatggctt
gcagctgtaccctgttggctgcacccaggacgcccttttcactgctaagcagtcctacctgaggcc
caggggctgccagattgacccataaataatctccggcgcctcagatccagaagctgctgagcctga
tcttagtgccttctcctttctctgtgtggcccccagcccctttccccactgccttgtgtccaagg
ccctttccttcatgtatccatggaggagagacaaaaatacacatcaataaaataagatagggaatc
cataaatagacattcagaagtatggccaacggatttatcttaaaaccaatggaggaagaagagttt
caataaatgttgtggacttccatttgtcaaagaccaaaacaaaggaaccccaaccttacatgtaat
acaaacttaactcaaaatggatcatatatctaaatgtaaaatggaaagctataaaactgaaaacag
actatctttacaacctaggcgtaggtatagtttttagacattacaccaaaagcacatgccgtaaaa
gaaaaaatagataaattggtggatttcattaaaattaaaaaactttttctctctgaaaaatcctgt
taagctgggcgctgtggttcatgcctgtaatcccagcactttgggaggctgagttgggaagaaatt
aatagcttgaggccaggagttcaagatcatcctgggcagcaaagtcatacactcttgagggaagag
agagaccttctcatattgttttatattgttttatactcagtacctgttttaagaaaaaaacaagga
agtgaaatcaaagacaggcagcccggcaccaggcctgaaaccagccctgggcctgcctggcctaaa
cctagtagttaaaaatcaacttacgacttagaacctgatgttatccgtagattccaagcattgtat
aaaaaaattgtgaaactccctgttgtgttctgtaccagtgcatgaaacccctgtcacatatcccct

agattgctcaatcaatcacgaccctttcatgtgaaatctttagtgttgtgagcccttaaaagggac
agaaattgtgcacttgaggagctcagattttaaggctgtagcttgccgatgctcccagctgaataa
agcccttccttct
(SEQ ID NO:70)

Homo sapiens ribosomal protein S25 (RPS25)
ataggtccaaccagctgtacatttggaaaaataaaactttattaaatc
(SEQ ID NO:71)

Homo sapiens ribosomal protein S26 (RPS26)

ggagctgagttcttaaagactgaagacaggctattctctggagaaaaataaaatggaaattgtact
t
(SEQ ID NO:72)

Homo sapiens ribosomal protein S27 (RPS27)
aagcactctgagtcaagatgagtgggaaaccatctcaataaacacattttggataaatcctg
(SEQ ID NO:73)

Homo sapiens ribosomal protein S27a (RPS27a)

ctgtatgagttaataaaagacatgaactaacatttattgttgggttttattgcagtaaaaagaatg
gtttttaagcaccaaattgatggtcacaccatttccttttagtagtgctactgctatcgctgtgtg
aatgttgcctctggggattatgtgacccagtggttctgtatacctgccaggtgccaaccacttgta
aaggtcttgatattttcaattcttagactacctatactttggcagaagttatatttaatgtaagtt
gtctaaatataa
(SEQ ID NO:74)

Homo sapiens ribosomal protein S28 (RPS28)

gcttggctgctcgctgggtcttggatgtcgggttcgaccacttggccgatgggaatggtctgtcac
agtctgctcctttttttgtccgccacacgtaactgagatgctcctttaaataaagcgtttgtgtt
tcaagtt
(SEQ ID NO:75)

Homo sapiens ribosomal protein S29 (RPS29)

atgctcttccttcagaggattatccggggcatctactcaatgaaaaaccatgataattctttgtat
ataaaataaacatttgaaaaacccttc
(SEQ ID NO:76)

Homo sapiens ribosomal protein L15 (RPL15)

tataagtaaagtttgtaaaattcatacttaataaacaatttaggacagtcatgtctgcttacaggt
gttatttgtctgttaaaactagtctgcagatgtttcttgaatgctttgtcaattaagaaagttaa
agtgcaataatgtttgaagacaataagtggtggtgtatcttgtttctaataagataaactttttg
tctttgctttatcttattagggagttgtatgtcagtgtataaaacatactgtgtggtataacaggc
ttaataaattctttaaaggagagaactgaaactagccctgtagatttgtctggtgcatgtgatga
aacctgcagctttatcggagtgatggcaatgctctgctggtttattttcaagtggctgcgtttttt
ttagtttggcaggtgtagacttttaagttgggctttagaaaatctgggttagcctgaagaaaatt
gcctcagcctccacagtaccattttaaattcacataaaaggtgaaagctcctggttcagtgccatg
gcttcatggcattcagtgattagtggtaatggtaaacactggtgtgttttgaagttgaatgtgcga
taaaattattagccttaagattggtaagctagcaatgaatgctagggtgggaagctggtgagccag
tggccattagataaatacctttcaagtgtgagcttagacgtcaaccctaaaatacttaaccgtaat
gctaattgtgatcattatgaatcccttcagtcacattagggggaaagtagttggctataagtacgt
cattcttagtccagtcagtcttaaaaacatcttgggttacccactctgtccactcccataggctac
agaaaaagtcacaagcgcatggtttccaaccatatgtgttttctgcagttatttctcttgttctgg

ccaaacaaccctaaaatccttaccattccacaaagttggaccatcacttgtgcacccactttgac
tatgagtataccaccacattgcatttctgtttgcaccatgtcttccaggagactagactactgttg
tccagggtcaatttgagtgtaaagaaaatgtagacaaggaattgcccaattttaaattctgacttt
gctgacttaatttaaatgctcgttctgaaccaattttctcctatcttctctaggggtttcaaaaga
ctcagttaattgatttccaggaagtactcatagcaagttcataaaagttcttgagacctaatttc
ttcacaaaaaaagaaaagatcttaagtcatacattttaattgtgtagaggttgttcaactgaagga
ataaatgtctattaaactaaaacaaatggaccttc
(SEQ ID NO:77)

Homo sapiens ribosomal protein L15 (RPL15)

gcaattcttctgcctcggcctcccaaatagccaggactacaggcgcacactgccatgcccagctaa
gttttgtatttttagtagagactgggtttcactatgttggccaggctggtctcgaactcctgacct
caagtgatccacctgccttggcctcccaaagtgctgggattacaggcgtgagccaccacccccagc
ccaatttttattttttgtacagacaggatctcactatgttgcccaggttggtctcaaactactggc
ctcaagcaatcctgccttggcctcccaaagtgctggaattataggaatgagccaccacaccgggcc
caaatttactttagtaataacaacaattggctgggtgcggtggctcacgcctgcaatcccaacact
ttcggtaaccaaggtgggcttgagctcatgagttagagagcagcctgagcaacgtggtgagagccc
atctcacaaaaaataacaaatcagctgggcatggtgttgcacgcctgtagtctccgaaatcacacc
actgcactcccatcttgggtgatagagccagaacttgtctcaaaaataacaattggtttcttacaa
tcccaaaaggtgcagttactagtattaatcctttttgccaatgaggaaacacaaagatgaagcaa
cttgctcaaagtcatacagtgacagtctgaattcaaatcctatacacttaaagtttatttgttttg
ttttggtttttttgagatggagtctcactgtgtcgcaaggctggagtgcagtggcacgatctcag
ctcactgcaacccgggttcaagcgattctcctgcctcagcctcccgagtagctgggactacaggca
cgcaccaccacacccagctaattttgtatttttagtagagacggtttcaccatgttggccaggat
ggtctcgagctcctgacctcaggtgatcctcccgccttggcctcccaaagtgccgggattacaggt
gtcagccactgcacgtggccaacttaaagtttttgatagataatacattaacgttaaaaattcaaa
agataagtataggctctacagtacaaacccttctgcctcctagttcctctccctggaggcaaggtg
atcagtttaacaatatttttttattttgagacagggtctcactgttgcccaggctggagtgtagtg
gcgcgttcacaacttactgtagcctcaacctcctggctcaagcaatcctcccacctcagcctgtcg
agtagctggaaccacaggtgcacaccaccatgccaggctaattttgtatttttgtagagacagg
gtttcaccatgttgttcaggctggtctcaaagtcctgggctcaagcaatcttcctgtctctgcttc
ccaaagtgctgggattacagatgtgggccacggtgcctggcctacatatgtatttttcctttct
tccccaagtggtaggatatgatacacattgttgatttttttgtttagttatgtatctcagagctta
ttctttatcagctcatgaggaacttcatttttttttttttttttgagatgtagttttgctcttata
gcccaggttggagtacagtaacacaatcttggctcgcagcaacttctgcctcccaggttcaagcga
ttctcctgcctcagcctccgagtagctaggattacaggtgcctgccactacatccagctattttg
tattttcagtagagacggggtttcaccattttggccaagctggtctcgaactcctgacctcaggtg
atccgcccatctcagcctcccaaagtagtgggattacaggcatgagcaaccgtgcccggctggaac
ttcattctttggtataactgcatggtatcccatcatgtggatgtaccatgattcattggatgtgg
accctcctgatggacatttaaatttcttccaatctgttgctattacaaaaagaaaaatgtgtgcat
acatctttattcatctgtagaataaattcttagaagt
(SEQ ID NO:78)

Homo sapiens ribosomal protein S2 (RPS2)
ggtttttatacaagaaaaataaagtgaattaagcgtg
(SEQ ID NO:79)

Homo sapiens ribosomal protein L14 (RPL14)

gtggcaatcataaaaagtaataaaggttctttttgacctgttgacaaatgtatttaagcctttgga
tttaaagcctgttgaggctggagttaggaggcagattgatagtaggattataataaacattaaata
atcagttc
(SEQ ID NO:80)

Homo sapiens ribosomal protein S14 (RPS14)
acaagattcctcaaaatattttctgttaataaattgccttcatgtaaactgtttc
(SEQ ID NO:81)

Homo sapiens ribosomal protein L10 (RPL10)

gggcttccaatgtgctgcccccctcttaatactcaccaataaattctacttcctgtccacctatgt
ctttgtatctacattcttgacggggaaggaacttcctctgggaacctttgggtcattgccctttca
cttcagaaacaggttgacaactcagccctgctcatgaggcagcaaaccctgcaaagggctgggact
ggtggccttatgtcagttgtctactctggagcttgacttggacctccccaggtcctaggcagtagg
ttgaaaaacactgaagtgcttttcatgaagcacagctgcagcaaagccttgcaatcccaggctggg
gtcagcctacagttgtgttgcttattacaacacatgcggaccaagaggggcttgtgggctagaggc
tgaccagcagcgtttatttagcaagggtaggtgtgcatcacattgggcttgttctcacccatctgg
tttggccattcctccttggtgggaatcatccaggtactgctgaggtcacctgcgatttgccccatt
tcctatctctagcaacctcctgggccccatgcccccaccccttctagaacctgcattcccagggcc
ttcaccacctgaccaaaggtctaggctaacctttggtcatttgtaacaagacctcggaacagacac
gtgtgtggcatggtttggcctggggatcttagatgtctgacctgaactattgtagaacagcgctgg
cttttggggagcagcaaaaatgagaggagtgctaggtgggtggcctgagcatctgtatccaggga
caggactccaaaggcttttggtcccagagctggggtatgttggccccagcccccagcctgtggctc
ccaaaaggcctctggtttttttgtaatctcagtttacagccatttcttaggtttttaattaccttta
ttttattttgccaaacatacctgggaatacctttttattttttttttaccttggggtgatggttcca
aaccataaatgtgattatagttaacacatgacccttctagcgtcccagccagtgtttttcctgacc
tctgttctttggagaggaggatggaagggaggggtccggcacgctgctggcattttgctgtgtcct
gcagcccctttccgggacacctgggttcacacagcttttttagcttacataactggtgcagattttc
tgtgtggagatgttgccttgaccagccttggctggactttaccaggcatgcagaagcctgtaccaa
cacagactacagcacccaggaggtgcgagtgtggctgctcagcggttataacaggcctgactgcat
tgttcaccggattataatgagccaaaatgtttcccggtgtttgctggtttcagggaaggagtttga
tatagcagattaaccaccctccttgtagctattggggcttaatggtttcctggtgattcttaccaa
tccacaataaacatggcccattggcatatctgc
(SEQ ID NO:82)

Homo sapiens ribosomal protein L10a (RPL10A)
ggcacatttgaataaattctattaccagttc
(SEQ ID NO:83)

Homo sapiens ribosomal protein L35 (RPL35)
ggggcgcattgtcaataaagcacagctggctgagactgc
(SEQ ID NO:84)

Homo sapiens ribosomal protein L13a (RPL13A)

gcccaataaagactgttaattcctcatgcgttgcctgcccttcctccattgttgccctggaatgta
cgggacccaggggcagcagcagtccaggtgccacaggcagccctgggacataggaagctgggagca
aggaaagggtcttagtcactgcctcccgaagttgcttgaaagcactcggagaattgtgcaggtgtc
atttatctatgaccaataggaagagcaaccagttactatgagtgaaagggagccagaagactgatt
ggagggccctatcttgtgagtggggcatctgttggactttccacctggtcatatactctgcagctg
ttagaatgtgcaagcacttggggacagcatgagcttgctgttgtacacagggtatttctagaagca
gaaatagactgggaagatgcacaaccaaggggttacaggcatcgcccatgctcctcacctgtattt
tgtaatcagaaataaattgctttt
(SEQ ID NO:85)

Homo sapiens ribosomal protein L36 (RPL36)
gcccctcccctgccctctccctgaaataaagaacagcttgacag
(SEQ ID NO:86)

Homo sapiens ribosomal protein L36a (RPL36A)

gtgtcatcttttattatgaagacaataaaatcttgagtttatgttcacttcatttgtttgctgttc atcttttgggagggaataagctagagccatcaatacaattccgcttgtggggaaatttatgcctct tactggtactacttgttttgcattgaagctgactggttgagttcacatcatatgttgcaattttct aatttggcacttcaatcactaggggccttatgaggcagtttgtcattatgcaatggttattggtta tcatgtgagtagacacatttcaggctaatagggagaagtcagtaacacattcatagtgaatatgag atgtctttgctaagagttaagtgtcagatctttgttataacagttaatttaataaagaattttggc attgttcttc
(SEQ ID NO:87)

Homo sapiens ribosomal protein L36a (RPL36A)

ttgccgtaaggatatgcacttgtctctagtccacacacttcatgatataggtatagcgttagttta gcgaagttttcactgcactgatatatctagtaggtgatggagctgggaatgcaactcatgtctgac tagtccacaatactgcactatttcagtgtttacgatttttatcctttcccttctgaagaggcaaa aaattgaggaatgtgccctgctttcctaagaactgaagtgtgagtacactggtaaatcctttcatt tgccttgttccttatctgtcaatatgtctgaatcctcgcttgttggttgcactaagaattgttctg ttgtttctcatcacagaaatctgcagtcaactacctgttctcgtgaagtcttaaaactcttataga atagccatttaggcctttctgctagcctcctgaattctgtattctcaggctgagcgagtttctgtt tactctcaaaccttaggtgatttggctaactcttaaagtaattagcacgatgattggaacggagca ttctctccaacacagcatttctttggcactttgcttcttgtgcagtttagctccagaaagtatta aggaatgactttagtgctcatttggatgcagtaagtggtttgatctcagggtggcaaaaagaatgc ttttttataccttttcacattcggataacttgtttagaagacagaggttctaactaggttttggc ctattaagaactgcaaactagcagcagcagaactctggctaaaggggcaagcttattaggaaattg agtatttaaaagttgagctaccatatgatccaacaatcccactgctgggtatatacccagaagaaa atcggtatatcaaagagatatctgcactcctatgtttgttgtagcactgtttataatagctaagat ttagaagcaaccttagtgtccatcggatgaatggataaagaaaatgtacctatacgcggccaggc acggtggcttgtgcctagcactttggaaagccgaggcgggtggatcacctgaggtcaggagttcga gaccagcctggccaagatagtgaaaccccgtctctagtaaaaatacaaaaattagccgggcttgtg gtgtgggcctgtaatctcagccacccgggaggctgaggcaggagaatcgctggaacctgggaggca gaggctgcagtgagccgagatcacgccactgtactccagcctgggcgacagagcaagactccatct caaaaaaaaaaaaaaaaaaaagggaaaaagaaaatgcacctatacacagtggtactattcagccat aaaaagaatgagatccagtcatttacaacaacatgggtggaactggagatcgttatgttaagtgaa ataggcacacaaagacaagcatcacatgttcttgtttgtgggatctaaaaatcaaaacaagtggac ttgtcatatagagagtagaaggatggttaccagaagctgagaacttctggtggcgggaggtgggga tggttaatgggtacaaaaagaaaaaagaatgaattagaccaactatttgatagcacgacagcgtga ctaaagtcaataacttagttacatattttaaaataacttagagtgtaattggattgtttgtacctc aaagaaaaaatgcaataaaactttacagtggagaaacctaacaagcactacctcagccaggtaatc aaggttaacatcaacagtcacgagtcatgttgatatataccccttgataaggtgtgatgaaaatgac acttaaacctaaaaatccataaccctatctaatgagaaaaataacaaatcccaagaggggcatttt acaaaatacttgaccagtagtgcggaaattgtcaaggtcatcaaaaaagtctgagaaattgccaca gccaaaggagtctagagacatgatgactaaatgttaggtggtgtcctgcgtggggtcctagaacag aaaaaggacattag
(SEQ ID NO:88)

Homo sapiens ribosomal protein L41 (RPL41)

accgctagcttgttgcaccgtggaggccacaggagcagaaacatggaatgccagacgctggggatg ctggtacaagttgtgggactgcatgctactgtctagagcttgtctcaatggatctagaacttcatc gccctctgatcgccgatcacctctgagacccaccttgctcataaacaaaatgcccatgttggtcct ctgccctggacctgtgacattctggactatttctgtgtttatttgtggccgagtgtaacaaccata taataaatcacctcttccgctgttttagctgaagaattaaatc

(SEQ ID NO:89)

Homo sapiens ribosomal protein S18 (RPS18)
gtctgtaggccttgtctgttaataaatagtttatatac
(SEQ ID NO:90)

Homo sapiens ribosomal protein S24 (RPS24)

agtgtctagcagtgagctggagattggatcacagccgaaggagtaaaggtgctgcaatgatgttag
ctgtggccactgtggatttttcgcaagaacattaataaactaaaaacttcatgtgtctggttgttt
g
(SEQ ID NO:91)

Homo sapiens ribosomal protein S24 (RPS24)

tgtcactgccatggccgccttgctgcatttctgaggatgcttcatctctccaccttcttctccact
cagcagccagcagggcactgtggaaatcggagtcacatgagctggcacctctgttcagaaccctcc
agggctccacatctctctcacccaaatgccaaagacctccccacgcccccacaatcccccacgacc
tggccactggcctcccaccaccttccagctccagcggctcctaccacatttaaggctttccttcct
agttttaatttttcctcgtcagcagttgatttattatttcttgtttattggtattttcccacta
gaaatgaagctgcgtgaagttagagatttttttttttggtctgtgttcctaattagctcattgcta
taccccctggcgcccagaacaatgccttggacacagtacgcagtagactaaataaatacttgttgaa
tgactgactgacggaatgacggctgtgtggggagtggattgggtcgtgaggcagaggctgcggtgg
aaactcaggcaggaggtgatggtggttcttggggctgcggaatgccaagtttagaagctcttcctc
tgctgtggcacatgaaccggtcactcgagaaggcttttagatttactttgcctaatcccctcttag
tgcatgtggggaaactgaggtacacaaaaggaattccccaccaagttaggggcagaacctagcccc
cttgtctcccagatggatatcttcttttttttttgagacggagtcttgctctgttgcccaggctgg
agtgcagtggtaccatcttggctcactgcaacctctgcttcccaggttcaagcgattctcctgcct
cagcctcctgagtgtctgcgattacaggtgcacacaaccacgcctggctaattttgtattttttag
tagagacggggtttcaccgtgttggtcagggtgacctcaaactcctgacctcatgatccacccagc
tcagcctcccaacgtgctgggattacaggcatgagccaccgtgcctggctggacatcttgttatta
aagcttcttctctctttgtaggggagggggagatgcctctggtggagaagaccagtgtggcagtga
ctgtgtctgttagtgaacctggtggctggttgagggtctgtcgtggtgactgaggacacatacaaa
gtgcttttctcagtggtcaccttggtgttggtgaataagggtcagaagatggctcctgtcctaggg
cactgccagtcggtttggaagctgaaatgcctgcttagcagtttgaggaaacacagaccttggagg
atcttctggttgcctcttcaagaattcattctattccccttctgctccccaaatttgcttttcttg
gggtgggtcttggttggcctaagccaagaaagtatggcatctactccttccatagcaatagctcag
gaataggcagtgacccagacctgaaccaatcagtgcatggaattacccctggccaaagtggttgat
tgaggctgggtgcaagcagagttgtgagaaggctcccatttggtggttggagagatcgcacttgct
ccagaggtcataatgtgcagatctgaggcttggaactgctgcagacattttgctaccacaagtgaa
gccaccctgacgacacagttgacaatttggagcagggcagagctgagagaacagcagggaaacagc
cagagtcttgctcaagcctccctgaagtatctataccccctggactctagttatggggctaataaa
tgttatatactgtttaaggt
(SEQ ID NO:92)

Homo sapiens ribosomal protein L8 (RPL8)
tgctgagggcctcaataaagtttgtgtttatgcc
(SEQ ID NO:93)

Homo sapiens ribosomal protein L34 (RPL34)

aaaaatgaaactttttttgagtaataaaaatgaaaagacgctgtccaatagaaaaagttggtgtgct
ggagctacctcacctcagcttgagagagccagttgtgtgcatctctttccagttttgcatccagtg
acgtctgcttggcatcttgagattgttatggtgagagtatttacacctcagcaaatgctgcaaaat
cctgtttttcccccagagagctggaggttaaatactaccagcacatccctagatactactcaagtta

cagtatatgatcactaatatagtatgctcttggtaccaggagctctgatatatatctggtacatgt
ttgataatgacttgattgttattataagtacttattaatacttcgattctgtaaagagtttagggt
ttgattttataaaatccaaaatgagccttttattgaatccagttctctatgtgaccagttctctgt
atgaatggaagggaaaagaattaaaaatcttgcaaagggg
(SEQ ID NO:94)

Homo sapiens ribosomal protein L34 (RPL34)

aaaaatgaaactttttttgagtaataaaaatgaaaagacgctgtccaatagaaaaagttggtgtgct
ggagctacctcacctcagcttgagagagccagttgtgtgcatctctttccagttttgcatccagtg
acgtctgcttggcatcttgagattgttatggtgagagtatttacacctcagcaaatgctgcaaaat
cctgttttcccccagagagctggaggttaaatactaccagcacatccctagatactactcaagtta
cagtatatgatcactaatatagtatgctcttggtaccaggagctctgatatatatctggtacatgt
ttgataatgacttgattgttattataagtacttattaatacttcgattctgtaaagagtttagggt
ttgattttataaaatccaaaatgagccttttattgaatccagttctctatgtgaccagttctctgt
atgaatggaagggaaaagaattaaaaatcttgcaaagggg
(SEQ ID NO:95)

Homo sapiens ribosomal protein S17 (RPS17)
attttttctgtagtgctgtattattttcaataaatctgggacaacagc
(SEQ ID NO:96)

Homo sapiens ribosomal protein SA (RPSA)

gctgttcttgcataggctcttaagcagcatggaaaaatggttgatggaaaataaacatcagtttct
aaaagttgtcttcatttagtttgctttttactccagatcagaatacctgggattgcatatcaaagc
ataataataaatacatgtctcgacatgagttgtacttct
(SEQ ID NO:97)

Homo sapiens ubiquitin A-52 residue ribosomal protein fusion product 1 (UBA52)

ggtggttctttccttgaagggcagcctcctgcccaggccccgtggccctggagcctcaataaagtg
tccctttcattgactggagcagcaattggtgtcctcatggctgatctgtccagggaggtggctgaa
gagtgggcatctcccttagggactctactcagcactccattctgtgccacctgtggggtcttctgt
cctagattctgtcacatcggcattggtccctgccctatgcccctgactctggatttgtcatctgta
aaactggagtaaaaacctcagtcgtgtaattggtgggactgaggatcagttttgtcattgctggga
tcctgtcaggcactttgaggtgtccctcaggccttggccctgaagtgtctaggtgtgtggagatgg
gtagaaaattaggtacacccaatggtgtagaacgttgattctcaaattttttattttatacaaat
ggggtctcactatgttgtccaggctggtcttgaactcctgggctcaagccatccgcccatctcagc
ccctcaaagtgttgggattacaagcaagaactgccatgcctgacccagttctcagtttttttgtttg
tttgtttgtttgtttgttttgagacggagtcttgctctgtcgcccaggctggagtgcagtggcgca
gtctcggcttactacaacctctgcctccggggttcacatccttctcctgcctcagcctcccgagta
gctgggactacaggtgcccgccacaactcctggctaatttttttgtatttttagtagagacggggtt
tcactgggttagccaggttggtctcgatctcctgaccttgtgatccattcgccttggcctcccaga
atgctggtattacaggcgtgagccagcacgcctggcccagttactcagttttgaatctgaggccgt
gacatcactcatggtctgcagtcagtgctctgcccctgagctgtaccctctcctatgataatcact
cttaagaagggcaacccttggtgtttcccccttaaggtcacccaggctggaatgcagtggtgtggt
catggctccctgtaccctggaactcaggcttgggtgatcctctctcctttgcctccgaagtagcca
ggactacaggtgtgcacccaccaccacactcagataattgctttggtgtttttaaagcttgtaatg
atcagtaggctgaggtgggcaaatcataaggtcaagagtttttttagatggggtgagcacagaccaa
ttcctgttttatttactgatttaaaattttgagacagtctcactgtcacccaggttggggtgcagt
ggtaggatcatagcttgctgcagccttgatctcccaggatcttgcctcagcctcccgagtagctgg
gactgcatgcttgtgccaccacactcggttaatattttgtagagatggggtcttgctatgttgccc
aggctggcttcaaactcctgaacttaaaagcctcctgtttagttttggtttttatcacttttttt

tttttttttgagatggagccttgctcccatcgtgcaggctggagtgcggtggcgcagtctcggct
cactgcagcttctgcctctcgggttcaagcgattctcctttctcagcctcttgagtagctggaatt
accagtgtgcgccaccaccacgcctggctagtttttctgtttttagtagagacagggttttgc
tatgttggccaggctggtcttgaactactgacctcttgtgatctacctgtcttggccttccaaagt
gctaggattacaagcgtaagccacagcgcctggccttgctacatttttttttttttttttttt
acagacatggtctcgctatgttgcccagaatggttttgcactgggtccaagcagttctgccgcagc
ctcccaaagtgctgggattacaggggtgaggcaccttgctggcccctgttttgattagggtgcagt
gctggtgaagccggtgcacgaggccagtgatgcatcctaatgaggggtggagttggcgggacttcc
tgggccagtttggggactttcacaaaagacccccatgactcagggttttgagttcttaactgatcg
aatgaaggattcaaaattaaccactccaagggggattgaaggaagaaccactcttaatggacaaa
aagaaagaaagggggagggagtaacagggatatgagctctagccgcccaagctagcaatggcaaccc
ttctgggtccccttccagcatgtggaagctttcctttcgcttcattcaataaacagctgctgctc
(SEQ ID NO:98)

Homo sapiens Finkel-Biskis-Reilly murine sarcoma virus (FBR-MuSV) ubiquitously expressed (FAU)
gtcttttgtaattctggctttctctaataaaaaagccacttagttcagtcatcgaaaa
(SEQ ID NO:99)

Homo sapiens ribosomal protein L22-like 1 (RPL22L1)

gcaaaggctccccttacagggctttgcttattaataaaataaatgaagtatacatgagaaatacca
agaaattggcttttagtttatcagtgaataaaaaatattatactcttgaacttttgtctcattttt
ttgagtatgctgtttatatgattttgatttccctctgataactatcaacagtatttaaatagctta
tagctggtataatttttcccacgatttccaaaatcttttatgtactcaggtaaaagtagcgttat
ataggaaatcttttttttagacactctcgttctgtcacccaggctggagtgcagtgactcagcttc
ctaaatagctggaattacaggtgtgagccaccatgcccggctaatttttgtacttttagtagagt
agggtttggccatgttggccaggctggtttcaaactcctgacctcaagtgatctacccacctcggc
ttcccaaagtgctgattatagctgtgaaccaccatgcccggccaggaaatcttactgtagaacaat
tttttatatagctgtataaaatgtatatgattgtcttgacagtctcaaatactgtttttaatagct
tgtaaatgtaatctcaagtgcttagaacagttcttacatataagttgctctgtagtttgctcttat
agttagcccaaagactctgggtgtgaggcctgctgtaaaccaatgttaaactgcttattagaaagc
cctaaccacctgctttgtaggcaccagaaactcaaaaccaaatctcaactcagctacagaatctac
tgtggtccttgtctgaaaaaattagttcactcggttggaatcttgtctcagagcatcctcatctct
ttctcaaaagcccctaccccaacaccggcgtgttggttgtctattgaaacttacaagtggatggac
cctttctcccgaataaactggcctttgaaagctctaatcgaaatggtttggcaaaatccatactgc
aggagattagggaggacaagaatgatgtgccttttttgtactgctgagcctgatggtggtgccacta
cttcaggtacttagatgagtcttgatgctaatagaattgtgtcgccaaacatatctggacagttac
aacctaatctatgcattaattggtttgggaattgcttgaaattattgtttaattcaatgttttaat
tcgttttcctaaaaatttaagtgcccccatcatcgtgcaatacctcagtgcagcaactccttgatt
cttggatgactgaacttcctaacttggctctgccccattgttcccatttttcatgtttttcacaaa
tagttaaccaggtacctactactgtgcaccgctgcagagcattgaggatgtatgtgatgagtaaaa
acacccagcctgctctgctgtgttagtattatgacggaaactgatcaaatcacatgtgaacaaatt
tactgctacaaaagggagggcttaataaaggaatttcatctgggaaggc
(SEQ ID NO:100)

Homo sapiens ribosomal protein S17 (RPS17)
attttttctgtagtgctgtattattttcaataaatctgggacaacagc
(SEQ ID NO:101)

Homo sapiens ribosomal protein L39-like (RPL39L)

ggaattgcacatgagatggcacacatatttatgctgtatcaagttcacgatcatcttacgatatca
agctgaaaatgtcaccactacctggacagttgcacatgttttactgggaatattttttttctgtttt
tctgtatgctctgtgctagtagggtggattcagtaataaatatgtgaaagcttttgtttcc
(SEQ ID NO:102)

Homo sapiens ribosomal protein L10-like (RPL10L)

aggttttggcagtactgtctccttgggccatgctggtctgacttatgcttactaataaattctgtt
tactggc
(SEQ ID NO:103)

Homo sapiens ribosomal protein L36a-like (RPL36AL)

actttgggatatttttcttcaattttgaagagaaaatggtgaagccatagaaaagttacccgaggg
aaaataaatacagtgatattcttacgc
(SEQ ID NO:104)

Homo sapiens ribosomal protein L3-like (RPL3L)

gctgtgtggggtggatgaaccctgaagcgcaccgcactgtctgccccaatgtctaacaaaggccgg
aggcgactcttcctgcgaggtctcagagcgctgtgtaaccgcccaaggggttcaccttgcctgctg
cctagacaaagccgattcattaagacaggggaattgcaatagagaaagagtaattcacacagagct
ggctgtgcgggagaccggagttttatgttttattattactcaaatcgatctctttgagc
(SEQ ID NO:105)

Homo sapiens ribosomal protein S27-like (RPS27L)

tgattcaaacagcttcctgaattttaattttgtgttgtctcacagaaagccttatcataaattcca
taattctaattaatttaccaagataatgtaattacatttggttttgtaaggtatacagcagtaatc
tcctattttggtgtcagtttttcaataaagttttgattatgggcaaatcccctctttttcttttt
taaaatatatttgagtatgccatacatttatatatatggtgtatatgaatttggtttaaacatttt
aaaatttattctgattagtttgtgtcttttttttttttttgagagagagagtcctgctctgtcac
tcaagctggagtgcagtggtgcgatctcggctcactgcaacctccgcctcccaggtccaagcaatt
ctcttgccttgtcctcccaagtagctgggattataggcacacaccaccatgcctggctaatttgtg
tctcattttcaagagtagaaaccctaaatatttatttcattcctttccaaattgctatgaatg
ggattaaaggattacagatgtaaagtctattatttgtgaattctaaatgtagttctgctgttgtac
ctgtggaaacatcttaaagaagtacatatttgcacgtcctgcacgtgtaccccagaacttaaact
ataattaaaaagaatagtttcaaaaaaataca
(SEQ ID NO:106)

Homo sapiens ribosomal protein L26-like 1 (RPL26L1)

atagaacctgttgtgcaaccacggtttaaccggagattttgaggctagggtgtgtttctttcgaac
ttttcggaatgtctggaacatttcatttcctgttttgttacctgtgcctctgtaaatctactttg
caattttaagtaataattttatgaataaaaatgggaaatgcttcctaattccacatagtatttgca
ttgttttataaataaattccacttactatc
(SEQ ID NO:107)

Homo sapiens ribosomal protein L7-like 1 (RPL7L1)

acccaggtgaggcagggctgaaaactgcccttgggctgacttttgataggccatgccttgccactt
tacaagttctttttgcatttactagtatttaagagtaaccttgagattgggaggaatagaggaggc
tggtacaaatagatggagacctgctgggatcagtgaatgcctgattaggacatggggctatgcata
gcctaagagttataggcttaaagatgtcgagtaactaaaaactgtattgctggccgggcgcggtgg
ctcacgcctgtaatcccagcactttgggaggccaaggcgggcagaccatgaggtcaggagattgag
accatcctggccaacatggtgaaaccctgtctctactaaaaatacaaaaatgagctgggtgtggtg
gcacgtgcctgtagtcccagctactcgagaggctaaggcaggaaaatcgcttgaacccaggaggca

gagattgcagtgagccaagattgcaccagtgcactccagctgggcgacagagcgagactccatctc
g
(SEQ ID NO:108)

Homo sapiens ribosomal protein L13a pseudogene (RPL13AP)

gtggaaaagaacatgaaaaagaaaactgacaaatacacacaggtctcctcaagatccatggacttc
tggtctgagcctaataaagactgtttgtttattcctcaaaaacaaacaaacaaaaaaaaaccctct
gtattataaattattctgtgtaatggtgtgttaccatacatt
(SEQ ID NO:109)

Homo sapiens ribosomal protein L37a pseudogene 8 (RPL37AP8)

atgctcctctactctttgagacatctctggcctataacaaatgggttaatttatgttaaaaaaaa
aaaagagagagagagtgaaacaacaatctacacaatcagagaaaatatttgcaaatcttatatctg
attagaaattagtatctggaacat
(SEQ ID NO:110)

Homo sapiens ribosomal protein S10 pseudogene 5 (RPS10P5)
aattggagaggattatttcacattgaataaacttacagccaaaaaa
(SEQ ID NO:111)

Homo sapiens ribosomal protein S26 pseudogene 11 (RPS26P11)

ggagctgagttcttaaagactgaagacaggctattctctggagaaaaataaaatggaaattgtact
t
(SEQ ID NO:112)

Homo sapiens ribosomal protein L39 pseudogene 5 (RPL39P5)

ggaattgaacatgagatggcacacatatttatgctgtctaaaggtcacaatcatgttaccatatca
agctgaaaatgtcaccactatctggacagttggacatgtttttttgggaatatactttttctctct
gaatctgttaggaactttctggttggctgggttccgtaataaatacatgagacctttcatttcaaa
aaaaagaaaaataggcctccttcccaggggctccggatttcatcagccttctgtgcatgcccagcc
atacaaaccacgcagggatggctccaagtg
(SEQ ID NO:113)

Homo sapiens ribosomal protein, large, P0 pseudogene 6 (RPLP0P6)

tcaccaaaaagcaaccaacttagccagctttatttgcaaaacaaggaaataaaggcttacttcttt
aaaaaataaataaataaataaataaataaataataaataaataaataaataaataaatagataaat
aaataaaaagttttctactcacactgaagtgacgaagtc
(SEQ ID NO:114)

Homo sapiens ribosomal protein L36 pseudogene 14 (RPL36P14)
gcccccttcccctgccctctccctgaaataaagaatagcttgacagaaa
(SEQ ID NO:115)

Further preferably, the at least one 3'-UTR element of the artificial nucleic acid molecule according to the present invention comprises or consists of a nucleic acid sequence which has an identity of at least about 1, 2, 3, 4, 5, 10, 15, 20, 30 or 40%, preferably of at least about 50%, preferably of at least about 60%, preferably of at least about 70%, more preferably of at least about 80%, more preferably of at least about 90%, even more preferably of at least

about 95%, even more preferably of at least about 99%, most preferably of 100% to the nucleic acid sequence of a 3'-UTR of a ribosomal protein gene, such as to the nucleic acid sequences according to SEQ ID NOs:116 to 205 or the corresponding RNA sequence:

Mus musculus ribosomal protein L9 (RPL9)

GGAGGCCTCAGTTCCTGGCCCCAGAAACGAGATCCTGACCACATGAACAATTTGGGCTCTTTTGGG
AGAATAAAAGACTTATATATTG
(SEQ ID NO:116)

Mus musculus ribosomal protein L3 (RPL3)

TTCCAGGACCACTTTGTGCAGATGGTGGGGTCTCACCAATAAAATATTTCTACTCACACTGGTTTT
CCC
(SEQ ID NO:117)

Mus musculus ribosomal protein L4 (RPL4)

ACTATTAAAAATTGTTAAATTCCAGAGAGCAAGTAGAGACCGCATATTTCAATAAATCAAACATGT
GGTGACAAACCCTTGTGTGACTCTTAAATTGTGGATGTTTCCAAGCCCCTTG
(SEQ ID NO:118)

Mus musculus ribosomal protein L5 (RPL5)

AGCAGTTTTCTATGAAGATTTTTTCATAAAGACAATAAACATATTGATCAAGCAGCTTTTTCTGTG
TTAAGCTGTTATTAATGAGACTATAGGAAATAGTGTGAAATTACAAAAGCAAAGAAGTAGATAGTT
ATTTAATTAATTAAATTAATTTTACCTTTTGTGTTGCACCATAACCTACCACTGGTGGGATTAAGG
GCAAGTATTACCATGCCTAGCTGAGAGTCTTTCTCCAGGAAAAACCAGCTTACATGGGTTCCTGCA
AATCTCATGAGTGTTTCTTGGGTTTCTAGTCTTCCTGGGAGGTGTCCTTATCTTTCAGATTTTCAG
ATCTGGTAATTAGCATGATCATCAGGACATTTATTACAAACAAATTGATTAGTGGGAAGAAAGTAT
CTCAAGGTCAATCTTGGAAGTGAACAACTGGTGCTAATCCATGGCTTTAAAGATTTGAGAACAACG
GTGAAATTTGGTTTGAGGAGAAGGGGGTGTCTAGGACGTTTCATTTTTATGGTACATGCCAGACAT
GAATGTACATAGGAAAATAACTTGAAAGGGTCAAATATTAAACCTTGAATATCAGGTTCACTTGGG
AAAGCATTAGGTGCTTATGCCTCTTAGTAAATAGCCCTTCATCCCAGAAGGAGCAAGAATTGTCTT
CCTGACTTAATCCAGTCTTAGCTGAGGTGCTGTGCATCTTTATCATCTTTGCCTTGCCTCACAGTG
TCAGGCTCTGTGGTACTGGGGCTACACAGGTCAGGTAAACAGTTAACTGCTTACCTACATCCCCAG
CAAAGATAATGTGACGATACTAAGATGAACCTATCAGAGCTTAAAGATAATGAGTTTCAGTCACAG
TGATAACTGCATGCTAACTTCAGCATGTAGAATATATGCCGAAGCTAAAAGCCATTCCACAGTTGA
CTCCATCTGAAGTTAAAGTGTGTAAGTACACAGTAAATCATGCTATATTAACTGAACTTTTTAATA
AATGAGTCATTTGAATTT
(SEQ ID NO:119)

Mus musculus ribosomal protein L6 (RPL6)

ATTGTTAACCTAATTAAACAGCTTCATAGGTTCTTTTGGTGTCCTTTTTGTGTGTTGTGTGTGCAC
ATGTTTGTTGGGTGGGTGTTTTGCTGGTGTCTTTTCCTCTGTGTCTTCCTCTGGCCCTTTCTGGAA
AGACCTGCTTAATCTGAAGCATGTGAGCTAGGCTAGTCCACTGGGTCCTGCTCTCTGCCCATCCCC
AGCTGGCTTTGGATTAGAGGCACATACACTGCCATGGCTGCCTTTTACTGTGGCTGTGGTTTTGCC
CTTTTTTTTTAAGCAAATAGAAAATGCTGCTGACTATACTGG
(SEQ ID NO:120)

Mus musculus ribosomal protein L7 (RPL7)
GGTGTCACCCATTGTATTTTTGTAATCTGGTCAGTTAATAAACAGTCACAGCTTGGCAAATTG

(SEQ ID NO:121)

Mus musculus ribosomal protein L7a (RPL7A)
ATGTACACTAAATTTTCTGTACCTAAATATAATTACAAAATTATCTTGA
(SEQ ID NO:122)

Mus musculus ribosomal protein L11 (RPL11)
ACTTGATCCAAAAAGCTAATAAAATTTTCTCAGAAATGC
(SEQ ID NO:123)

Mus musculus ribosomal protein L12 (RPL12)
GAAGCAACAAGAAAATATTCCAATAAAAGACTATCTGATAACCAGTG
(SEQ ID NO:124)

Mus musculus ribosomal protein L13 (RPL13)
TTCTGTGTTGGAGAGCTGCAATAAATTTTCCATAAAGCAAAA
(SEQ ID NO:125)

Mus musculus ribosomal protein L23 (RPL23)

TTCTCCAGTGTATTTGTAAAATATATTCATTAAAGTCTCTGCTCTGAGAGCTGGTCTTCTTGACAC
CTTTTCCAATATCAGCTTTGCAGAAGGAAACTTAAATTTCAGTTCAGGGCATGACCTTCATGACCT
TGCAGAACTTCTTCACTTTCCAGGTTAAGTAAAGGCGATCTTTAGGGGCTGTCCAGATGGATCAGC
TATAAAGATTCAATTGTAGAAGGTTCACGTCTCAATGCCCACGTGGTAGCTGTAACTTCAATTAAA
AAACAAAAACAGCCGGGCGTGGTGGTGCACGCCTTTAATCCCAGCACTTGGGAGGCAGAGGCAGGC
GGATTTCTGAGGCCAGCCTGATCTACAGAGTGAGTTCCAGGACAGCCAGGAATACACAGAGAAACC
CTTGTCTCCAAAAACCAAAAAAAACAAAACACGCATTCTTTTCAGGTCTTTGCTGGGACCAGGTAC
ACATAACACAGATAAATATTAGAGCAAACCATGCACATATGGTAAATTATCTTTGGGTTTTGGGTC
CCTAAAATAAAGTGGTGTGTTCATTGTG
(SEQ ID NO:126)

Mus musculus ribosomal protein L18 (RPL18)
CCCTGGATCTTAACTGTTAATAAAAAAAACATTGGATGATGATGGTA
(SEQ ID NO:127)

Mus musculus ribosomal protein L18a (RPL18A)

ACACAGAGACCCACTGAATAAAAACTTGAGACTGTCCTTGCTTGTTTGCTTCTATGTCCCTGGAGA
GGTCCCAGTTGGTCCCGTCCCTAACAACATGCTAGCCCTGCTCACCTGCCTGTCAGCCTTGCTCAG
TGGCATCTTTCCATAGGTGTGTATCCCCTTAGATTAGCTTCAGCCCCACTACGATTTGTCTAGGAC
ATAGCCTGAGCCCTGCCTGTGACACTGAGGGGTAGCAGTCTGTTTCTGGACTCCAGGGTGCTGCTG
TCTCAGGCCTAAGAATTCCAGACATGACTATAATCCAAGCCTGGGGACCTGGTTGAGCTTTTTATC
CTGCTGGCTCTAAGCTTCAGCTAGGTGGAAATGAGGCCAGCCAAGCCCCACAGTGAGCTTGCAAGC
TTTAGATGGGGACAGGGTTACGCTTTGGTGAATGATGGAGGAAACATGGGGGTTCCTTTTGTTGGG
TGCAGCCAGCACGGCATCATCATGGTGCCCAATCTTGAAAGGGCACAGGCCTGAAGCTTCCTGGGA
CTGTTCTGTCACAGGGAGGAACCTACTGCAGTTGCCTACAATTGCTACCTCTGAGGGACTTGCCTC
TGGCCCCTTGTAGACATTTCCATGTCTACACATGGCCCAGAGTACTTTCAGGGATAGCAATGTGTG
AATGGCACTTAGAAGATAACATGTGAAAGCCAT
(SEQ ID NO:128)

Mus musculus ribosomal protein L19 (RPL19)

AGCTTCCCTCGTGTCTGTACATAGCGGCCTGGCTGTGGCCTCATGTGGATCAGTCTTTAAAATAAA
ACAAGCCTTTGTCTGTTGCCCTCTTGTTTAGC
(SEQ ID NO:129)

Mus musculus ribosomal protein L21 (RPL21)

TGTACACAAAGAAATAAAATACCAGCACCAGGACTGTGAAGTGTTTTCCTTAGACTGTAGTGTGGG
GTTTGCTCATTGGCTTTCTTGTTCAGATTTTACTAATTGTTCTAAATGATACAGCTTAGTGTGCAG
AAAATATCCTCTTGATTGGAAAATAGCCAAATATTTACAAAACAGGTATACTAGTTTGAAGAGGCT
CTATATGGGGGGAGGGGGTGCTGGAAAACATTAGTGGGTGACCAGTAATGGTGGTACAGCTCTAAC
TCCTAGCACCAGGAGTCCGAGGCAGGGGGATCTTCAGGCTGCATGATGTGCATAGTAGCATGCTGG
TATTAGGGAGTGGGTATCTGTGGTTCCCACTTTGAAAATAACCAAAATTCTCCAAAGTGGGCAGAC
CTAAGCCAGGAGAGGGCTGGCCACAGACATTTGGTACTGCTTGCTGAGAAAGCACTGATTGTTTTC
CTAACCTAAAGATTATATATGGCCCACCATACCATCTTTGAAACAATGTGTACTGGCCTTTGGTTC
ACCTTTCTTGTCTTTGAAGTTGTACTTGGTGGGTGCATTTAACCTTGCCACAGAGTGGGGAGGATA
GAGTCTAATGGACCTTAAGTGGTCTCTGGTGGCCATGTCGGCAGTGCTTAGGTTGTAGCCCAGGGT
TGGAGTCGGCAGTGACAAGCAAATAACTATATTCTTGCTTGCTGTGGCAGCTATACAGAAATTTAC
GGTATAGGTAAGAGGGTTCTCTAGAAGTACTACCTGTCTTAGTGAAAGAGATTGCTTGGTTAACAT
CCTGTTATGTAGTGGGGCTACTTTTAAACTGTGTGAAGTCCCCATTAGCCACCTCCATAGGCAATG
GAGCTAACATTCTTGCTACAGTGGCCGCAGCTCATTAACACCTAATGATGTGTTTAACATGTGTCC
ACATGGTGTGAATGTGGGTACGCATGTGCCCAGTATTCAGTTCACAGAATTGTCATCATCTTCCAT
CATGTCTTCAGTGAGGGACTCTGCAGATGCCCACCCCAGTCCTTGGTTGTGGTGATTCTGTTAGCA
TTAAATGCACTGGAGAGCTTC
(SEQ ID NO:130)

Mus musculus ribosomal protein L22 (RPL22)

GACACATTGGTCTGCAATGTTTTGTATTAATTCATAAATAAAATTTAGGAACAAAACCGGTGGTTT
ATCCTTGCATCTCTGCAGTGTGGATTGGACAGGAAGTTGGAAATGACAGGGACTTTAACTGGGCTG
CTGCTCCTTTGTATATAGACACTTTTTTCCTGCTCAGAAACTTGAGTTCTCCAGTAGCAATGGCCA
AACAGAAGAACCAGGCTAGGGGGCTGCATCTGACAGAGCAAGTAGACGAGAGGCTGGGTGGTGGGC
TCCGGCCAGCCCGAGTCTTAGAGCTGGTGGTTGGTTATATCTGGTGCCTGTCTCGAGGAGGGCTTG
AGACACAGTGTGGTGCTCCTCAGAAGCAGACAGGTGATTTCTTTGTGTGATTTTTCTTTTCCCCTG
GGACAATGACAGTCAGTAAGACAGGTTTCAGGGACTTTTGTGTCCAGGTCTGAGCACTAGTCGCTC
ACAGTTGTGTGTACTAACCTTCTTCCTTCCTATTGAAATGGCAGGGGTCTTTGAGTCTCACTGCTG
CATGTTCTGCCTTCATAGGGATCTGTAAGTATGCTGGGCATCTGGGCTTTTAGGGGGCTCTCTATA
GGGTGTCTGAGATAGAGGTCAACAAGGGCTTATAGACAACTCAAACAAAGCCCATGGCTTGAGCAA
GTCTGCAACAAGCTGTTTGTCTAGCCTCCAGCAGAGGGCGAGGGAGACAGCTTCCAGATGTTCCCA
GTAGGTGGAGCCCCTCCAAGCCCAGGGCTCAGGAGGCTTACAGGGTGGGAACTCCAATACTGGTGG
AGGGAGGAGGGCGTTTGATGGGAAGATAGGGAAGTTGCTGCTTCCTAAACTGTCACAACTGGGCTT
GGATAGGAGTCATAGTCTGGGACCACAGCCCTGTGGTAGAATGCTAGCCTGGTGTGCTCCAGGTTT
AATCTCCATCACTGCAGAAATGAGTCCAAGCTGTGTGTACCTCCAGGGCACTGGGCATGGGGTTCC
CTTGCCATTGTGTGTGCCCGGAGAACTGGCAGGCGGGAAATGTCTTTATCAAGGGTTACCTTGGAA
GAGGTCCCAACACTGTAGGGTGCTCCTGTTGTCAAAACCTATGCAGAGGCATCTGCTTGCTCTCTA
ATAACAGTATGCAATGCTAAAGGGCTCGCTTACAGCCGGTGGCCACACTGGAGGCCTGCACATCAG
GTGGCCACAAGTTCTGCTGCTGCGCCTCCGAGGAAACACTTGGTCCTCCGATCGATTTTAACCTGT
TGAGGCTTTGCAATCCCCCTGTGGCAAAGGCTCCAGTGTTTTCTATTTCTATGCAAATTTCTTGAA
GCAGAACTGTTACTGTCTTTCTCCTCTGCCCTGGGAGGAGGCGCTAGCGTTTCCTTCCAACTTCAG
GTGCAGCCCCCCTCGTGGTTAGCGGTCTTAAGTTCGTGACTTGGGTTTGCAGATCTTTTTTGTTAC
ATCGCCGGACCATGTGGTGGTCTTTAGCTGTAAACAACATTAACCCTGGGTTGATTAGCATATGCT
TCTAAAAGATGGTCCCAGATTCTGCGACTTGTAATAAAATGGAAACTTGCTGGTTTTTATGCCTTT
CTAACTCTTGTATTTGAATGAATGTTGATCACTTTTTGTATTAAAGTGGCTGACACATGGCTACTG
TCACTGTG
(SEQ ID NO:131)

Mus musculus ribosomal protein L22 (RPL22)

AATATCTCACCAAAAAATATTTGAAGAAGAACAACCTCCGAGACTGGCTGCGTGTTGTCGCCAACA
GCAAAGAGAGTTACGAGCTGCGTTACTTCCAGATTAACCAGGATGAAGAGGAGGAGGAAGACGAGG
ATTAGGACACATTGGTCTGCAATGTTTTGTATTAATTCATAAATAAAATTTAGGAACAAAACCGGT

GGTTTATCCTTGCATCTCTGCAGTGTGGATTGGACAGGAAGTTGGAAATGACAGGGACTTTAACTG
GGCTGCTGCTCCTTTGTATATAGACACTTTTTTCCTGCTCAGAAACTTGAGTTCTCCAGTAGCAAT
GGCCAAACAGAAGAACCAGGCTAGGGGGCTGCATCTGACAGAGCAAGTAGACGAGAGGCTGGGTGG
TGGGCTCCGGCCAGCCCGAGTCTTAGAGCTGGTGGTTGGTTATATCTGGTGCCTGTCTCGAGGAGG
GCTTGAGACACAGTGTGGTGCTCCTCAGAAGCAGACAGGTGATTTCTTTGTGTGATTTTTCTTTTC
CCCTGGGACAATGACAGTCAGTAAGACAGGTTTCAGGGACTTTTGTGTCCAGGTCTGAGCACTAGT
CGCTCACAGTTGTGTGTACTAACCTTCTTCCTTCCTATTGAAATGGCAGGGGTCTTTGAGTCTCAC
TGCTGCATGTTCTGCCTTCATAGGGATCTGTAAGTATGCTGGGCATCTGGGCTTTTAGGGGGCTCT
CTATAGGGTGTCTGAGATAGAGGTCAACAAGGGCTTATAGACAACTCAAACAAAGCCCATGGCTTG
AGCAAGTCTGCAACAAGCTGTTTGTCTAGCCTCCAGCAGAGGGCGAGGGAGACAGCTTCCAGATGT
TCCCAGTAGGTGGAGCCCCTCCAAGCCCAGGGCTCAGGAGGCTTACAGGGTGGGAACTCCAATACT
GGTGGAGGGAGGAGGGCGTTTGATGGGAAGATAGGGAAGTTGCTGCTTCCTAAACTGTCACAACTG
GGCTTGGATAGGAGTCATAGTCTGGGACCACAGCCCTGTGGTAGAATGCTAGCCTGGTGTGCTCCA
GGTTTAATCTCCATCACTGCAGAAATGAGTCCAAGCTGTGTGTACCTCCAGGGCACTGGGCATGGG
GTTCCCTTGCCATTGTGTGTGCCCGGAGAACTGGCAGGCGGGAAATGTCTTTATCAAGGGTTACCT
TGGAAGAGGTCCCAACACTGTAGGGTGCTCCTGTTGTCAAAACCTATGCAGAGGCATCTGCTTGCT
CTCTAATAACAGTATGCAATGCTAAAGGGCTCGCTTACAGCCGGTGGCCACACTGGAGGCCTGCAC
ATCAGGTGGCCACAAGTTCTGCTGCTGCGCCTCCGAGGAAACACTTGGTCCTCCGATCGATTTTAA
CCTGTTGAGGCTTTGCAATCCCCCTGTGGCAAAGGCTCCAGTGTTTTCTATTTCTATGCAAATTTC
TTGAAGCAGAACTGTTACTGTCTTTCTCCTCTGCCCTGGGAGGAGGCGCTAGCGTTTCCTTCCAAC
TTCAGGTGCAGCCCCCCTCGTGGTTAGCGGTCTTAAGTTCGTGACTTGGGTTTGCAGATCTTTTTT
GTTACATCGCCGGACCATGTGGTGGTCTTTAGCTGTAAACAACATTAACCCTGGGTTGATTAGCAT
ATGCTTCTAAAAGATGGTCCCAGATTCTGCGACTTGTAATAAAATGGAAACTTGCTGGTTTTTATG
CCTTTCTAACTCTTGTATTTGAATGAATGTTGATCACTTTTTGTATTAAAGTGGCTGACACATGGC
TACTGTCACTGTG
(SEQ ID NO:132)

Mus musculus ribosomal protein L23a (RPL23A)
ACTGAGTCCAGATGGCTAATTCTAAATATATACTTTTTTCACCATAAA
(SEQ ID NO:133)

Mus musculus ribosomal protein L17 (RPL17)

ATTCAGCATAAAATAAAGGCAGATAAAGTTAAAGGTCTTCTGGTGGTCTTTAATGAGCCCTGTTGG
GAGTGAGGTGCTTTAACATGGAGAAGCATGTTATTAAACAGTGAAATAGATGGTTCAAAACCACGT
GACCATGT
(SEQ ID NO:134)

Mus musculus ribosomal protein L24 (RPL24)
TGTGGTAGAGCAGAGTTGGAAATAAAGCTCTATCTTTAACTCTAGG
(SEQ ID NO:135)

Mus musculus ribosomal protein L26 (RPL26)
AGACATCTCGTGCACGGCTTTCATTAAAGACTGCTTAAGT
(SEQ ID NO:136)

Mus musculus ribosomal protein L27 (RPL27)

GTATATTTTTGTTTTGGTCATTAAAAATTAAAAAAAAAAAAAATACAAGTGTCTGCCTATTGCATTT
GTGTGGGAAGAGACTGG GGAAATAAAACAGGTGTGCTGTTGTG
(SEQ ID NO:137)

Mus musculus ribosomal protein L30 (RPL30)
ACAAGAAAGTTTTCCTTTAATAAAACTTTGCCAGAGCTCCTTTTG
(SEQ ID NO:138)

Mus musculus ribosomal protein L27a (RPL27A)

AAGCCACACCGGAGGTTAATTAAATGCTAACATTTTCCATGTGGTCTTTGCATCCTTCCTTGTCTG
CATGTTGGAAATCTGCCTAACATTCTAGGAAGAGGTGAGGTGTGGGCCCTTGAGAGTCAGTCTGTG
GGAATAAGTGTAGCCCAACTATGCACAGTTGTAAATTCCTACATCCCCGTGTGTATTGGTCTTGAT
ATTCAAAGAATTGATGAATGCCATTACTTTCAGTCCAAAGTGAAGAAACCTGGTCTCAAAAAATCC
CGAGGACCAGAAATGAGATGGGTTTTCCTGAAAATCTAAAGTTCTTGAAAAACCTTGCCATCCAGA
TTGCTAGCAACTGCCTAGCTTTGTAAGCTTACTGTGATGGACAGGTAGCTCAGGACGACTGGTCAC
TTAATACTGGACAGATTAGCATGGAAAACTTAAGGGGAGGAGGAGGTAGTAGGTTCCATCCAGCTT
CGCTTTGTTGGTGGCATCTAGGTGTTGTTCCAAGGGAGCATGCCTACCTGCAACAGGACATCACTG
GTTGGGAATACTGTAGAACCAGAGCTGTGACCTTTGAACTACTAGAAAGATGAAATTTTATGTAAA
GAGTACCTTGGAGTAAATAAATAAAGCCCAAGATCCTGATTGTCTA
(SEQ ID NO:139)

Mus musculus ribosomal protein L28 (RPL28)
GCCCCACACGCCCGAAGCAATAAAGAGTCCACTGACTTCC
(SEQ ID NO:140)

Mus musculus ribosomal protein L29 (RPL29)

AAAAGGCTCCTGCCAGTGTGAAGACAGACGGACTGCTGTGACACACCTCCCCACACACTATTTGCA
GATGACCAGTGTCCTATGCTGTTCTTACAAATAAACTCAGGCAAGATCTGTTAGCTTG
(SEQ ID NO:141)

Mus musculus ribosomal protein L31 (RPL31)

CCTGCTCGTGTCAAATAAAGTTGCAGAACTGCCTTCAGGGTTTGGTTTTCCTTTCTGTTGTCTGCC
TCATGGGTGGAATTTTTGGGTCTACAGGGTGTTGGAAATTAATCTGAGAATCTCTGTTCTGGGTAC
ATGGGAAATTAGAAATACGTGAAACATTCTTTTCACAGAAGTCACTTTATTAGGATTGTGGATTTG
GGTTGGTTTTGAAACAGGGTTTCTTGTGGCACTGCTTGTTCTATAGAATAGGGTGGCCTTGAACTC
AGAAATCCACCTGCCTTTTCCTCCCTAGTATTGGCAATTAAATGCCCAGCTTGTTTGTAAGCTCTC
ATTTTCAGTTCCAGGTTTATGTGTGAGCCTAAGATTAGGTAAAGATTGAGGTTATAACTTAAACGT
ACTGAATTAACTTATGTTGTGTGGGTCCCAGGAATTGGACCTGGGACATCAACTCTGCCTTTCCAG
CCATCTTTGCCAACCAGTAGCTCATCTCTGGGATGTGTCTGCCCTCAAAATGACATTTTAAAAAAG
TCAGTACAAAAGAACGATTTTTATTAAAAACCTTGAGGACAAACATT
(SEQ ID NO:142)

Mus musculus ribosomal protein L32 (RPL32)
ATGGCTTGTGTGCATGTTTTATGTTTAAATAAAATCACAAAACCTGCCGTCGTA
(SEQ ID NO:143)

Mus musculus ribosomal protein L35a (RPL35A)

ACTAATGGAGAGTAAATAAATAAAAGTAGATTTGTGCTCTTGTATTTTTTTTCACATCTGTCCTA
AA
(SEQ ID NO:144)

Mus musculus ribosomal protein L37 (RPL37)

GGATTTCAATCAGTCATAAAATAAATGTTCTGCTTTCAAAAATTCTGTGGTGATCTAAGGTACTTT
AACATCGGTTCAGAGTTCGGTTATATGATTGCTCTGGGATCCTACGCTTCTTCCTTCATAGTTCCT
GTGGGTCCGAAGCTGGGAGGGGCTGGGTGGACTCTCGGGAAAGATACTCTGAGCCTGTCTCGGTCC
CCATCGTGTTTGCTTGGCCCTGGGCATGGAAGTGGGTGAGTGATGAGCTGAACGAGCAGGCTTGCT
AGAGATGAGGACAGTTACTGGTGTGGTTATATCACTACCATGCCTACAGTGTCTTAAGACGCTTAC
AGTCTGTAAGGGACTTAAATGATTTGAGCTCTTACTTATCCTGTAGTTTCTGATTTTTAACATTTA

CTTGAATAAAGCCAAGCAAGATAAGCCTTTATTCCCAGCACTTGGTGACAGGTGGATCTATGAGTT
GGGGATCAGAGCTACACATTAAAACTCTTAATTCATCTTACT
(SEQ ID NO:145)

Mus musculus ribosomal protein L37 (RPL37)

GGATTTCAATCAGTCATAAAATAAATGTTCTGCTTTCAAAAAAAAAAAAAATTAATCCTCTGTGAT
GGCCAGCAGTTAACATTCAACAGTTTCTCTCTAGGCTCTTGATTCTCTGACTATTGTAGGGATTCG
ATCAGCACTCGCATACCAGAAGTGTGAGATGGTCCGTCCTTTTTCAAGACAAGATTTCTCTGTGTA
GCCCTGGCTGTCCTGGAACCCACTCTGTAGACCAGGCTGGCCTTGAATTTACAGAGATCCCCTTGC
CTCCGCTTGCTGAGTGCTAGGATTAAAGGCATGCGCACTATG
(SEQ ID NO:146)

Mus musculus ribosomal protein L37a (RPL37A)

AAGCCCTGCTGTCTGAGACTTGCCTAGCCTGCAATAAACGGGTTATTTACGTAACTTTTTTTTTT
TGCCTTGTTTGTGGTTAATTAAAACATTTGGTGTGTGTTCTATTTTTATTTTCGAAAGATGCTTG
TTTTGAGACATACTGTGTGACCCTGGCTGGCCTTGAGTGCCTGGTTCTCCTTACAAGTGTAGATAC
ATCTGGCTTAAGATTTTAGTCTTTCAGAAATAAAAATGTTGCTAAGAC
(SEQ ID NO:147)

Mus musculus ribosomal protein L38 (RPL38)
ACCAGCCCTCTGCGTGTGACTATTAAAAACCCTGAAAAGTG
(SEQ ID NO:148)

Mus musculus ribosomal protein L39 (RPL39)

GGATTCACACAATGGCAAGACTGAGGATTTATACTGAATTGTCATCAATCAGTCCTACCAGATGGA
TTTCAACATTTAAACCTGGAGACTCTTCGTGTCTTGAATTAGGATGTTTGTCCAGTAATAAAATAT
AGAACCTTTCAAAATGCTTTTCTGGTTTATAAAGTACTGAATTGCCCTT
(SEQ ID NO:149)

Mus musculus ribosomal protein, large, P0 (RPLP0)

TCCCGCCAAAGCAACCAAGTCAGCCTGCTTAATTTGAGAAAGATGGAAATAAAGGCTTACTTCTCT
TAAAACTCCGGTCTGGATTTATTTAGTTTGTTCACTTAAGCAGGATGAAAAAGCAAAACCGCTACT
GTTTACTTTGTGTTGGCATCTTTGTTTCTAAAATTAAAGCTCCTAGTGTTTTTGTGGGCTTTGTTT
GTTTTTTGAGACAGTCTCTTGACTTGGTGCCATAGCTAGTCTGGGACAAAGATTTTCCAGGTGTGA
ATTAAAGGTGTATGTCATCAA
(SEQ ID NO:150)

Mus musculus ribosomal protein, large, P1 (RPLP1)
ACTGCTTTTGTTAAGTTGGCTAATAAAGAGCTGAACCTGT
(SEQ ID NO:151)

Mus musculus ribosomal protein, large, P2 (RPLP2)
ATTCCTGCTCCCCTGCAAATAAAGTCTTTTTATGTATCTTGA
(SEQ ID NO:152)

Mus musculus ribosomal protein S3 (RPS3)

CAGGGTCTTGGCAGCTGCATCTGGAGGCATTTAATAAAATAAAGACATTTAATAAAATCTTGAACA
AAGACAAGGCCTGACTGGATTGTGTCCAGTATTCAACTGAGTTATGTTGTCTATGGAGCCATGCTT
ATTCTGTTGGTTTAAGCTGGAGGGCATGAGCAGAGCTGACCAGAGAAGTCATGAAGTTGGTGACCC
TGTGTTGAACAATTGAGGGTTAGAAGAGCAGTTTGGTTTTGGTGCTCTTGATGGAACCCAGGTGCT
TGGACATAGTAAGCACACATAAGACAGAGTAAGACTGCTGTGTCTCTGGCCTGGAGTAGTCTTTCT
TGCTTTTTTTTTTTTTTTTTTTTTTCTCTAGAATGAAAGCAGATGGCCCAGCGAGTTAGGTGCTTC

CTATGAAAGCATGTGTGCTGGTTTGTCATGCACACAGCCCTGCAGGAGAGAGTATGGCAACACAGC
CGCTCAGCATCCCAAGATAAAAAGGGAGTTTCTACTGCCATTTTGAGCTTGGGAGTTTGAAATGTA
AAGCCTGTCCATATGTTTTAAGGATCCATGTATTTCTGTTTTGTTTGTTTTTCAAAACAGGGTTTC
TCTGTAGCCCTGGCTGTCCTGGAACCCACTCTGTATGTAGACCAGGCTGGCCTTGAACTCAGAAAT
CCACCTGCCTCTGGCGATCCATATATTTCTAAGTCCTGTACTTAGACGCTGTTTTGGAAAATTCAT
TTTGGAAGCATTTACTGTTGGTGTGTTTTGTGGGGAATGAATGATAGCTTGGGAATTCTTTTCTGT
TTGGTGAGAGTGAAGCTGTCAGCCCGGTTGTAGCCTGGCTGGTGCTCAAAGGCTTTCTCTCATTGT
CTTCACCTACGTAGCTTTACGTGGGGTAAGGACTTAAGTTACTTAAGTTGGGTGCACACTGACCAT
GTCCACAACCTGTTAACCAACTCTACATGATGAGTACAGATGTACCTTTTTAGAAAGTGTTAATGT
GTAGCCCTGGCTGGCCTCTGCCTCAGGGTATTATGAATAAAGTGTGCAACCTTCATCTGGTTGATT
AAA
(SEQ ID NO:153)

Mus musculus ribosomal protein S3A (RPS3A)
AATCCAGATTTTTAATAGTGACAAATAAAAAGTCCTATTTGTGATCGTT
(SEQ ID NO:154)

Mus musculus ribosomal protein S4, X linked (RPS4X)

AATGGTCTCTAGGAGACATGCTGGAAAAGTGTTTGTACAAGCCTTTCTAGGCAACATACATGCTAG
ATTAAACAGCATGGTGAAACT
(SEQ ID NO:155)

Mus musculus ribosomal protein S4-like (RPS41)

AGTGGACTCTGAGGGACATTGCGGGGAAGGGGCGTTTACGTTTGTTTATACTTAAAAGTTTTTTAA
GCAGCATGTTGAATTAAAAAAGAAAGCAAGCTTC
(SEQ ID NO:156)

Mus musculus ribosomal protein S5 (RPS5)
TTTCCCAGCTGCTGCCTAATAAACTGTGTCCTTTGGAACAACTAT
(SEQ ID NO:157)

Mus musculus ribosomal protein S6 (RPS6)

GTCTTTAAGAGCAACAAATAAATAATGACCTTGAATCTTTCATTGGCTTTCATTAATAGTGTAACT
AGATAAATGATGGGAAAGATGAGACAGAAGAAGGAATACATCTATAGGACTGCTAGAATATGGGGA
GAGTGATTATTTTCAAATTAATATGTATCGAGCTTCTACCCCAAGGTAAATAAATAACATTTGGAG
ACCATTAAAATGTAGGATGGCATAGAAGAGGCCTTTACTAAGATTAATAATTAAAGAAACACAGCC
TTTAAAGTAAAAAACACACTGTGCCTTTGAAACTTGCTAAAAAGATTAACTTCTGTCCCAAAAGGT
ATCAGCCATGCGCTACCAGCCTCCCTGCCCCTACAGTGGCAGTGGCTGCATTCTTGGTGAATGGTA
GTGGAAGGGATTAAACCTAGGCCTCAGTCATGCTTCCCAGTCACTGGTACTGATTTGTATGCACCC
GCTTAGGTGTGAAGGTAGTTTTGGTGTGTATCACAAGTTAGCCTGTGTAGCGAAGACAGGTTTTCT
CCACCGTGTTTTTTGTTACACATGACTATTCACAAATGTGCTGCAGACAGTAAAATGAGAAATACC
CTTCCAAGG
(SEQ ID NO:158)

Mus musculus ribosomal protein S7 (RPS7)

AGAAAATGACTGAATAAAGTGTCATTCATAGTATTTGGTTGTAGTAACTTGTCAAAATCTCAGGGC
CATGGGTGCACGACAGCAGTAGCTTCTTGAATGAACTGAAGTTTTCAAGAGGTGCCTGGAAGGTGA
AAAACACACTGAAGCCAGTCATGTTGATATGGGGGCATTCTGCTGCTGTGAAACAGACTGGGGTTC
ACACCCACCTTGCGGGATTAGAACTTCACTGCCCTCCAACTTCTTTCTTTGTAAACAACTGTCCAC
ATTTT
(SEQ ID NO:159)

Mus musculus ribosomal protein S8 (RPS8)
GCCTCATGTGTAGTGTAATAAAGGTGTCTGCTGTTCTATCTG
(SEQ ID NO:160)

Mus musculus ribosomal protein S9 (RPS9)

TTAATACTTGGCTGAACTGGAGGATTGTCTAGTTTTCCAGCTGAAAAATAAAAAAGAATTGATACT
TGG
(SEQ ID NO:161)

Mus musculus ribosomal protein S10 (RPS10)
AGTTGGAGTTTATGTTGTATTGAATAAACTTTAAAG
(SEQ ID NO:162)

Mus musculus ribosomal protein S11 (RPS11)
GGGGACTCTGGCCAATGCCCTAGAACAAATAAAGTTATTTTCCAACG
(SEQ ID NO:163)

Mus musculus ribosomal protein S12 (RPS12)
ATAAATTTTGGCTGATTTTTCTCTTGTATTTCTTGTTTGCTGGTATAAAA
(SEQ ID NO:164)

Mus musculus ribosomal protein S13 (RPS13)
ATGCTGTTGTGTGCACAAGCAATAAAATCACTTTGAGTAACTT
(SEQ ID NO:165)

Mus musculus ribosomal protein S15 (RPS15)
CCGAGGCCAATAAAGACTGGTTTTGGTCCCTGGA

(SEQ ID NO:166)

Mus musculus ribosomal protein S15a (RPS15A)

ACGTAAAGCATAAATAAAAAGCCTTTGTGGACTGTGCTCAGGGTCAGTCCTTTTGAATCTCTGCAG
CAGAGTAGCTGGCTGTGCTGACTGGTGACACTTCTGGTGATGCTCAGCTGTGAGGTTTTATGTAGA
TATTGAAAGCATGACCATTGTCTTCACTTCACCTCCAGCTTGGGTTGTATGCCAGTAACATCAGCA
TAAGGTGGTTAATGACAGGATGGTCCCTTGAGTGTGCAGTGAGTCTGGTTTATTTGCCAATGAGAA
GCACAGGCCTCCTGTATGGGTCTTTGCCTACAGCCCCCTTTCATCACCCAGACTTGGTAGACTTAC
ATTCTGTCACACTGTTGGCTCTTAATCTCAGCCCTGAAAAATGCCATTTCTTGGGTATCAAGGCTA
GTCTAGATTCAGAAACCATATAAAGGTTGACAGCTGGTTTAAAAAAAAAAAGGCTTGGAGCTTGAG
TTGGGTTCGCAGGTTATTCCAGGGTATCTGTCTGCACTTTGTCTCCCAGATTTAAAGGTAAGTGCC
ACCATGCCTAGCATGGTGACTTATTAGCTTTGTTGCTGTGGAACATACATCAAATGAAACATTGGT
ATGGCTCTGGTTTCACTGTCCATGGTTAGTATCTGGTGGGTGGACACCTGGTGGAACCAAGCTGCT
CATCCCAGAACTAAGGAGCCATTCCCCAGAGACTTGTCTTCCTACTAAGTTCCATCCCTACAGCTT
CTAGTAGTAGCTTCAGAGGTTGTGAATTGTGGACTTAGTCTGCCATAACATTTAAAATAGGTATTA
AATTCAAGTCATTTGGTCACTCAGCACCCACGTGGCTCTTCAGAACAACAGAAGCCCCTCGGACTT
GTCTGTTGGAAAAACCAGTTTGAAATAATGTACCTGCTTTAGTTGAGAAAACGCTACAACTGGTGC
TGTGTCCTGCCATGCTGATGAGCTCTGCTCTGCGACCTGCCGAACTTGGGGGATCTCTACCCCCAG
ACTTTGCTCAGATCTGTTGATGATTTGTCCATGCAGGAAAGTTTACAAGGTCTCTGTGTGTCTACT
ACTTACTAGTTGCTGTGACAAAAATACTGAAAGTGTTTACTGTGTGTGAGGCACAAAGTTTGTGGG
AAGCTGTACCCTCCCTCATTTTGGTGCTGCTCCTGCCTTGACTGACAGGATGAGCTGCCCCAACCA
TCGTTGCCATCTTCCATAAGAAGCAGGTGGCTCTATTGAGTTCCCTGGAGGTGATCCCAAGGGAAG
GAGGAGCCTGGGAAAGTGGATCTCAAGTCCCCTAGTCTGGCAGTTGGCTGTTTAGGAAAGTCCAGT
GTCAGTGTTTGATATGTTGTAAGGAAACAAATTCAGTTTTATTTAGCTTATTGGCTCTGGGGAAAT
GGCAGTTCCCATTAATTGGTGCTGTCTTTCTCTTTGAGGATCAAAATTAGCTTCCTGTTCAGTTGT

TAAGCATATTTCATAGTCAAATAATCCTCTTATCTTTACAAGTGAGGTTTTCCTTCGAGTGGATAT
CAGAGTCCCTCCCACGGCTTCTATCCCTCCTATCCTTGTGTAGGAAGTTAAGCTTGCTCATTTGTA
GATTAGTGGTCGGTTACACTGCAATTTAGAGTATCATGTGTACTCTACACATGATTGATTCTAAGC
CCCCTTCCCTTTCCATGTCCTTCAAAAATTTTTTATTCTGAGACAGTGTTAGGATTTTTCCTGGG
CTAGCCAAATGCAAGAAGTGTTGGTCCCAGACTTGTAATCTTTCTGCCTTAGCCTCCCAAATTCTA
GGATTATAGATTTATGTCATGTGATGAGTGGTCTTTTAAAGATTATCTTTTATTTTTTTGAGATG
GGGTTTTTCTGTGTAGTTTTGGCTTTCTTGGGACTTGCTCTGTAGACCCAGGATGGTCTTTACTCA
GATCTGCTTGCCTCTGCCTCCCAACTGCTAGGATTAAAGCATGAGCCTGAGCCTTCATGCCTGGCT
GACAGGTGAATTCTCAATACCTACCTAGCCATAGGGAAAGTGATTGTGTCCCCTTCCTCATAGAGG
GGCATAGTCACCCAGGCCATACCTTTAGCTTGGGCTTTTGGTCAGTGAAGAAGTATGAAGGGACAA
GAACACTATCAAGAGCCTAATGTGCTCTGGCCTGGATGGTCAGCACAAAATGAATAGACTTACCAA
ATTCTGCTGTCTCCTTGGTAGATGTGAAGTTGTTGGAAGAGTCCTAAATTTAGCAGACTATACTGT
CAGCCTATCAGACTATAGGCTGCCGGAGGGCAAGTCTGCTACCTATTTCCATCTCATGCCTGCATT
GTTCATCCCCCTATGTAACCCACCTACCTGTCACTCATTCCTCCATCCAAAAACTATTGTAGGTTC
AGTGGAAATTTCAAGCTTGCCTGTCTCAGCATCTTTCTTACCTTACCCCTAAGGATGGCATCTCTC
TTGGCTACATCTTTGGTTTATCTGGAGATCCTTGATTAATTTGAACAAGAGCTACCTTGGGTTATG
CAGTTTATGCCTCCAGTGTCCCAGAGACCGGCATTTGAGAGATCCCTGATAGCAAACCCATAGGGT
GGCCTTTTTTTCATCCACCCCATTCTCCCTCCCACCTCCCTCTTTTGACCTTGAGTCCTCACCAGA
GAGAAACCAGGCCCACTTAATTAGTTCTACATGTGTACACTACATGGGTGCAGTGCCCAAGCAGGA
GAGGTGTTAGATTCCCTTTTAACTATAGTTAATAGACAGTTTTTAAGCCCCAGTGGATGCTGGGAA
GCAAACCTACATCCTCTAGAAGAGCAGCTATTTCTCTGAGCCATTTCTCCAGCACCATTTTCCCCT
CTTTTAAAAGCAGGTCTTGCAGTGTGGCCTAGTCTGGCCCCCTGAGGTGTTTGCATTGCATGGCAG
GCATGTCCACAGGAACACCATAGTTCTCACCACTCGTACAGCACAGCAAGTGGGGTGCCGCAGGGG
ATTATCACTTGAGTATAAAATAAGGGTTGCTTTAGATTGAATAGGATAACCACGCGTTCTCAGAAC
AATCAAGGAAGGCTGGGGTGAGCCAGCACCGACCTTAATTGTTTACTTAGTAAACTACTAAATGTA
TGCACGTGTAAGCTTTTGCCTTGATTGAGGTCAAGCTGTCGAGAAATGGTTCTCTTTACAGTGGAT
CCAGTCAGGATTGGCAGCAAGCACCTTTGCCTGCTGAGCCTACATGTTGTATAGAATGGCAACGTT
GTGTAGAATGGCAGTACATTAAATGGGTTTTTCATTTA
(SEQ ID NO:167)

Mus musculus ribosomal protein S16 (RPS16)
GCCCATCTCAAGGATCGGGGTTTACCTTTGTAATAAACATCCTAGGATTTTAACGTTCC
(SEQ ID NO:168)

Mus musculus ribosomal protein S19 (RPS19)
AACAAAGGATGCTGGGTTAATAAATTGCCTCATTC
(SEQ ID NO:169)

Mus musculus ribosomal protein S20 (RPS20)

GACAACTGAATAAATCGTCTTAATGGTCAAATTTTGCTGGCTTTTGTTCAGGTTTTTTTTTTTTAA
TTCATGTTTATGAGTGTAAATGTGTGTGTCACAGGGGTGTCAGATGTTCTTTGAACCACCATGTAG
GTGCTGGAAACCCAACCTGCATCCTCGGAGAGAACAGGTTCCTTAACCACTGAGCAAGTACTGAAG
CATTAAACTGCTTTTAAAAATGAAGGTGTGCTAACAGATTGGTCAGGTGAAAAAGAGACGTTAGGT
TTCCTGCAGGGGGCGCTAAGCCAATTTAAAGACTAAGTTGGGTTAGAAAAGAGCAGATTGCATCCT
TGATCTTTTAAGCCTGGGGATTTTGTTTTGTTTTGGGATAGGGTCCCAACACAGAACAGGCTGACC
TCATTAAATATCAATCTTATTTGATTGCCTCTGCTCCCAGAGTACTGGAATTAAAGGCAGGGACCA
CTGTATTAGCCATTCTGAGTTATTTGAAATGGACTCTGCAGGCCATACTTGGTCAAAATTCTGCCT
TCTCAATTACAGGCATGAGCCACTATGCCTGGTTTACTTACTAATAGATGTCCAAAGACTAGTGTA
TGAAAATTTTGCTTTTCCAGGTGATTTGTGAAAGGCAGGGTGGCCTCTCCCATGTCACACTACTTG
GGTTACTCATGTTGCAACATATCTGCAACTTTAGGTTGAGGGGATTTGAGCCTGCATGTGCCACTT
TGGCCAACTGAACTAATCTTTAATTCCATCTAAAACTTTTAAATCTCAGTCATGTGTTCAACTGGA
AAATAACCTAGAGTGTGCTATGTTGACTTCAGGTACACATCAAAGCAGGTTTTAGTGATGTAGAAG

CTGTGTTTGAGTTGAACTAGTGTTGAGGCTAGGCTTAGGTACCATAGAACTTTGGTTTTTCAAGAC
AGGGTTTCTCTGTGTAGCCCTGGCTGTCCTGGAACTCACTGTAGACCAGGCTGGCCTTGAACTCAG
AAATCTGCCTGCCTGTGCCTCCCAACACACCCAGCTCTAGCTTTAAATTCCTTGCACCAAGAGATG
CTTTATCCCTCCGCTGAGAATACAGGTGCATGTCAGCATGCTAAACTCTAGATAAAATTTCATCTT
GTTTGAAAGGACAATAATATAAGAAAAGTGTATTTGCACTGTATACCATGCCCTTTTGTGTTTAAA
GTTAAACTGGCAACAGTGTCCCATAGAGGTTCCAGAAGAAACTGCTTCTAAGGGGAGGACCATAAA
GGGAAATGCTTACCATAGAACTTTTTAAATGTTCCTACAGGTTGTACTCTGGATAGGTATATGAAC
ACCTTTCTATTAGAACAGTTTTATAGTAGTACTTAGTGAATATGTAAATAATACTATTTGTGAAAT
AGTTTGAGGTTTCTCTATAGTCCTGGCTGGCTTGAGCTCTATACCAGATTGGCTTCTAAATCAGAA
ATCTGCCTCTATCTCCTGAATGGTTATGATAGAGATGCAAGTTACTTAATTTCTTACATGAATTGC
ACTTTGTACATGCTTTTGGATATGGGCCTAGGCTTTTGCTTTTGGATTAGACTGTCTTTATTACAT
TTACTGGCTTGATACTTTACAGTCTTAAGCCCACTTGCCTGGGTGTGATGCACACCTTTAATCCCA
GCACTTTGGGATTTCTGAGTTCCAGGACAGCCAGGGCTACGCAGAGAAACCCTGTCTTGGAGGGGA
AAAAAAGAAACTTTGGAAATCAAAACTTCTTGGAAAGCCACTTTTAGAGACTTGAATCTAAGGATA
ACTAACCAGGTAGTAACCACGAGTCATTGATTCTGTGAATCTTGTATGAGTGGGTTACAGGCAGAA
ATTAACTTCCTCTGAGAGCACTGCTGTTTTAGAAATGCGACCTAGTAATTACCAAAGGCATTGAAG
CCACTTGACTACAGTCTCAGGTTTCTGCATCTGACATTGCTGGGACTGTGTGGGGTTTATGGGTGT
AAAATAAAACAGGCGAAAGGATGTTGAGTGGAAAGCTTTGGCTTCAGAATCAAATTCCAAATCAAT
TACCAGATCAAATCAAATGCCAGATCAAATTACAAGACTTCCTTATGACTAAATTCTTCAGTGACT
TAGGATACTAATAGTATCTGCCTCAAAAGTGTATGTGGTTATTTTTGTCCCAGTGAAGCCAAGATT
CACATGCTATGGGCATGGATTTCCTGAGGACATGCTAGCCTATGGTATGAGTTACTTGAAAGGACT
CTGAGAAATCTTAGTCCCCAGCTGTGAGGTTTTTAAAGATCATGCTCAATGGAAAGTGGGGCAGTA
ATTTGAGAGCATGGCACAAATGAGGTTTTATCTTTTGAAACTAAGTGAATCTATGTCCTTGGACTA
GCATATTTTAAATCACACATCAAATGAAATTTCTGTTCAATTCCTATAAACAGTTTATTTCATATT
TTGTAGTTACCATTTTCATTAACAGCATACACCCTTCAATTGTGTTGCTAAAACTGAGTCACATTA
TTCTGTAAGAACTTACTCCAGTATCACAACTTGGTGCTCATCCAATATTTTTATTTTCATTCTATT
TTCCCTGTCTAGCCACGTATGGCCCTATTCTCTCTTCTTGGATTGACCCTAGCTTCAACACAAGAA
AGCTTGCAGTATTTTTTTTTTTGCGCCTGGTTCATTGTTTTGTCCTCAAGTTCTATCCATGTGTCCA
GAAATTCGAGGATTTTTTAAAATTTACTTTTCTGCCTAAATACTGGTATGTGGATAGTCTGTTATT
TTTACTGTTGGTTGCATATCTGTAGCATATTTCTGTATTTGCAATGACTACATTGAATGTCCCTTA
GTTAACCTCATTCTTCTTAACTATTTTGTAGGCGTTTGCTATTCAAAGCACAATCTCAATTAAAAT
GTTTTTAATAGCATCTTTCCACTTGGATGTGTAAAGAAAGTATTTCTAGAAGTCTGAATTTTTGTT
GCATTGTAGATGTGTACACAATAGGGCTGGGAAAGTAGCTCACAGTGGGTAAAAGCCATTTGTTGC
CAATCCTAACAGCCTGAGTTCAATCCCAGAATCTATAAGGTAGAAGAAAAAAACCCCAGCTCCCAC
AAGTTATCTGGCCCTCTGCACACATATAAATAGTGCAATAAAAATTAACCATTTAAAAATATAAA
(SEQ ID NO:170)

Mus musculus ribosomal protein S21 (RPS21)
GCAGAAGAAATCGGGAATTTGTTACAAATAAAAGTTTTAAGTACCTGTGACAGTTAAG
(SEQ ID NO:171)

Mus musculus ribosomal protein S23 (RPS23)
AATTTGACAATGGAAACAGAGTAATAAATTTTCATATTCTGAAAAAATA
(SEQ ID NO:172)

Mus musculus ribosomal protein S25 (RPS25)

ACAGGTTCAATCAGCTGTACATTTGGAAAAATAAAACTTTATTGAATCAAATGAATGGGTGCATCT
GTTTCCTAAGGCAGCCGGGGAGGATTTGGTCTTAGGAATAATAGCTGGAATTGGTTTGTTGGCCAT
GAAGTCAGATGCAATTGCGCCTGGGAACCTTCAGCTTTTCCCTTTACGTGGTTGCTTGCTTCTTGT
TGCAGCTTCGGTTTTGAATTGATGCCTGAAAGAAAATAAAAACTTAGCAAGACTAATGGTAAATCT
(SEQ ID NO:173)

Mus musculus ribosomal protein S26 (RPS26)

AGAGGCCGTTTTGTAAGGACGGAAGGAAAATTACCCTGGAAAAATAAAATGGAAGTTGTACTTTAC
ATGGC
(SEQ ID NO:174)

Mus musculus ribosomal protein S27 (RPS27)
AAGCCCCTGATTGAAGATGAGTGGGAACCTTCCCAATAAACACGTTTTGGATATAT
(SEQ ID NO:175)

Mus musculus ribosomal protein S27a (RPS27a)
TTGTGTATGCGTTAATAAAAAGAAGGAACTCGTACTTA
(SEQ ID NO:176)

Mus musculus ribosomal protein S28 (RPS28)

TCTTGCAGCTGGGTTCTGGATATCCACTACTTAGCCCACGGAATGATCTGCAACTGTTAAATAAAG
CATTTATATTAATTCTTGTCTAGAAA
(SEQ ID NO:177)

Mus musculus ribosomal protein S29 (RPS29)

GCGACCTTGAATGGATTCGACTGACTACTACCAAGTGGAACCGATCATGCTAGTCTTTGTACACAA
AGAATAAAAATGTGAAGAACTTTAA
(SEQ ID NO:178)

Mus musculus ribosomal protein L15 (RPL15)

TACACGTGATATTTGTAAAATTCATATCCAATAAACAATTTAGGACAGTCATTTCTGCTTAAAGGT
GTTATTTGTTAAAACTAGTCTACAGATTGTCATGAGTGTTCTGTGAAAATGTAGAAGTTAAGTGCA
ATAATTGAAAACTGCAGGTGATGGCATATCTTGTTTCTGATGTACTTTGCATTATCTGCTTATGAG
ATTAAGTGTATATAGTGTTTGTGCCAAGTGGTGTTCTGTGTGTAAGACCCTGTAAGAGGTAAAAAG
TCCTGAAACTGACCCTGGATGTGTTGGTGCATGAGATAGAATCTACAGCTTTACGATGGCATCTTT
TGGTTCACTGAAGTGGCTGCTTGGGAGTTTGATGAGTACAAACTTTATAGAGTTGGATTTTGCTTA
GAAATGTGTAGGAAGAGAGGGTTTCAAAACCTGTTTTGTGCATAGAAAAGTGAGATCAACTATAAC
CCACATTTTGAGAATTGAATCCAGTGTTATTTTCAGAAGACCAGGTAAATTTGTTGGAAGAGGTTT
CACTTTCTGTTGGATCTAGAGTATGGATTTGGAGATGAAGGTTGAACATTGGATGTAGTAGGGCTT
ATTTAGGGCAGATTATTTCCATTAGATTTGTAAACTTGGTTGTGGTTGCAAGTTAATATCAACCAA
GCAAATATAAGTTTGATTAAGCTTGCAGACATTAAGCTTTTCTAGCAGCTGGTGTGTGCAGAGGCA
GATGGCTCTCTGGTTCCAGGACTACCTACAATATAGAGAAATTTTCTCTATTCCAGTCAATTCATC
ATGGGTAAGAATAGTCCATTTTGGTAGTGTTATTTCATCGTTTACAATCTACCTATGGGTAGTGGC
TGGTAACTGCCTGGATGTTGACATTTCACAAAGGCCATACTTAACCACACTTTTATTTCTATTTGT
GCGATGCCTTGGAGTAGTTTCCCAAAGTGATTTTGAGTGTGGAAGAAATGGTATTGTCCCCGAACA
GCTGGCTTGGTCTCAAAATCTAATTGATGCTTTTATTAAATTGGTTTTCCTTTGGAGATTTTAAAA
GGATGATTTGATTTCCAGAAAATACTAGACTCAAAATCTTGATAGCTAAAATTCTTTTCTATTCAG
CAAAACAAGTCACTGTATAGAGGTTGTTCAAATCAACTAAAGTAATAAATGTCTTAAACAAGTGG
(SEQ ID NO:179)

Mus musculus ribosomal protein S2 (RPS2)
GGGTTTTTATATGAGAAAAATAAAAGAATTAAGTCTGCTGATT
(SEQ ID NO:180)

Mus musculus ribosomal protein L14 (RPL14)

GGCTACACAGAATAATAAAGGTTCTTTTTGACGGTGGTAAATCTCATGTGTGGACTCTAAGCTTGT
CGCCAAGTGGGAAATAGACTGGTGGGATTGTAGATAGGATGGGCTACTTAAACTCATTCTACCCAG

GCCTTAGTACTTAGCATACAGCCAGAGTCAAACTGATCCTTTATACAGGGGGTACCATGACAGTAC
AACAGTGTCGTTAACCCTAACAAATAAATTTCCCACCAACGGGTGGAATTCCTTCATTTTG
(SEQ ID NO:181)

Mus musculus ribosomal protein S14 (RPS14)
ACAGGACTTCTCATTATTTTCTGTTAATAAATTGCTTTGTGTAAGCTA
(SEQ ID NO:182)

Mus musculus ribosomal protein L10 (RPL10)

AGGCTTCAATAGTTCTCCTATACCCTACCAAATCGTTCAATAATAAAATCTCGCATCAAGTTCGCT
T
(SEQ ID NO:183)

Mus musculus ribosomal protein L10a (RPL10A)
GATGCTCCAATAAACCTCACTGCTGCCACTCAG
(SEQ ID NO:184)

Mus musculus ribosomal protein L35 (RPL35)
GATGACAACGACAATAAAGTGCGAGACTGACTGGCT
(SEQ ID NO:185)

Mus musculus ribosomal protein L13a (RPL13A)

ACCCAATAAAGACTGTTTGCCTCATGCCTGCCTGGCCTGCCCTTCCTCCGCCGCCAACTAGGGAAG
TGGGGACCAAAGGTTCCTTAGGCACTGCTCCTGTGGGTAGAGGGGACATTAGAGAGCTGACAGCGC
ACCACCTGCATGAGTTTTTATTAAAGTGCAAACCATGGGATGAATCAGTTGAGCTTCAGTGTTGAA
AATGAGTAGCAGGGCTGCCCCACCCACCTGACCAAGTACCCTATTCTGCAGCTATGAAAATGAGAT
CTGCACATGAGCTGGGGTTCACAAGTGCACACTTGGAGCACTGCCTTGCTCCTTCCCAGCAGACCA
CAAAGCAGTATTTTTCTGGAGGATTTTATGTGCTAATAAATTATTTGACTTAAGTGTG
(SEQ ID NO:186)

Mus musculus ribosomal protein L36 (RPL36)
TGAACCCTCCCCCAATAAAAGATGGTTCCTAC
(SEQ ID NO:187)

Mus musculus ribosomal protein L36a (RPL36A)

GCAGATTTTGTTATGAAGACAATAAAATCTTGACCTTTCAACCCCTTTGATTGCAGTTGTTCGTTT
GGGAGGGAATACATTAAAAGCTTTCAGAAATTACCTG
(SEQ ID NO:188)

Mus musculus ribosomal protein L41 (RPL41)

GCCAGCCCGTGCACCTACGACGCCTGCAGGAGCAGAAGTGAGGGATGCTGAGGGCCGGGACAAGCT
ATCGGACTGTGTGCTGCCATCGGTAATGAGTCTCAGTAGACCTGGAACGTCACCTCGCCGCGATCG
CCTGGAGAAATGACCGCCTTTCTTACAACCAAAACAGTCCCTCTGCCCTGGACCCCCGGCACTCTG
GACTAGCTCTGTTCTCTTGTGGCCAAGTGTAGCTCGTGTACAATAAACCCTCTTGCAGTCAGCTGA
AGAATCAAACTGC
(SEQ ID NO:189)

Mus musculus ribosomal protein S18 (RPS18)
GTCTCTGGGCCTTTGCTGTTAATAAATAGTTTATATACCTATGA
(SEQ ID NO:190)

Mus musculus ribosomal protein S24 (RPS24)

AATACCTAGCAGTGAGTGGAGATTGGATACAGCCAAAGGAGTAGATCTGCGGTGACTTGATGTTTT
GCTGTGATGTGCAGATTTCTGAGAGGACAAATAAACTAAAAAGCTCCTACACGTCTGCTCTGCTGC
TTATTGGGCATTAGAAGAATCAGGTGGCTGCTTGGGTGTTGATGCAGTCAAGTGCACTGGGCTTGG
TGAAAAGCCCAGTGTAAGAGGCCGGTACAGATCCTTCCTGGCAGAGGGTGGTGATGGAGAGAACAT
AAATAACTACATGGGCAAAGTGTAGGACCAATTACCCTGTTAGCATCGTCTTTGCTCAACACCTTT
CTGTGTCCCTAGACTCTGAGTTTTTTTCTAATTGATTTTTATTGAACACTGAGTGTTTTGAGGTTT
TATTTTTT
(SEQ ID NO:191)

Mus musculus ribosomal protein L8 (RPL8)
AGTTCAGGAGCTAATAAAGTACGTCCTTTGGCTAATCCG
(SEQ ID NO:192)

Mus musculus ribosomal protein L34 (RPL34)
ATATGCACATTTTTTAAGTAATAAAAATCAAGACTTGATCTACGCTTC
(SEQ ID NO:193)

Mus musculus ribosomal protein S17 (RPS17)
TCTGTTATGCCATATTTTCAATAAACCTGAAAACAA
(SEQ ID NO:194)

Mus musculus ribosomal protein SA (RPSA)

GCTGCTGTGCAGGTGCCTGAGCAAAGGGAAAAAAGATGGAAGGAAAATAAAGTTGCTAAAAGCTGT
CTTATGGTCCTCACTGCAGACTGTACCTGGATTGGCATTTGGCTATACAACAGAGGCATGGTCCTA
CTGACATGTTTTGTGTTGAATACTTAAGCATGTGAACACATGGGTTTTTTTTTTTTTTAATGTAAA
ATGTAGTAACACAATGTTTAGGTGGCTTTGGTGTTAGCTCTGGAGACTTCATGTGTCATCTAGGTG
AGGTGTTCTTTAACACAGGGTCTCTGTTCTGTCATGCCTCATAGATCCTTCTACCTCCAGATTGGA
GAGGGAAAAGGCTTATGTCACTGAACCTGGCCAGATTGGGATTTTGTGTCCCAGGAACAAAGTTAA
TGCTAAAAAGTTAATGCCTTGGTGAGACTGATAGTCTGATGGTGTGAATTCACAGTAAGTGGTTGG
GATTGCCAGATGGAATTCCCTGAGCTGCCGTGACAGGTGGCATTGCAGAAGTGAAGGATTCAGGAA
TTTGAGTGTTGGGTGGGGGCCTGTGAATAGCACTTGGGCTGGGAGGGGAGACTGCTGCCCCTGAAT
GTCCTGGAATTCAAGGACAGTACCTGGTTAAATGTTTTTCTAGCTTTTCTAAAAAGTTTGTTAGGC
CTGGCATTGGCGGCGCACACCTTTAATCCCAGCACTTGGGAGTCAGGCAGGTGGATTTCTGGCCTG
GTCTACAGAGAGTTCCAGGATAGCCAGGGATACACAGAAATCCTGCCTCAGGAAAAAACCAAAAAG
AAGTTTGCTAAAAATAAGCATTTTTGCTTGATGGTATTGAAGATTGTAAGACATTAAATTGTGTCA
TTACTTCTCCAGGTACT
(SEQ ID NO:195)

Mus musculus ubiquitin A-52 residue ribosomal protein fusion product 1 (UBA52)

AGCTGTGGTCATACCTGGCATGTGACCCCGGGACCAAATAAAGTCCCCTTCCATCCACTGGAGCAG
C
(SEQ ID NO:196)

Mus musculus Finkel-Biskis-Reilly murine sarcoma virus (FBR-MuSV) ubiquitously expressed (FAU)
GTCCTATTGCCACCCTGCCATGCTAATAAAGCCACTGTGTCCAGACTTCT
(SEQ ID NO:197)

Mus musculus ribosomal protein L22-like 1 (RPL22L1)

TTGGCCTCTGCTTGTAATACAATGAAAGTATTCTAAAGAAATATAAAATTGGACTTTATGAGAAAA
TAAAAGTCATTTCACTCT

(SEQ ID NO:198)

Mus musculus ribosomal protein S17 (RPS17)
TCTGTTATGCCATATTTTCAATAAACCTGAAAACAA
(SEQ ID NO:199)

Mus musculus ribosomal protein L39-like (RPL39L)

GGAACCACACGTACTTATGCTGTAACTTACTGTAGCGTTTACAGCGTTACCGCTGTCTGGACAGCT
GAGTGTGTTTCTTAGGAATATAAGTTTTCTTTCTGTGCTTTAGTGAGTTCATTCAGCAGTTCAGTA
ATAAATATGTGAAACCTTTTGTTTCGAAAAAAATTGCTTCTGTGTTACAACTTACTTTGTTTTATG
GTTCAGGATCATCTGCATAATAGACAAGTATTCTATCAGTGAGCGATATCCTGGATCTTGTTTGTG
TAGTTTGGGGTTGAGACAAGTCCAGACTGCCCTAAAACTCACGATCTTCCTTCCTCGGCCTTCTAA
ATGATTGGAGGACTCCAAACCTAAGTGATGGAAGTAAAAAGAAACTTATATGTCAAGACTCATTTT
CTCTATCATTTCATGTGACAATTGAAATTAGATTATTTCTTTTTTCAATC
(SEQ ID NO:200)

Mus musculus ribosomal protein L10-like (RPL10L)

GGACTTTGCTGGCAGAAAAGAAGTACAGATGAGGTTATAGTTTGAAAAACGTTAATTCCGTTTATT
GACTTTAGAATGTTACTTTGCATAGTATAGACCATTACAATGGAAAGTACCTGCCTTAAGAAACAA
GAAAACTGCAGTTTATAGAGAAAGAATTTTCAATTTTGACCCATGTACTTAAAATTTTTGGTGTAT
ACTGCAGTGTAGCAAATGTTTTGTGGTGACGGTATAAATGGTACTGTTTGTTATCTTGGATTAAGA
GTGGCTAGAGAAGTTGGAAGACGTGTGAGAAGTTCTTTATAGAGAATTAAACATGAAAATTACATC
TC
(SEQ ID NO:201)

Mus musculus ribosomal protein L36a-like (RPL36AL)

AACTTGTGTTCTCTGAGAGGAAAATACTGAAGCAGTAGAGAAATGACCTGCTAGAGAATAAAGTTA
CTGTTAATGATACC
(SEQ ID NO:202)

Mus musculus ribosomal protein L3-like (RPL3L)

GATGTCTGGAATGGAGCACACCTGGGATGTAGCACTCTTGCTATCTGTCCGGTCCTTTTTGTTCAA
TAAAGTCCTGAGGCAACTCTCTCTGTC
(SEQ ID NO:203)

Mus musculus ribosomal protein S27-like (RPS27L)

TGAGCTATGAAGTTCCGGAATTTGTGTTTTTCACAGAAAGCCTTACCAACTTCAGTTACTTTACCA
AGACAATGTAATTATTGTTTGATTTTATAAAGTCTACAACAATGATCTCCTATTTTGGTGTCAGTT
TTTCAATAAAGTTTTAATTA
(SEQ ID NO:204)

Mus musculus ribosomal protein L7-like 1 (RPL7L1)

AGCAGGAGCAGGTTTTCCAAAAGCACCCCTCGGAAGTGTTTTTGTCGTCGTTTAAAATTATCAAGT
ATCTTCAGAGAAGATTATTTTCTGCCTTCAGAAACTGAAGGAAGGCTTGGGCCTAGAGAACGACAG
TAAGGTGCGAGCACCGGAGACACTTAACACAGCTCAGTCCATGGAAGGACGAGTTCCCTCATTGGC
TGCCTGTCTCGAAATCCACGCAAGCTGTGGAGGAAAGAATTACCCTGCTCATCCTGCCTTCTATCT
TGGTGTTTAATGTTGGGTGGGCAACAAGCACAAACCTCCCTCCCACCCCCTCCAAGACTGTTAGAG
CAGTGGGCCAGACCAAGCGGCGCACTTGAACATGGATCAAGAGGGTCCCGGTTTTACTTTTTATTT
TTGTCAGGGTAGGCAGTCTTGTGTTTGCTTTGTTCAAAGCAGGGTCTCCCTGTTGGCCCTGGCTGG
CCTTATACTCCACAGCAGTCCTGCCTCCTCCTCCTAGGTGCTGGGATTAAAGGCGTGCGCCACCAC
GCCCGGCTACAGCCTGCATTTTTATGCACATTGGTCTGTTAAGCTAGTTGCATTCTGTGCTACCGG
AGGGGACTGAAGTTTAATCACTTGTCTTCTATTAAAAACTAGTGTTTGCCTGGGCCTGGTGTGTAT

ACCTTTAGTTGCAGCGCTTGGGAGGCAGAGGCAGGCAGACTTCATGAGTTCAGGGACAGGCAAGCC
TGCTCTACAGATTTCCAGGATACCCAGGGCTACACATAGAGAAAATGTTAAAGATAAACAAAAAGC
TGGACAGTGGGGGAGCACACCTTTAATCCCAGCACTCGGGAGGCAGAGGCAGGCGGATTTCTGAGT
TCGAGGCCAGCCTGGTCTACAGAGTGAGTTCCAGGACAGCCAGGACTACACAGAGAAACCCTGTCT
CAGAAAAAAAACAAAACAAAAACCAATGCAGTGATAGATTGTTGTTTCCTAAACCACATGTACCCA
GGAAATGCCCACTAAATTTCACCTGGATCAGTGTTAACTGATCATTGGGAAATGAGGTGACCAAAA
ATGCATCGCAACCTTGGACAAACAGCATGGCTATTTAACATTCTGGGATCCTGCAGAATCCTGCAT
CTTCCTAAGTAGGGAAGCACTGTAGCATTGGAGAGAGGCCTGGGCGAGCAGAGCTAAGGCTTCCAT
TTCTGGCTTGCTTGGAATTTAAAACAAGCTTTTTTCTATATAGTAAAAGATTGTTTTTAAGATTTT
TGCGTGTGAGTACATGCCAAAGTAGCCAGGAAGTGTCACTTGCCCTGGAGCTAGAATTACTGGCAA
ATGAAGGCTCAGAGGTGGATGCTGGGACCAATTCTAGGCCCTCTGAGAAAGCAGGTGCACTTGGCT
TGTGCCTCCAGCCCCAAAGGCGATGGCTTATTGTGAGCCTGAGGCCAGCCAGGGTTACAGAGACTC
AAGAAACAAGTGGGGTTGTCCATGTTGCTGGAGATGACCCAGGTCTATTAGGACCTTGACTACATG
GATAGACATTCTGGCAGCTAGTATACTGCCATTGGGGCCTATGGAGAAGTAGCCACCGAGCCTATT
TAGAAAGAAGGACTGCTGGCAAGCTTGGTGTCACTATGAAGGCAGACAAAGATCGATGTATTAACG
ACCCCGACTCCAAAAACACTCGAGGGGGCCCAAGGTGGGCTCAGTGGTTAAGAGCCGTTCGCCCAG
GGGCTGGAGAGTTGGCTCAGTGGTACCACATGGTGGCTCACAACCATCTGTAATGAGATCTGACGC
CCTCTTCTGGTGTATCTGAAGACAGCTACAGTGTACTTACATAAAATAAATAAATCTTT
(SEQ ID NO:205)

In a preferred embodiment, the at least one 3'-UTR element of the artificial nucleic acid molecule according to the present invention comprises or consists of a nucleic acid sequence which has an identity of at least about 40%, preferably of at least about 50%, preferably of at least about 60%, preferably of at least about 70%, more preferably of at least about 80%, more preferably of at least about 90%, even more preferably of at least about 95%, even more preferably of at least about 99%, most preferably of 100% to the 3'-UTR sequence of ribosomal protein Small 9 (RPS9). Most preferably, the at least one 3'-UTR element of the artificial nucleic acid molecule according to the present invention comprises or consists of a nucleic acid sequence which has an identity of at least about 40%, preferably of at least about 50%, preferably of at least about 60%, preferably of at least about 70%, more preferably of at least about 80%, more preferably of at least about 90%, even more preferably of at least about 95%, even more preferably of at least about 99%, most preferably of 100% to SEQ ID NO: 1 or SEQ ID NO: 2

SEQ ID NO: 1

GTCCACCTGTCCCTCCTGGGCTGCTGGATTGTCTCGTTTTCCTGCCAAATAAACAGGATCAGCGCT
TTAC

SEQ ID NO: 2

GUCCACCUGUCCCUCCUGGGCUGCUGGAUUGUCUCGUUUUCCUGCCAAAUAAACAGGAUCAGCGCU
UUAC

The at least one 3'-UTR element of the artificial nucleic acid molecule according to the present invention may also comprise or consist of a fragment of a nucleic acid sequence which has an identity of at least about 40%, preferably of at least about 50%, preferably of at least about 60%, preferably of at least about 70%, more preferably of at least about 80%, more preferably of at least about 90%, even more preferably of at least about 95%, even more preferably of at least about 99%, most preferably of 100% to the nucleic acid sequence of the 3'-UTR of a ribosomal protein gene, such as to the 3'-UTR of a sequence according to SEQ ID NOs:10 to 205, wherein the fragment is preferably a functional fragment or a functional variant fragment as described above. Such fragment preferably exhibits a length of at least about 3 nucleotides, preferably of at least about 5 nucleotides, more preferably of at least about 10, 15, 20, 25 or 30 nucleotides, even more preferably of at least about 50 nucleotides, most preferably of at least about 70 nucleotides. In a preferred embodiment, the fragment or variant thereof exhibits a length of between 3 and about 500 nucleotides, preferably of between 5 and about 150 nucleotides, more preferably of between 10 and 100 nucleotides, even more preferably of between 15 and 90, most preferably of between 20 and 70.

Preferably, said variants, fragments or variant fragments are functional variants, functional fragments, or functional variant fragments as described above, exhibiting at least one function of the nucleic acid sequence according to SEQ ID NOs:10 to 205, such as stabilization of the artificial nucleic acid molecule according to the invention, stabilizing and/or prolonging protein expression from the artificial nucleic acid molecule according to the invention, and/or increasing protein production, preferably with an efficiency of at least 40%, more preferably of at least 50%, more preferably of at least 60%, even more preferably of at least 70%, even more preferably of at least 80%, most preferably of at least 90% of the stabilizing efficiency and/or protein production increasing efficiency exhibited by an artificial nucleic acid molecule comprising the nucleic acid sequence according to SEQ ID No. 1 or SEQ ID NO. 2.

Preferably, the at least one 3'-UTR element of the artificial nucleic acid molecule according to the present invention exhibits a length of at least about 3 nucleotides, preferably of at least about 5 nucleotides, more preferably of at least about 10, 15, 20, 25 or 30 nucleotides, even more preferably of at least about 50 nucleotides, most preferably of at least about 70 nucleotides. The upper limit for the length of the 3'-UTR element may be 500 nucleotides or less, e.g. 400, 300, 200, 150 or 100 nucleotides. For other embodiments the upper limit may be chosen within the range of 50 to 100 nucleotides. For example, the fragment or variant thereof may exhibit a length of between 3 and about 500 nucleotides, preferably of between 5 and about 150 nucleotides, more preferably of between 10 and 100 nucleotides, even more preferably of between 15 and 90, most preferably of between 20 and 70.

Furthermore, the artificial nucleic acid molecule according to the present invention may comprise more than one 3'-UTR elements as described above. For example, the artificial nucleic acid molecule according to the present invention may comprise one, two, three, four or more 3'-UTR elements, wherein the individual 3'-UTR elements may be the same or they may be different. For example, the artificial nucleic acid molecule according to the present invention may comprise two essentially identical 3'-UTR elements as described above, e.g. two 3'-UTR elements comprising or consisting of a nucleic acid sequence, which is derived from the 3'-UTR of a ribosomal protein gene, such as from a sequence according to SEQ ID NO: 10 to 205, or from a fragment or variant of the 3'-UTR of a ribosomal protein gene, such as a nucleic acid sequence according to SEQ ID No. 1 or 2, functional variants thereof, functional fragments thereof, or functional variant fragments thereof as described above.

Surprisingly, the inventors found that an artificial nucleic acid molecule comprising a 3'-UTR element as described above may represent or may provide an mRNA molecule, which allows for enhanced, prolonged and/or stabilized protein production. Thus, a 3'-UTR element as described herein may improve stability of protein expression from an mRNA molecule and/or improve translational efficiency.

The artificial nucleic acid molecule according to the present invention may be RNA, such as mRNA, DNA, such as a DNA vector, or may be a modified RNA or DNA molecule. It may be provided as a double-stranded molecule having a sense strand and an anti-sense strand, for example, as a DNA molecule having a sense strand and an anti-sense strand.

The artificial nucleic acid molecule according to the present invention may further comprise optionally a 5'-UTR and/or a 5'-cap. The optional 5'-cap and/or the 5'-UTR are preferably located 5' to the ORF within the artificial nucleic acid molecule according to the present invention.

Preferably, the artificial nucleic acid molecule according to the present invention further comprises a poly(A) sequence and/or a polyadenylation signal. Preferably, the optional poly(A) sequence is located 3' to the at least one 3'-UTR element, preferably the optional poly(A) sequence is connected to the 3'-end of the 3'-UTR element. The connection may be direct or indirect, for example, via a stretch of 2, 4, 6, 8, 10, 20 etc. nucleotides, such as via a linker of 1-50, preferably

of 1-20 nucleotides, e.g. comprising or consisting of one or more restriction sites.

In one embodiment, the optional polyadenylation signal is located downstream of the 3' of the 3'-UTR element. Preferably, the polyadenylation signal comprises the consensus sequence NN(U/T)ANA, with N = A or U, preferably AA(U/T)AAA or A(U/T)(U/T)AAA. Such consensus sequence may be recognised by most animal and bacterial cell-systems, for example by the polyadenylation-factors, such as cleavage/polyadenylation specificity factor (CPSF) cooperating with CstF, PAP, PAB2, CFI and/or CFII. Preferably, the polyadenylation signal, preferably the consensus sequence NNUANA, is located less than about 50 nucleotides, more preferably less than about 30 bases, most preferably less than about 25 bases, for example 21 bases, downstream of the 3'-end of the 3'-UTR element.

Transcription of an artificial nucleic acid molecule according to the present invention, e.g. of an artificial DNA molecule, comprising a polyadenylation signal downstream of the 3'-UTR element will result in a premature-RNA containing the polyadenylation signal downstream of its 3'-UTR element. For example, transcription of a DNA molecule comprising a 3'-UTR element according to SEQ ID No. 1 will result in an RNA having a 3'-UTR element according to the sequence SEQ ID No. 2.

Using an appropriate transcription system will then lead to attachment of a poly(A) sequence to the premature-RNA. For example, the inventive artificial nucleic acid molecule may be a DNA molecule comprising a 3'-UTR element as described above and a polyadenylation signal, which may result in polyadenylation of an RNA upon transcription of this DNA molecule. Accordingly, a resulting RNA may comprise a combination of the inventive 3'-UTR element followed by a poly(A) sequence.

Potential transcription systems are *in vitro* transcription systems or cellular transcription systems etc. Accordingly, transcription of an artificial nucleic acid molecule according to the invention, e.g. transcription of an artificial nucleic acid molecule comprising an open reading frame, a 3'-UTR element and a polyadenylation-signal, may result in an mRNA molecule comprising an open reading frame, a 3'-UTR element and a poly(A) sequence.

Accordingly, the invention also provides an artificial nucleic acid molecule, which is an mRNA molecule comprising an open reading frame, a 3'-UTR element as described above and a poly(A) sequence.

In one embodiment, the invention provides an artificial nucleic acid molecule, which is an artificial DNA molecule comprising an open reading frame and a sequence according to SEQ ID No. 1 or a sequence having an identity of at least about 40% or more to SEQ ID No. 1 or a fragment thereof as described above. Furthermore, the invention provides an artificial nucleic acid molecule which is an artificial RNA molecule comprising an open reading frame and a sequence according to SEQ ID NO. 2 or a sequence having an identity of at least about 40 % or more to SEQ ID No. 2 or a fragment thereof as described above.

Accordingly, the invention provides an artificial nucleic acid molecule, which may serve as a template for an RNA molecule, preferably for an mRNA molecule, which is stabilised and optimized with respect to translation efficiency. In other words, the artificial nucleic acid molecule may be a DNA, which may be used as a template for production of an mRNA. The obtainable mRNA, may, in turn, be translated for production of a desired peptide or protein encoded by the open reading frame. If the artificial nucleic acid molecule is a DNA, it may, for example, be used as a double-stranded storage form for continued and repetitive *in vitro* or *in vivo* production of mRNA.

In another embodiment, the 3'-UTR of the artificial nucleic acid molecule according to the invention does not comprise a polyadenylation signal or a poly(A) sequence. Further preferably, the artificial nucleic acid molecule according to the invention does not comprise a polyadenylation signal or a poly(A) sequence. More preferably, the 3'-UTR of the artificial nucleic acid molecule, or the inventive artificial nucleic acid molecule as such, does not comprise a polyadenylation signal, in particular it does not comprise the polyadenylation signal AAU/TAAA.

In one embodiment, the artificial nucleic acid molecule according to the present invention further comprises a poly(A) sequence. For example, a DNA molecule comprising an ORF followed by the ribosomal 3' UTR may contain a stretch of thymidine nucleotides which can be transcribed into a poly(A) sequence in the resulting mRNA. The length of the poly(A) sequence may vary. For example, the poly(A) sequence may have a length of about 20 adenine nucleotides up to about 300 adenine nucleotides, preferably of about 40 to about 200 adenine nucleotides, more preferably from about 50 to about 100 adenine nucleotides, such as about 60, 70, 80, 90 or 100 adenine nucleotides. Most preferably, the inventive nucleic acid comprises a poly(A) sequence of about 60 to about 70 nucleotides, most preferably 64 adenine nucleotides.

For example, the artificial nucleic acid molecule according to the present invention may comprise a nucleic acid sequence corresponding to the DNA-sequence

GTCCACCTGTCCCTCCTGGGCTGCTGGATTGTCTCGTTTTCCTGCCAAATAAACAGGATCAGCGCT
TTACAGATCTAAAAAAAAAAAAAAAAAAAAAAAAAAAAAAAAAAAAAAAAAAAAAAAA
AAAAAAAA (SEQ ID No. 3).

Transcription of such sequences may result in artificial nucleic acid molecules comprising the sequence

GUCCACCUGUCCCUCCUGGGCUGCUGGAUUGUCUCGUUUUCCUGCCAAAUAAACAGGAUCAGCGCU
UUACAGAUCUAAAAAAAAAAAAAAAAAAAAAAAAAAAAAAAAAAAAAAAAAAAAAAAA
AAAAAAAA (SEQ ID No. 4).

Such artificial RNA-molecules, i.e. artificial nucleic acid molecules comprising a sequence according to SEQ ID No. 4 may also be obtainable *in vitro* by common methods of chemical-synthesis without being necessarily transcribed from a DNA-progenitor.

In a particularly preferred embodiment, the artificial nucleic acid molecule according to the present invention is an RNA molecule, preferably an mRNA molecule comprising in 5'-to-3'-direction an open reading frame, a 3'-UTR element as described above and a poly(A) sequence.

Preferably, the open reading frame does not code for a ribosomal protein. In a preferred embodiment, the open reading frame does not code for a ribosomal protein, from which the 3'-UTR element of the inventive artificial nucleic acid is derived, particularly not for a mammalian ribosomal protein, provided that the 3'-UTR element is identical to the 3'-UTR of a mammalian ribosomal protein gene. In some further preferred embodiments, the open reading frame does not code for RPS9 or variants thereof, provided that the 3'-UTR element is a sequence which is identical to SEQ ID No. 1 or SEQ ID No. 2.

In a preferred embodiment, the ORF does not encode human or plant, in particular Arabidopsis, ribosomal proteins, in particular does not encode human ribosomal protein S6 (RPS6), human ribosomal protein L36a-like (RPL36AL) or Arabidopsis ribosomal protein S16 (RPS16). In a further preferred embodiment, the open reading frame (ORF) does not encode ribosomal protein S6 (RPS6), ribosomal protein L36a-like (RPL36AL) or ribosomal protein S16 (RPS16) of whatever origin.

In one embodiment, the invention provides an artificial DNA molecule comprising an open reading frame, preferably an open reading frame which encodes a peptide or protein other than the ribosomal protein, from which the 3'-UTR is derived; a 3'-UTR element comprising or consisting of a sequence which has at least about 60%, preferably at least about 70%, more preferably at least about 80%, more preferably at least about 90%, even more preferably at least about 95%; even more preferably at least 99%; even more preferably 100% sequence identity to SEQ ID No. 1; and a polyadenylation signal and/or a poly(A) sequence. Furthermore, the invention provides an artificial DNA molecule comprising an open reading frame, preferably an open reading frame which encodes any peptide or protein other than the ribosomal protein, from which the 3'-UTR is derived; a 3'-UTR element comprising or consisting of a sequence, which has at least about 60%, preferably at least about 70%, more preferably at least about 80%, more preferably at least about 90%, even more preferably at least about 95%; even more preferably at least 99%; even more preferably 100% sequence identity to SEQ ID No. 3.

Furthermore, the invention provides an artificial RNA molecule, preferably an artificial mRNA molecule or an artificial viral RNA molecule, comprising an open reading frame, preferably an open reading frame which encodes a peptide or protein other than the ribosomal protein, from which the 3'-UTR is derived; a 3'-UTR element comprising or consisting of a sequence which has at least about 60%, preferably at least about 70%, more preferably at least about 80%, more preferably at least about 90%, even more preferably at least about 95%; even more preferably at least 99%; even more preferably 100% sequence identity to SEQ ID No. 2; and a polyadenylation signal and/or a poly(A) sequence. Furthermore, the invention provides an artificial RNA molecule, preferably an artificial mRNA molecule or an artificial viral RNA molecule, comprising an open reading frame, preferably an open reading frame which encodes a peptide or protein other than the ribosomal protein, from which the 3'-UTR is derived; a 3'-UTR element comprising or consisting of a sequence which has at least about 60%, preferably at least about 70%, more preferably at least about 80%, more preferably at least about 90%, even more preferably at least about 95%; even more preferably at least 99%; even more preferably 100% sequence identity to SEQ ID No. 4.

The invention provides an artificial nucleic acid molecule, preferably an artificial mRNA, which may be characterized by enhanced stability and prolonged expression of the encoded peptide or protein. Without being bound by any theory, enhanced stability of protein expression and thus prolonged protein expression may result from reduction in degradation of the artificial nucleic acid molecule, such as an artificial mRNA molecule according to the present invention. Accordingly, the inventive 3'-UTR element may prevent the artificial nucleic acid from degradation and decay.

In some embodiments, the artificial nucleic acid molecule may comprise a histone stem-loop in addition to the nucleic acid sequence derived from the 3'-UTR of a ribosomal protein gene. Such artificial nucleic acid molecule according to the present invention, for example, may comprise in 5'-to-3'-direction an ORF, an 3'-UTR element, an optional histone stem-loop sequence, an optional poly(A) sequence or polyadenylation signal and an optional poly(C) sequence. It may also comprise in 5'-to-3'-direction an ORF, an 3'-UTR element, an optional poly(A) sequence, an optional poly (C)

sequence and an optional histone stem-loop sequence.

In a preferred embodiment, the artificial nucleic acid molecule according to the invention comprises at least one histone stem-loop sequence.

Such histone stem-loop sequences are preferably selected from histone stem-loop sequences as disclosed in WO 2012/019780, whose disclosure is incorporated herewith by reference.

A histone stem-loop sequence, suitable to be used within the present invention, is preferably selected from at least one of the following formulae (I) or (II):

formula (1) (stem-loop sequence without stem bordering elements):

$$\underbrace{[N_{0-2}GN_{3-5}]}_{\text{stem1}}\ \underbrace{[N_{0-4}(U/T)N_{0-4}]}_{\text{loop}}\ \underbrace{[N_{3-5}CN_{0-2}]}_{\text{stem2}}$$

formula (II) (stem-loop sequence with stem bordering elements):

$$\underbrace{N_{1-6}}_{\substack{\text{stem1}\\ \text{bordering element}}}\ \underbrace{[N_{0-2}GN_{3-5}]}_{\text{stem1}}\ \underbrace{[N_{0-4}(U/T)N_{0-4}]}_{\text{loop}}\ \underbrace{[N_{3-5}CN_{0-2}]}_{\text{stem2}}\ \underbrace{N_{1-6}}_{\substack{\text{stem2}\\ \text{bordering element}}}$$

wherein:

| | |
|---|---|
| stem1 or stem2 bordering elements $N_{1-6}$ | is a consecutive sequence of 1 to 6, preferably of 2 to 6, more preferably of 2 to 5, even more preferably of 3 to 5, most preferably of 4 to 5 or 5 N, wherein each N is independently from another selected from a nucleotide selected from A, U, T, G and C, or a nucleotide analogue thereof; |
| stem1 $[N_{0-2}GN_{3-5}]$ | is reverse complementary or partially reverse complementary with element stem2, and is a consecutive sequence between of 5 to 7 nucleotides; |
| | wherein $N_{0-2}$ is a consecutive sequence of 0 to 2, preferably of 0 to 1, more preferably of 1 N, wherein each N is independently from another selected from a nucleotide selected from A, U, T, G and C or a nucleotide analogue thereof; |
| | wherein $N_{3-5}$ is a consecutive sequence of 3 to 5, preferably of 4 to 5, more preferably of 4 N, wherein each N is independently from another selected from a nucleotide selected from A, U, T, G and C or a nucleotide analogue thereof, and |
| | wherein G is guanosine or an analogue thereof, and may be optionally replaced by a cytidine or an analogue thereof, provided that its complementary nucleotide cytidine in stem2 is replaced by guanosine; |
| loop sequence $[N_{0-4}(U/T)N_{0-4}]$ | is located between elements stem1 and stem2, and is a consecutive sequence of 3 to 5 nucleotides, more preferably of 4 nucleotides; |
| | wherein each $N_{0-4}$ is independent from another a consecutive sequence of 0 to 4, preferably of 1 to 3, more preferably of 1 to 2 N, wherein each N is independently from another selected from a nucleotide selected from A, U, T, G and C or a nucleotide analogue thereof; and wherein U/T represents uridine, or optionally thymidine; |
| stem2 $[N_{3-5}CN_{0-2}]$ | is reverse complementary or partially reverse complementary with element stem1, and is a consecutive sequence between of 5 to 7 nucleotides; |
| | wherein $N_{3-5}$ is a consecutive sequence of 3 to 5, preferably of 4 to 5, more preferably of 4 N, wherein each N is independently from another selected from a nucleotide selected from A, U, T, G and C or a nucleotide analogue thereof; |
| | wherein $N_{0-2}$ is a consecutive sequence of 0 to 2, preferably of 0 to 1, more preferably of 1 N, wherein each N is independently from another selected from a nucleotide selected from A, U, T, G or C or a nucleotide analogue thereof; and |

(continued)

wherein C is cytidine or an analogue thereof, and may be optionally replaced by a guanosine or an analogue thereof provided that its complementary nucleoside guanosine in stem1 is replaced by cytidine;

wherein
stem1 and stem2 are capable of base pairing with each other forming a reverse complementary sequence, wherein base pairing may occur between stem1 and stem2, e.g. by Watson-Crick base pairing of nucleotides A and U/T or G and C or by non-Watson-Crick base pairing e.g. wobble base pairing, reverse Watson-Crick base pairing, Hoogsteen base pairing, reverse Hoogsteen base pairing or are capable of base pairing with each other forming a partially reverse complementary sequence, wherein an incomplete base pairing may occur between stem1 and stem2, on the basis that one ore more bases in one stem do not have a complementary base in the reverse complementary sequence of the other stem.

According to a further preferred embodiment the histone stem-loop sequence may be selected according to at least one of the following specific formulae (Ia) or (IIa):

formula (Ia) (stem-loop sequence without stem bordering elements):

$$\underbrace{[N_{0-1}GN_{3-5}]}_{\text{stem1}}\ \underbrace{[N_{1-3}(U/T)N_{0-2}]}_{\text{loop}}\ \underbrace{[N_{3-5}CN_{0-1}]}_{\text{stem2}}$$

formula (IIa) (stem-loop sequence with stem bordering elements):

$$\underbrace{N_{2-5}}_{\substack{\text{stem1}\\\text{bordering element}}}\ \underbrace{[N_{0-1}GN_{3-5}]}_{\text{stem1}}\ \underbrace{[N_{1-3}(U/T)N_{0-2}]}_{\text{loop}}\ \underbrace{[N_{3-5}CN_{0-1}]}_{\text{stem2}}\ \underbrace{N_{2-5}}_{\substack{\text{stem2}\\\text{bordering element}}}$$

wherein:

N, C, G, T and U are as defined above.

According to a further more particularly preferred embodiment of the first aspect, the artificial nucleic acid molecule sequence may comprise at least one histone stem-loop sequence according to at least one of the following specific formulae (Ib) or (IIb):

formula (Ib) (stem-loop sequence without stem bordering elements):

$$\underbrace{[N_{1}GN_{4}]}_{\text{stem1}}\ \underbrace{[N_{2}(U/T)N_{1}]}_{\text{loop}}\ \underbrace{[N_{4}CN_{1}]}_{\text{stem2}}$$

formula (IIb) (stem-loop sequence with stem bordering elements):

$$\underbrace{N_{4-5}}_{\substack{\text{stem1}\\\text{bordering element}}}\ \underbrace{[N_{1}GN_{4}]}_{\text{stem1}}\ \underbrace{[N_{2}(U/T)N_{1}]}_{\text{loop}}\ \underbrace{[N_{4}CN_{1}]}_{\text{stem2}}\ \underbrace{N_{4-5}}_{\substack{\text{stem2}\\\text{bordering element}}}$$

wherein:

N, C, G, T and U are as defined above.

A particular preferred histone stem-loop sequence is the sequence according to SEQ ID NO: 5: CAAAGGCTCTTTTCAGAGCCACCA or more preferably the corresponding RNA sequence of the nucleic acid sequence according to SEQ ID NO: 5.

As an example, the single elements may be present in the artificial nucleic acid molecule in the following order:

5'-cap - 5'-UTR - ORF - 3'-UTR element - histone stem-loop - poly(A)/(C) sequence;
5'-cap - 5'-UTR - ORF - 3'-UTR element - poly(A)/(C) sequence - histone stem-loop;
5'-cap - 5'-UTR - ORF - IRES - ORF - 3'-UTR element - histone stem-loop - poly(A)/(C) sequence;
5'-cap - 5'-UTR - ORF - IRES - ORF - 3'-UTR element - histone stem-loop - poly(A)/(C) sequence - poly(A)/(C) sequence;
5'-cap - 5'-UTR - ORF - IRES - ORF - 3'-UTR element - poly(A)/(C) sequence - histone stem-loop;
5'-cap - 5'-UTR - ORF - IRES - ORF - 3'-UTR element - poly(A)/(C) sequence - poly(A)/(C) sequence - histone stem-loop;
5'-cap - 5'-UTR - ORF - 3'-UTR element - poly(A)/(C) sequence - poly(A)/(C) sequence;
5'-cap - 5'-UTR - ORF - 3'-UTR element - poly(A)/(C) sequence - poly(A)/(C) sequence - histone stem loop; etc.

In some embodiments, the artificial nucleic acid molecule comprises further elements such as a 5'-cap, a poly(C) sequence and/or an IRES-motif. A 5'-cap may be added during transcription or post-transcriptionally to the 5'end of an RNA. Furthermore, the inventive artificial nucleic acid molecule, particularly if the nucleic acid is in the form of an mRNA or codes for an mRNA, may be modified by a sequence of at least 10 cytidines, preferably at least 20 cytidines, more preferably at least 30 cytidines (so-called "poly(C) sequence"). In particular, the inventive artificial nucleic acid molecule may contain, especially if the nucleic acid is in the form of an (m)RNA or codes for an mRNA, a poly(C) sequence of typically about 10 to 200 cytidine nucleotides, preferably about 10 to 100 cytidine nucleotides, more preferably about 10 to 70 cytidine nucleotides or even more preferably about 20 to 50 or even 20 to 30 cytidine nucleotides. Most preferably, the inventive nucleic acid comprises a poly(C) sequence of 30 cytidine residues. Thus, preferably the artificial nucleic acid molecule according to the present invention comprises, preferably in 5'-to-3' direction, an ORF, at least one 3'-UTR element as described above, a poly(A) sequence or a polyadenylation signal, and a poly(C) sequence.

An internal ribosome entry site (IRES) sequence or IRES-motif may separate several open reading frames, for example if the artificial nucleic acid molecule encodes for two or more peptides or proteins. An IRES-sequence may be particularly helpful if the artificial nucleic acid molecule is a bi- or multicistronic nucleic acid molecule.

Furthermore, the artificial nucleic acid molecule may comprise additional 5'-elements, preferably a 5'-UTR, a promoter, or a 5'-UTR and a promoter containing-sequence. The promoter may drive and or regulate transcription of the artificial nucleic acid molecule according to the present invention, for example of an artificial DNA-molecule according to the present invention. Furthermore, the 5'-UTR may consist or may comprise the 5'-UTR of a gene as defined herein. Furthermore the 5'-UTR may interact with the inventive 3'-UTR element and thus may support the stabilising effect of the inventive 3'-UTR element. Such elements may further support stability and translational efficiency. Accordingly, in some embodiments, the invention provides artificial nucleic acid molecules, preferably mRNA-molecules, comprising in 5'-to-3'-direction at least one of the following structures
5'-cap - 5'-UTR - ORF - 3'-UTR element - histone stem-loop - poly(A)/(C) sequence;
5'-cap - 5'-UTR - ORF - 3'-UTR element - poly(A)/(C) sequence - histone stem-loop;
5'-cap - 5'-UTR - ORF - IRES - ORF - 3'-UTR element - histone stem-loop - poly(A)/(C) sequence;
5'-cap - 5'-UTR - ORF - IRES - ORF - 3'-UTR element - histone stem-loop - poly(A)/(C) sequence - poly(A)/(C) sequence;
5'-cap - 5'-UTR - ORF - IRES - ORF - 3'-UTR element - poly(A)/(C) sequence - histone stem-loop;
5'-cap - 5'-UTR - ORF - IRES - ORF - 3'-UTR element - poly(A)/(C) sequence - poly(A)/(C) sequence - histone stem-loop;
5'-cap - 5'-UTR - ORF - 3'-UTR element - poly(A)/(C) sequence - poly(A)/(C) sequence;
5'-cap - 5'-UTR - ORF - 3'-UTR element - poly(A)/(C) sequence - poly(A)/(C) sequence - histone stem loop.

In a particularly preferred embodiment of the present invention the artificial nucleic acid molecule comprises at least one 5'-untranslated region element (5'UTR element) of a length of less than 500, 400, 300, 250, 200, 150 or 100 nucleotides and/or of more than 20, 30, 40, 50 or 60 nucleotides, which preferably comprises or consists of a nucleic acid sequence which is derived from the 5'UTR of a TOP gene or which is derived from a fragment, homolog or variant of the 5'UTR of a TOP gene.

It is particularly preferred that the 5'UTR element does not comprise a TOP-motif or a 5'TOP, as defined above.

The nucleic acid sequence, which is derived from the 5'UTR of a TOP gene is derived from a eukaryotic TOP gene, preferably a plant or animal TOP gene, more preferably a chordate TOP gene, even more preferably a vertebrate

TOP gene, most preferably a mammalian TOP gene, such as a human TOP gene.

For example, the 5'UTR element is preferably selected from 5'-UTR elements comprising or consisting of a nucleic acid sequence, which is derived from a nucleic acid sequence selected from the group consisting of SEQ ID NOs. 1-1363, SEQ ID NO. 1395, SEQ ID NO. 1421 and SEQ ID NO. 1422 of the patent application WO2013/143700 whose disclosure is incorporated herein by reference, from the homologs of SEQ ID NOs. 1-1363, SEQ ID NO. 1395, SEQ ID NO. 1421 and SEQ ID NO. 1422 of the patent application WO2013/143700, from a variant thereof, or preferably from a corresponding RNA sequence. The term "homologs of SEQ ID NOs. 1-1363, SEQ ID NO. 1395, SEQ ID NO. 1421 and SEQ ID NO. 1422 of the patent application WO2013/143700" refers to sequences of other species than homo sapiens, which are homologous to the sequences according to SEQ ID NOs. 1-1363, SEQ ID NO. 1395, SEQ ID NO. 1421 and SEQ ID NO. 1422 of the patent application WO2013/143700.

In a preferred embodiment, the 5'UTR element comprises or consists of a nucleic acid sequence, which is derived from a nucleic acid sequence extending from nucleotide position 5 (i.e. the nucleotide that is located at position 5 in the sequence) to the nucleotide position immediately 5' to the start codon (located at the 3' end of the sequences), e.g. the nucleotide position immediately 5' to the ATG sequence, of a nucleic acid sequence selected from SEQ ID NOs. 1-1363, SEQ ID NO. 1395, SEQ ID NO. 1421 and SEQ ID NO. 1422 of the patent application WO2013/143700, from the homologs of SEQ ID NOs. 1-1363, SEQ ID NO. 1395, SEQ ID NO. 1421 and SEQ ID NO. 1422 of the patent application WO2013/143700, from a variant thereof, or a corresponding RNA sequence. It is particularly preferred that the 5' UTR element is derived from a nucleic acid sequence extending from the nucleotide position immediately 3' to the 5'TOP to the nucleotide position immediately 5' to the start codon (located at the 3' end of the sequences), e.g. the nucleotide position immediately 5' to the ATG sequence, of a nucleic acid sequence selected from SEQ ID NOs. 1-1363, SEQ ID NO. 1395, SEQ ID NO. 1421 and SEQ ID NO. 1422 of the patent application WO2013/143700, from the homologs of SEQ ID NOs. 1-1363, SEQ ID NO. 1395, SEQ ID NO. 1421 and SEQ ID NO. 1422 of the patent application WO2013/143700, from a variant thereof, or a corresponding RNA sequence.

In a particularly preferred embodiment, the 5'UTR element comprises or consists of a nucleic acid sequence, which is derived from a 5'UTR of a TOP gene encoding a ribosomal protein or from a variant of a 5'UTR of a TOP gene encoding a ribosomal protein. For example, the 5'UTR element comprises or consists of a nucleic acid sequence which is derived from a 5'UTR of a nucleic acid sequence according to any of SEQ ID NOs: 170, 232, 244, 259, 1284, 1285, 1286, 1287, 1288, 1289, 1290, 1291, 1292, 1293, 1294, 1295, 1296, 1297, 1298, 1299, 1300, 1301, 1302, 1303, 1304, 1305, 1306, 1307, 1308, 1309, 1310, 1311, 1312, 1313, 1314, 1315, 1316, 1317, 1318, 1319, 1320, 1321, 1322, 1323, 1324, 1325, 1326, 1327, 1328, 1329, 1330, 1331, 1332, 1333, 1334, 1335, 1336, 1337, 1338, 1339, 1340, 1341, 1342, 1343, 1344, 1346, 1347, 1348, 1349, 1350, 1351, 1352, 1353, 1354, 1355, 1356, 1357, 1358, 1359, or 1360 of the patent application WO2013/143700, a corresponding RNA sequence, a homolog thereof, or a variant thereof as described herein, preferably lacking the 5'TOP motif. As described above, the sequence extending from position 5 to the nucleotide immediately 5' to the ATG (which is located at the 3'end of the sequences) corresponds to the 5'UTR of said sequences.

Preferably, the 5'UTR element comprises or consists of a nucleic acid sequence, which is derived from a 5'UTR of a TOP gene encoding a ribosomal Large protein (RPL) or from a homolog or variant of a 5'UTR of a TOP gene encoding a ribosomal Large protein (RPL). For example, the 5'UTR element comprises or consists of a nucleic acid sequence which is derived from a 5'UTR of a nucleic acid sequence according to any of SEQ ID NOs: SEQ ID NOs: 67, 259, 1284-1318, 1344, 1346, 1348-1354, 1357, 1358, 1421 and 1422 of the patent application WO2013/143700, a corresponding RNA sequence, a homolog thereof, or a variant thereof as described herein, preferably lacking the 5'TOP motif.

In a particularly preferred embodiment, the 5'UTR element comprises or consists of a nucleic acid sequence which is derived from the 5'UTR of a ribosomal protein Large 32 gene, preferably from a vertebrate ribosomal protein Large 32 (L32) gene, more preferably from a mammalian ribosomal protein Large 32 (L32) gene, most preferably from a human ribosomal protein Large 32 (L32) gene, or from a variant of the 5'UTR of a ribosomal protein Large 32 gene, preferably from a vertebrate ribosomal protein Large 32 (L32) gene, more preferably from a mammalian ribosomal protein Large 32 (L32) gene, most preferably from a human ribosomal protein Large 32 (L32) gene, wherein preferably the 5'UTR element does not comprise the 5'TOP of said gene.

Accordingly, in a particularly preferred embodiment, the 5'UTR element comprises or consists of a nucleic acid sequence, which has an identity of at least about 40%, preferably of at least about 50%, preferably of at least about 60%, preferably of at least about 70%, more preferably of at least about 80%, more preferably of at least about 90%, even more preferably of at least about 95%, even more preferably of at least about 99% to the nucleic acid sequence according to SEQ ID No. 6 (5'-UTR of human ribosomal protein Large 32 lacking the 5' terminal oligopyrimidine tract: GCCGCTGCCTACGCAGGTGCCAGCCATCTCCTTCTCGGCATC; corresponding to SEQ ID No. 1368 of the patent application WO2013/143700) or preferably to a corresponding RNA sequence, or wherein the at least one 5'UTR element comprises or consists of a fragment of a nucleic acid sequence which has an identity of at least about 40%, preferably of at least about 50%, preferably of at least about 60%, preferably of at least about 70%, more preferably of at least about 80%, more preferably of at least about 90%, even more preferably of at least about 95%, even more preferably

of at least about 99% to the nucleic acid sequence according to SEQ ID No. 6 or more preferably to a corresponding RNA sequence, wherein, preferably, the fragment is as described above, i.e. being a continuous stretch of nucleotides representing at least 20% etc. of the full-length 5'UTR. Preferably, the fragment exhibits a length of at least about 20 nucleotides or more, preferably of at least about 30 nucleotides or more, more preferably of at least about 40 nucleotides or more. Preferably, the fragment is a functional fragment as described herein.

In some embodiments, the artificial nucleic acid molecule comprises a 5'UTR element which comprises or consists of a nucleic acid sequence which is derived from the 5'UTR of a vertebrate TOP gene, such as a mammalian, e.g. a human TOP gene, selected from RPSA, RPS2, RPS3, RPS3A, RPS4, RPS5, RPS6, RPS7, RPS8, RPS9, RPS10, RPS11, RPS12, RPS13, RPS14, RPS15, RPS15A, RPS16, RPS17, RPS18, RPS19, RPS20, RPS21, RPS23, RPS24, RPS25, RPS26, RPS27, RPS27A, RPS28, RPS29, RPS30, RPL3, RPL4, RPL5, RPL6, RPL7, RPL7A, RPL8, RPL9, RPL10, RPL10A, RPL11, RPL12, RPL13, RPL13A, RPL14, RPL15, RPL17, RPL18, RPL18A, RPL19, RPL21, RPL22, RPL23, RPL23A, RPL24, RPL26, RPL27, RPL27A, RPL28, RPL29, RPL30, RPL31, RPL32, RPL34, RPL35, RPL35A, RPL36, RPL36A, RPL37, RPL37A, RPL38, RPL39, RPL40, RPL41, RPLP0, RPLP1, RPLP2, RPLP3, RPLP0, RPLP1, RPLP2, EEF1A1, EEF1B2, EEF1D, EEF1G, EEF2, EIF3E, EIF3F, EIF3H, EIF2S3, EIF3C, EIF3K, EIF3EIP, EIF4A2, PABPC1, HNRNPA1, TPT1, TUBB1, UBA52, NPM1, ATP5G2, GNB2L1, NME2, UQCRB or from a homolog or variant thereof, wherein preferably the 5'UTR element does not comprise a TOP-motif or the 5'TOP of said genes, and wherein optionally the 5'UTR element starts at its 5'-end with a nucleotide located at position 1, 2, 3, 4, 5, 6, 7, 8, 9 or 10 downstream of the 5'terminal oligopyrimidine tract (TOP) and wherein further optionally the 5'UTR element which is derived from a 5'UTR of a TOP gene terminates at its 3'-end with a nucleotide located at position 1, 2, 3, 4, 5, 6, 7, 8, 9 or 10 upstream of the start codon (A(U/T)G) of the gene it is derived from.

Preferably, the artificial nucleic acid molecule according to the present invention, preferably the open reading frame, is at least partially G/C modified. Thus, the inventive artificial nucleic acid molecule may be thermodynamically stabilized by modifying the G (guanosine)/C (cytidine) content of the molecule. The G/C content of the open reading frame of an artificial nucleic acid molecule according to the present invention may be increased compared to the G/C content of the open reading frame of a corresponding wild type sequence, preferably by using the degeneration of the genetic code. Thus, the encoded amino acid sequence of the artificial nucleic acid molecule is preferably not modified by the G/C modification compared to the coded amino acid sequence of the particular wild type sequence. The codons of the coding sequence or the whole artificial nucleic acid molecule, e.g. an mRNA, may therefore be varied compared to the wild type coding sequence, such that they include an increased amount of G/C nucleotides while the translated amino acid sequence is maintained. Due to the fact that several codons code for one and the same amino acid (so-called degeneration of the genetic code), it is feasible to alter codons while not altering the encoded peptide/protein sequence (so-called alternative codon usage). Hence, it is possible to specifically introduce certain codons (in exchange for the respective wild-type codons encoding the same amino acid), which are more favourable with respect to stability of RNA and/or with respect to codon usage in a subject (so-called codon optimization).

Depending on the amino acid to be encoded by the coding region of the inventive artificial nucleic acid molecule as defined herein, there are various possibilities for modification of the nucleic acid sequence, e.g. the open reading frame, compared to its wild type coding region. In the case of amino acids, which are encoded by codons which contain exclusively G or C nucleotides, no modification of the codon is necessary. Thus, the codons for Pro (CCC or CCG), Arg (CGC or CGG), Ala (GCC or GCG) and Gly (GGC or GGG) require no modification, since no A or U/T is present.

In contrast, codons which contain A and/or U/T nucleotides may be modified by substitution of other codons which code for the same amino acids but contain no A and/or U/T. For example
the codons for Pro can be modified from CC(U/T) or CCA to CCC or CCG;
the codons for Arg can be modified from CG(U/T) or CGA or AGA or AGG to CGC or CGG;
the codons for Ala can be modified from GC(U/T) or GCA to GCC or GCG;
the codons for Gly can be modified from GG(U/T) or GGA to GGC or GGG.

In other cases, although A or (U/T) nucleotides cannot be eliminated from the codons, it is however possible to decrease the A and (U/T) content by using codons which contain a lower content of A and/or (U/T) nucleotides. Examples of these are:

The codons for Phe can be modified from (U/T)(U/T)(U/T) to (U/T) (U/T)C;
the codons for Leu can be modified from (U/T) (U/T)A, (U/T) (U/T)G, C(U/T) (U/T) or C(U/T)A to C(U/T)C or C(U/T)G;
the codons for Ser can be modified from (U/T)C(U/T) or (U/T)CA or AG(U/T) to (U/T)CC, (U/T)CG or AGC;
the codon for Tyr can be modified from (U/T)A(U/T) to (U/T)AC;
the codon for Cys can be modified from (U/T)G(U/T) to (U/T)GC;
the codon for His can be modified from CA(U/T) to CAC;
the codon for Gln can be modified from CAA to CAG;
the codons for lie can be modified from A(U/T)(U/T) or A(U/T)A to A(U/T)C;
the codons for Thr can be modified from AC(U/T) or ACA to ACC or ACG;

the codon for Asn can be modified from AA(U/T) to AAC;
the codon for Lys can be modified from AAA to AAG;
the codons for Val can be modified from G(U/T)(U/T) or G(U/T)A to G(U/T)C or G(U/T)G;
the codon for Asp can be modified from GA(U/T) to GAC;
the codon for Glu can be modified from GAA to GAG;
the stop codon (U/T)AA can be modified to (U/T)AG or (U/T)GA.

In the case of the codons for Met (A(U/T)G) and Trp ((U/T)GG), on the other hand, there is no possibility of sequence modification without altering the encoded amino acid sequence.

The substitutions listed above can be used either individually or in all possible combinations to increase the G/C content of the open reading frame of the inventive artificial nucleic acid molecule as defined herein, compared to its particular wild type open reading frame (i.e. the original sequence). Thus, for example, all codons for Thr occurring in the wild type sequence can be modified to ACC (or ACG).

Preferably, the G/C content of the open reading frame of the inventive artificial nucleic acid molecule as defined herein is increased by at least 7%, more preferably by at least 15%, particularly preferably by at least 20%, compared to the G/C content of the wild type coding region without altering the encoded amino acid sequence, i.e. using the degeneracy of the genetic code. According to a specific embodiment at least 5%, 10%, 20%, 30%, 40%, 50%, 60%, more preferably at least 70 %, even more preferably at least 80% and most preferably at least 90%, 95% or even 100% of the substitutable codons in the open reading frame of the inventive artificial nucleic acid molecule or a fragment, variant or derivative thereof are substituted, thereby increasing the G/C content of said open reading frame.

In this context, it is particularly preferable to increase the G/C content of the open reading frame of the inventive artificial nucleic acid molecule as defined herein, to the maximum (i.e. 100% of the substitutable codons), compared to the wild type open reading frame, without altering the encoded amino acid sequence.

Furthermore, the open reading frame is preferably at least partially codon-optimized. Codon-optimization is based on the finding that the translation efficiency may be determined by a different frequency in the occurrence of transfer RNAs (tRNAs) in cells. Thus, if so-called "rare codons" are present in the coding region of the inventive artificial nucleic acid molecule as defined herein, to an increased extent, the translation of the corresponding modified nucleic acid sequence is less efficient than in the case where codons coding for relatively "frequent" tRNAs are present.

Thus, the open reading frame of the inventive artificial nucleic acid molecule is preferably modified compared to the corresponding wild type coding region such that at least one codon of the wild type sequence which codes for a tRNA, which is relatively rare in the cell, is exchanged for a codon, which codes for a tRNA, which is comparably frequent in the cell and carries the same amino acid as the relatively rare tRNA. By this modification, the open reading frame of the inventive artificial nucleic acid molecule as defined herein, is modified such that codons, for which frequently occurring tRNAs are available may replace codons, which correspond to rare tRNAs. In other words, according to the invention, by such a modification all codons of the wild type open reading frame, which code for a rare tRNA, may be exchanged for a codon, which codes for a tRNA, which is more frequent in the cell and which carries the same amino acid as the rare tRNA. Which tRNAs occur relatively frequently in the cell and which, in contrast, occur relatively rarely is known to a person skilled in the art; cf. e.g. Akashi, Curr. Opin. Genet. Dev. 2001, 11(6): 660-666. Accordingly, preferably, the open reading frame is codon-optimized, preferably with respect to the system in which the artificial nucleic acid molecule according to the present invention is to be expressed, preferably with respect to the system in which the artificial nucleic acid molecule according to the present invention is to be translated. Preferably, the codon usage of the open reading frame is codon-optimized according to mammalian codon usage, more preferably according to human codon usage. Preferably, the open reading frame is codon-optimized and G/C-content modified.

For further improving degradation resistance, e.g. resistance to *in vivo* degradation by an exo- or endonuclease, and/or for further improving stability of protein expression from the artificial nucleic acid molecule according to the present invention, the artificial nucleic acid molecule may further comprise modifications, such as backbone modifications, sugar modifications and/or base modifications, e.g., lipid-modifications or the like. Preferably, the transcription and/or the translation of the artificial nucleic acid molecule according to the present invention is not significantly impaired by said modifications.

Generally, the artificial nucleic acid molecule of the present invention may comprise any native (= naturally occurring) nucleotide, e.g. guanosine, uracil, adenosine, and/or cytosine or an analogue thereof. In this respect, nucleotide analogues are defined as natively and non-natively occurring variants of the naturally occurring nucleotides adenosine, cytosine, thymidine, guanosine and uridine. Accordingly, analogues are e.g. chemically derivatized nucleotides with non-natively occurring functional groups, which are preferably added to or deleted from the naturally occurring nucleotide or which substitute the naturally occurring functional groups of a nucleotide. Accordingly, each component of the naturally occurring nucleotide may be modified, namely the base component, the sugar (ribose) component and/or the phosphate component forming the backbone (see above) of the RNA sequence. Analogues of guanosine, uridine, adenosine, thymidine and cytosine include, without implying any limitation, any natively occurring or non-natively occurring guanosine, uridine, adenosine, thymidine or cytosine that has been altered e.g. chemically, for example by acetylation, methylation, hydroxylation, etc., including 1-methyl-adenosine, 1-methyl-guanosine, 1-methyl-inosine, 2,2-dimethyl-guanosine, 2,6-diaminopurine, 2'-Amino-2'-deoxyadenosine, 2'-Amino-2'-deoxycytidine, 2'-Amino-2'-deoxyguanosine, 2'-Amino-2'-deoxyuridine, 2-Amino-6-chloropurineriboside, 2-Aminopurine-riboside, 2'-Araadenosine, 2'-Aracytidine, 2'-Arauridine, 2'-Azido-2'-deoxyadenosine, 2'-Azido-2'-deoxycytidine, 2'-Azido-2'-deoxyguanosine, 2'-Azido-2'-deoxyuridine, 2-Chloroadenosine, 2'-Fluoro-2'-deoxyadenosine, 2'-Fluoro-2'-deoxycytidine, 2'-Fluoro-2'-deoxyguanosine, 2'-Fluoro-2'-deoxyuridine, 2'-Fluorothymidine, 2-methyl-adenosine, 2-methyl-guanosine, 2-methyl-thio-N6-isopenenyl-adenosine, 2'-O-Methyl-2-aminoadenosine, 2'-O-Methyl-2'-deoxyadenosine, 2'-O-Methyl-2'-deoxycytidine, 2'-O-Methyl-2'-deoxyguanosine, 2'-O-Methyl-2'-deoxyuridine, 2'-O-Methyl-5-methyluridine, 2'-O-Methylinosine, 2'-O-Methylpseudouridine, 2-Thiocytidine, 2-thio-cytosine, 3-methyl-cytosine, 4-acetyl-cytosine, 4-Thiouridine, 5-(carboxyhydroxymethyl)-uracil, 5,6-Dihydrouridine, 5-Aminoallylcytidine, 5-Aminoallyl-deoxy-uridine, 5-Bromouridine, 5-carboxymehtylaminomethyl-2-thio-uracil, 5-carboxymethylamonomethyl-uracil, 5-Chloro-Ara-cytosine, 5-Fluoro-uridine, 5-Iodouridine, 5-methoxycarbonylmethyl-uridine, 5-methoxy-uridine, 5-methyl-2-thiouridine, 6-Azacytidine, 6-Azauridine, 6-Chloro-7-deaza-guanosine, 6-Chloropurineriboside, 6-Mercapto-guanosine, 6-Methyl-mercaptopurine-riboside, 7-Deaza-2'-deoxy-guanosine, 7-Deazaadenosine, 7-methyl-guanosine, 8-Azaadenosine, 8-Bromo-adenosine, 8-Bromo-guanosine, 8-Mercapto-guanosine, 8-Oxoguanosine, Benzimidazole-riboside, Beta-D-mannosyl-queosine, Dihydro-uracil, Inosine, N1-Methyladenosine, N6-([6-Aminohexyl]carbamoylmethyl)-adenosine, N6-isopentenyl-adenosine, N6-methyl-adenosine, N7-Methyl-xanthosine, N-uracil-5-oxyacetic acid methyl ester, Puromycin, Queosine, Uracil-5-oxyacetic acid, Uracil-5-oxyacetic acid methyl ester, Wybutoxosine, Xanthosine, and Xylo-adenosine. The preparation of such analogues is known to a person skilled in the art, for example from US Patents 4,373,071, US 4,401,796, US 4,415,732, US 4,458,066, US 4,500,707, US 4,668,777, US 4,973,679, US 5,047,524, US 5,132,418, US 5,153,319, US 5,262,530 and 5,700,642. In the case of an analogue as described above, particular preference may be given according to certain embodiments of the invention to those analogues that increase the protein expression of the encoded peptide or protein or that increase the immunogenicity of the artificial nucleic acid molecule of the invention and/or do not interfere with a further modification of the artificial nucleic acid molecule that has been introduced.

According to a particular embodiment, the artificial nucleic acid molecule of the present invention can contain a lipid modification.

In a preferred embodiment, the artificial nucleic acid molecule comprises, preferably from 5' to 3' direction, the following elements:

a 5'-UTR;
at least one open reading frame (ORF), wherein the ORF preferably comprises at least one modification with respect to the wildtype sequence;
a 3'-UTR derived from the 3'-UTR of a ribosomal protein, preferably from a nucleic acid sequence according to any of SEQ ID NOs: 10 to 115, more preferably of the 3'-UTR of RPS9, more preferably of the 3'-UTR of human RPS9;
a poly(A) sequence, preferably comprising 64 adenylates;
a poly(C) sequence, preferably comprising 30 cytidylates;
a histone stem-loop sequence.

In another preferred embodiment, the artificial nucleic acid molecule comprises or consists of a nucleotide sequence as shown according to SEQ ID NO: 7 (see Fig. 3) or the complementary DNA sequence.

In a particularly preferred embodiment, the artificial nucleic acid molecule according to the invention may further comprise one or more of the modifications described in the following:

Chemical modifications:

The term "modification" as used herein with regard to the artificial nucleic acid molecule may refer to chemical modifications comprising backbone modifications as well as sugar modifications or base modifications.

In this context, the artificial nucleic acid molecule, preferably an RNA molecule, as defined herein may contain nucleotide analogues/modifications, e.g. backbone modifications, sugar modifications or base modifications. A backbone modification in connection with the present invention is a modification, in which phosphates of the backbone of the nucleotides contained in a nucleic acid molecule as defined herein are chemically modified. A sugar modification in connection with the present invention is a chemical modification of the sugar of the nucleotides of the nucleic acid molecule as defined herein. Furthermore, a base modification in connection with the present invention is a chemical modification of the base moiety of the nucleotides of the nucleic acid molecule of the nucleic acid molecule. In this context, nucleotide analogues or modifications are preferably selected from nucleotide analogues which are applicable for transcription and/or translation.

Sugar Modifications:

The modified nucleosides and nucleotides, which may be incorporated into the artificial nucleic acid molecule, preferably an RNA, as described herein, can be modified in the sugar moiety. For example, the 2' hydroxyl group (OH) of an RNA molecule can be modified or replaced with a number of different "oxy" or "deoxy" substituents. Examples of "oxy" -2' hydroxyl group modifications include, but are not limited to, alkoxy or aryloxy (-OR, e.g., R = H, alkyl, cycloalkyl, aryl, aralkyl, heteroaryl or sugar); polyethyleneglycols (PEG), -0(CH2CH2o)nCH2CH2OR; "locked" nucleic acids (LNA) in which the 2' hydroxyl is connected, e.g., by a methylene bridge, to the 4' carbon of the same ribose sugar; and amino groups (-O-amino, wherein the amino group, e.g., NRR, can be alkylamino, dialkylamino, heterocyclyl, arylamino, diarylamino, heteroarylamino, or diheteroaryl amino, ethylene diamine, polyamino) or aminoalkoxy.

"Deoxy" modifications include hydrogen, amino (e.g. NH2; alkylamino, dialkylamino, heterocyclyl, arylamino, diaryl amino, heteroaryl amino, diheteroaryl amino, or amino acid); or the amino group can be attached to the sugar through a linker, wherein the linker comprises one or more of the atoms C, N, and O.

The sugar group can also contain one or more carbons that possess the opposite stereochemical configuration than that of the corresponding carbon in ribose. Thus, a modified nucleic acid molecule can include nucleotides containing, for instance, arabinose as the sugar.

Backbone Modifications:

The phosphate backbone may further be modified in the modified nucleosides and nucleotides, which may be incorporated into the artificial nucleic acid molecule, preferably an RNA, as described herein. The phosphate groups of the backbone can be modified by replacing one or more of the oxygen atoms with a different substituent. Further, the modified nucleosides and nucleotides can include the full replacement of an unmodified phosphate moiety with a modified phosphate as described herein. Examples of modified phosphate groups include, but are not limited to, phosphorothioate, phosphoroselenates, borano phosphates, borano phosphate esters, hydrogen phosphonates, phosphoroamidates, alkyl or aryl phosphonates and phosphotriesters. Phosphorodithioates have both non-linking oxygens replaced by sulfur. The phosphate linker can also be modified by the replacement of a linking oxygen with nitrogen (bridged phosphoroamidates), sulfur (bridged phosphorothioates) and carbon (bridged methylene-phosphonates).

Base Modifications:

The modified nucleosides and nucleotides, which may be incorporated into the artificial nucleic acid molecule, preferably an RNA molecule, as described herein, can further be modified in the nucleobase moiety. Examples of nucleobases found in RNA include, but are not limited to, adenine, guanine, cytosine and uracil. For example, the nucleosides and nucleotides described herein can be chemically modified on the major groove face. In some embodiments, the major groove chemical modifications can include an amino group, a thiol group, an alkyl group, or a halo group.

In particularly preferred embodiments of the present invention, the nucleotide analogues/modifications are selected from base modifications, which are preferably selected from 2-amino-6-chloropurineriboside-5'-triphosphate, 2-Aminopurine-riboside-5'-triphosphate; 2-aminoadenosine-5'-triphosphate, 2'-Amino-2'-deoxycytidine-triphosphate, 2-thiocytidine-5'-triphosphate, 2-thiouridine-5'-triphosphate, 2'-Fluorothymidine-5'-triphosphate, 2'-O-Methyl inosine-5'-triphosphate 4-thiouridine-5'-triphosphate, 5-aminoallylcytidine-5'-triphosphate, 5-aminoallyluridine-5'-triphosphate, 5-bromocytidine-5'-triphosphate, 5-bromouridine-5'-triphosphate, 5-Bromo-2'-deoxycytidine-5'-triphosphate, 5-Bromo-2'-deoxyuridine-5'-triphosphate, 5-iodocytidine-5'-triphosphate, 5-lodo-2'-deoxycytidine-5'-triphosphate, 5-iodouridine-5'-triphosphate, 5-lodo-2'-deoxyuridine-5'-triphosphate, 5-methylcytidine-5'-triphosphate, 5-methyluridine-5'-triphosphate, 5-Propynyl-2'-deoxycytidine-5'-triphosphate, 5-Propynyl-2'-deoxyuridine-5'-triphosphate, 6-azacytidine-S'-triphosphate, 6-azauridine-5'-triphosphate, 6-chloropurineriboside-5'-triphosphate, 7-deazaadenosine-5'-triphosphate, 7-deazaguanosine-5'-triphosphate, 8-azaadenosine-5'-triphosphate, 8-azidoadenosine-5'-triphosphate, benzimidazole-riboside-5'-triphosphate, N1-methyladenosine-5'-triphosphate, N1-methylguanosine-5'-triphosphate, N6-methyladenosine-5'-triphosphate, O6-methylguanosine-5'-triphosphate, pseudouridine-5'-triphosphate, or puromycin-5'-triphosphate, xanthosine-5'-triphosphate. Particular preference is given to nucleotides for base modifications selected from the group of base-modified nucleotides consisting of 5-methylcytidine-5'-triphosphate, 7-deazaguanosine-5'-triphosphate, 5-bromocytidine-5'-triphosphate, and pseudouridine-5'-triphosphate.

In some embodiments, modified nucleosides include pyridin-4-one ribonucleoside, 5-azauridine, 2-thio-5-azauridine, 2-thiouridine, 4-thio-pseudouridine, 2-thio-pseudouridine, 5-hydroxyuridine, 3-methyluridine, 5-carboxymethyl-uridine, 1-carboxymethyl-pseudouridine, 5-propynyl-uridine, 1-propynyl-pseudouridine, 5-taurinomethyluridine, 1-taurinomethyl-pseudouridine, 5-taurinomethyl-2-thio-uridine, 1-taurinomethyl-4-thiouridine, 5-methyl-uridine, 1-methyl-pseudouridine, 4-thio- 1-methyl-pseudouridine, 2-thio-1-methyl-pseudouridine, 1 -methyl- 1-deaza-pseudouridine, 2-thio- 1 -methyl- 1 -deaza-pseudouridine, dihydrouridine, dihydropseudouridine, 2-thio-dihydrouridine, 2-thio-dihydropseudouridine, 2-methoxyuridine, 2-methoxy-4-thio-uridine, 4-methoxy-pseudouridine, and 4-methoxy-2-thio-pseudouridine.

In some embodiments, modified nucleosides include 5-aza-cytidine, pseudoisocytidine, 3-methyl-cytidine, N4-acetylcytidine, 5-formylcytidine, N4-methylcytidine, 5-hydroxymethylcytidine, 1-methyl-pseudoisocytidine, pyrrolo-cytidine, pyrrolo-pseudoisocytidine, 2-thio-cytidine, 2-thio-5-methyl-cytidine, 4-thio-pseudoisocytidine, 4-thio- 1-methyl-pseudoisocytidine, 4-thio- 1 -methyl- 1 -deaza-pseudoisocytidine, 1 -methyl-1 -deaza-pseudoisocytidine, zebularine, 5-aza-zebularine, 5-methyl-zebularine, 5-aza-2-thio-zebularine, 2-thio-zebularine, 2-methoxy-cytidine, 2-methoxy-5-methyl-cytidine, 4-methoxy-pseudoisocytidine, and 4-methoxy-l-methyl-pseudoisocytidine.

In other embodiments, modified nucleosides include 2-aminopurine, 2, 6-diaminopurine, 7-deaza-adenine, 7-deaza-8-aza-adenine, 7-deaza-2-aminopurine, 7-deaza-8-aza-2-aminopurine, 7-deaza-2,6-diaminopurine, 7-deaza-8-aza-2,6-diaminopurine, 1-methyladenosine, N6-methyladenosine, N6-isopentenyladenosine, N6-(cis-hydroxyisopentenyl)adenosine, 2-methylthio-N6-(cis-hydroxyisopentenyl) adenosine, N6-glycinylcarbamoyladenosine, N6-threonylcarbamoyladenosine, 2-methylthio-N6-threonyl carbamoyladenosine, N6,N6-dimethyladenosine, 7-methyladenine, 2-methylthio-adenine, and 2-methoxy-adenine.

In other embodiments, modified nucleosides include inosine, 1-methyl-inosine, wyosine, wybutosine, 7-deaza-guanosine, 7-deaza-8-aza-guanosine, 6-thio-guanosine, 6-thio-7-deaza-guanosine, 6-thio-7-deaza-8-aza-guanosine, 7-methyl-guanosine, 6-thio-7-methyl-guanosine, 7-methylinosine, 6-methoxy-guanosine, 1-methylguanosine, N2-methylguanosine, N2,N2-dimethylguanosine, 8-oxo-guanosine, 7-methyl-8-oxo-guanosine, I-methyl-6-thio-guanosine, N2-methyl-6-thio-guanosine, and N2,N2-dimethyl-6-thio-guanosine.

In some embodiments, the nucleotide can be modified on the major groove face and can include replacing hydrogen on C-5 of uracil with a methyl group or a halo group.

In specific embodiments, a modified nucleoside is 5'-O-(1-Thiophosphate)-Adenosine, 5'-O-(1-Thiophosphate)-Cytidine, 5'-O-(1-Thiophosphate)-Guanosine, 5'-O-(1-Thiophosphate)-Uridine or 5'-O-(1-Thiophosphate)-Pseudouridine.

In further specific embodiments the artificial nucleic acid molecule, preferably an RNA molecule, may comprise nucleoside modifications selected from 6-aza-cytidine, 2-thio-cytidine, alpha-thio-cytidine, Pseudo-iso-cytidine, 5-aminoallyl-uridine, 5-iodo-uridine, N1-methyl-pseudouridine, 5,6-dihydrouridine, alpha-thio-uridine, 4-thio-uridine, 6-aza-uridine, 5-hydroxy-uridine, deoxy-thymidine, 5-methyl-uridine, Pyrrolo-cytidine, inosine, alpha-thio-guanosine, 6-methyl-guanosine, 5-methyl-cytdine, 8-oxo-guanosine, 7-deaza-guanosine, N1-methyl-adenosine, 2-amino-6-Chloro-purine, N6-methyl-2-amino-purine, Pseudo-iso-cytidine, 6-Chloro-purine, N6-methyl-adenosine, alpha-thio-adenosine, 8-azido-adenosine, 7-deaza-adenosine.

Lipid modification:

According to a further embodiment, the artificial nucleic acid molecule, preferably an RNA, as defined herein can contain a lipid modification. Such a lipid-modified RNA typically comprises an RNA as defined herein. Such a lipid-modified RNA molecule as defined herein typically further comprises at least one linker covalently linked with that RNA molecule, and at least one lipid covalently linked with the respective linker. Alternatively, the lipid-modified RNA molecule comprises at least one RNAmolecule as defined herein and at least one (bifunctional) lipid covalently linked (without a linker) with that RNA molecule. According to a third alternative, the lipid-modified RNA molecule comprises an artificial nucleic acid molecule, preferably an RNA molecule, as defined herein, at least one linker covalently linked with that RNA molecule, and at least one lipid covalently linked with the respective linker, and also at least one (bifunctional) lipid covalently linked (without a linker) with that RNA molecule. In this context, it is particularly preferred that the lipid modification is present at the terminal ends of a linear RNA sequence.

Modification of the 5'-end of the modified RNA:

According to another preferred embodiment of the invention, the artificial nucleic acid molecule, preferably an RNA molecule, as defined herein, can be modified by the addition of a so-called "5' CAP" structure.

A 5'-cap is an entity, typically a modified nucleotide entity, which generally "caps" the 5'-end of a mature mRNA. A 5'-cap may typically be formed by a modified nucleotide, particularly by a derivative of a guanine nucleotide. Preferably, the 5'-cap is linked to the 5'-terminus via a 5'-5'-triphosphate linkage. A 5'-cap may be methylated, e.g. m7GpppN, wherein N is the terminal 5' nucleotide of the nucleic acid carrying the 5'-cap, typically the 5'-end of an RNA. m7GpppN is the 5'-CAP structure which naturally occurs in mRNA transcribed by polymerase II and is therefore not considered as modification comprised in the modified RNA according to the invention. This means the artificial nucleic acid molecule, preferably an RNA molecule, according to the present invention may comprise a m7GpppN as 5'-CAP, but additionally the artificial nucleic acid molecule, preferably an RNA molecule, comprises at least one further modification as defined herein.

Further examples of 5'cap structures include glyceryl, inverted deoxy abasic residue (moiety), 4',5' methylene

nucleotide, 1-(beta-D-erythrofuranosyl) nucleotide, 4'-thio nucleotide, carbocyclic nucleotide, 1,5-anhydrohexitol nucleotide, L-nucleotides, alpha-nucleotide, modified base nucleotide, threo-pentofuranosyl nucleotide, acyclic 3',4'-seco nucleotide, acyclic 3,4-dihydroxybutyl nucleotide, acyclic 3,5 dihydroxypentyl nucleotide, 3'-3'-inverted nucleotide moiety, 3'-3'-inverted abasic moiety, 3'-2'-inverted nucleotide moiety, 3'-2'-inverted abasic moiety, 1,4-butanediol phosphate, 3'-phosphoramidate, hexylphosphate, aminohexyl phosphate, 3'-phosphate, 3'phosphorothioate, phosphorodithioate, or bridging or non-bridging methylphosphonate moiety. These modified 5'-CAP structures are regarded as at least one modification comprised in the artificial nucleic acid molecule, preferably in an RNA molecule, according to the present invention.

Particularly preferred modified 5'-CAP structures are CAP1 (methylation of the ribose of the adjacent nucleotide of m7G), CAP2 (methylation of the ribose of the $2^{nd}$ nucleotide downstream of the m7G), CAP3 (methylation of the ribose of the $3^{rd}$ nucleotide downstream of the m7G), CAP4 (methylation of the ribose of the $4^{th}$ nucleotide downstream of the m7G), ARCA (anti-reverse CAP analogue, modified ARCA (e.g. phosphothioate modified ARCA), inosine, N1-methyl-guanosine, 2'-fluoro-guanosine, 7-deaza-guanosine, 8-oxo-guanosine, 2-amino-guanosine, LNA-guanosine, and 2-azido-guanosine.

In a preferred embodiment, the at least one open reading frame encodes a therapeutic protein or peptide. In another embodiment, an antigen is encoded by the at least one open reading frame, such as a pathogenic antigen, a tumour antigen, an allergenic antigen or an autoimmune antigen. Therein, the administration of the artificial nucleic acid molecule encoding the antigen is used in a genetic vaccination approach against a disease involving said antigen.

In an alternative embodiment, an antibody is encoded by the at least one open reading frame of the artificial nucleic acid molecule according to the invention.

Antigens:

Pathogenic antigens:

The artificial nucleic acid molecule according to the present invention may encode a protein or a peptide, which comprises a pathogenic antigen or a fragment, variant or derivative thereof. Such pathogenic antigens are derived from pathogenic organisms, in particular bacterial, viral or protozoological (multicellular) pathogenic organisms, which evoke an immunological reaction in a subject, in particular a mammalian subject, more particularly a human. More specifically, pathogenic antigens are preferably surface antigens, e.g. proteins (or fragments of proteins, e.g. the exterior portion of a surface antigen) located at the surface of the virus or the bacterial or protozoological organism.

Pathogenic antigens are peptide or protein antigens preferably derived from a pathogen associated with infectious disease which are preferably selected from antigens derived from the pathogens Acinetobacter baumannii, Anaplasma genus, Anaplasma phagocytophilum, Ancylostoma braziliense, Ancylostoma duodenale, Arcanobacterium haemolyticum, Ascaris lumbricoides, Aspergillus genus, Astroviridae, Babesia genus, Bacillus anthracis, Bacillus cereus, Bartonella henselae, BK virus, Blastocystis hominis, Blastomyces dermatitidis, Bordetella pertussis, Borrelia burgdorferi, Borrelia genus, Borrelia spp, Brucella genus, Brugia malayi, Bunyaviridae family, Burkholderia cepacia and other Burkholderia species, Burkholderia mallei, Burkholderia pseudomallei, Caliciviridae family, Campylobacter genus, Candida albicans, Candida spp, Chlamydia trachomatis, Chlamydophila pneumoniae, Chlamydophila psittaci, CJD prion, Clonorchis sinensis, Clostridium botulinum, Clostridium difficile, Clostridium perfringens, Clostridium perfringens, Clostridium spp, Clostridium tetani, Coccidioides spp, coronaviruses, Corynebacterium diphtheriae, Coxiella burnetii, Crimean-Congo hemorrhagic fever virus, Cryptococcus neoformans, Cryptosporidium genus, Cytomegalovirus (CMV), Dengue viruses (DEN-1, DEN-2, DEN-3 and DEN-4), Dientamoeba fragilis, Ebolavirus (EBOV), Echinococcus genus, Ehrlichia chaffeensis, Ehrlichia ewingii, Ehrlichia genus, Entamoeba histolytica, Enterococcus genus, Enterovirus genus, Enteroviruses, mainly Coxsackie A virus and Enterovirus 71 (EV71), Epidermophyton spp, Epstein-Barr Virus (EBV), Escherichia coli O157:H7, O111 and O104:H4, Fasciola hepatica and Fasciola gigantica, FFI prion, Filarioidea superfamily, Flaviviruses, Francisella tularensis, Fusobacterium genus, Geotrichum candidum, Giardia intestinalis, Gnathostoma spp, GSS prion, Guanarito virus, Haemophilus ducreyi, Haemophilus influenzae, Helicobacter pylori, Henipavirus (Hendra virus Nipah virus), Hepatitis A Virus, Hepatitis B Virus (HBV), Hepatitis C Virus (HCV), Hepatitis D Virus, Hepatitis E Virus, Herpes simplex virus 1 and 2 (HSV-1 and HSV-2), Histoplasma capsulatum, HIV (Human immunodeficiency virus), Hortaea werneckii, Human bocavirus (HBoV), Human herpesvirus 6 (HHV-6) and Human herpesvirus 7 (HHV-7), Human metapneumovirus (hMPV), Human papillomavirus (HPV), Human parainfluenza viruses (HPIV), Japanese encephalitis virus, JC virus, Junin virus, Kingella kingae, Klebsiella granulomatis, Kuru prion, Lassa virus, Legionella pneumophila, Leishmania genus, Leptospira genus, Listeria monocytogenes, Lymphocytic choriomeningitis virus (LCMV), Machupo virus, Malassezia spp, Marburg virus, Measles virus, Metagonimus yokagawai, Microsporidia phylum, Molluscum contagiosum virus (MCV), Mumps virus, Mycobacterium leprae and Mycobacterium lepromatosis, Mycobacterium tuberculosis, Mycobacterium ulcerans, Mycoplasma pneumoniae, Naegleria fowleri, Necator americanus, Neisseria gonorrhoeae, Neisseria meningitidis, Nocardia asteroides, Nocardia spp, Onchocerca volvulus, Orientia tsutsugamushi, Orthomyxoviridae family (Influenza), Paracoccidioides brasiliensis, Paragonimus spp, Paragonimus westermani, Parvovirus B19, Pasteurella genus, Plasmodium genus, Pneumocystis jirovecii, Poliovirus, Rabies virus, Respiratory syncytial virus (RSV), Rhinovirus, rhinoviruses, Rickettsia akari, Rickettsia genus, Rickettsia prowazekii, Rickettsia rickettsii, Rickettsia typhi, Rift Valley fever virus, Rotavirus, Rubella virus, Sabia virus, Salmonella genus, Sarcoptes scabiei, SARS coronavirus, Schistosoma genus, Shigella genus, Sin Nombre virus, Hantavirus, Sporothrix schenckii, Staphylococcus genus, Staphylococcus genus, Streptococcus agalactiae, Streptococcus pneumoniae, Streptococcus pyogenes, Strongyloides stercoralis, Taenia genus, Taenia solium, Tick-borne encephalitis virus (TBEV), Toxocara canis or Toxocara cati, Toxoplasma gondii, Treponema pallidum, Trichinella spiralis, Trichomonas vaginalis, Trichophyton spp, Trichuris trichiura, Trypanosoma brucei, Trypanosoma cruzi, Ureaplasma urealyticum, Varicella zoster virus (VZV), Varicella zoster virus (VZV), Variola major or Variola minor, vCJD prion, Venezuelan equine encephalitis virus, Vibrio cholerae, West Nile virus, Western equine encephalitis virus, Wuchereria bancrofti, Yellow fever virus, Yersinia enterocolitica, Yersinia pestis, and Yersinia pseudotuberculosis.

In this context particularly preferred are antigens from the pathogens selected from Influenza virus, respiratory syncytial virus (RSV), Herpes simplex virus (HSV), human Papilloma virus (HPV), Human immunodeficiency virus (HIV), Plasmodium, Staphylococcus aureus, Dengue virus, Chlamydia trachomatis, Cytomegalovirus (CMV), Hepatitis B virus (HBV), Mycobacterium tuberculosis, Rabies virus, and Yellow Fever Virus.

Tumour antigens:

In a further embodiment the artificial nucleic acid molecule according to the present invention may encode a protein or a peptide, which comprises a peptide or protein comprising a tumour antigen, a fragment, variant or derivative of said tumour antigen, preferably, wherein the tumour antigen is a melanocyte-specific antigen, a cancer-testis antigen or a tumour-specific antigen, preferably a CT-X antigen, a non-X CT-antigen, a binding partner for a CT-X antigen or a binding partner for a non-X CT-antigen or a tumour-specific antigen, more preferably a CT-X antigen, a binding partner for a non-X CT-antigen or a tumour-specific antigen or a fragment, variant or derivative of said tumour antigen; and wherein each of the nucleic acid sequences encodes a different peptide or protein; and wherein at least one of the nucleic acid sequences encodes for 5T4, 707-AP, 9D7, AFP, AlbZIP HPG1, alpha-5-beta-1-integrin, alpha-5-beta-6-integrin, alpha-actinin-4/m, alpha-methylacyl-coenzyme A racemase, ART-4, ARTC1/m, B7H4, BAGE-1, BCL-2, bcr/abl, beta-catenin/m, BING-4, BRCA1/m, BRCA2/m, CA 15-3/CA 27-29, CA 19-9, CA72-4, CA125, calreticulin, CAMEL, CASP-8/m, cathepsin B, cathepsin L, CD19, CD20, CD22, CD25, CDE30, CD33, CD4, CD52, CD55, CD56, CD80, CDC27/m, CDK4/m, CDKN2A/m, CEA, CLCA2, CML28, CML66, COA-1/m, coactosin-like protein, collage XXIII, COX-2, CT-9/BRD6, Cten, cyclin B1, cyclin D1, cyp-B, CYPB1, DAM-10, DAM-6, DEK-CAN, EFTUD2/m, EGFR, ELF2/m, EMMPRIN, EpCam, EphA2, EphA3, ErbB3, ETV6-AML1, EZH2, FGF-5, FN, Frau-1, G250, GAGE-1, GAGE-2, GAGE-3, GAGE-4, GAGE-5, GAGE-6, GAGE7b, GAGE-8, GDEP, GnT-V, gp100, GPC3, GPNMB/m, HAGE, HAST-2, hepsin, Her2/neu, HERV-K-MEL, HLA-A*0201-R171, HLA-A11/m, HLA-A2/m, HNE, homeobox NKX3.1, HOM-TES-14/SCP-1, HOM-TES-85, HPV-E6, HPV-E7, HSP70-2M, HST-2, hTERT, iCE, IGF-1R, IL-13Ra2, IL-2R, IL-5, immature laminin receptor, kallikrein-2, kallikrein-4, Ki67, KIAA0205, KIAA0205/m, KK-LC-1, K-Ras/m, LAGE-A1, LDLR-FUT, MAGE-A1, MAGE-A2, MAGE-A3, MAGE-A4, MAGE-A6, MAGE-A9, MAGE-A10, MAGE-A12, MAGE-B1, MAGE-B2, MAGE-B3, MAGE-B4, MAGE-B5, MAGE-B6, MAGE-B10, MAGE-B16, MAGE-B17, MAGE-C1, MAGE-C2, MAGE-C3, MAGE-D1, MAGE-D2, MAGE-D4, MAGE-E1, MAGE-E2, MAGE-F1, MAGE-H1, MAGEL2, mammaglobin A, MART-1/melan-A, MART-2, MART-2/m, matrix protein 22, MC1R, M-CSF, ME1/m, mesothelin, MG50/PXDN, MMP11, MN/CA IX-antigen, MRP-3, MUC-1, MUC-2, MUM-1/m, MUM-2/m, MUM-3/m, myosin class I/m, NA88-A, N-acetylglucosaminyltransferase-V, Neo-PAP, Neo-PAP/m, NFYC/m, NGEP, NMP22, NPM/ALK, N-Ras/m, NSE, NY-ESO-1, NY-ESO-B, OA1, OFA-iLRP, OGT, OGT/m, OS-9, OS-9/m, osteocalcin, osteopontin, p15, p190 minor bcr-abl, p53, p53/m, PAGE-4, PAI-1, PAI-2, PAP, PART-1, PATE, PDEF, Pim-1-Kinase, Pin-1, Pml/PARalpha, POTE, PRAME, PRDX5/m, prostein, proteinase-3, PSA, PSCA, PSGR, PSM, PSMA, PTPRK/m, RAGE-1, RBAF600/m, RHAMM/CD168, RU1, RU2, S-100, SAGE, SART-1, SART-2, SART-3, SCC, SIRT2/m, Sp17, SSX-1, SSX-2/HOM-MEL-40, SSX-4, STAMP-1, STEAP-1, survivin, survivin-2B, SYT-SSX-1, SYT-SSX-2, TA-90, TAG-72, TARP, TEL-AML1, TGFbeta, TGFbetaRII, TGM-4, TPI/m, TRAG-3, TRG, TRP-1, TRP-2/6b, TRP/INT2, TRP-p8, tyrosinase, UPA, VEGFR1, VEGFR-2/FLK-1, WT1 and a immunoglobulin idiotype of a lymphoid blood cell or a T cell receptor idiotype of a lymphoid blood cell, or a fragment, variant or derivative of said tumour antigen; preferably survivin or a homologue thereof, an antigen from the MAGE-family or a binding partner thereof or a fragment, variant or derivative of said tumour antigen. Particularly preferred in this context are the tumour antigens NY-ESO-1, 5T4, MAGE-C1, MAGE-C2, Survivin, Muc-1, PSA, PSMA, PSCA, STEAP and PAP.

In a preferred embodiment, the artificial nucleic acid molecule encodes a protein or a peptide, which comprises a therapeutic protein or a fragment, variant or derivative thereof.

Therapeutic proteins as defined herein are peptides or proteins, which are beneficial for the treatment of any inherited or acquired disease or which improves the condition of an individual. Particularly, therapeutic proteins play an important role in the creation of therapeutic agents that could modify and repair genetic errors, destroy cancer cells or

pathogen infected cells, treat immune system disorders, treat metabolic or endocrine disorders, among other functions. For instance, Erythropoietin (EPO), a protein hormone can be utilized in treating patients with erythrocyte deficiency, which is a common cause of kidney complications. Furthermore adjuvant proteins, therapeutic antibodies are encompassed by therapeutic proteins and also hormone replacement therapy which is e.g. used in the therapy of women in menopause. In more recent approaches, somatic cells of a patient are used to reprogram them into pluripotent stem cells, which replace the disputed stem cell therapy. Also these proteins used for reprogramming of somatic cells or used for differentiating of stem cells are defined herein as therapeutic proteins. Furthermore, therapeutic proteins may be used for other purposes, e.g. wound healing, tissue regeneration, angiogenesis, etc. Furthermore, antigen-specific B cell receptors and fragments and variants thereof are defined herein as therapeutic proteins.

Therefore therapeutic proteins can be used for various purposes including treatment of various diseases like e.g. infectious diseases, neoplasms (e.g. cancer or tumour diseases), diseases of the blood and blood-forming organs, endocrine, nutritional and metabolic diseases, diseases of the nervous system, diseases of the circulatory system, diseases of the respiratory system, diseases of the digestive system, diseases of the skin and subcutaneous tissue, diseases of the musculoskeletal system and connective tissue, and diseases of the genitourinary system, independently if they are inherited or acquired.

In this context, particularly preferred therapeutic proteins, which can be used inter alia in the treatment of metabolic or endocrine disorders, are selected from (in brackets the particular disease for which the therapeutic protein is used in the treatment): Acid sphingomyelinase (Niemann-Pick disease), Adipotide (obesity), Agalsidase-beta (human galactosidase A) (Fabry disease; prevents accumulation of lipids that could lead to renal and cardiovascular complications), Alglucosidase (Pompe disease (glycogen storage disease type II)), alpha-galactosidase A (alpha-GAL A, Agalsidase alpha) (Fabry disease), alpha-glucosidase (Glycogen storage disease (GSD), Morbus Pompe), alpha-L-iduronidase (mucopolysaccharidoses (MPS), Hurler syndrome, Scheie syndrome), alpha-N-acetylglucosaminidase (Sanfilippo syndrome), Amphiregulin (cancer, metabolic disorder), Angiopoietin ((Ang1, Ang2, Ang3, Ang4, ANGPTL2, ANGPTL3, ANGPTL4, ANGPTL5, ANGPTL6, ANGPTL7) (angiogenesis, stabilize vessels), Betacellulin (metabolic disorder), Beta-glucuronidase (Sly syndrome), Bone morphogenetic protein BMPs (BMP1, BMP2, BMP3, BMP4, BMP5, BMP6, BMP7, BMP8a, BMP8b, BMP10, BMP15) (regenerative effect, bone-related conditions, chronic kidney disease (CKD)), CLN6 protein (CLN6 disease - Atypical Late Infantile, Late Onset variant, Early Juvenile, Neuronal Ceroid Lipofuscinoses (NCL)), Epidermal growth factor (EGF) (wound healing, regulation of cell growth, proliferation, and differentiation), Epigen (metabolic disorder), Epiregulin (metabolic disorder), Fibroblast Growth Factor (FGF, FGF-1, FGF-2, FGF-3, FGF-4, FGF-5, FGF-6, FGF-7, FGF-8, FGF-9, FGF-10, FGF-11, FGF-12, FGF-13, FGF-14, FGF-16, FGF-17, FGF-17, FGF-18, FGF-19, FGF-20, FGF-21, FGF-22, FGF-23) (wound healing, angiogenesis, endocrine disorders, tissue regeneration), Galsulphase (Mucopolysaccharidosis VI), Ghrelin (irritable bowel syndrome (IBS), obesity, Prader-Willi syndrome, type II diabetes mellitus), Glucocerebrosidase (Gaucher's disease), GM-CSF (regenerative effect, production of white blood cells, cancer), Heparin-binding EGF-like growth factor (HB-EGF) (wound healing, cardiac hypertrophy and heart development and function), Hepatocyte growth factor HGF (regenerative effect, wound healing), Hepcidin (iron metabolism disorders, Beta-thalassemia), Human albumin (Decreased production of albumin (hypoproteinaemia), increased loss of albumin (nephrotic syndrome), hypovolaemia, hyperbilirubinaemia), Idursulphase (Iduronate-2-sulphatase) (Mucopolysaccharidosis II (Hunter syndrome)), Integrins $\alpha V\beta 3$, $\alpha Y\beta 5$ and $\alpha 5\beta 1$ (Bind matrix macromolecules and proteinases, angiogenesis), Iuduronate sulfatase (Hunter syndrome), Laronidase (Hurler and Hurler-Scheie forms of mucopolysaccharidosis I), N-acetylgalactosamirie-4-sulfatase (rhASB; galsulfase, Arylsulfatase A (ARSA), Arylsulfatase B (ARSB)) (arylsulfatase B deficiency, Maroteaux-Lamy syndrome, mucopolysaccharidosis VI), N-acetylglucosamine-6-sulfatase (Sanfilippo syndrome), Nerve growth factor (NGF, Brain-Derived Neurotrophic Factor (BDNF), Neurotrophin-3 (NT-3), and Neurotrophin 4/5 (NT-4/5) (regenerative effect, cardiovascular diseases, coronary atherosclerosis, obesity, type 2 diabetes, metabolic syndrome, acute coronary syndromes, dementia, depression, schizophrenia, autism, Rett syndrome, anorexia nervosa, bulimia nervosa, wound healing, skin ulcers, corneal ulcers, Alzheimer's disease), Neuregulin (NRG1, NRG2, NRG3, NRG4) (metabolic disorder, schizophrenia), Neuropilin (NRP-1, NRP-2) (angiogenesis, axon guidance, cell survival, migration), Obestatin (irritable bowel syndrome (IBS), obesity, Prader-Willi syndrome, type II diabetes mellitus), Platelet Derived Growth factor (PDGF (PDFF-A, PDGF-B, PDGF-C, PDGF-D) (regenerative effect, wound healing, disorder in angiogenesis, Arteriosclerosis, Fibrosis, cancer), TGF beta receptors (endoglin, TGF-beta 1 receptor, TGF-beta 2 receptor, TGF-beta 3 receptor) (renal fibrosis, kidney disease, diabetes, ultimately end-stage renal disease (ESRD), angiogenesis), Thrombopoietin (THPO) (Megakaryocyte growth and development factor (MGDF)) (platelets disorders, platelets for donation, recovery of platelet counts after myelosuppressive chemotherapy), Transforming Growth factor (TGF (TGF-alpha, TGF-beta (TGFbeta1, TGFbeta2, and TGFbeta3))) (regenerative effect, wound healing, immunity, cancer, heart disease, diabetes, Marfan syndrome, Loeys-Dietz syndrome), VEGF (VEGF-A, VEGF-B, VEGF-C, VEGF-D, VEGF-E, VEGF-F und PIGF) (regenerative effect, angiogenesis, wound healing, cancer, permeability), Nesiritide (Acute decompensated congestive heart failure), Trypsin (Decubitus ulcer, varicose ulcer, debridement of eschar, dehiscent wound, sunburn, meconium ileus), adrenocorticotrophic hormone (ACTH) ("Addison's disease, Small cell carcinoma, Adrenoleukodystrophy, Congenital adrenal hyperplasia, Cushing's syndrome, Nelson's syndrome,

Infantile spasms), Atrial-natriuretic peptide (ANP) (endocrine disorders), Cholecystokinin (diverse), Gastrin (hypogastrinemia), Leptin (Diabetes, hypertriglyceridemia, obesity), Oxytocin (stimulate breastfeeding, non-progression of parturition), Somatostatin (symptomatic treatment of carcinoid syndrome, acute variceal bleeding, and acromegaly, polycystic diseases of the liver and kidney, acromegaly and symptoms caused by neuroendocrine tumors), Vasopressin (antidiuretic hormone) (diabetes insipidus), Calcitonin (Postmenopausal osteoporosis, Hypercalcaemia, Paget's disease, Bone metastases, Phantom limb pain, Spinal Stenosis), Exenatide (Type 2 diabetes resistant to treatment with metformin and a sulphonylurea), Growth hormone (GH), somatotropin (Growth failure due to GH deficiency or chronic renal insufficiency, Prader-Willi syndrome, Turner syndrome, AIDS wasting or cachexia with antiviral therapy), Insulin (Diabetes mellitus, diabetic ketoacidosis, hyperkalaemia), Insulin-like growth factor 1 IGF-1 (Growth failure in children with GH gene deletion or severe primary IGF1 deficiency, neurodegenerative disease, cardiovascular diseases, heart failure), Mecasermin rinfabate, IGF-1 analog (Growth failure in children with GH gene deletion or severe primary IGF1 deficiency, neurodegenerative disease, cardiovascular diseases, heart failure), Mecasermin, IGF-1 analog (Growth failure in children with GH gene deletion or severe primary IGF1 deficiency, neurodegenerative disease, cardiovascular diseases, heart failure), Pegvisomant (Acromegaly), Pramlintide (Diabetes mellitus, in combination with insulin), Teriparatide (human parathyroid hormone residues 1-34) (Severe osteoporosis), Becaplermin (Debridement adjunct for diabetic ulcers), Dibotermin-alpha (Bone morphogenetic protein 2) (Spinal fusion surgery, bone injury repair), Histrelin acetate (gonadotropin releasing hormone; GnRH) (Precocious puberty), Octreotide (Acromegaly, symptomatic relief of VIP-secreting adenoma and metastatic carcinoid tumours), and Palifermin (keratinocyte growth factor; KGF) (Severe oral mucositis in patients undergoing chemotherapy, wound healing).

These and other proteins are understood to be therapeutic, as they are meant to treat the subject by replacing its defective endogenous production of a functional protein in sufficient amounts. Accordingly, such therapeutic proteins are typically mammalian, in particular human proteins.

For the treatment of blood disorders, diseases of the circulatory system, diseases of the respiratory system, cancer or tumour diseases, infectious diseases or immunedeficiencies following therapeutic proteins may be used: Alteplase (tissue plasminogen activator; tPA) (Pulmonary embolism, myocardial infarction, acute ischaemic stroke, occlusion of central venous access devices), Anistreplase (Thrombolysis), Antithrombin III (AT-III) (Hereditary AT-III deficiency, Thromboembolism), Bivalirudin (Reduce blood-clotting risk in coronary angioplasty and heparin-induced thrombocytopaenia), Darbepoetin-alpha (Treatment of anaemia in patients with chronic renal insufficiency and chronic renal failure (+/- dialysis)), Drotrecogin-alpha (activated protein C) (Severe sepsis with a high risk of death), Erythropoietin, Epoetin-alpha, erythropoetin, erthropoyetin (Anaemia of chronic disease, myleodysplasia, anaemia due to renal failure or chemotherapy, preoperative preparation), Factor IX (Haemophilia B), Factor VIIa (Haemorrhage in patients with haemophilia A or B and inhibitors to factor VIII or factor IX), Factor VIII (Haemophilia A), Lepirudin (Heparin-induced thrombocytopaenia), Protein C concentrate (Venous thrombosis, Purpura fulminans), Reteplase (deletion mutein of tPA) (Management of acute myocardial infarction, improvement of ventricular function), Streptokinase (Acute evolving transmural myocardial infarction, pulmonary embolism, deep vein thrombosis, arterial thrombosis or embolism, occlusion of arteriovenous cannula), Tenecteplase (Acute myocardial infarction), Urokinase (Pulmonary embolism), Angiostatin (Cancer), Anti-CD22 immunotoxin (Relapsed CD33+ acute myeloid leukaemia), Denileukin diftitox (Cutaneous T-cell lymphoma (CTCL)), Immunocyanin (bladder and prostate cancer), MPS (Metallopanstimulin) (Cancer), Aflibercept (Non-small cell lung cancer (NSCLC), metastatic colorectal cancer (mCRC), hormone-refractory metastatic prostate cancer, wet macular degeneration), Endostatin (Cancer, inflammatory diseases like rheumatoid arthritis as well as Crohn's disease, diabetic retinopathy, psoriasis, and endometriosis), Collagenase (Debridement of chronic dermal ulcers and severely burned areas, Dupuytren's contracture, Peyronie's disease), Human deoxy-ribonuclease I, dornase (Cystic fibrosis; decreases respiratory tract infections in selected patients with FVC greater than 40% of predicted), Hyaluronidase (Used as an adjuvant to increase the absorption and dispersion of injected drugs, particularly anaesthetics in ophthalmic surgery and certain imaging agents), Papain (Debridement of necrotic tissue or liquefication of slough in acute and chronic lesions, such as pressure ulcers, varicose and diabetic ulcers, burns, postoperative wounds, pilonidal cyst wounds, carbuncles, and other wounds), L-Asparaginase (Acute lymphocytic leukaemia, which requires exogenous asparagine for proliferation), Peg-asparaginase (Acute lymphocytic leukaemia, which requires exogenous asparagine for proliferation), Rasburicase (Paediatric patients with leukaemia, lymphoma, and solid tumours who are undergoing anticancer therapy that may cause tumour lysis syndrome), Human chorionic gonadotropin (HCG) (Assisted reproduction), Human follicle-stimulating hormone (FSH) (Assisted reproduction), Lutropin-alpha (Infertility with luteinizing hormone deficiency), Prolactin (Hypoprolactinemia, serum prolactin deficiency, ovarian dysfunction in women, anxiety, arteriogenic erectile dysfunction, premature ejaculation, oligozoospermia, asthenospermia, hypofunction of seminal vesicles, hypoandrogenism in men), alpha-1-Proteinase inhibitor (Congenital antitrypsin deficiency), Lactase (Gas, bloating, cramps and diarrhoea due to inability to digest lactose), Pancreatic enzymes (lipase, amylase, protease) (Cystic fibrosis, chronic pancreatitis, pancreatic insufficiency, post-Billroth II gastric bypass surgery, pancreatic duct obstruction, steatorrhoea, poor digestion, gas, bloating), Adenosine deaminase (pegademase bovine, PEG-ADA) (Severe combined immunodeficiency disease due to adenosine deaminase deficiency), Abatacept (Rheumatoid arthritis (especially when

refractory to TNFalpha inhibition)), Alefacept (Plaque Psoriasis), Anakinra (Rheumatoid arthritis), Etanercept (Rheumatoid arthritis, polyarticular-course juvenile rheumatoid arthritis, psoriatic arthritis, ankylosing spondylitis, plaque psoriasis, ankylosing spondylitis), Interleukin-1 (IL-1) receptor antagonist, Anakinra (inflammation and cartilage degradation associated with rheumatoid arthritis), Thymulin (neurodegenerative diseases, rheumatism, anorexia nervosa), TNF-alpha antagonist (autoimmune disorders such as rheumatoid arthritis, ankylosing spondylitis, Crohn's disease, psoriasis, hidradenitis suppurativa, refractory asthma), Enfuvirtide (HIV-1 infection), and Thymosin $\alpha$1 (Hepatitis B and C).
(in brackets is the particular disease for which the therapeutic protein is used in the treatment)

In a further aspect, the present invention provides a vector comprising

a. an open reading frame (ORF) and/or a cloning site, e.g. for insertion of an open reading frame or a sequence comprising an open reading frame; and
b. at least one 3'-untranslated region element (3'-UTR element) comprising a nucleic acid sequence which is derived from the 3'-UTR of a ribosomal protein gene.

The at least one 3'-UTR element and the ORF are as described above for the artificial nucleic acid molecule according to the present invention. The cloning site may be any sequence that is suitable for introducing an open reading frame or a sequence comprising an open reading frame, such as one or more restriction sites. Thus, the vector comprising a cloning site is preferably suitable for inserting an open reading frame into the vector, preferably for inserting an open reading frame 5' to the 3'-UTR element. Preferably, the cloning site or the ORF is located 5' to the 3'-UTR element, preferably in close proximity to the 5'-end of the 3'-UTR element. For example, the cloning site or the ORF may be directly connected to the 5'-end of the 3'-UTR element or they may be connected via a stretch of nucleotides, such as by a stretch of 2, 4, 6, 8, 10, 20 etc. nucleotides as described above for the artificial nucleic acid molecule according to the present invention.

Preferably, the vector according to the present invention is suitable for producing the artificial nucleic acid molecule according to the present invention, preferably for producing an artificial mRNA according to the present invention, for example, by optionally inserting an open reading frame or a sequence comprising an open reading frame into the vector and transcribing the vector. Thus, preferably, the vector comprises elements needed for transcription, such as a promoter, e.g. an RNA polymerase promoter. Preferably, the vector is suitable for transcription using eukaryotic, prokaryotic, viral or phage transcription systems, such as eukaryotic cells, prokaryotic cells, or eukaryotic, prokaryotic, viral or phage *in vitro* transcription systems. Thus, for example, the vector may comprise a promoter sequence, which is recognized by a polymerase, such as by an RNA polymerase, e.g. by a eukaryotic, prokaryotic, viral, or phage RNA polymerase. In a preferred embodiment, the vector comprises a phage RNA polymerase promoter such as an SP6, T3 or T7, preferably a T7 promoter. Preferably, the vector is suitable for *in vitro* transcription using a phage based *in vitro* transcription system, such as a T7 RNA polymerase based *in vitro* transcription system.

In another preferred embodiment, the vector may be used directly for expression of the encoded peptide or protein in cells or tissue. For this purpose, the vector comprises particular elements, which are necessary for expression in those cells/tissue e.g. particular promoter sequences, such as a CMV promoter.

The vector may further comprise a poly(A) sequence and/or a polyadenylation signal as described above for the artificial nucleic acid molecule according to the present invention.

The vector may be an RNA vector or a DNA vector. Preferably, the vector is a DNA vector. The vector may be any vector known to the skilled person, such as a viral vector or a plasmid vector. Preferably, the vector is a plasmid vector, preferably a DNA plasmid vector.

In a preferred embodiment, the vector according to the present invention comprises the artificial nucleic acid molecule according to the present invention.

In one embodiment, a DNA vector according to the invention comprises a nucleic acid sequence, which has an identity of at least about 1, 2, 3, 4, 5, 10, 15, 20, 30 or 40%, preferably of at least about 50%, preferably of at least about 60%, preferably of at least about 70%, more preferably of at least about 80%, more preferably of at least about 90%, even more preferably of at least about 95%, even more preferably of at least about 99%, most preferably of 100% to the nucleic acid sequence of a 3'-UTR of a ribosomal protein gene, such as to the nucleic acid sequences according to SEQ ID NOs:10 to 115.

Preferably, a DNA vector according to the present invention comprises a sequence according to SEQ ID No. 1, SEQ ID No. 3, a sequence complementary to SEQ ID No. 7 or a sequence having an identity of at least about 40%, preferably of at least about 50%, preferably of at least about 60%, preferably of at least about 70%, more preferably of at least about 80%, more preferably of at least about 90%, even more preferably of at least about 95%; even more preferably of at least about 99% sequence identity to the nucleic acid sequence according to SEQ ID No. 1, SEQ ID No. 3, SEQ ID No. 7 or a fragment thereof as described above, preferably a functional fragment thereof.

Preferably, an RNA vector according to the present invention comprises a sequence according to SEQ ID No. 2, SEQ ID No. 4, SEQ ID No. 7 or a sequence having an identity of at least about 40%, preferably of at least about 50%,

preferably of at least about 60%, preferably of at least about 70%, more preferably of at least about 80%, more preferably of at least about 90%, even more preferably of at least about 95%; even more preferably of at least about 99% sequence identity to the nucleic acid sequence according to SEQ ID No. 2, SEQ ID No. 4, SEQ ID No. 7 or a fragment thereof, preferably a functional fragment thereof.

Preferably, the vector is a circular molecule. Preferably, the vector is a double-stranded molecule, such as a double-stranded DNA molecule. Such circular, preferably double stranded DNA molecule may be used conveniently as a storage form for the inventive artificial nucleic acid molecule. Furthermore, it may be used for transfection of cells, for example, cultured cells. Also it may be used for *in vitro* transcription for obtaining an artificial RNA molecule according to the invention.

Preferably, the vector, preferably the circular vector, is linearizable, for example, by restriction enzyme digestion. In a preferred embodiment, the vector comprises a cleavage site, such as a restriction site, preferably a unique cleavage site, located immediately 3' to the 3'-UTR element, or - if present - located 3' to the poly(A) sequence or polyadenylation signal, or - if present - located 3' to the poly(C) sequence, or - if present - located 3' to the histone stem-loop. Thus, preferably, the product obtained by linearizing the vector terminates at the 3'end with the 3'-end of the 3'-UTR element, or - if present - with the 3'-end of the poly(A) sequence or polyadenylation signal, or - if present - with the 3'-end of the poly(C) sequence. In the embodiment, wherein the vector according to the present invention comprises the artificial nucleic acid molecule according to the present invention, a restriction site, preferably a unique restriction site, is preferably located immediately 3' to the 3'-end of the artificial nucleic acid molecule.

In a further aspect, the present invention relates to a cell comprising the artificial nucleic acid molecule according to the present invention or the vector according to present invention. The cell may be any cell, such as a bacterial cell, insect cell, plant cell, vertebrate cell, e.g. a mammalian cell. Such cell may be, e.g., used for replication of the vector of the present invention, for example, in a bacterial cell. Furthermore, the cell may be used for transcribing the artificial nucleic acid molecule or the vector according to the present invention and/or translating the open reading frame of the artificial nucleic acid molecule or the vector according to the present invention. For example, the cell may be used for recombinant protein production.

The cells according to the present invention are, for example, obtainable by standard nucleic acid transfer methods, such as standard transfection, transduction or transformation methods. For example, the artificial nucleic acid molecule or the vector according to the present invention may be transferred into the cell by electroporation, lipofection, e.g. based on cationic lipids and/or liposomes, calcium phosphate precipitation, nanoparticle based transfection, virus based transfection, or based on cationic polymers, such as DEAE-dextran or polyethylenimine etc.

Preferably, the cell is a mammalian cell, such as a cell of human subject, a domestic animal, a laboratory animal, such as a mouse or rat cell. Preferably the cell is a human cell. The cell may be a cell of an established cell line, such as a CHO, BHK, 293T, COS-7, HELA, HEK, etc. or the cell may be a primary cell, such as a human dermal fibroblast (HDF) cell etc., preferably a cell isolated from an organism. In a preferred embodiment, the cell is an isolated cell of a mammalian subject, preferably of a human subject. For example, the cell may be an immune cell, such as a dendritic cell, a cancer or tumor cell, or any somatic cell etc., preferably of a mammalian subject, preferably of a human subject.

In a further aspect, the present invention provides a pharmaceutical composition comprising the artificial nucleic acid molecule according to the present invention, the vector according the present invention, or the cell according to the present invention. The pharmaceutical composition according to the invention may be used, e.g., as a vaccine, for example, for genetic vaccination. Thus, the ORF may, e.g., encode an antigen to be administered to a patient for vaccination. Thus, in a preferred embodiment, the pharmaceutical composition according to the present invention is a vaccine. Furthermore, the pharmaceutical composition according to the present invention may be used, e.g., for gene therapy.

Preferably, the pharmaceutical composition further comprises one or more pharmaceutically acceptable vehicles, diluents and/or excipients and/or one or more adjuvants. In the context of the present invention, a pharmaceutically acceptable vehicle typically includes a liquid or non-liquid basis for the inventive pharmaceutical composition. In one embodiment, the pharmaceutical composition is provided in liquid form. In this context, preferably, the vehicle is based on water, such as pyrogen-free water, isotonic saline or buffered (aqueous) solutions, e.g phosphate, citrate etc. buffered solutions. The buffer may be hypertonic, isotonic or hypotonic with reference to the specific reference medium, i.e. the buffer may have a higher, identical or lower salt content with reference to the specific reference medium, wherein preferably such concentrations of the afore mentioned salts may be used, which do not lead to damage of mammalian cells due to osmosis or other concentration effects. Reference media are e.g. liquids occurring in *"in vivo"* methods, such as blood, lymph, cytosolic liquids, or other body liquids, or e.g. liquids, which may be used as reference media in *"in vitro"* methods, such as common buffers or liquids. Such common buffers or liquids are known to a skilled person. Ringer-Lactate solution is particularly preferred as a liquid basis.

One or more compatible solid or liquid fillers or diluents or encapsulating compounds suitable for administration to a patient may be used as well for the inventive pharmaceutical composition. The term "compatible" as used herein preferably means that these components of the inventive pharmaceutical composition are capable of being mixed with

the inventive artificial nucleic acid, vector or cells as defined herein in such a manner that no interaction occurs which would substantially reduce the pharmaceutical effectiveness of the inventive pharmaceutical composition under typical use conditions.

The pharmaceutical composition according to the present invention may optionally further comprise one or more additional pharmaceutically active components. A pharmaceutically active component in this context is a compound that exhibits a therapeutic effect to heal, ameliorate or prevent a particular indication or disease. Such compounds include, without implying any limitation, peptides or proteins, nucleic acids, (therapeutically active) low molecular weight organic or inorganic compounds (molecular weight less than 5000, preferably less than 1000), sugars, antigens or antibodies, therapeutic agents already known in the prior art, antigenic cells, antigenic cellular fragments, cellular fractions, cell wall components (e.g. polysaccharides), modified, attenuated or de-activated (e.g. chemically or by irradiation) pathogens (virus, bacteria etc.).

Furthermore, the inventive pharmaceutical composition may comprise a carrier for the artificial nucleic acid molecule or the vector. Such a carrier may be suitable for mediating dissolution in physiological acceptable liquids, transport and cellular uptake of the pharmaceutical active artificial nucleic acid molecule or the vector. Accordingly, such a carrier may be a component, which may be suitable for depot and delivery of an artificial nucleic acid molecule or vector according to the invention. Such components may be, for example, cationic or polycationic carriers or compounds, which may serve as transfection or complexation agent.

Particularly preferred transfection or complexation agents, in this context, are cationic or polycationic compounds, including protamine, nucleoline, spermine or spermidine, or other cationic peptides or proteins, such as poly-L-lysine (PLL), poly-arginine, basic polypeptides, cell penetrating peptides (CPPs), including HIV-binding peptides, HIV-1 Tat (HIV), Tat-derived peptides, Penetratin, VP22 derived or analog peptides, HSV VP22 (Herpes simplex), MAP, KALA or protein transduction domains (PTDs), PpT620, proline-rich peptides, arginine-rich peptides, lysine-rich peptides, MPG-peptide(s), Pep-1, L-oligomers, Calcitonin peptide(s), Antennapedia-derived peptides (particularly from *Drosophila antennapedia*), pAntp, pIsl, FGF, Lactoferrin, Transportan, Buforin-2, Bac715-24, SynB, SynB(1), pVEC, hCT-derived peptides, SAP, or histones.

Furthermore, such cationic or polycationic compounds or carriers may be cationic or polycationic peptides or proteins, which preferably comprise or are additionally modified to comprise at least one -SH moiety. Preferably, a cationic or polycationic carrier is selected from cationic peptides having the following sum formula (I):

$$\{(Arg)_l;(Lys)_m;(His)_n;(Orn)_o;(Xaa)_x\}; \qquad \text{formula (I)}$$

wherein l + m + n + o + x = 3-100, and l, m, n or o independently of each other is any number selected from 0, 1, 2, 3, 4, 5, 6, 7, 8, 9, 10, 11, 12, 13, 14, 15, 16, 17, 18, 19, 20, 21-30, 31-40, 41-50, 51-60, 61-70, 71-80, 81-90 and 91-100 provided that the overall content of Arg (Arginine), Lys (Lysine), His (Histidine) and Orn (Ornithine) represents at least 10% of all amino acids of the oligopeptide; and Xaa is any amino acid selected from native (= naturally occurring) or non-native amino acids except of Arg, Lys, His or Orn; and x is any number selected from 0, 1, 2, 3, 4, 5, 6, 7, 8, 9, 10, 11, 12, 13, 14, 15, 16, 17, 18, 19, 20, 21-30, 31-40, 41-50, 51-60, 61-70, 71-80, 81-90, provided, that the overall content of Xaa does not exceed 90 % of all amino acids of the oligopeptide. Any of amino acids Arg, Lys, His, Orn and Xaa may be positioned at any place of the peptide. In this context cationic peptides or proteins in the range of 7-30 amino acids are particular preferred.

Further, the cationic or polycationic peptide or protein, when defined according to formula $\{(Arg)_l;(Lys)_m;(His)_n;(Orn)_o;(Xaa)_x\}$ (formula (I)) as shown above and which comprise or are additionally modified to comprise at least one -SH moeity, may be, without being restricted thereto, selected from subformula (Ia):

$$\{(Arg)_l;(Lys)_m;(His)_n;(Orn)_o;(Xaa')_x(Cys)_y\} \qquad \text{subformula (Ia)}$$

wherein $(Arg)_l;(Lys)_m;(His)_n;(Orn)_o$; and x are as defined herein, Xaa' is any amino acid selected from native (= naturally occurring) or non-native amino acids except of Arg, Lys, His, Orn or Cys and y is any number selected from 0, 1, 2, 3, 4, 5, 6, 7, 8, 9, 10, 11, 12, 13, 14, 15, 16, 17, 18, 19, 20, 21-30, 31-40, 41-50, 51-60, 61-70, 71-80 and 81-90, provided that the overall content of Arg (Arginine), Lys (Lysine), His (Histidine) and Orn (Ornithine) represents at least 10% of all amino acids of the oligopeptide. Further, the cationic or polycationic peptide may be selected from subformula (Ib):

$$Cys_1 \{(Arg)_l;(Lys)_m;(His)_n;(Orn)_o;(Xaa)_x\} Cys_2 \qquad \text{subformula (Ib)}$$

wherein empirical formula $\{(Arg)_l;(Lys)_m;(His)_n;(Orn)_o;(Xaa)_x\}$ (formula (III)) is as defined herein and forms a core of an amino acid sequence according to (semiempirical) formula (III) and wherein $Cys_1$ and $Cys_2$ are Cysteines proximal to, or terminal to $(Arg)_l;(Lys)_m;(His)_n;(Orn)_o;(Xaa)_x$.

Further preferred cationic or polycationic compounds, which can be used as transfection or complexation agent

may include cationic polysaccharides, for example chitosan, polybrene, cationic polymers, e.g. polyethyleneimine (PEI), cationic lipids, e.g. DOTMA: [1-(2,3-sioleyloxy)propyl)]-N,N,N-trimethylammonium chloride, DMRIE, di-C14-amidine, DOTIM, SAINT, DC-Chol, BGTC, CTAP, DOPC, DODAP, DOPE: Dioleyl phosphatidylethanol-amine, DOSPA, DODAB, DOIC, DMEPC, DOGS: Dioctadecylamidoglicylspermin, DIMRI: Dimyristo-oxypropyl dimethyl hydroxyethyl ammonium bromide, DOTAP: dioleoyloxy-3-(trimethylammonio)propane, DC-6-14: O,O-ditetradecanoyl-N-(α-trimethylammonioacetyl)diethanolamine chloride, CLIP1: rac-[(2,3-dioctadecyloxypropyl)(2-hydroxyethyl)]-dimethylammonium chloride, CLIP6: rac-[2(2,3-dihexadecyloxypropyl-oxymethyloxy)ethyl]-trimethylammonium, CLIP9: rac-[2(2,3-dihexadecyloxypropyl-oxysuccinyloxy)ethyl]-trimethylammonium, oligofectamine, or cationic or polycationic polymers, e.g. modified polyaminoacids, such as β-aminoacid-polymers or reversed polyamides, etc., modified polyethylenes, such as PVP (poly(N-ethyl-4-vinylpyridinium bromide)), etc., modified acrylates, such as pDMAEMA (poly(dimethylaminoethyl methylacrylate)), etc., modified Amidoamines such as pAMAM (poly(amidoamine)), etc., modified polybetaaminoester (PBAE), such as diamine end modified 1,4 butanediol diacrylate-co-5-amino-1-pentanol polymers, etc., dendrimers, such as polypropylamine dendrimers or pAMAM based dendrimers, etc., polyimine(s), such as PEI: poly(ethyleneimine), poly(propyleneimine), etc., polyallylamine, sugar backbone based polymers, such as cyclodextrin based polymers, dextran based polymers, chitosan, etc., silan backbone based polymers, such as PMOXA-PDMS copolymers, etc., blockpolymers consisting of a combination of one or more cationic blocks (e.g. selected from a cationic polymer as mentioned above) and of one or more hydrophilic or hydrophobic blocks (e.g polyethyleneglycole); etc.

According to another embodiment, the pharmaceutical composition according to the invention may comprise an adjuvant in order to enhance the immunostimulatory properties of the pharmaceutical composition. In this context, an adjuvant may be understood as any compound, which is suitable to support administration and delivery of the components such as the artificial nucleic acid molecule or vector comprised in the pharmaceutical composition according to the invention. Furthermore, such an adjuvant may, without being bound thereto, initiate or increase an immune response of the innate immune system, i.e. a non-specific immune response. With other words, when administered, the pharmaceutical composition according to the invention typically initiates an adaptive immune response directed to the antigen encoded by the artificial nucleic acid molecule. Additionally, the pharmaceutical composition according to the invention may generate an (supportive) innate immune response due to addition of an adjuvant as defined herein to the pharmaceutical composition according to the invention.

Such an adjuvant may be selected from any adjuvant known to a skilled person and suitable for the present case, i.e. supporting the induction of an immune response in a mammal. Preferably, the adjuvant may be selected from the group consisting of, without being limited thereto, TDM, MDP, muramyl dipeptide, pluronics, alum solution, aluminium hydroxide, ADJUMER™ (polyphosphazene); aluminium phosphate gel; glucans from algae; algammulin; aluminium hydroxide gel (alum); highly protein-adsorbing aluminium hydroxide gel; low viscosity aluminium hydroxide gel; AF or SPT (emulsion of squalane (5%), Tween 80 (0.2%), Pluronic L121 (1.25%), phosphate-buffered saline, pH 7.4); AVRIDINE™ (propanediamine); BAY R1005™ ((N-(2-deoxy-2-L-leucylamino-b-D-glucopyranosyl)-N-octadecyl-dodecanoylamide hydroacetate); CALCITRIOL™ (1-alpha,25-dihydroxy-vitamin D3); calcium phosphate gel; CAP™ (calcium phosphate nanoparticles); cholera holotoxin, cholera-toxin-A1-protein-A-D-fragment fusion protein, sub-unit B of the cholera toxin; CRL 1005 (block copolymer P1205); cytokine-containing liposomes; DDA (dimethyldioctadecylammonium bromide); DHEA (dehydroepiandrosterone); DMPC (dimyristoylphosphatidylcholine); DMPG (dimyristoylphosphatidylglycerol); DOC/alum complex (deoxycholic acid sodium salt); Freund's complete adjuvant; Freund's incomplete adjuvant; gamma inulin; Gerbu adjuvant (mixture of: i) N-acetylglucosaminyl-(P1-4)-N-acetylmuramyl-L-alanyl-D-glutamine (GMDP), ii) dimethyldioctadecylammonium chloride (DDA), iii) zinc-L-proline salt complex (ZnPro-8); GM-CSF); GMDP (N-acetylglucosaminyl-(beta-1-4)-N-acetylmuramyl-L-alanyl-D-isoglutamine); imiquimod (1-(2-methylpropyl)-1H-imidazo[4,5-c]quinoline-4-amine); ImmTher™ (N-acetylglucosaminyl-N-acetylmuramyl-L-Ala-D-isoGlu-L-Ala-glycerol dipalmitate); DRVs (immunoliposomes prepared from dehydration-rehydration vesicles); interferon-gamma; interleukin-lbeta; interleukin-2; interleukin-7; interleukin-12; ISCOMS™; ISCOPREP 7.0.3. ™; liposomes; LOXORIBINE™ (7-allyl-8-oxoguanosine); LT oral adjuvant (E.coli labile enterotoxin-protoxin); microspheres and microparticles of any composition; MF59™; (squalene-water emulsion); MONTANIDE ISA 51™ (purified incomplete Freund's adjuvant); MONTANIDE ISA 720™ (metabolisable oil adjuvant); MPL™ (3-Q-desacyl-4'-monophosphoryl lipid A); MTP-PE and MTP-PE liposomes ((N-acetyl-L-alanyl-D-isoglutaminyl-L-alanine-2-(1,2-dipalmitoyl-sn-glycero-3-(hydroxyphosphoryloxy))-ethylamide, monosodium salt); MURAMETIDE™ (Nac-Mur-L-Ala-D-Gln-OCH3); MURAPALMITINE™ and D-MURAPALMITINE™ (Nac-Mur-L-Thr-D-isoGln-sn-glyceroldipalmitoyl); NAGO (neuraminidase-galactose oxidase); nanospheres or nanoparticles of any composition; NISVs (non-ionic surfactant vesicles); PLEURAN™ (beta-glucan); PLGA, PGA and PLA (homo- and co-polymers of lactic acid and glycolic acid; microspheres/nanospheres); PLURONIC L121™; PMMA (polymethyl methacrylate); PODDSTM (proteinoid microspheres); polyethylene carbamate derivatives; poly-rA: poly-rU (polyadenylic acid-polyuridylic acid complex); polysorbate 80 (Tween 80); protein cochleates (Avanti Polar Lipids, Inc., Alabaster, AL); STIMULON™ (QS-21); Quil-A (Quil-A saponin); S-28463 (4-amino-otec-dimethyl-2-ethoxymethyl-1H-imidazo[4,5 c]quinoline-1-ethanol); SAF-1™ ("Syntex adjuvant formulation"); Sendai proteoliposomes and Sendai-containing lipid matrices; Span-85 (sorbitan trioleate); Specol (emulsion of Marcol 52, Span 85 and Tween 85); squalene

or Robane® (2,6,10,15,19,23-hexamethyltetracosan and 2,6,10,15,19,23-hexamethyl-2,6,10,14, 18,22-tetracosahexane); stearyltyrosine (octadecyltyrosine hydrochloride); Theramid® (N-acetylglucosaminyl-N-acetylmuramyl-L-Ala-D-isoGlu-L-Ala-dipalmitoxypropylamide); Theronyl-MDP (TermurtideTM or [thr 1]-MDP; N-acetylmuramyl-L-threonyl-D-isoglutamine); Ty particles (Ty-VLPs or virus-like particles); Walter-Reed liposomes (liposomes containing lipid A adsorbed on aluminium hydroxide), and lipopeptides, including Pam3Cys, in particular aluminium salts, such as Adju-phos, Alhydrogel, Rehydragel; emulsions, including CFA, SAF, IFA, MF59, Provax, TiterMax, Montanide, Vaxfectin; copolymers, including Optivax (CRL1005), L121, Poloaxmer4010), etc.; liposomes, including Stealth, cochleates, including BIORAL; plant derived adjuvants, including QS21, Quil A, Iscomatrix, ISCOM; adjuvants suitable for costimulation including Tomatine, biopolymers, including PLG, PMM, Inulin,; microbe derived adjuvants, including Romurtide, DETOX, MPL, CWS, Mannose, CpG nucleic acid sequences, CpG7909, ligands of human TLR 1-10, ligands of murine TLR 1-13, ISS-1018, IC31, Imidazoquinolines, Ampligen, Ribi529, IMOxine, IRIVs, VLPs, cholera toxin, heat-labile toxin, Pam3Cys, Flagellin, GPI anchor, LNFPIII/Lewis X, antimicrobial peptides, UC-1V150, RSV fusion protein, cdiGMP; and adjuvants suitable as antagonists including CGRP neuropeptide.

Suitable adjuvants may also be selected from cationic or polycationic compounds wherein the adjuvant is preferably prepared upon complexing the artificial nucleic acid molecule or the vector of the pharmaceutical composition with the cationic or polycationic compound. Association or complexing the artificial nucleic acid molecule or the vector of the pharmaceutical composition with cationic or polycationic compounds as defined herein preferably provides adjuvant properties and confers a stabilizing effect to the artificial nucleic acid molecule or the vector of the pharmaceutical composition. Particularly such preferred, such cationic or polycationic compounds are selected from cationic or polycationic peptides or proteins, including protamine, nucleoline, spermin or spermidine, or other cationic peptides or proteins, such as poly-L-lysine (PLL), poly-arginine, basic polypeptides, cell penetrating peptides (CPPs), including HIV-binding peptides, Tat, HIV-1 Tat (HIV), Tat-derived peptides, Penetratin, VP22 derived or analog peptides, HSV VP22 (Herpes simplex), MAP, KALA or protein transduction domains (PTDs, PpT620, prolin-rich peptides, arginine-rich peptides, lysine-rich peptides, MPG-peptide(s), Pep-1, L-oligomers, Calcitonin peptide(s), Antennapedia-derived peptides (particularly from Drosophila antennapedia), pAntp, pIsl, FGF, Lactoferrin, Transportan, Buforin-2, Bac715-24, SynB, SynB(1), pVEC, hCT-derived peptides, SAP, protamine, spermine, spermidine, or histones. Further preferred cationic or polycationic compounds may include cationic polysaccharides, for example chitosan, polybrene, cationic polymers, e.g. polyethyleneimine (PEI), cationic lipids, e.g. DOTMA: 1-(2,3-sioleyloxy)propyl) -N,N,N-trimethylammonium chloride, DMRIE, di-C14-amidine, DOTIM, SAINT, DC-Chol, BGTC, CTAP, DOPC, DODAP, DOPE: Dioleyl phosphatidylethanol-amine, DOSPA, DODAB, DOIC, DMEPC, DOGS: Dioctadecylamidoglicylspermin, DIMRI: Dimyristo-oxypropyl dimethyl hydroxyethyl ammonium bromide, DOTAP: dioleoyloxy-3-(trimethylammonio)propane, DC-6-14: O,O-ditetradecanoyl-N-(-trimethylammonioacetyl)diethanolamine chloride, CLIP1: rac-[(2,3-dioctadecyloxypropyl)(2-hydroxyethyl)]-dimethylammonium chloride, CLIP6: rac-[2(2,3-dihexadecyloxypropyl-oxymethyloxy)ethyl]-trimethylammonium, CLIP9: rac-[2(2,3-dihexadecyloxypropyl-oxysuccinyloxy)ethyl]-trimethylammonium, oligofectamine, or cationic or polycationic polymers, e.g. modified polyaminoacids, such as -aminoacid-polymers or reversed polyamides, etc., modified polyethylenes, such as PVP (poly(N-ethyl-4-vinylpyridinium bromide)), etc., modified acrylates, such as pDMAEMA (poly(dimethylaminoethyl methylacrylate)), etc., modified Amidoamines such as pAMAM (poly(amidoamine)), etc., modified polybetaaminoester (PBAE), such as diamine end modified 1,4 butanediol diacrylate-co-5-amino-1-pentanol polymers, etc., dendrimers, such as polypropylamine dendrimers or pAMAM based dendrimers, etc., polyimine(s), such as PEI: poly(ethyleneimine), poly(propyleneimine), etc., polyallylamine, sugar backbone based polymers, such as cyclodextrin based polymers, dextran based polymers, Chitosan, etc., silan backbone based polymers , such as PMOXA-PDMS copolymers, etc., Blockpolymers consisting of a combination of one or more cationic blocks (e.g. selected of a cationic polymer as mentioned above) and of one or more hydrophilic- or hydrophobic blocks (e.g polyethyleneglycole); etc.

Additionally, preferred cationic or polycationic proteins or peptides, which can be used as an adjuvant by complexing the artificial nucleic acid molecule or the vector, preferably an RNA, of the composition, may be selected from following proteins or peptides having the following total formula (1): (Arg)l;(Lys)m;(His)n;(Orn)o;(Xaa)x, wherein l + m + n+o + x = 8-15, and l, m, n or o independently of each other may be any number selected from 0, 1, 2, 3, 4, 5, 6, 7, 8, 9, 10, 11, 12, 13, 14 or 15, provided that the overall content of Arg, Lys, His and Orn represents at least 50% of all amino acids of the oligopeptide; and Xaa may be any amino acid selected from native (= naturally occurring) or non-native amino acids except of Arg, Lys, His or Orn; and x may be any number selected from 0, 1, 2, 3 or 4, provided, that the overall content of Xaa does not exceed 50 % of all amino acids of the oligopeptide. Particularly preferred oligoarginines in this context are e.g. Arg7, Arg8, Arg9, Arg7, H3R9, R9H3, H3R9H3, YSSR9SSY, (RKH)4, Y(RKH)2R, etc.

The ratio of the artificial nucleic acid or the vector to the cationic or polycationic compound may be calculated on the basis of the nitrogen/phosphate ratio (N/P-ratio) of the entire nucleic acid complex. For example, 1 μg RNA typically contains about 3 nmol phosphate residues, provided the RNA exhibits a statistical distribution of bases. Additionally, 1 μg peptide typically contains about x nmol nitrogen residues, dependent on the molecular weight and the number of basic amino acids. When exemplarily calculated for (Arg)9 (molecular weight 1424 g/mol, 9 nitrogen atoms), 1 μg (Arg)9 contains about 700 pmol (Arg)9 and thus 700 x 9=6300 pmol basic amino acids = 6.3 nmol nitrogen atoms.

For a mass ratio of about 1:1 RNA/(Arg)9 an N/P ratio of about 2 can be calculated. When exemplarily calculated for protamine (molecular weight about 4250 g/mol, 21 nitrogen atoms, when protamine from salmon is used) with a mass ratio of about 2:1 with 2 μg RNA, 6 nmol phosphate are to be calulated for the RNA; 1 μg protamine contains about 235 pmol protamine molecues and thus 235 x 21 = 4935 pmol basic nitrogen atoms = 4.9 nmol nitrogen atoms. For a mass ratio of about 2:1 RNA/protamine an N/P ratio of about 0.81 can be calculated. For a mass ratio of about 8:1 RNA/protamine an N/P ratio of about 0.2 can be calculated. In the context of the present invention, an N/P-ratio is preferably in the range of about 0.1-10, preferably in a range of about 0.3-4 and most preferably in a range of about 0.5-2 or 0.7-2 regarding the ratio of nucleic acid:peptide in the complex, and most preferably in the range of about 0.7-1.5.

Patent application WO2010/037539, the disclosure of which is incorporated herein by reference, describes an immunostimulatory composition and methods for the preparation of an immunostimulatory composition. Accordingly, in a preferred embodiment of the invention, the composition is obtained in two separate steps in order to obtain both, an efficient immunostimulatory effect and efficient translation of the artificial nucleic acid molecule according to the invention. Therein, a so called "adjuvant component" is prepared by complexing - in a first step - the artificial nucleic acid molecule or vector, preferably an RNA, of the adjuvant component with a cationic or polycationic compound in a specific ratio to form a stable complex. In this context, it is important, that no free cationic or polycationic compound or only a neglibly small amount remains in the adjuvant component after complexing the nucleic acid. Accordingly, the ratio of the nucleic acid and the cationic or polycationic compound in the adjuvant component is typically selected in a range that the nucleic acid is entirely complexed and no free cationic or polycationic compound or only a neclectably small amount remains in the composition. Preferably the ratio of the adjuvant component, i.e. the ratio of the nucleic acid to the cationic or polycationic compound is selected from a range of about 6:1 (w/w) to about 0,25:1 (w/w), more preferably from about 5:1 (w/w) to about 0,5:1 (w/w), even more preferably of about 4:1 (w/w) to about 1:1 (w/w) or of about 3:1 (w/w) to about 1:1 (w/w), and most preferably a ratio of about 3:1 (w/w) to about 2:1 (w/w).

According to a preferred embodiment, the artificial nucleic acid molecule or vector, preferably an RNA molecule, according to the invention is added in a second step to the complexed nucleic acid molecule, preferably an RNA, of the adjuvant component in order to form the (immunostimulatory) composition of the invention. Therein, the artificial acid molecule or vector, preferably an RNA, of the invention is added as free nucleic acid, i.e. nucleic acid, which is not complexed by other compounds. Prior to addition, the free artificial nucleic acid molecule or vector is not complexed and will preferably not undergo any detectable or significant complexation reaction upon the addition of the adjuvant component.

Suitable adjuvants may furthermore be selected from nucleic acids having the formula (II): GlXmGn, wherein: G is guanosine, uracil or an analogue of guanosine or uracil; X is guanosine, uracil, adenosine, thymidine, cytosine or an analogue of the above-mentioned nucleotides; l is an integer from 1 to 40, wherein when l = 1 G is guanosine or an analogue thereof, when l > 1 at least 50% of the nucleotides are guanosine or an analogue thereof; m is an integer and is at least 3; wherein when m = 3 X is uracil or an analogue thereof, when m > 3 at least 3 successive uracils or analogues of uracil occur; n is an integer from 1 to 40, wherein when n = 1 G is guanosine or an analogue thereof, when n > 1 at least 50% of the nucleotides are guanosine or an analogue thereof.

Other suitable adjuvants may furthermore be selected from nucleic acids having the formula (III): ClXmCn, wherein: C is cytosine, uracil or an analogue of cytosine or uracil; X is guanosine, uracil, adenosine, thymidine, cytosine or an analogue of the above-mentioned nucleotides; l is an integer from 1 to 40, wherein when l = 1 C is cytosine or an analogue thereof, when l > 1 at least 50% of the nucleotides are cytosine or an analogue thereof; m is an integer and is at least 3; wherein when m = 3 X is uracil or an analogue thereof, when m > 3 at least 3 successive uracils or analogues of uracil occur; n is an integer from 1 to 40, wherein when n = 1 C is cytosine or an analogue thereof, when n > 1 at least 50% of the nucleotides are cytosine or an analogue thereof.

The pharmaceutical composition according to the present invention preferably comprises a "safe and effective amount" of the components of the pharmaceutical composition, particularly of the inventive artificial nucleic acid molecule, the vector and/or the cells as defined herein. As used herein, a "safe and effective amount" means an amount sufficient to significantly induce a positive modification of a disease or disorder as defined herein. At the same time, however, a "safe and effective amount" preferably avoids serious side-effects and permits a sensible relationship between advantage and risk. The determination of these limits typically lies within the scope of sensible medical judgment.

In a further aspect, the present invention provides the artificial nucleic acid molecule according to the present invention, the vector according to the present invention, the cell according to the present invention, or the pharmaceutical composition according to the present invention for use as a medicament, for example, as vaccine (in genetic vaccination) or in gene therapy.

The artificial nucleic acid molecule according to the present invention, the vector according to the present invention, the cell according to the present invention, or the pharmaceutical composition according to the present invention are particularly suitable for any medical application which makes use of the therapeutic action or effect of peptides, polypeptides or proteins, or where supplementation of a particular peptide or protein is needed. Thus, the present invention provides the artificial nucleic acid molecule according to the present invention, the vector according to the

present invention, the cell according to the present invention, or the pharmaceutical composition according to the present invention for use in the treatment or prevention of diseases or disorders amenable to treatment by the therapeutic action or effect of peptides, polypeptides or proteins or amenable to treatment by supplementation of a particular peptide, polypeptide or protein. For example, the artificial nucleic acid molecule according to the present invention, the vector according to the present invention, the cell according to the present invention, or the pharmaceutical composition according to the present invention may be used for the treatment or prevention of genetic diseases, autoimmune diseases, cancerous or tumour-related diseases, infectious diseases, chronic diseases or the like, e.g., by genetic vaccination or gene therapy.

In particular, such therapeutic treatments which benefit from a stable and prolonged presence of therapeutic peptides, polypeptides or proteins in a subject to be treated are especially suitable as medical application in the context of the present invention, since the inventive 3'-UTR element provides for a stable and prolonged expression of the encoded peptide or protein of the inventive artificial nucleic acid molecule or vector. Thus, a particularly suitable medical application for the artificial nucleic acid molecule according to the present invention, the vector according to the present invention, the cell according to the present invention, or the pharmaceutical composition according to the present invention is vaccination. Thus, the present invention provides the artificial nucleic acid molecule according to the present invention, the vector according to the present invention, the cell according to the present invention, or the pharmaceutical composition according to the present invention for vaccination of a subject, preferably a mammalian subject, more preferably a human subject. Preferred vaccination treatments are vaccination against infectious diseases, such as bacterial, protozoal or viral infections, and anti-tumour-vaccination. Such vaccination treatments may be prophylactic or therapeutic.

Depending on the disease to be treated or prevented, the ORF may be selected. For example, the open reading frame may code for a protein that has to be supplied to a patient suffering from total lack or at least partial loss of function of a protein, such as a patient suffering from a genetic disease. Additionally the open reading frame may be chosen from an ORF coding for a peptide or protein, which beneficially influences a disease or the condition of a subject. Furthermore, the open reading frame may code for a peptide or protein which effects down-regulation of a pathological overproduction of a natural peptide or protein or elimination of cells expressing pathologically a protein or peptide. Such lack, loss of function or overproduction may, e.g., occur in the context of tumour and neoplasia, autoimmune diseases, allergies, infections, chronic diseases or the like. Furthermore, the open reading frame may code for an antigen or immunogen, e.g. for an epitope of a pathogen or for a tumour antigen. Thus, in preferred embodiments, the artificial nucleic acid molecule or the vector according to the present invention comprises an ORF encoding an amino acid sequence comprising or consisting of an antigen or immunogen, e.g. an epitope of a pathogen or a tumour-associated antigen, a 3'-UTR element as described above, and optional further components, such as a poly(A) sequence etc.

In the context of medical application, in particular, in the context of vaccination, it is preferred that the artificial nucleic acid molecule according to the present invention is RNA, preferably mRNA, since DNA harbours the risk of eliciting an anti-DNA immune response and tends to insert into genomic DNA. However, in some embodiments, for example, if a viral delivery vehicle, such as an adenoviral delivery vehicle is used for delivery of the artificial nucleic acid molecule or the vector according to the present invention, e.g., in the context of gene therapeutic treatments, it may be desirable that the artificial nucleic acid molecule or the vector is a DNA molecule.

The artificial nucleic acid molecule according to the present invention, the vector according to the present invention, the cell according to the present invention, or the pharmaceutical composition according to the present invention may be administered orally, parenterally, by inhalation spray, topically, rectally, nasally, buccally, vaginally, via an implanted reservoir or via jet injection. The term parenteral as used herein includes subcutaneous, intravenous, intramuscular, intra-articular, intra-synovial, intrasternal, intrathecal, intrahepatic, intralesional, intracranial, transdermal, intradermal, intrapulmonal, intraperitoneal, intracardial, intraarterial, and sublingual injection or infusion techniques. In a preferred embodiment, the artificial nucleic acid molecule according to the present invention, the vector according to the present invention, the cell according to the present invention, or the pharmaceutical composition according to the present invention is administered via needle-free injection (e.g. jet injection).

Preferably, the artificial nucleic acid molecule according to the present invention, the vector according to the present invention, the cell according to the present invention, or the pharmaceutical composition according to the present invention is administered parenterally, e.g. by parenteral injection, more preferably by subcutaneous, intravenous, intramuscular, intra-articular, intra-synovial, intrasternal, intrathecal, intrahepatic, intralesional, intracranial, transdermal, intradermal, intrapulmonal, intraperitoneal, intracardial, intraarterial, sublingual injection or via infusion techniques. Particularly preferred is intradermal and intramuscular injection. Sterile injectable forms of the inventive pharmaceutical composition may be aqueous or oleaginous suspension. These suspensions may be formulated according to techniques known in the art using suitable dispersing or wetting agents and suspending agents. Preferably, the solutions or suspensions are administered via needle-free injection (e.g. jet injection).

The artificial nucleic acid molecule according to the present invention, the vector according to the present invention, the cell according to the present invention, or the pharmaceutical composition according to the present invention may also be administered orally in any orally acceptable dosage form including, but not limited to, capsules, tablets,

aqueous suspensions or solutions.

The artificial nucleic acid molecule according to the present invention, the vector according to the present invention, the cell according to the present invention, or the pharmaceutical composition according to the present invention may also be administered topically, especially when the target of treatment includes areas or organs readily accessible by topical application, e.g. including diseases of the skin or of any other accessible epithelial tissue. Suitable topical formulations are readily prepared for each of these areas or organs. For topical applications, the artificial nucleic acid molecule according to the present invention, the vector according to the present invention, the cell according to the present invention, or the pharmaceutical composition according to the present invention may be formulated in a suitable ointment suspended or dissolved in one or more carriers.

In one embodiment, the use as a medicament comprises the step of transfection of mammalian cells, preferably *in vitro* or *ex vivo* transfection of mammalian cells, more preferably *in vitro* transfection of isolated cells of a subject to be treated by the medicament. If the use comprises the *in vitro* transfection of isolated cells, the use as a medicament may further comprise the readministration of the transfected cells to the patient. The use of the inventive artificial nucleic acid molecules or the vector as a medicament may further comprise the step of selection of successfully transfected isolated cells. Thus, it may be beneficial if the vector further comprises a selection marker. Also, the use as a medicament may comprise *in vitro* transfection of isolated cells and purification of an expression-product, i.e. the encoded peptide or protein from these cells. This purified peptide or protein may subsequently be administered to a subject in need thereof.

The present invention also provides a method for treating or preventing a disease or disorder as described above comprising administering the artificial nucleic acid molecule according to the present invention, the vector according to the present invention, the cell according to the present invention, or the pharmaceutical composition according to the present invention to a subject in need thereof.

Furthermore, the present invention provides a method for treating or preventing a disease or disorder comprising transfection of a cell with an artificial nucleic acid molecule according to the present invention or with the vector according to the present invention. Said transfection may be performed *in vitro, ex vivo* or *in vivo.* In a preferred embodiment, transfection of a cell is performed *in vitro* and the transfected cell is administered to a subject in need thereof, preferably to a human patient. Preferably, the cell which is to be transfected *in vitro* is an isolated cell of the subject, preferably of the human patient. Thus, the present invention provides a method of treatment comprising the steps of isolating a cell from a subject, preferably from a human patient, transfecting the isolated cell with the artificial nucleic acid according to the present invention or the vector according to the present invention, and administering the transfected cell to the subject, preferably the human patient.

The method of treating or preventing a disorder according to the present invention is preferably a vaccination method or a gene therapy method as described above.

As described above, the inventive 3'-UTR element is capable of stabilizing an mRNA molecule and/or of enhancing, stabilizing and/or prolonging the protein production from an mRNA molecule. Thus, in a further aspect, the present invention relates to a method for stabilizing an RNA molecule, preferably an mRNA molecule, comprising the step of associating the RNA molecule, preferably the mRNA molecule, or a vector encoding the RNA molecule, with a 3'-UTR element comprising or consisting of a nucleic acid sequence which is derived from the 3'-UTR of a ribosomal protein gene or from a variant of the 3'-UTR of a ribosomal protein gene, preferably with the 3'-UTR element as described above.

Furthermore, the present invention relates to a method for enhancing, stabilizing and / or prolonging protein production from an artificial nucleic acid molecule or from a vector, preferably from an mRNA molecule, and/or for stabilizing and/or prolonging protein production from an artificial nucleic acid molecule or from a vector, preferably from an mRNA molecule, the method comprising the step of associating the artificial nucleic acid molecule or the vector, preferably the mRNA molecule, with a 3'-UTR element which comprises or consists of a nucleic acid sequence which is derived from the 3'-UTR of a ribosomal protein gene or from a variant of the 3'-UTR of a ribosomal protein gene, preferably with the 3'-UTR element as described above.

The term "associating the artificial nucleic acid molecule or the vector with a 3'-UTR element" in the context of the present invention preferably means functionally associating or functionally combining the artificial nucleic acid molecule or the vector with the 3'-UTR element. This means that the artificial nucleic acid molecule or the vector and the 3'-UTR element, preferably the 3'-UTR element as described above, are associated or coupled such that the function of the 3'-UTR element, e.g., the RNA and/or protein production stabilizing function, is exerted. Typically, this means that the 3'-UTR element is integrated into the artificial nucleic acid molecule or the vector, preferably the mRNA molecule, 3' to an open reading frame, preferably immediately 3' to an open reading frame, preferably between the open reading frame and a poly(A) sequence or a polyadenylation signal. Preferably, the 3'-UTR element is integrated into the artificial nucleic acid molecule or the vector, preferably the mRNA, as 3'-UTR, i.e. such that the 3'-UTR element is the 3'-UTR of the artificial nucleic acid molecule or the vector, preferably the mRNA, i.e., such that it extends from the 3'-side of the open reading frame to the 5'-side of a poly(A) sequence or a polyadenylation signal, optionally connected via a short linker, such as a sequence comprising or consisting of one or more restriction sites. Thus, preferably, the term "associating

the artificial nucleic acid molecule or the vector with a 3'-UTR element" means functionally associating the 3'-UTR element with an open reading frame located within the artificial nucleic acid molecule or the vector, preferably within the mRNA molecule. The 3'-UTR and the ORF are as described above for the artificial nucleic acid molecule according to the present invention, for example, preferably the ORF and the 3'-UTR are heterologous, e.g. derived from different genes, as described above.

In a further aspect, the present invention provides the use of a 3'-UTR element, preferably the 3'-UTR element as described above, for increasing the stability of an RNA molecule, preferably of an mRNA molecule, wherein the 3'-UTR element comprises or consists of a nucleic acid sequence, which is derived from the 3'-UTR of a ribosomal protein gene or from a variant of the 3'-UTR of a ribosomal protein gene.

Furthermore, the present invention provides the use of a 3'-UTR element, preferably the 3'-UTR element as described above, for increasing protein production from an artificial nucleic acid molecule or a vector, preferably from an mRNA molecule, and/or for stabilizing and/or prolonging protein production from an artificial nucleic acid molecule or a vector molecule, preferably from an mRNA molecule, wherein the 3'-UTR element comprises or consists of a nucleic acid sequence which is derived from the 3'-UTR of a ribosomal protein gene or from a variant of the 3'-UTR of a ribosomal protein gene as described above.

The uses according to the present invention preferably comprise associating the artificial nucleic acid molecule, the vector, or the RNA with the 3'-UTR element as described above.

The compounds and ingredients of the inventive pharmaceutical composition may also be manufactured and traded separately of each other. Thus, the invention relates further to a kit or kit of parts comprising an artificial nucleic acid molecule according to the invention, a vector according to the invention, a cell according to the invention, and/or a pharmaceutical composition according to the invention. Preferably, such kit or kits of parts may, additionally, comprise instructions for use, cells for transfection, an adjuvant, a means for administration of the pharmaceutical composition, a pharmaceutically acceptable carrier and/or a pharmaceutically acceptable solution for dissolution or dilution of the artificial nucleic acid molecule, the vector, the cells or the pharmaceutical composition.

The following Figures, Sequences and Examples are intended to illustrate the invention further. They are not intended to limit the subject matter of the invention thereto.

Figures 1 to 3 show sequences encoding mRNAs that can be obtained by in vitro transcription. The following abbreviations are used:

- rpl32: 5'-UTR of human ribosomal protein Large 32 lacking the 5' terminal oligopyrimidine tract
- PpLuc (GC): GC-enriched mRNA sequence coding for *Photinus pyralis* luciferase
- A64: poly(A)-sequence with 64 adenylates
- ag: center, $\alpha$-complex-binding portion of the 3'-UTR of human $\alpha$-globin
- rps9: 3'-UTR element derived from the 3'-UTR of human ribosomal protein Small 9
- C30: poly(C)-sequence with 30 cytidylates
- histoneSL: A histone stem-loop sequence taken from (Cakmakci, Lerner, Wagner, Zheng, & William F Marzluff, 2008. Mol. Cell. Biol. 28(3):1182-94);
- albumin: 3'-UTR of human albumin.

Fig. 1: shows the sequence encoding the mRNA rpl32 - PpLuc(GC) - A64 - C30-histoneSL (SEQ ID NO:8)

Fig. 2: shows the sequence encoding the mRNA rpl32 - PpLuc(GC) - ag - A64 - C30-histoneSL (SEQ ID NO:9). The center, $\alpha$-complex-binding portion of the 3'-UTR of human $\alpha$-globin was inserted between ORF and poly(A). The PpLuc(GC) ORF is highlighted in italics. The 3'-UTR element derived from $\alpha$-globin is underlined.

Fig. 3: mRNA sequence of rpl32 - PpLuc(GC) - rps9 - A64 - C30 - hSL (SEQ ID NO:7). The 3'-UTR of human ribosomal protein Small 9 was inserted between ORF and poly(A). The PpLuc(GC) ORF is highlighted in italics, the 3' UTR element derived from rps9 is underlined.

Fig. 4: shows that the 3'-UTR of ribosomal protein Small 9 markedly increases protein expression from mRNA.
The effect of the inventive 3'-UTR of human ribosomal protein Small 9 on luciferase expression from mRNA was examined, compared to luciferase expression from mRNA lacking a 3'-UTR or containing the human $\alpha$-globin 3'-UTR. Therefore different mRNAs were transfected into human dermal fibroblasts (HDF) by lipofection. Luciferase levels were measured at 6, 24, 48, and 72 hours after transfection. Luciferase was clearly expressed from mRNA lacking a 3'-UTR. However, luciferase expression was not increased by the well-known $\alpha$-globin 3'-UTR. In contrast, the 3'-UTR of ribosomal protein Small 9 increased luciferase expression markedly. Data are graphed as mean RLU $\pm$ SEM (relative light units $\pm$ standard error) for triplicate transfections. RLU are summarized in Example 5.1.

Fig. 5: shows the sequence encoding the mRNA rpl32 - PpLuc(GC) - albumin - A64-C30 - histoneSL (SEQ ID NO: 206). The 3'-UTR of human albumin was inserted between ORF and poly(A). The PpLuc(GC) ORF is highlighted in italics. The 3'-UTR element derived from albumin is underlined.

Fig. 6: shows that the 3'-UTRs derived from the murine ribosomal protein genes rps21, rps29, rps9, rps27, rps28, rps19, rpl35a, rpl13, rpl36 and rpl23a increase protein expression from mRNA in HDF cells (human dermal fibroblasts) at least to the same extent as an mRNA comprising the 3'-UTR of the albumin gene, which was already shown to increase protein expression from mRNA (WO2013143698).

Fig. 7: shows that the 3'-UTRs derived from the murine ribosomal protein genes rps21, rps29, rps9, rps27, rps28, rps19, rpl35a, rpl13, rpl36 and rpl23a increase protein expression from mRNA in HeLa cells at least to the same extent as an mRNA comprising the 3'-UTR of the albumin gene, which was already shown to increase protein expression from mRNA (WO2013143698).

Fig. 8: shows that the 3'-UTRs derived from the murine ribosomal protein genes rpl23, uba52, rpl22l1, rpl36a, rps4x, rpl27, rpl3, rps23, rps13, rpl26, rps17, rps18, rps8, Fau, rps13, rpl11 and rpl38 increase protein expression from mRNA in HDF cells (human dermal fibroblasts) at least to the same extent as an mRNA comprising the 3'-UTR of the albumin gene, which was already shown to increase protein expression from mRNA (WO2013143698).

Fig. 9: shows that the 3'-UTRs derived from the murine ribosomal protein genes rpl23, uba52, rpl22l1, rpl36a, rps4x, rpl27, rpl3, rps23, rps13, rpl26, rps17, rps18, rps8, Fau, rps13, rpl11 and rpl38 increase protein expression from mRNA in HeLa cells at least to the same extent as an mRNA comprising the 3'-UTR of the albumin gene, which was already shown to increase protein expression from mRNA (WO2013143698).

## Examples

1. Preparation of DNA-templates

A vector for *in vitro* transcription was constructed containing a T7 promoter and a GC-enriched sequence coding for *Photinus pyralis* luciferase (PpLuc(GC)). The 5' untranslated region (5'-UTR) of ribosomal protein Large 32 was inserted 5' of PpLuc(GC). An A64 poly(A) sequence, followed by C30 and a histone stem-loop sequence, was inserted 3' of PpLuc(GC). The histone stem-loop sequence was followed by a restriction site used for linearization of the vector before in vitro transcription. mRNA obtained from this vector accordingly by *in vitro* transcription is designated as "rpl32 - PpLuc(GC) - A64 - C30-histoneSL".

This vector was modified to include untranslated sequences 3' of the open reading frame (3'-UTR). In summary, vectors comprising the following mRNA encoding sequences have been generated (some of the mRNA encoding sequences are depicted as examples in Figures 1 to 3):

rpl32 - PpLuc(GC) - A64 - C30 - histoneSL (SEQ ID NO:8, Fig. 1)
rpl32 - PpLuc(GC) - ag - A64 - C30 - histoneSL (SEQ ID NO:9, Fig. 2)
rpl32 - PpLuc(GC) - rps9 - A64 - C30 - histoneSL (SEQ ID NO:7, Fig. 3)
rpl32 - PpLuc(GC) - rps21 - A64 - C30 - histoneSL
rpl32 - PpLuc(GC) - rps29 - A64 - C30 - histoneSL
rpl32 - PpLuc(GC) - rps9 - A64 - C30 - histoneSL
rpl32 - PpLuc(GC) - rps27 - A64 - C30 - histoneSL
rpl32 - PpLuc(GC) - rps28 - A64 - C30 - histoneSL
rpl32 - PpLuc(GC) - rps19 - A64 - C30 - histoneSL
rpl32 - PpLuc(GC) - rpl35a - A64 - C30 - histoneSL
rpl32 - PpLuc(GC) - rpl13 - A64 - C30 - histoneSL
rpl32 - PpLuc(GC) - rpl36 - A64 - C30 - histoneSL
rpl32 - PpLuc(GC) - rpl23a - A64 - C30 - histoneSL
rpl32 - PpLuc(GC) - rpl23 - A64 - C30 - histoneSL
rpl32 - PpLuc(GC) - uba52 - A64 - C30 - histoneSL
rpl32 - PpLuc(GC) - rpl22l1 - A64 - C30 - histoneSL
rpl32 - PpLuc(GC) - rpl36a - A64 - C30 - histoneSL
rpl32 - PpLuc(GC) - rps4x - A64 - C30 - histoneSL
rpl32 - PpLuc(GC) - rpl27 - A64 - C30 - histoneSL

rpl32 - PpLuc(GC) - rpl3 - A64 - C30 - histoneSL
rpl32 - PpLuc(GC) - rps23 - A64 - C30 - histoneSL
rpl32 - PpLuc(GC) - rps13 - A64 - C30 - histoneSL
rpl32 - PpLuc(GC) - rpl26 - A64 - C30 - histoneSL
rpl32 - PpLuc(GC) - rps17 - A64 - C30 - histoneSL
rpl32 - PpLuc(GC) - rps18 - A64 - C30 - histoneSL
rpl32 - PpLuc(GC) - Fau - A64 - C30 - histoneSL
rpl32 - PpLuc(GC) - rps13 - A64 - C30 - histoneSL
rpl32 - PpLuc(GC) - rpl11 - A64 - C30 - histoneSL
rpl32 - PpLuc(GC) - rpl38 - A64 - C30 - histoneSL

2. *In vitro* transcription

The DNA-template according to Example 1 was linearized and transcribed *in vitro* using T7-RNA polymerase. The DNA template was then digested by DNase-treatment. mRNA transcripts contained a 5'-CAP structure obtained by adding an excess of N7-Methyl-Guanosine-5'-Triphosphate-5'-Guanosine to the transcription reaction. mRNA thus obtained was purified and resuspended in water.

3. Luciferase expression by mRNA lipofection

Human dermal fibroblasts (HDF) or HeLa cells were seeded in 24 well plates three days before transfection at a density of $3x10^4$ cells per well in medium (RPMI 1640 medium with L-glutamine and 25mM Hepes (Lonza, Basel, Switzerland) to which 10% FCS, 1% Pen/Strep, 1%Glutamine were added). Immediately before lipofection, cells were washed in Opti-MEM. Cells were lipofected with 25 ng of PpLuc-encoding mRNA per well complexed with Lipofectamine2000. mRNA coding for Renilla reniformis luciferase (RrLuc) was transfected together with PpLuc mRNA to control for transfection efficiency (2.5 ng of RrLuc mRNA per well). 90 minutes after start of transfection, Opti-MEM was exchanged for medium. 6, 24, 48, and 72 hours after transfection, medium was aspirated and cells were lysed in 100 μl of lysis buffer (Passive Lysis Buffer, Promega). Lysates were stored at -80°C until luciferase activity was measured.

4. Luciferase measurement

Luciferase activity was measured as relative light units (RLU) in a Hidex Chameleon plate reader. PpLuc activity was measured at 2 seconds measuring time using 20 μl of lysate and 50 μl of luciferin buffer (Beetle-Juice, PJK GmbH). RrLuc activity was measured at 2 seconds measuring time using 20 μl of lysate and 50 μl of coelenterazin buffer (Renilla-Juice, PJK GmbH).

5. Results

5.1 The 3'-UTR of ribosomal protein genes increases protein expression.

To investigate the effect of the 3'-UTR of ribosomal protein genes on protein expression from mRNA, mRNAs with different UTRs were synthesized: mRNAs either lacked a 3'-UTR, or contained the center, α-complex-binding portion of the 3'-UTR of human α-globin (ag), or contained the 3'-UTR of human ribosomal protein Small 9 (rps9). Luciferase-encoding mRNAs were transfected into human dermal fibroblasts (HDF). Luciferase levels were measured at 6, 24, 48, and 72 hours after transfection. From these data, total protein expressed from 0 to 72 hours was calculated as the area under the curve (AUC) (see following Table 1 and Figure 4).

Table 1:

| 3'-UTR | RLU at 6 hours | RLU at 24 hours | RLU at 48 hours | RLU at 72 hours | AUC |
|---|---|---|---|---|---|
| none | 1183752 | 2703805 | 2040979 | 536076 | 126400000 |
| ag | 696317 | 2188117 | 1630769 | 273142 | 96720000 |
| rps9 | 1650962 | 4513651 | 4273634 | 755401 | 226200000 |

Luciferase was clearly expressed from mRNA lacking a 3'-UTR. However, luciferase expression was not increased by the well-known α-globin 3'-UTR. In contrast, the 3'-UTR of ribosomal protein Small 9 increased luciferase

expression markedly.

5.2 The 3'-UTRs of murine ribosomal protein genes increase protein expression.

To investigate the effect of 3'-UTRs of murine ribosomal protein genes on protein expression from mRNA, mRNAs with different UTRs were synthesized: mRNAs contained the 3'-UTR of different murine ribosomal proteins (rps21, rps29, rps9, rps27, rps28, rps19, rpl35a, rpl13, rpl36 and rpl23a) and for comparison the 3'-UTR of albumin, which is known to increase protein expression from mRNA (WO2013143698). Luciferase-encoding mRNAs were transfected into human dermal fibroblasts (HDF). Luciferase levels were measured at 24, 48, and 72 hours after transfection. (see following Table 2 and Figure 6).

Table 2:

| | RLU at 24 hours | RLU at 48 hours | RLU at 72 hours |
|---|---|---|---|
| rpl32- PpLuc(GC)-albumin | 1468876 | 720609 | 199437 |
| rpl32-PpLuc(GC)-albumin | 1407897 | 580822 | 181030 |
| rpl32- PpLuc(GC)-rps21 | 2974366 | 779239 | 191021 |
| rpl32-PpLuc(GC)-rps29 | 4040760 | 856996 | 216321 |
| rpl32-PpLuc(GC)-rps9 | 2930305 | 802405 | 219681 |
| rpl32- PpLuc(GC)-rps27 | 4503067 | 1090230 | 274320 |
| rpl32-PpLuc(GC)-rps28 | 2026219 | 448372 | 103269 |
| rpl32- PpLuc(GC)-rps19 | 4249503 | 1075621 | 206846 |
| rpl32-PpLuc(GC)-rpl35a | 3907863 | 1223672 | 262108 |
| rpl32- PpLuc(GC)-rpl13 | 2543231 | 782597 | 165374 |
| rpl32- PpLuc(GC)-rpl36 | 2880198 | 683174 | 143539 |
| rpl32-PpLuc(GC)-rpl23a | 2872413 | 606451 | 120915 |
| cells | 118 | 213 | 191 |

The results show that more Luciferase was expressed from mRNA comprising a 3'-UTR of murine ribosomal proteins compared to mRNA comprising the 3'-UTR of albumin, which has already been described to increase protein production.

5.3 The 3'-UTRs of murine ribosomal protein genes increase protein expression.

To investigate the effect of 3'-UTRs of murine ribosomal protein genes on protein expression from mRNA, mRNAs with different UTRs were synthesized: mRNAs contained the 3'-UTR of different murine ribosomal proteins (rps21, rps29, rps9, rps27, rps28, rps19, rpl35a, rpl13, rpl36 and rpl23a) and for comparison the 3'-UTR of albumin. Luciferase-encoding mRNAs were transfected into HeLa cells. Luciferase levels were measured at 24, 48, and 72 hours after transfection. (see following Table 3 and Figure 7).

Table 3:

| | RLU at 24 hours | RLU at 48 hours | RLU at 72 hours |
|---|---|---|---|
| rpl32- PpLuc(GC)-albumin | 482053 | 131028 | 16580 |
| rpl32- PpLuc(GC)-albumin | 525908 | 86636 | 21971 |
| rpl32- PpLuc(GC)-rps21 | 1452649 | 307127 | 38317 |
| rpl32- PpLuc(GC)-rps29 | 1413088 | 293924 | 27114 |
| rpl32- PpLuc(GC)-rps9 | 1399948 | 304061 | 36627 |
| rpl32- PpLuc(GC)-rps27 | 1460812 | 235486 | 28608 |

(continued)

| | RLU at 24 hours | RLU at 48 hours | RLU at 72 hours |
|---|---|---|---|
| rpl32- PpLuc(GC)-rps28 | 1384794 | 201359 | 25787 |
| rpl32- PpLuc(GC)-rps19 | 1446100 | 303612 | 40591 |
| rpl32- PpLuc(GC)-rpl35a | 1848259 | 307848 | 47293 |
| rpl32- PpLuc(GC)-rpl13 | 772371 | 138186 | 27188 |
| rpl32- PpLuc(GC)-rpl36 | 837643 | 147108 | 36641 |
| rpl32- PpLuc(GC)-rpl23a | 887643 | 157330 | 24916 |
| cells | 97 | 193 | 117 |

The results show that more Luciferase was expressed from mRNA comprising a 3'-UTR of murine ribosomal proteins compared to mRNA comprising the 3'-UTR of albumin, which has already been described to increase protein production.

5.4 The 3'-UTRs of murine ribosomal protein genes increase protein expression.

To investigate the effect of 3'-UTRs of murine ribosomal protein genes on protein expression from mRNA, mRNAs with different UTRs were synthesized: mRNAs contained the 3'-UTR of different murine ribosomal proteins (rpl23, uba52, rpl22l1, rpl36a, rps4x, rpl27, rpl3, rps23, rps13, rpl26, rps17, rps18, rps8, Fau, rps13, rpl11 and rpl38) and for comparison the 3'-UTR of albumin. Luciferase-encoding mRNAs were transfected into human dermal fibroblasts (HDF). Luciferase levels were measured at 24, 48, and 72 hours after transfection. (see following Table 4 and Figure 8).

Table 4:

| | RLU at 24 hours | RLU at 48 hours | RLU at 72 hours |
|---|---|---|---|
| rpl32-PpLuc(GC)-albumin | 2181914 | 921116 | 474521 |
| rpl32-PpLuc(GC)-rpl23 | 4836067 | 1365445 | 444623 |
| rpl32-PpLuc(GC)-uba52 | 4404508 | 1572170 | 517874 |
| rpl32-PpLuc(GC)-rpl22l1 | 4124152 | 1057264 | 346186 |
| rpl32-PpLuc(GC)-rpl36a | 4326843 | 1328764 | 510755 |
| rpl32-PpLuc(GC)-rps4x | 2879407 | 724050 | 246616 |
| rpl32-PpLuc(GC)-rpl27 | 3391506 | 830016 | 254254 |
| rpl32-PpLuc(GC)-rpl3 | 4889331 | 1074353 | 427856 |
| rpl32-PpLuc(GC)-albumi n | 2870659 | 1252124 | 437208 |
| rpl32-PpLuc(GC)-rps23 | 4321886 | 911262 | 308560 |
| rpl32-PpLuc(GC)-rps13 | 4313724 | 1173245 | 373579 |
| rpl32-PpLuc(GC)-rpl26 | 4405685 | 1450797 | 455755 |
| rpl32-PpLuc(GC)-rps17 | 4661966 | 1138370 | 359931 |
| rpl32-PpLuc(GC)-rps18 | 4580492 | 1224085 | 329321 |
| rpl32-PpLuc(GC)-rps8 | 5043669 | 1255188 | 380314 |
| rpl32-PpLuc(GC)-Fau | 5495042 | 1277753 | 416901 |
| rpl32-PpLuc(GC)-rps13 | 4879517 | 1307823 | 361588 |
| rpl32-PpLuc(GC)-rpl1 1 | 4273583 | 1264210 | 436367 |
| rpl32-PpLuc(GC)-rpl38 | 4891163 | 1477135 | 464923 |

The results show that more Luciferase was expressed from mRNA comprising a 3'-UTR of murine ribosomal proteins compared to mRNA comprising the 3'-UTR of albumin, which has already been described to increase protein production.

5.5 The 3'-UTRs of murine ribosomal protein genes increase protein expression.

To investigate the effect of 3'-UTRs of murine ribosomal protein genes on protein expression from mRNA, mRNAs with different UTRs were synthesized: mRNAs contained the 3'-UTR of different murine ribosomal proteins (rpl23, uba52, rpl22l1, rpl36a, rps4x, rpl27, rpl3, rps23, rps13, rpl26, rps17, rps18, rps8, Fau, rps13, rpl11 and rpl38) and for comparison the 3'-UTR of albumin. Luciferase-encoding mRNAs were transfected into HeLa cells. Luciferase levels were measured at 24, 48, and 72 hours after transfection. (see following Table 5 and Figure 9).

Table 5:

| | RLU at 24 hours | RLU at 48 hours | RLU at 72 hours |
|---|---|---|---|
| rpl32-PpLuc(GC)-albumin | 1370770 | 383733 | 69772 |
| rpl32-PpLuc(GC)-rpl23 | 2860370 | 711666 | 78196 |
| rpl32-PpLuc(GC)-uba52 | 2954670 | 820157 | 97648 |
| rpl32-PpLuc(GC)-rpl22l1 | 3211757 | 822579 | 86780 |
| rpl32-PpLuc(GC)-rpl36a | 3198982 | 878820 | 115778 |
| rpl32-PpLuc(GC)-rps4x | 3282181 | 500040 | 45538 |
| rpl32-PpLuc(GC)-rpl27 | 2544927 | 533978 | 66691 |
| rpl32-PpLuc(GC)-rpl3 | 3081117 | 653787 | 83858 |
| rpl32-PpLuc(GC)-albumin | 1692963 | 397629 | 73285 |
| rpl32-PpLuc(GC-rps23 | 3828806 | 864761 | 94624 |
| rpl32-PpLuc(GC)-rps13 | 3627626 | 898377 | 135420 |
| rpl32-PpLuc(GC)-rpl26 | 3981814 | 839608 | 149750 |
| rpl32-PpLuc(GC)-rps17 | 4607487 | 1029902 | 182204 |
| rpl32-PpLuc(GC)-rps18 | 4071557 | 742337 | 101819 |
| rpl32-PpLuc(GC)-rps8 | 4459446 | 1032970 | 125825 |
| rpl32-PpLuc(GC)-Fau | 4486972 | 1066363 | 147843 |
| rpl32-PpLuc(GC)-rps13 | 4811136 | 955703 | 123026 |
| rpl32-PpLuc(GC)-rpl11 | 4405071 | 1231676 | 252711 |
| rpl32-PpLuc(GC)-rpl38 | 5485224 | 1023424 | 170059 |

The results show that more Luciferase was expressed from mRNA comprising a 3'-UTR of murine ribosomal proteins compared to mRNA comprising the 3'-UTR of albumin, which has already been described to increase protein production.

Items

1. An artificial nucleic acid molecule comprising

a. at least one open reading frame (ORF); and

b. at least one 3'-untranslated region element (3'-UTR element) comprising a nucleic acid sequence which is derived from the 3'-UTR of a ribosomal protein gene or from a variant of the 3'-UTR of a ribosomal protein gene.

2. The artificial nucleic acid molecule according to item 1, wherein the at least one 3'-UTR element enhances, stabilizes and/or prolongs protein production from said artificial nucleic acid molecule.

3. The artificial nucleic acid molecule according to item 1 or 2, wherein the 3'-UTR is heterologous to the ORF.

4. The artificial nucleic acid molecule according to any one of items 1 to 3, wherein the at least one 3'-UTR element comprises a nucleic acid sequence which is derived from the 3'-UTR of a eukaryotic ribosomal protein gene, preferably from the 3'-UTR of a vertebrate ribosomal protein gene, more preferably from the 3'-UTR of a mammalian ribosomal protein gene, even more preferably from the 3'-UTR of a primate ribosomal protein gene, in particular of a human ribosomal protein gene, or of a rodent ribosomal protein gene, in particular of a murine ribosomal protein gene.

5. The artificial nucleic acid molecule according to any one of items 1 to 4, wherein the open reading frame (ORF) does not encode a reporter gene or is not derived from a reporter gene, wherein the reporter gene is preferably not selected from group consisting of globin proteins (particularly beta-globin), luciferase protein, beta-glucuronidase (GUS) and GFP proteins or variants thereof, preferably not EGFP, or variants exhibiting at least 70% sequence identity to a globin protein, a luciferase protein, or a GFP protein.

6. The artificial nucleic acid molecule according to any one of items 1 to 5, wherein the open reading frame (ORF) does not encode a ribosomal protein, preferably does not encode an eukaryotic ribosomal protein.

7. The artificial nucleic acid molecule according to any one of items 1 to 6, wherein the open reading frame (ORF) does not encode a human or a plant ribosomal protein, in particular human ribosomal protein S6 (RPS6), human ribosomal protein L36a-like (RPL36AL) or Arabidopsis ribosomal protein S16 (RPS16).

8. The artificial nucleic acid molecule according to any one of items 1 to 7, wherein the open reading frame (ORF) does not encode ribosomal protein S6 (RPS6), ribosomal protein L36a-like (RPL36AL) or ribosomal protein S16 (RPS16).

9. The artificial nucleic acid molecule according to any one of items 1 to 8, wherein the 3'-UTR is not derived from a virus.

10. The artificial nucleic acid molecule according to any one of items 1 to 9, wherein the 3'-UTR, preferably the artificial nucleic acid molecule, does not comprise a poly(A) sequence or a polyadenylation signal,
or
wherein the 3'-UTR, preferably the artificial nucleic acid molecule, further comprises a poly(A) sequence and/or a polyadenylation signal.

11. The artificial nucleic acid molecule according to item 10, wherein the poly(A) sequence or the polyadenylation signal is located 3'-terminal to the 3'-UTR element.

12. The artificial nucleic acid molecule according to item 10 or 11, wherein the polyadenylation signal comprises the consensus sequence NN(U/T)ANA, with N = A or U, preferably AA(U/T)AAA or A(U/T)(U/T)AAA.

13. The artificial nucleic acid molecule according to any one of items 10 to 12, wherein the polyadenylation signal, preferably the consensus sequence NNUANA, is located less than about 50 nucleotides downstream of the 3'-terminus of the 3'-UTR element.

14. The artificial nucleic acid molecule according to any one of items 10 to 13, wherein the poly(A) sequence has a length of about 20 to about 300 adenine nucleotides, preferably of about 40 to about 200 adenine nucleotides, more preferably of about 50 to about 100 adenine nucleotides, even more preferably of about 60 to about 70 adenine nucleotides.

15. The artificial nucleic acid molecule according to any one of items 1 to 14, wherein the nucleic acid comprises an additional 5'-terminal element, preferably a 5'-UTR, a promoter, or a 5'-UTR and a promoter containing-sequence.

16. The artificial nucleic acid molecule according to item 15, wherein the 5'-UTR is a 5'-TOP UTR.

17. The artificial nucleic acid molecule according to item 15 or 16, wherein the 5'-UTR does not comprise a 5' TOP motif.

18. The artificial nucleic acid molecule according to any one of items 1 to 17, wherein the artificial nucleic acid molecule further comprises a 5'-UTR and wherein all of the 3'-UTR, the ORF and the 5'-UTR are heterologous to each other.

19. The artificial nucleic acid molecule according to any one of items 1 to 18, wherein the at least one 3'-UTR element comprises a nucleic acid sequence which is derived from the 3'-UTR of a sequence selected from the group consisting of ribosomal protein L9 (RPL9), ribosomal protein L3 (RPL3), ribosomal protein L4 (RPL4), ribosomal protein L5 (RPL5), ribosomal protein L6 (RPL6), ribosomal protein L7 (RPL7), ribosomal protein L7a (RPL7A), ribosomal protein L11 (RPL11), ribosomal protein L12 (RPL12), ribosomal protein L13 (RPL13), ribosomal protein L23 (RPL23), ribosomal protein L18 (RPL18), ribosomal protein L18a (RPL18A), ribosomal protein L19 (RPL19), ribosomal protein L21 (RPL21), ribosomal protein L22 (RPL22), ribosomal protein L23a (RPL23A), ribosomal protein L17 (RPL17), ribosomal protein L24 (RPL24), ribosomal protein L26 (RPL26), ribosomal protein L27 (RPL27), ribosomal protein L30 (RPL30), ribosomal protein L27a (RPL27A), ribosomal protein L28 (RPL28), ribosomal protein L29 (RPL29), ribosomal protein L31 (RPL31), ribosomal protein L32 (RPL32), ribosomal protein L35a (RPL35A), ribosomal protein L37 (RPL37), ribosomal protein L37a (RPL37A), ribosomal protein L38 (RPL38), ribosomal protein L39 (RPL39), ribosomal protein, large, P0 (RPLP0), ribosomal protein, large, P1 (RPLP1), ribosomal protein, large, P2 (RPLP2), ribosomal protein S3 (RPS3), ribosomal protein S3A (RPS3A), ribosomal protein S4, X- linked (RPS4X), ribosomal protein S4, Y-linked 1 (RPS4Y1), ribosomal protein S5 (RPS5), ribosomal protein S6 (RPS6), ribosomal protein S7 (RPS7), ribosomal protein S8 (RPS8), ribosomal protein S9 (RPS9), ribosomal protein S10 (RPS10), ribosomal protein S11 (RPS11), ribosomal protein S12 (RPS12), ribosomal protein S13 (RPS13), ribosomal protein S15 (RPS15), ribosomal protein S15a (RPS15A), ribosomal protein S16 (RPS16), ribosomal protein S19 (RPS19), ribosomal protein S20 (RPS20), ribosomal protein S21 (RPS21), ribosomal protein S23 (RPS23), ribosomal protein S25 (RPS25), ribosomal protein S26 (RPS26), ribosomal protein S27 (RPS27), ribosomal protein S27a (RPS27a), ribosomal protein S28 (RPS28), ribosomal protein S29 (RPS29), ribosomal protein L15 (RPL15), ribosomal protein S2 (RPS2), ribosomal protein L14 (RPL14), ribosomal protein S14 (RPS14), ribosomal protein L10 (RPL10), ribosomal protein L10a (RPL10A), ribosomal protein L35 (RPL35), ribosomal protein L13a (RPL13A), ribosomal protein L36 (RPL36), ribosomal protein L36a (RPL36A), ribosomal protein L41 (RPL41), ribosomal protein S18 (RPS18), ribosomal protein S24 (RPS24), ribosomal protein L8 (RPL8), ribosomal protein L34 (RPL34), ribosomal protein S17 (RPS17), ribosomal protein SA (RPSA), ubiquitin A-52 residue ribosomal protein fusion product 1 (UBA52), Finkel-Biskis-Reilly murine sarcoma virus (FBR-MuSV) ubiquitously expressed (FAU), ribosomal protein L22-like 1 (RPL22L1), ribosomal protein S17 (RPS17), ribosomal protein L39-like (RPL39L), ribosomal protein L10-like (RPL10L), ribosomal protein L36a-like (RPL36AL), ribosomal protein L3-like (RPL3L), ribosomal protein S27-like (RPS27L), ribosomal protein L26-like 1 (RPL26L1), ribosomal protein L7-like 1 (RPL7L1), ribosomal protein L13a pseudogene (RPL13AP), ribosomal protein L37a pseudogene 8 (RPL37AP8), ribosomal protein S10 pseudogene 5 (RPS10P5), ribosomal protein S26 pseudogene 11 (RPS26P11), ribosomal protein L39 pseudogene 5 (RPL39P5), ribosomal protein, large, P0 pseudogene 6 (RPLP0P6) and ribosomal protein L36 pseudogene 14 (RPL36P14).

20. The artificial nucleic acid molecule according to any one of items 1 to 19, wherein the at least one 3'-UTR element comprises or consists of a nucleic acid sequence which has an identity of at least about 1, 2, 3, 4, 5, 10, 15, 20, 30 or 40%, preferably of at least about 50%, preferably of at least about 60%, preferably of at least about 70%, more preferably of at least about 80%, more preferably of at least about 90%, even more preferably of at least about 95%, even more preferably of at least about 99% to a nucleic acid sequence selected from the group consisting of SEQ ID NOs:10 to 205 or wherein the at least one 3'-UTR element comprises or consists of a fragment of a nucleic acid sequence which has an identity of at least about 40%, preferably of at least about 50%, preferably of at least about 60%, preferably of at least about 70%, more preferably of at least about 80%, more preferably of at least about 90%, even more preferably of at least about 95%, even more preferably of at least about 99% to a nucleic acid sequence selected from the group consisting of SEQ ID NOs:10 to 205.

21. The artificial nucleic acid molecule according to item 20, wherein the fragment exhibits a length of between 3 and about 500 nucleotides, preferably of between 5 and about 150 nucleotides, more preferably of between 10 and 100 nucleotides, even more preferably of between 15 and 90, most preferably of between 20 and 70.

22. The artificial nucleic acid molecule according to any one of items 1 to 21, wherein the at least one 3'-UTR element exhibits a length of between 3 and about 500 nucleotides, preferably of between 5 and about 150 nucleotides, more preferably of between 10 and 100 nucleotides, even more preferably of between 15 and 90, most preferably of

between 20 and 70.

23. The artificial nucleic acid molecule according to any one of items 1 to 22, further comprising a 5'-cap structure, a poly(C) sequence, a histone stem-loop, and/or an IRES-motif.

24. The artificial nucleic acid molecule according to any one of items 1 to 23, wherein the histone stem-loop comprises a sequence according to SEQ ID NO:5.

25. The artificial nucleic acid molecule according to any one of items 1 to 24, wherein the artificial nucleic acid molecule, preferably the open reading frame, is at least partially G/C modified, preferably wherein the G/C content of the open reading frame is increased compared to the wild type open reading frame.

26. The artificial nucleic acid molecule according to any one of items 1 to 25, wherein the open reading frame comprises a codon-optimized region, preferably, wherein the open reading frame is codon-optimized.

27. The artificial nucleic acid molecule according to any one of items 1 to 26, which is an RNA, preferably an mRNA molecule.

28. A vector comprising

a. an open reading frame and/or a cloning site; and

b. at least one 3'-untranslated region element (3'-UTR element) comprising a nucleic acid sequence which is derived from the 3'-UTR of a ribosomal protein gene or from a variant of the 3'-UTR of a ribosomal protein gene.

29. The vector according to item 28, wherein the at least one 3'-UTR element comprises or consists of a nucleic acid sequence which is derived from the 3'-UTR of a eukaryotic ribosomal protein gene, preferably from the 3'-UTR of a vertebrate ribosomal protein gene, more preferably from the 3'-UTR of a mammalian ribosomal protein gene, even more preferably from the 3'-UTR of a primate ribosomal protein gene, in particular of a human ribosomal protein gene.

30. The vector according to item 28 or 29, wherein the open reading frame (ORF) does not encode a reporter gene or is not derived from a reporter gene, wherein the reporter gene is preferably not selected from group consisting of globin proteins (particularly beta-globin), luciferase protein, beta-glucuronidase (GUS) and GFP proteins or variants thereof, preferably not EGFP, or variants exhibiting at least 70% sequence identity to a globin protein, a luciferase protein, or a GFP protein.

31. The vector according to any one of items 28 to 30, wherein the open reading frame (ORF) does not encode human ribosomal protein S6 (RPS6), human ribosomal protein L36a-like (RPL36AL) or Arabidopsis ribosomal protein S16 (RPS16).

32. The vector according to any one of items 28 to 31, wherein the open reading frame (ORF) does not encode ribosomal protein S6 (RPS6), ribosomal protein L36a-like (RPL36AL) or ribosomal protein S16 (RPS16).

33. The vector according to any one of items 28 to 32, wherein the open reading frame (ORF) does not encode a ribosomal protein gene, preferably does not encode an eukaryotic ribosomal protein gene.

34. The vector to any one of items 28 to 33, wherein the 3'-UTR is not derived from a virus.

35. The vector according to any one of items 28 to 34, wherein the vector comprises an additional 5'-element, preferably a 5'-UTR, a promoter, or a 5'-UTR and a promoter containing-sequence.

36. The vector according to item 35, wherein the 5'-UTR is a 5'-TOP UTR.

37. The vector according to item 35 or 36, wherein the 5'-UTR does not comprise a 5' TOP motif.

38. The vector according to any one of items 28 to 37, wherein the 3'-UTR is heterologous to the ORF.

39. The vector according to any one of items 28 to 38, wherein the artificial nucleic acid molecule further comprises a 5'-UTR and wherein all of the 3'-UTR, the ORF and the 5'-UTR are heterologous to each other.

40. The vector according to any one of items 28 to 39, wherein the 3'-UTR, preferably the vector, does not comprise a poly(A) sequence or a polyadenylation signal,
or
wherein the 3'-UTR, preferably the vector, further comprises a poly(A) sequence and/or a polyadenylation signal.

41. The vector according to item 40, wherein the poly(A) sequence or the polyadenylation signal is located 3' of the 3'-UTR element.

42. The vector according to item 40 or 41, wherein the polyadenylation signal comprises the consensus sequence NN(U/T)ANA, with N = A or U, preferably AA(U/T)AAA or A(U/T)(U/T)AAA.

43. The vector according to any one of items 40 to 42, wherein the polyadenylation signal, preferably the consensus sequence NNUANA, is located less than about 50 nucleotides downstream of the 3'-end of the 3'-UTR element.

44. The vector according to any one of items 40 to 43, wherein the poly(A) sequence has a length of about 20 to about 300 adenine nucleotides, preferably of about 40 to about 200 adenine nucleotides, more preferably of about 50 to about 100 adenine nucleotides, even more preferably of about 60 to about 70 adenine nucleotides.

45. The vector according to any one of items 28 to 44, wherein the at least one 3'-UTR element comprises or consists of a nucleic acid sequence which is derived from the 3'-UTR of a sequence selected from the group consisting of ribosomal protein L9 (RPL9), ribosomal protein L3 (RPL3), ribosomal protein L4 (RPL4), ribosomal protein L5 (RPL5), ribosomal protein L6 (RPL6), ribosomal protein L7 (RPL7), ribosomal protein L7a (RPL7A), ribosomal protein L11 (RPL11), ribosomal protein L12 (RPL12), ribosomal protein L13 (RPL13), ribosomal protein L23 (RPL23), ribosomal protein L18 (RPL18), ribosomal protein L18a (RPL18A), ribosomal protein L19 (RPL19), ribosomal protein L21 (RPL21), ribosomal protein L22 (RPL22), ribosomal protein L23a (RPL23A), ribosomal protein L17 (RPL17), ribosomal protein L24 (RPL24), ribosomal protein L26 (RPL26), ribosomal protein L27 (RPL27), ribosomal protein L30 (RPL30), ribosomal protein L27a (RPL27A), ribosomal protein L28 (RPL28), ribosomal protein L29 (RPL29), ribosomal protein L31 (RPL31), ribosomal protein L32 (RPL32), ribosomal protein L35a (RPL35A), ribosomal protein L37 (RPL37), ribosomal protein L37a (RPL37A), ribosomal protein L38 (RPL38), ribosomal protein L39 (RPL39), ribosomal protein, large, P0 (RPLP0), ribosomal protein, large, P1 (RPLP1), ribosomal protein, large, P2 (RPLP2), ribosomal protein S3 (RPS3), ribosomal protein S3A (RPS3A), ribosomal protein S4, X- linked (RPS4X), ribosomal protein S4, Y-linked 1 (RPS4Y1), ribosomal protein S5 (RPS5), ribosomal protein S6 (RPS6), ribosomal protein S7 (RPS7), ribosomal protein S8 (RPS8), ribosomal protein S9 (RPS9), ribosomal protein S10 (RPS10), ribosomal protein S11 (RPS11), ribosomal protein S12 (RPS12), ribosomal protein S13 (RPS13), ribosomal protein S15 (RPS15), ribosomal protein S15a (RPS15A), ribosomal protein S16 (RPS16), ribosomal protein S19 (RPS19), ribosomal protein S20 (RPS20), ribosomal protein S21 (RPS21), ribosomal protein S23 (RPS23), ribosomal protein S25 (RPS25), ribosomal protein S26 (RPS26), ribosomal protein S27 (RPS27), ribosomal protein S27a (RPS27a), ribosomal protein S28 (RPS28), ribosomal protein S29 (RPS29), ribosomal protein L15 (RPL15), ribosomal protein S2 (RPS2), ribosomal protein L14 (RPL14), ribosomal protein S14 (RPS14), ribosomal protein L10 (RPL1 0), ribosomal protein L10a (RPL10A), ribosomal protein L35 (RPL35), ribosomal protein L13a (RPL13A), ribosomal protein L36 (RPL36), ribosomal protein L36a (RPL36A), ribosomal protein L41 (RPL41), ribosomal protein S18 (RPS18), ribosomal protein S24 (RPS24), ribosomal protein L8 (RPL8), ribosomal protein L34 (RPL34), ribosomal protein S17 (RPS17), ribosomal protein SA (RPSA), ubiquitin A-52 residue ribosomal protein fusion product 1 (UBA52), Finkel-Biskis-Reilly murine sarcoma virus (FBR-MuSV) ubiquitously expressed (FAU), ribosomal protein L22-like 1 (RPL22L1), ribosomal protein S17 (RPS17), ribosomal protein L39-like (RPL39L), ribosomal protein L10-like (RPL10L), ribosomal protein L36a-like (RPL36AL), ribosomal protein L3-like (RPL3L), ribosomal protein S27-like (RPS27L), ribosomal protein L26-like 1 (RPL26L1), ribosomal protein L7-like 1 (RPL7L1), ribosomal protein L13a pseudogene (RPL13AP), ribosomal protein L37a pseudogene 8 (RPL37AP8), ribosomal protein S10 pseudogene 5 (RPS10P5), ribosomal protein S26 pseudogene 11 (RPS26P11), ribosomal protein L39 pseudogene 5 (RPL39P5), ribosomal protein, large, P0 pseudogene 6 (RPLP0P6) and ribosomal protein L36 pseudogene 14 (RPL36P14).

46. The vector according to any one of items 28 to 45, wherein the at least one 3'-UTR element comprises or consists of a nucleic acid sequence which has an identity of at least about 40%, preferably of at least about 50%, preferably of at least about 60%, preferably of at least about 70%, more preferably of at least about 80%, more preferably of at least about 90%, even more preferably of at least about 95%, even more preferably of at least about 99% to a

sequence selected from the group consisting of SEQ ID NOs:10 to 205 or wherein the at least one 3'-UTR element comprises or consists of a fragment of a nucleic acid sequence which has an identity of at least about 40%, preferably of at least about 50%, preferably of at least about 60%, preferably of at least about 70%, more preferably of at least about 80%, more preferably of at least about 90%, even more preferably of at least about 95%, even more preferably of at least about 99% to a sequence selected from the group consisting of SEQ ID NOs:10 to 205.

47. The vector according to item 46, wherein the fragment exhibits a length of between 3 and about 500 nucleotides, preferably of between 5 and about 150 nucleotides, more preferably of between 10 and 100 nucleotides, even more preferably of between 15 and 90, most preferably of between 20 and 70.

48. The vector according to any one of items 28-47, wherein the at least one 3'-UTR element exhibits a length of between 3 and about 500 nucleotides, preferably of between 5 and about 150 nucleotides, more preferably of between 10 and 100 nucleotides, even more preferably of between 15 and 90, most preferably of between 20 and 70.

49. The vector according to any one of items 28 to 48, further comprising a poly(C) sequence, a histone stem-loop, and/or an IRES-motif.

50. The vector according to any one of items 28 to 49, which is at least partially G/C modified, preferably wherein the open reading frame is at least partially G/C modified, preferably wherein the G/C content of the open reading frame is increased compared to the wild type open reading frame.

51. The vector according to any one of items 28 to 50, wherein the open reading frame comprises a codon-optimized region, preferably wherein the open reading frame is codon-optimized.

52. The vector according to any one of items 28 to 51, which is a DNA vector.

53. The vector according to any one of items 28 to 52, which is a plasmid vector or a viral vector, preferably a plasmid vector.

54. The vector according to any one of items 28 to 53, which comprises an artificial nucleic acid molecule according to any one of items 1-25.

55. The vector according to any one of items 28 to 54, which is a circular molecule.

56. The vector according to item 55, wherein the poly(A) sequence, the poly(C) sequence, the histone stem loop or the 3'-UTR element of the coding strand is followed in 5'→3' direction by a restriction site for linearization of the circular vector molecule.

57. A cell comprising the artificial nucleic acid molecule according to any one of items 1-27 or the vector according to any one of items 28 to 56.

58. The cell according to item 57, which is a mammalian cell.

59. The cell according to item 57 or 58, which is a cell of a mammalian subject, preferably an isolated cell of a mammalian subject, preferably of a human subject.

60. A pharmaceutical composition comprising the artificial nucleic acid molecule according to any one of items 1-27, the vector according to any one of items 28 to 56, or the cell according to any one of items 57 to 59.

61. The pharmaceutical composition according to item 60, further comprising one or more pharmaceutically acceptable vehicles, diluents and/or excipients and/or one or more adjuvants.

62. The artificial nucleic acid molecule according to any one of items 1-27, the vector according to any one of items 28 to 56, the cell according to any one of items 57 to 59, or the pharmaceutical composition according to item 60 or 61 for use as a medicament.

63. The artificial nucleic acid molecule according to any one of items 1-27, the vector according to any one of items 28 to 56, the cell according to any one of items 57 to 59, or the pharmaceutical composition according to item 60

or 61 for use as a vaccine or for use in gene therapy.

64. A method for treating or preventing a disorder comprising administering the artificial nucleic acid molecule according to any one of items 1-27, the vector according to any one of items 28 to 56, the cell according to any one of items 57 to 59, or the pharmaceutical composition according to item 60 or 61 to a subject in need thereof.

65. A method of treating or preventing a disorder comprising transfection of a cell with an artificial nucleic acid molecule according to any one of items 1-27 or with the vector according to any one of items 28 to 56.

66. The method according to item 65, wherein transfection of a cell is performed *in vitro*/*ex vivo* and the transfected cell is administered to a subject in need thereof, preferably to a human patient.

67. The method according to item 66, wherein the cell which is to be transfected *in vitro* is an isolated cell of the subject, preferably of the human patient.

68. The method according to any one of items 64 to 67, which is a vaccination method or a gene therapy method.

69. A method for enhancing, stabilizing and/or prolonging protein production from an artificial nucleic acid molecule, preferably from an mRNA molecule or a vector, the method comprising the step of associating the nucleic acid molecule, preferably the mRNA molecule or the vector, with an 3'-UTR element, wherein the 3'-UTR element comprises or consists of a nucleic acid sequence which is derived from the 3'-UTR of a ribosomal protein gene.

70. Use of an 3'-UTR element for enhancing, stabilizing and/or prolonging protein production from a nucleic acid molecule, preferably from an mRNA molecule or a vector, wherein the 3'-UTR element comprises or consists of a nucleic acid sequence which is derived from the 3'-UTR of a ribosomal protein gene.

71. The method according to item 69 or the use according to item 70, wherein the 3'-UTR element comprises or consists of a nucleic acid sequence which is derived from the 3'-UTR of eukaryotic ribosomal protein gene, preferably from the 3'-UTR of a vertebrate ribosomal protein gene, more preferably from the 3'-UTR of a mammalian ribosomal protein gene, even more preferably from the 3'-UTR of a primate ribosomal protein gene, in particular of a human ribosomal protein gene.

72. The method or the use according to any one of items 69 to 71, wherein the 3'-UTR element comprises or consists of a nucleic acid sequence which is derived from the 3'-UTR of a sequence selected from the group consisting of ribosomal protein L9 (RPL9), ribosomal protein L3 (RPL3), ribosomal protein L4 (RPL4), ribosomal protein L5 (RPL5), ribosomal protein L6 (RPL6), ribosomal protein L7 (RPL7), ribosomal protein L7a (RPL7A), ribosomal protein L11 (RPL11), ribosomal protein L12 (RPL12), ribosomal protein L13 (RPL13), ribosomal protein L23 (RPL23), ribosomal protein L18 (RPL18), ribosomal protein L18a (RPL18A), ribosomal protein L19 (RPL19), ribosomal protein L21 (RPL21), ribosomal protein L22 (RPL22), ribosomal protein L23a (RPL23A), ribosomal protein L17 (RPL17), ribosomal protein L24 (RPL24), ribosomal protein L26 (RPL26), ribosomal protein L27 (RPL27), ribosomal protein L30 (RPL30), ribosomal protein L27a (RPL27A), ribosomal protein L28 (RPL28), ribosomal protein L29 (RPL29), ribosomal protein L31 (RPL31), ribosomal protein L32 (RPL32), ribosomal protein L35a (RPL35A), ribosomal protein L37 (RPL37), ribosomal protein L37a (RPL37A), ribosomal protein L38 (RPL38), ribosomal protein L39 (RPL39), ribosomal protein, large, P0 (RPLP0), ribosomal protein, large, P1 (RPLP1), ribosomal protein, large, P2 (RPLP2), ribosomal protein S3 (RPS3), ribosomal protein S3A (RPS3A), ribosomal protein S4, X- linked (RPS4X), ribosomal protein S4, Y-linked 1 (RPS4Y1), ribosomal protein S5 (RPS5), ribosomal protein S6 (RPS6), ribosomal protein S7 (RPS7), ribosomal protein S8 (RPS8), ribosomal protein S9 (RPS9), ribosomal protein S10 (RPS10), ribosomal protein S11 (RPS11), ribosomal protein S12 (RPS12), ribosomal protein S13 (RPS13), ribosomal protein S15 (RPS15), ribosomal protein S15a (RPS15A), ribosomal protein S16 (RPS16), ribosomal protein S19 (RPS19), ribosomal protein S20 (RPS20), ribosomal protein S21 (RPS21), ribosomal protein S23 (RPS23), ribosomal protein S25 (RPS25), ribosomal protein S26 (RPS26), ribosomal protein S27 (RPS27), ribosomal protein S27a (RPS27a), ribosomal protein S28 (RPS28), ribosomal protein S29 (RPS29), ribosomal protein L15 (RPL15), ribosomal protein S2 (RPS2), ribosomal protein L14 (RPL14), ribosomal protein S14 (RPS14), ribosomal protein L10 (RPL10), ribosomal protein L10a (RPL10A), ribosomal protein L35 (RPL35), ribosomal protein L13a (RPL13A), ribosomal protein L36 (RPL36), ribosomal protein L36a (RPL36A), ribosomal protein L41 (RPL41), ribosomal protein S18 (RPS18), ribosomal protein S24 (RPS24), ribosomal protein L8 (RPL8), ribosomal protein L34 (RPL34), ribosomal protein S17 (RPS17), ribosomal protein SA (RPSA), ubiquitin A-52 residue ribosomal protein fusion product 1 (UBA52), Finkel-Biskis-Reilly murine sarcoma virus (FBR-MuSV) ubiquitously expressed (FAU), ribosomal protein L22-like 1

(RPL22L1), ribosomal protein S17 (RPS17), ribosomal protein L39-like (RPL39L), ribosomal protein L10-like (RPL10L), ribosomal protein L36a-like (RPL36AL), ribosomal protein L3-like (RPL3L), ribosomal protein S27-like (RPS27L), ribosomal protein L26-like 1 (RPL26L1), ribosomal protein L7-like 1 (RPL7L1), ribosomal protein L13a pseudogene (RPL13AP), ribosomal protein L37a pseudogene 8 (RPL37AP8), ribosomal protein S10 pseudogene 5 (RPS10P5), ribosomal protein S26 pseudogene 11 (RPS26P11), ribosomal protein L39 pseudogene 5 (RPL39P5), ribosomal protein, large, P0 pseudogene 6 (RPLP0P6) and ribosomal protein L36 pseudogene 14 (RPL36P14).

73. The method or the use according to any one of items 69 to 72, wherein the 3'-UTR element comprises or consists of a nucleic acid sequence which has an identity of at least about 40%, preferably of at least about 50%, preferably of at least about 60%, preferably of at least about 70%, more preferably of at least about 80%, more preferably of at least about 90%, even more preferably of at least about 95%, even more preferably of at least about 99% to a sequence selected from the group consisting of SEQ ID NOs:10 to 205 or wherein the 3'-UTR element comprises or consists of a fragment of a nucleic acid sequence that has an identity of at least about 40%, preferably of at least about 50%, preferably of at least about 60%, preferably of at least about 70%, more preferably of at least about 80%, more preferably of at least about 90%, even more preferably of at least about 95%, even more preferably of at least about 99% to a sequence selected from the group consisting of SEQ ID NOs:10 to 205.

74. The method or the use according to item 73, wherein the fragment exhibits a length of between 3 and about 500 nucleotides, preferably of between 5 and about 150 nucleotides, more preferably of between 10 and 100 nucleotides, even more preferably of between 15 and 90, most preferably of between 20 and 70.

75. The method or the use according to any one of items 69 to 73, wherein the 3'-UTR element exhibits a length of between 3 and about 500 nucleotides, preferably of between 5 and about 150 nucleotides, more preferably of between 10 and 100 nucleotides, even more preferably of between 15 and 90, most preferably of between 20 and 70.

76. A kit or kit of parts comprising an artificial nucleic acid molecule according to any one of items 1-27, a vector according to any one of items 28 to 56, a cell according to any one of items 57 to 59, and/or a pharmaceutical composition according to item 60 or 61.

77. The kit according to item 76 further comprising instructions for use, cells for transfection, an adjuvant, a means for administration of the pharmaceutical composition, a pharmaceutically acceptable carrier and/or a pharmaceutically acceptable solution for dissolution or dilution of the artificial nucleic acid molecule, the vector, the cells or the pharmaceutical composition.

## Claims

1. An artificial nucleic acid molecule comprising

    a. at least one open reading frame (ORF); and
    b. at least one 3'-untranslated region element (3'-UTR element) comprising a nucleic acid sequence which is derived from the 3'-UTR of ribosomal protein gene S10 (RPS10), or from a variant of the 3'-UTR of ribosomal protein gene S10 (RPS10).

2. The artificial nucleic acid molecule according to claim 1, wherein the at least one 3'-UTR element enhances, stabilizes and/or prolongs protein production from said artificial nucleic acid molecule.

3. The artificial nucleic acid molecule according to claim 1 or 2, wherein the 3'-UTR is heterologous to the ORF.

4. The artificial nucleic acid molecule according to any one of claims 1 to 3, wherein the open reading frame (ORF) does not encode a reporter gene or is not derived from a reporter gene, wherein the reporter gene is preferably not selected from group consisting of globin proteins (particularly beta-globin), luciferase protein, beta-glucuronidase (GUS) and GFP proteins or variants thereof, preferably not EGFP, or variants exhibiting at least 70% sequence identity to a globin protein, a luciferase protein, or a GFP protein,
or
wherein the open reading frame (ORF) does not encode a ribosomal protein, preferably does not encode an eukaryotic ribosomal protein.

5. The artificial nucleic acid molecule according to any one of claims 1 to 4, wherein the 3'-UTR, preferably the artificial nucleic acid molecule, does not comprise a poly(A) sequence or a polyadenylation signal,
or
wherein the 3'-UTR, preferably the artificial nucleic acid molecule, further comprises a poly(A) sequence and/or a polyadenylation signal, wherein the poly(A) sequence or the polyadenylation signal is preferably located 3'-terminal to the 3'-UTR element,
and/or
wherein the polyadenylation signal preferably comprises the consensus sequence NN(U/T)ANA, with N = A or U, preferably AA(U/T)AAA or A(U/T)(U/T)AAA,
wherein the polyadenylation signal, preferably the consensus sequence NNUANA, is more preferably located less than about 50 nucleotides downstream of the 3'-terminus of the 3'-UTR element.

6. The artificial nucleic acid molecule according to any one of claims 1 to 5, wherein the nucleic acid comprises an additional 5'-terminal element, preferably a 5'-UTR, a promoter, or a 5'-UTR and a promoter containing-sequence, wherein the 5'-UTR is more preferably a 5'-TOP UTR,
wherein the 5'-UTR does more preferably not comprise a 5' TOP motif.

7. The artificial nucleic acid molecule according to any one of claims 1 to 6, wherein the artificial nucleic acid molecule further comprises a 5'-UTR and wherein all of the 3'-UTR, the ORF and the 5'-UTR are heterologous to each other.

8. The artificial nucleic acid molecule according to any one of claims 1 to 7, wherein the at least one 3'-UTR element comprises or consists of a nucleic acid sequence which has an identity of at least about 1, 2, 3, 4, 5, 10, 15, 20, 30

or 40%, preferably of at least about 50%, preferably of at least about 60%, preferably of at least about 70%, more preferably of at least about 80%, more preferably of at least about 90%, even more preferably of at least about 95%, even more preferably of at least about 99% to a nucleic acid sequence selected from the group consisting of SEQ ID NOs: 59 and 162, or the corresponding RNA sequence thereof or wherein the at least one 3'-UTR element comprises or consists of a fragment of a nucleic acid sequence which has an identity of at least about 40%, preferably of at least about 50%, preferably of at least about 60%, preferably of at least about 70%, more preferably of at least about 80%, more preferably of at least about 90%, even more preferably of at least about 95%, even more preferably of at least about 99% to a nucleic acid sequence selected from the group consisting of SEQ ID NOs: 59 or 162, or the corresponding RNA sequence thereof,
wherein the fragment preferably exhibits a length of between 3 and about 500 nucleotides, preferably of between 5 and about 150 nucleotides, more preferably of between 10 and 100 nucleotides, even more preferably of between 15 and 90, most preferably of between 20 and 70.

**9.** The artificial nucleic acid molecule according to any one of claims 1 to 8, further comprising a 5'-cap structure, a poly(C) sequence, a histone stem-loop, and/or an IRES-motif.

**10.** The artificial nucleic acid molecule according to any one of claims 1 to 9, wherein the histone stem-loop comprises a sequence according to SEQ ID NO:5,
and/or
wherein the artificial nucleic acid molecule, preferably the open reading frame, is at least partially G/C modified, preferably wherein the G/C content of the open reading frame is increased compared to the wild type open reading frame,
and/or
wherein the open reading frame comprises a codon-optimized region, preferably, wherein the open reading frame is codon-optimized.

**11.** The artificial nucleic acid molecule according to any one of claims 1 to 10, which is an RNA, preferably an mRNA molecule.

**12.** A vector comprising

a. an open reading frame and/or a cloning site; and
b. at least one 3'-untranslated region element (3'-UTR element) comprising a nucleic acid sequence which is derived from the 3'-UTR of ribosomal protein gene S10 (RPS10) or from a variant of the 3'-UTR of ribosomal protein gene S10 (RPS10).

**13.** The vector according to claim 12, which comprises an artificial nucleic acid molecule according to any one of claims 1-11.

**14.** The vector according to claims 12 or 13, which is a circular molecule,
wherein the poly(A) sequence, the poly(C) sequence, the histone stem loop or the 3'-UTR element of the coding strand is preferably followed in 5'→3' direction by a restriction site for linearization of the circular vector molecule.

**15.** A cell comprising the artificial nucleic acid molecule according to any one of claims 1-11 or the vector according to any one of claims 12 to 14,
which is preferably a mammalian cell,
more preferably of a cell of a human subject.

**16.** A pharmaceutical composition comprising the artificial nucleic acid molecule according to any one of claims 1-11, the vector according to any one of claims 12 to 14, or the cell according to claim 15,
optionally further comprising one or more pharmaceutically acceptable vehicles, diluents and/or excipients and/or one or more adjuvants.

**17.** The artificial nucleic acid molecule according to any one of claims 1-11, the vector according to any one of claims 12 to 14, the cell according to claim 15, or the pharmaceutical composition according to claim 16 for use as a medicament, preferably for use as a vaccine or for use in gene therapy.

**18.** A method for enhancing, stabilizing and/or prolonging protein production from an artificial nucleic acid molecule,

preferably from an mRNA molecule, according to any of claims 1 to 11, or a vector according to any of claims 12 to 14, the method comprising the step of associating the nucleic acid molecule, preferably the mRNA molecule or the vector, with an 3'-UTR element, wherein the 3'-UTR element comprises or consists of a nucleic acid sequence which is derived from the 3'-UTR of a ribosomal protein gene.

**19.** The method or the use according to claim 18, wherein the 3'-UTR element comprises or consists of a nucleic acid sequence which has an identity of at least about 40%, preferably of at least about 50%, preferably of at least about 60%, preferably of at least about 70%, more preferably of at least about 80%, more preferably of at least about 90%, even more preferably of at least about 95%, even more preferably of at least about 99% to a sequence selected from the group consisting of SEQ ID NOs: 59 and 162 or a corresponding RNA sequence thereof, or wherein the 3'-UTR element comprises or consists of a fragment of a nucleic acid sequence that has an identity of at least about 40%, preferably of at least about 50%, preferably of at least about 60%, preferably of at least about 70%, more preferably of at least about 80%, more preferably of at least about 90%, even more preferably of at least about 95%, even more preferably of at least about 99% to a sequence selected from the group consisting of SEQ ID NOs: 59 or 162 or a corresponding RNA sequence thereof.

**20.** A kit or kit of parts comprising an artificial nucleic acid molecule according to any one of claims 1-11, a vector according to any one of claims 12 to 14, a cell according to claim 15, and/or a pharmaceutical composition according to claim 16, optionally further comprising instructions for use, cells for transfection, an adjuvant, a means for administration of the pharmaceutical composition, a pharmaceutically acceptable carrier and/or a pharmaceutically acceptable solution for dissolution or dilution of the artificial nucleic acid molecule, the vector, the cells or the pharmaceutical composition.

rpl32 – PpLuc(GC) – A64 – C30 – histoneSL:

GGGGCGCTGCCTACGGAGGTGGCAGCCATCTCCTTCTCGGCATCAAGCTTGAGG*ATGGAG*
*GACGCCAAGAACATCAAGAAGGGCCCGGCGCCCTTCTACCCGCTGGAGGACGGGACCGCC*
*GGCGAGCAGCTCCACAAGGCCATGAAGCGGTACGCCCTGGTGCCGGGCACGATCGCCTTC*
*ACCGACGCCCACATCGAGGTCGACATCACCTACGCGGAGTACTTCGAGATGAGCGTGCGC*
*CTGGCCGAGGCCATGAAGCGGTACGGCCTGAACACCAACCACCGGATCGTGGTGTGCTCG*
*GAGAACAGCCTGCAGTTCTTCATGCCGGTGCTGGGCGCCCTCTTCATCGGCGTGGCCGTC*
*GCCCCGGCGAACGACATCTACAACGAGCGGGAGCTGCTGAACAGCATGGGGATCAGCCAG*
*CCGACCGTGGTGTTCGTGAGCAAGAAGGGCCTGCAGAAGATCCTGAACGTGCAGAAGAAG*
*CTGCCCATCATCCAGAAGATCATCATCATGGACAGCAAGACCGACTACCAGGGCTTCCAG*
*TCGATGTACACGTTCGTGACCAGCCACCTCCCGCCGGGCTTCAACGAGTACGACTTCGTC*
*CCGGAGAGCTTCGACCGGGACAAGACCATCGCCCTGATCATGAACAGCAGCGGCAGCACC*
*GGCCTGCCGAAGGGGGTGGCCCTGCCGCACCGGACCGCCTGCGTGCGCTTCTCGCACGCC*
*CGGGACCCCATCTTCGGCAACCAGATCATCCCGGACACCGCCATCCTGAGCGTGGTGCCG*
*TTCCACCACGGCTTCGGCATGTTCACGACCCTGGGCTACCTCATCTGCGGCTTCCGGGTG*
*GTCCTGATGTACCGGTTCGAGGAGGAGCTGTTCCTGCGGAGCCTGCAGGACTACAAGATC*
*CAGAGCGCGCTGCTCGTGCCGACCCTGTTCAGCTTCTTCGCCAAGAGCACCCTGATCGAC*
*AAGTACGACCTGTCGAACCTGCACGAGATCGCCAGCGGGGGCGCCCCGCTGAGCAAGGAG*
*GTGGGCGAGGCCGTGGCCAAGCGGTTCCACCTCCCGGGCATCCGCCAGGGCTACGGCCTG*
*ACCGAGACCACGAGCGCGATCCTGATCACCCCCGAGGGGGACGACAAGCCGGGCGCCGTG*
*GGCAAGGTGGTCCCGTTCTTCGAGGCCAAGGTGGTGGACCTGGACACCGGCAAGACCCTG*
*GGCGTGAACCAGCGGGGCGAGCTGTGCGTGCGGGGGCCGATGATCATGAGCGGCTACGTG*
*AACAACCCGGAGGCCACCAACGCCCTCATCGACAAGGACGGCTGGCTGCACAGCGGCGAC*
*ATCGCCTACTGGGACGAGGACGAGCACTTCTTCATCGTCGACCGGCTGAAGTCGCTGATC*
*AAGTACAAGGGCTACCAGGTGGCGCCGGCCGAGCTGGAGAGCATCCTGCTCCAGCACCCC*
*AACATCTTCGACGCCGGCGTGGCCGGGCTGCCGGACGACGACGCCGGCGAGCTGCCGGCC*
*GCGGTGGTGGTGCTGGAGCACGGCAAGACCATGACGGAGAAGGAGATCGTCGACTACGTG*
*GCCAGCCAGGTGACCACCGCCAAGAAGCTGCGGGGCGGCGTGGTGTTCGTGGACGAGGTC*
*CCGAAGGGCCTGACCGGGAAGCTCGACGCCCGGAAGATCCGCGAGATCCTGATCAAGGCC*
*AAGAAGGGCGGCAAGATCGCCGTGTAA*GACTAGTAGATCTAAAAAAAAAAAAAAAAAAAA
AAAAAAAAAAAAAAAAAAAAAAAAAAAAAAAAAAAAAAAAAAAATGCATCCCCCCCCCCC
CCCCCCCCCCCCCCCCCCCAAAGGCTCTTTTCAGAGCCACCAGAATT

Fig. 1

rpl32 – PpLuc(GC) – ag – A64 – C30 – histoneSL:

GGGGCGCTGCCTACGGAGGTGGCAGCCATCTCCTTCTCGGCATCAAGCTTGAGGATGGAG
GACGCCAAGAACATCAAGAAGGGCCCGGCGCCCTTCTACCCGCTGGAGGACGGGACCGCC
GGCGAGCAGCTCCACAAGGCCATGAAGCGGTACGCCCTGGTGCCGGGCACGATCGCCTTC
ACCGACGCCCACATCGAGGTCGACATCACCTACGCGGAGTACTTCGAGATGAGCGTGCGC
CTGGCCGAGGCCATGAAGCGGTACGGCCTGAACACCAACCACCGGATCGTGGTGTGCTCG
GAGAACAGCCTGCAGTTCTTCATGCCGGTGCTGGGCGCCCTCTTCATCGGCGTGGCCGTC
GCCCCGGCGAACGACATCTACAACGAGCGGGAGCTGCTGAACAGCATGGGGATCAGCCAG
CCGACCGTGGTGTTCGTGAGCAAGAAGGGCCTGCAGAAGATCCTGAACGTGCAGAAGAAG
CTGCCCATCATCCAGAAGATCATCATCATGGACAGCAAGACCGACTACCAGGGCTTCCAG
TCGATGTACACGTTCGTGACCAGCCACCTCCCGCCGGGCTTCAACGAGTACGACTTCGTC
CCGGAGAGCTTCGACCGGGACAAGACCATCGCCCTGATCATGAACAGCAGCGGCAGCACC
GGCCTGCCGAAGGGGGTGGCCCTGCCGCACCGGACCGCCTGCGTGCGCTTCTCGCACGCC
CGGGACCCCATCTTCGGCAACCAGATCATCCCGGACACCGCCATCCTGAGCGTGGTGCCG
TTCCACCACGGCTTCGGCATGTTCACGACCCTGGGCTACCTCATCTGCGGCTTCCGGGTG
GTCCTGATGTACCGGTTCGAGGAGGAGCTGTTCCTGCGGAGCCTGCAGGACTACAAGATC
CAGAGCGCGCTGCTCGTGCCGACCCTGTTCAGCTTCTTCGCCAAGAGCACCCTGATCGAC
AAGTACGACCTGTCGAACCTGCACGAGATCGCCAGCGGGGGCGCCCCGCTGAGCAAGGAG
GTGGGCGAGGCCGTGGCCAAGCGGTTCCACCTCCCGGGCATCCGCCAGGGCTACGGCCTG
ACCGAGACCACGAGCGCGATCCTGATCACCCCCGAGGGGGACGACAAGCCGGGCGCCGTG
GGCAAGGTGGTCCCGTTCTTCGAGGCCAAGGTGGTGGACCTGGACACCGGCAAGACCCTG
GGCGTGAACCAGCGGGGCGAGCTGTGCGTGCGGGGGCCGATGATCATGAGCGGCTACGTG
AACAACCCGGAGGCCACCAACGCCCTCATCGACAAGGACGGCTGGCTGCACAGCGGCGAC
ATCGCCTACTGGGACGAGGACGAGCACTTCTTCATCGTCGACCGGCTGAAGTCGCTGATC
AAGTACAAGGGCTACCAGGTGGCGCCGGCCGAGCTGGAGAGCATCCTGCTCCAGCACCCC
AACATCTTCGACGCCGGCGTGGCCGGGCTGCCGGACGACGACGCCGGCGAGCTGCCGGCC
GCGGTGGTGGTGCTGGAGCACGGCAAGACCATGACGGAGAAGGAGATCGTCGACTACGTG
GCCAGCCAGGTGACCACCGCCAAGAAGCTGCGGGGCGGCGTGGTGTTCGTGGACGAGGTC
CCGAAGGGCCTGACCGGGAAGCTCGACGCCCGGAAGATCCGCGAGATCCTGATCAAGGCC
AAGAAGGGCGGCAAGATCGCCGTGTAAGACTAGTTATAAGACTGACTAGCCCGATGGGCC
TCCCAACGGGCCCTCCTCCCCTCCTTGCACCGAGATTAATAGATCTAAAAAAAAAAAAAA
AAAAAAAAAAAAAAAAAAAAAAAAAAAAAAAAAAAAAAAAAAAAAAAAAATGCATCCCCC
CCCCCCCCCCCCCCCCCCCCCCCCCAAAGGCTCTTTTCAGAGCCACCAGAATT

Fig. 2

rpl32 – PpLuc(GC) – rps9 – A64 – C30 – histoneSL ):

GGGGCGCTGCCTACGGAGGTGGCAGCCATCTCCTTCTCGGCATCAAGCTTGAGG*ATGGAG*
*GACGCCAAGAACATCAAGAAGGGCCCGGCGCCCTTCTACCCGCTGGAGGACGGGACCGCC*
*GGCGAGCAGCTCCACAAGGCCATGAAGCGGTACGCCCTGGTGCCGGGCACGATCGCCTTC*
*ACCGACGCCCACATCGAGGTCGACATCACCTACGCGGAGTACTTCGAGATGAGCGTGCGC*
*CTGGCCGAGGCCATGAAGCGGTACGGCCTGAACACCAACCACCGGATCGTGGTGTGCTCG*
*GAGAACAGCCTGCAGTTCTTCATGCCGGTGCTGGGCGCCCTCTTCATCGGCGTGGCCGTC*
*GCCCCGGCGAACGACATCTACAACGAGCGGGAGCTGCTGAACAGCATGGGGATCAGCCAG*
*CCGACCGTGGTGTTCGTGAGCAAGAAGGGCCTGCAGAAGATCCTGAACGTGCAGAAGAAG*
*CTGCCCATCATCCAGAAGATCATCATCATGGACAGCAAGACCGACTACCAGGGCTTCCAG*
*TCGATGTACACGTTCGTGACCAGCCACCTCCCGCCGGGCTTCAACGAGTACGACTTCGTC*
*CCGGAGAGCTTCGACCGGGACAAGACCATCGCCCTGATCATGAACAGCAGCGGCAGCACC*
*GGCCTGCCGAAGGGGGTGGCCCTGCCGCACCGGACCGCCTGCGTGCGCTTCTCGCACGCC*
*CGGGACCCCATCTTCGGCAACCAGATCATCCCGGACACCGCCATCCTGAGCGTGGTGCCG*
*TTCCACCACGGCTTCGGCATGTTCACGACCCTGGGCTACCTCATCTGCGGCTTCCGGGTG*
*GTCCTGATGTACCGGTTCGAGGAGGAGCTGTTCCTGCGGAGCCTGCAGGACTACAAGATC*
*CAGAGCGCGCTGCTCGTGCCGACCCTGTTCAGCTTCTTCGCCAAGAGCACCCTGATCGAC*
*AAGTACGACCTGTCGAACCTGCACGAGATCGCCAGCGGGGGCGCCCCGCTGAGCAAGGAG*
*GTGGGCGAGGCCGTGGCCAAGCGGTTCCACCTCCCGGGCATCCGCCAGGGCTACGGCCTG*
*ACCGAGACCACGAGCGCGATCCTGATCACCCCCGAGGGGGACGACAAGCCGGGCGCCGTG*
*GGCAAGGTGGTCCCGTTCTTCGAGGCCAAGGTGGTGGACCTGGACACCGGCAAGACCCTG*
*GGCGTGAACCAGCGGGGCGAGCTGTGCGTGCGGGGGCCGATGATCATGAGCGGCTACGTG*
*AACAACCCGGAGGCCACCAACGCCCTCATCGACAAGGACGGCTGGCTGCACAGCGGCGAC*
*ATCGCCTACTGGGACGAGGACGAGCACTTCTTCATCGTCGACCGGCTGAAGTCGCTGATC*
*AAGTACAAGGGCTACCAGGTGGCGCCGGCCGAGCTGGAGAGCATCCTGCTCCAGCACCCC*
*AACATCTTCGACGCCGGCGTGGCCGGGCTGCCGGACGACGACGCCGGCGAGCTGCCGGCC*
*GCGGTGGTGGTGCTGGAGCACGGCAAGACCATGACGGAGAAGGAGATCGTCGACTACGTG*
*GCCAGCCAGGTGACCACCGCCAAGAAGCTGCGGGGCGGCGTGGTGTTCGTGGACGAGGTC*
*CCGAAGGGCCTGACCGGGAAGCTCGACGCCCGGAAGATCCGCGAGATCCTGATCAAGGCC*
*AAGAAGGGCGGCAAGATCGCCGTGTAA*GACTAGTGTCCACCTGTCCCTCCTGGGCTGCTG
GATTGTCTCGTTTTCCTGCCAAATAAACAGGATCAGCGCTTTACAGATCTAAAAAAAAAA
AAAAAAAAAAAAAAAAAAAAAAAAAAAAAAAAAAAAAAAAAAAAAAAAAAAAAATGCATC
CCCCCCCCCCCCCCCCCCCCCCCCCCCCCCAAAGGCTCTTTTCAGAGCCACCAGAATT

Fig. 3

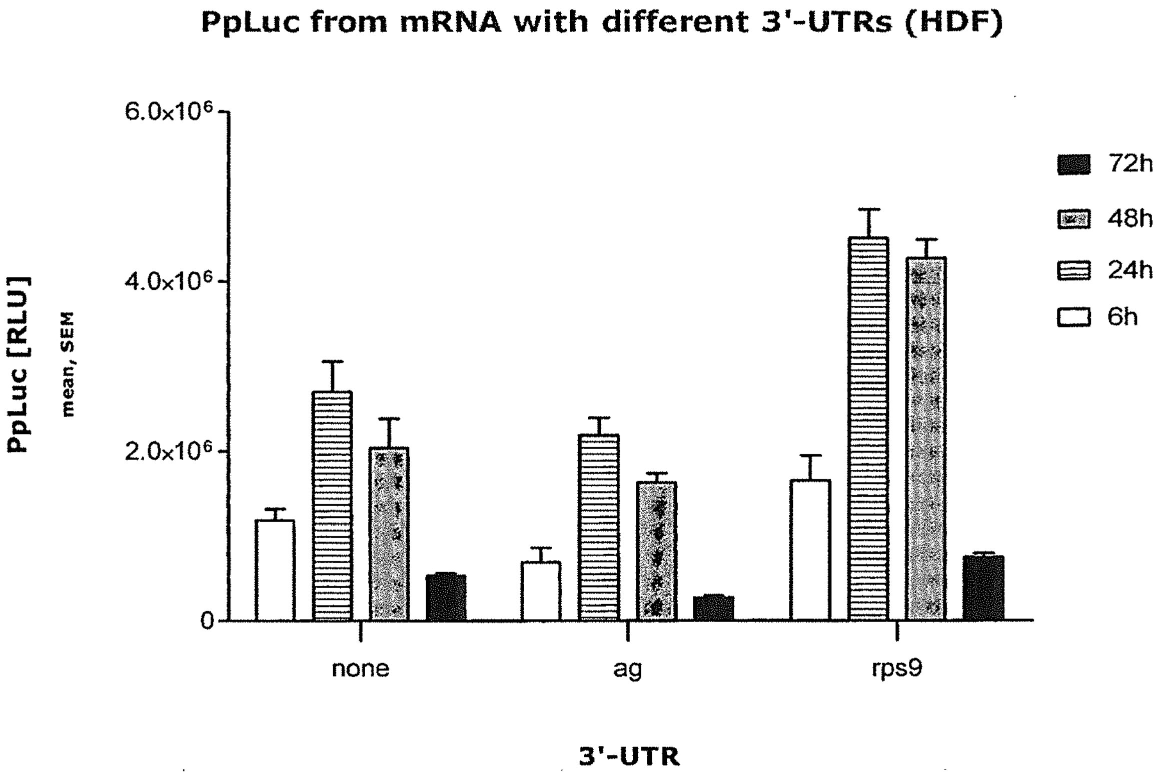
PpLuc from mRNA with different 3'-UTRs (HDF)
PpLuc [RLU]
mean, SEM
6.0x10^6
4.0x10^6
2.0x10^6
0
none
ag
rps9
3'-UTR
72h
48h
24h
6h

Fig. 4

rpl32 – PpLuc(GC) – albumin7 – A64 – C30 – histoneSL

GGGGCGCTGCCTACGGAGGTGGCAGCCATCTCCTTCTCGGCATCAAGCTTGAGGATGGAG
GACGCCAAGAACATCAAGAAGGGCCCGGCGCCCTTCTACCCGCTGGAGGACGGGACCGCC
GGCGAGCAGCTCCACAAGGCCATGAAGCGGTACGCCCTGGTGCCGGGCACGATCGCCTTC
ACCGACGCCCACATCGAGGTCGACATCACCTACGCGGAGTACTTCGAGATGAGCGTGCGC
CTGGCCGAGGCCATGAAGCGGTACGGCCTGAACACCAACCACCGGATCGTGGTGTGCTCG
GAGAACAGCCTGCAGTTCTTCATGCCGGTGCTGGGCGCCCTCTTCATCGGCGTGGCCGTC
GCCCCGGCGAACGACATCTACAACGAGCGGGAGCTGCTGAACAGCATGGGGATCAGCCAG
CCGACCGTGGTGTTCGTGAGCAAGAAGGGCCTGCAGAAGATCCTGAACGTGCAGAAGAAG
CTGCCCATCATCCAGAAGATCATCATCATGGACAGCAAGACCGACTACCAGGGCTTCCAG
TCGATGTACACGTTCGTGACCAGCCACCTCCCGCCGGGCTTCAACGAGTACGACTTCGTC
CCGGAGAGCTTCGACCGGGACAAGACCATCGCCCTGATCATGAACAGCAGCGGCAGCACC
GGCCTGCCGAAGGGGGTGGCCCTGCCGCACCGGACCGCCTGCGTGCGCTTCTCGCACGCC
CGGGACCCCATCTTCGGCAACCAGATCATCCCGGACACCGCCATCCTGAGCGTGGTGCCG
TTCCACCACGGCTTCGGCATGTTCACGACCCTGGGCTACCTCATCTGCGGCTTCCGGGTG
GTCCTGATGTACCGGTTCGAGGAGGAGCTGTTCCTGCGGAGCCTGCAGGACTACAAGATC
CAGAGCGCGCTGCTCGTGCCGACCCTGTTCAGCTTCTTCGCCAAGAGCACCCTGATCGAC
AAGTACGACCTGTCGAACCTGCACGAGATCGCCAGCGGGGGCGCCCCGCTGAGCAAGGAG
GTGGGCGAGGCCGTGGCCAAGCGGTTCCACCTCCCGGGCATCCGCCAGGGCTACGGCCTG
ACCGAGACCACGAGCGCGATCCTGATCACCCCCGAGGGGGACGACAAGCCGGGCGCCGTG
GGCAAGGTGGTCCCGTTCTTCGAGGCCAAGGTGGTGGACCTGGACACCGGCAAGACCCTG
GGCGTGAACCAGCGGGGCGAGCTGTGCGTGCGGGGGCCGATGATCATGAGCGGCTACGTG
AACAACCCGGAGGCCACCAACGCCCTCATCGACAAGGACGGCTGGCTGCACAGCGGCGAC
ATCGCCTACTGGGACGAGGACGAGCACTTCTTCATCGTCGACCGGCTGAAGTCGCTGATC
AAGTACAAGGGCTACCAGGTGGCGCCGGCCGAGCTGGAGAGCATCCTGCTCCAGCACCCC
AACATCTTCGACGCCGGCGTGGCCGGGCTGCCGGACGACGACGCCGGCGAGCTGCCGGCC
GCGGTGGTGGTGCTGGAGCACGGCAAGACCATGACGGAGAAGGAGATCGTCGACTACGTG
GCCAGCCAGGTGACCACCGCCAAGAAGCTGCGGGGCGGCGTGGTGTTCGTGGACGAGGTC
CCGAAGGGCCTGACCGGGAAGCTCGACGCCCGGAAGATCCGCGAGATCCTGATCAAGGCC
AAGAAGGGCGGCAAGATCGCCGTGTAAGACTAGTGCATCACATTTAAAAGCATCTCAGCC
TACCATGAGAATAAGAGAAAGAAAATGAAGATCAATAGCTTATTCATCTCTTTTTCTTTT
TCGTTGGTGTAAAGCCAACACCCTGTCTAAAAAACATAAATTTCTTTAATCATTTTGCCT
CTTTTCTCTGTGCTTCAATTAATAAAAAATGGAAAGAACCTAGATCTAAAAAAAAAAAAA
AAAAAAAAAAAAAAAAAAAAAAAAAAAAAAAAAAAAAAAAAAAAAAAAAAATGCATCCCC
CCCCCCCCCCCCCCCCCCCCCCCCCCAAAGGCTCTTTTCAGAGCCACCAGAATT

Fig. 5

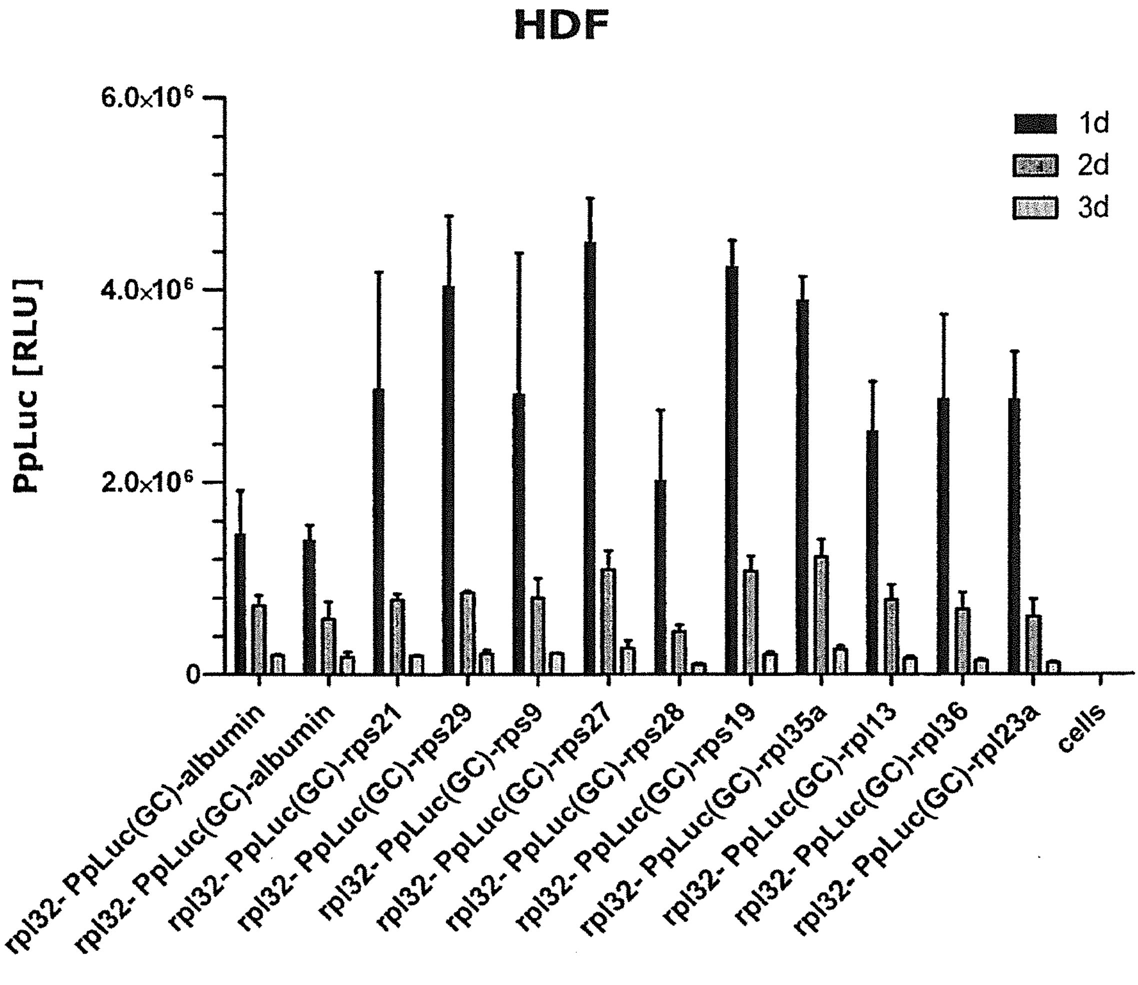
HDF
PpLuc [RLU]
6.0×10⁶
4.0×10⁶
2.0×10⁶
0
1d
2d
3d
rpl32- PpLuc(GC)-albumin
rpl32- PpLuc(GC)-albumin
rpl32- PpLuc(GC)-rps21
rpl32- PpLuc(GC)-rps29
rpl32- PpLuc(GC)-rps9
rpl32- PpLuc(GC)-rps27
rpl32- PpLuc(GC)-rps28
rpl32- PpLuc(GC)-rps19
rpl32- PpLuc(GC)-rpl35a
rpl32- PpLuc(GC)-rpl13
rpl32- PpLuc(GC)-rpl36
rpl32- PpLuc(GC)-rpl23a
cells

Fig. 6

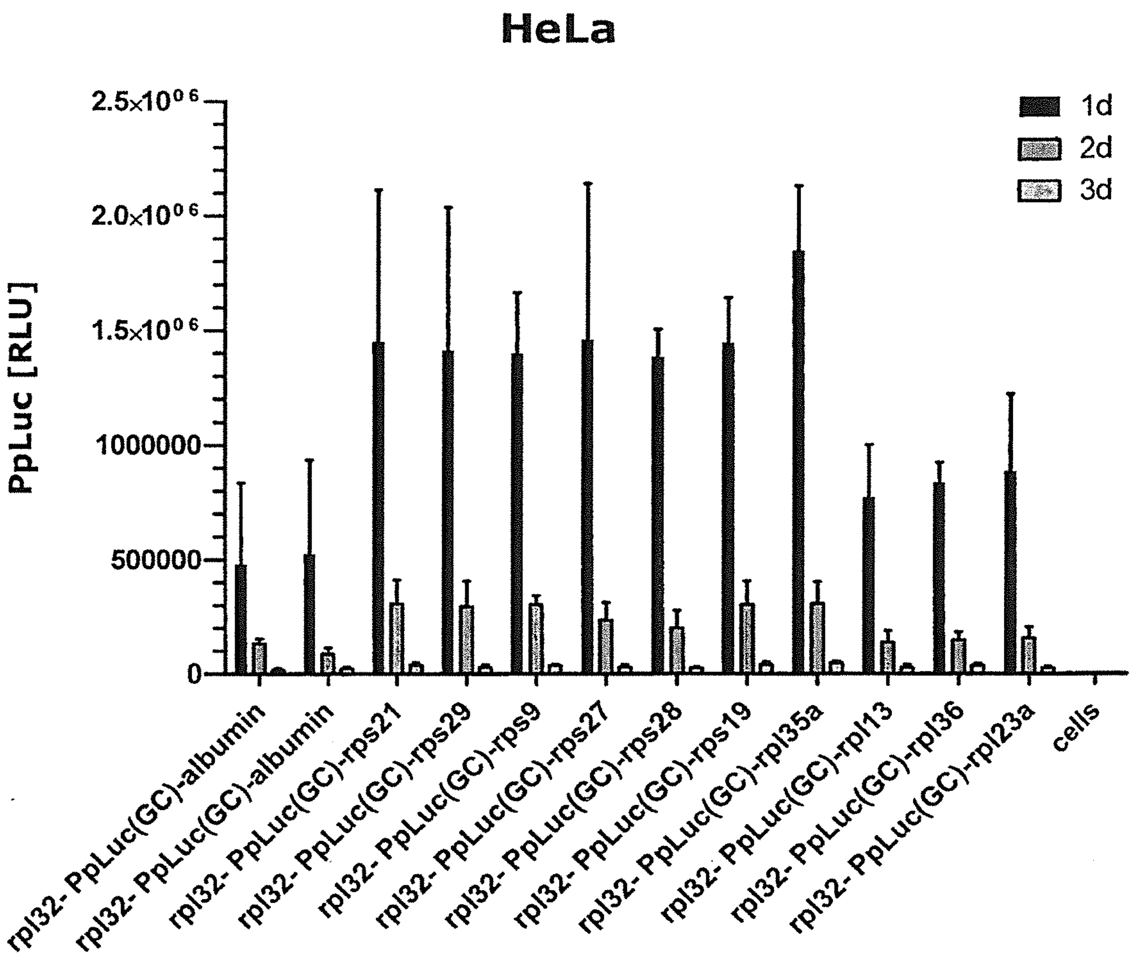
HeLa
PpLuc [RLU]
2.5×10⁰⁶
2.0×10⁰⁶
1.5×10⁰⁶
1000000
500000
0
1d
2d
3d
rpl32- PpLuc(GC)-albumin
rpl32- PpLuc(GC)-albumin
rpl32- PpLuc(GC)-rps21
rpl32- PpLuc(GC)-rps29
rpl32- PpLuc(GC)-rps9
rpl32- PpLuc(GC)-rps27
rpl32- PpLuc(GC)-rps28
rpl32- PpLuc(GC)-rps19
rpl32- PpLuc(GC)-rpl35a
rpl32- PpLuc(GC)-rpl13
rpl32- PpLuc(GC)-rpl36
rpl32- PpLuc(GC)-rpl23a
cells

Fig. 7

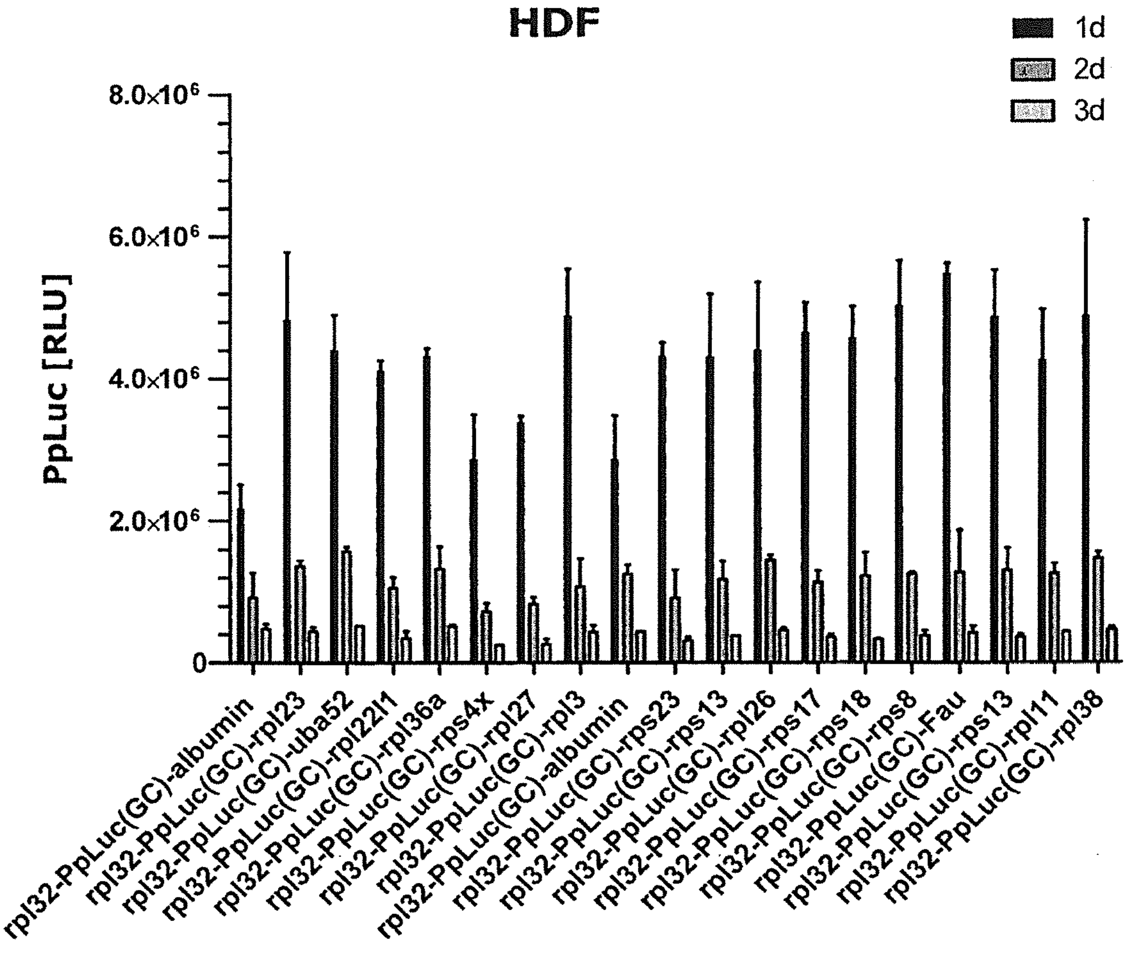
HDF
1d
2d
3d
PpLuc [RLU]
8.0×10⁶
6.0×10⁶
4.0×10⁶
2.0×10⁶
0
rpl32-PpLuc(GC)-albumin
rpl32-PpLuc(GC)-rpl23
rpl32-PpLuc(GC)-uba52
rpl32-PpLuc(GC)-rpl22l1
rpl32-PpLuc(GC)-rpl36a
rpl32-PpLuc(GC)-rps4x
rpl32-PpLuc(GC)-rpl27
rpl32-PpLuc(GC)-rpl3
rpl32-PpLuc(GC)-albumin
rpl32-PpLuc(GC)-rps23
rpl32-PpLuc(GC)-rps13
rpl32-PpLuc(GC)-rpl26
rpl32-PpLuc(GC)-rps17
rpl32-PpLuc(GC)-rps18
rpl32-PpLuc(GC)-rps8
rpl32-PpLuc(GC)-Fau
rpl32-PpLuc(GC)-rps13
rpl32-PpLuc(GC)-rpl11
rpl32-PpLuc(GC)-rpl38

Fig. 8

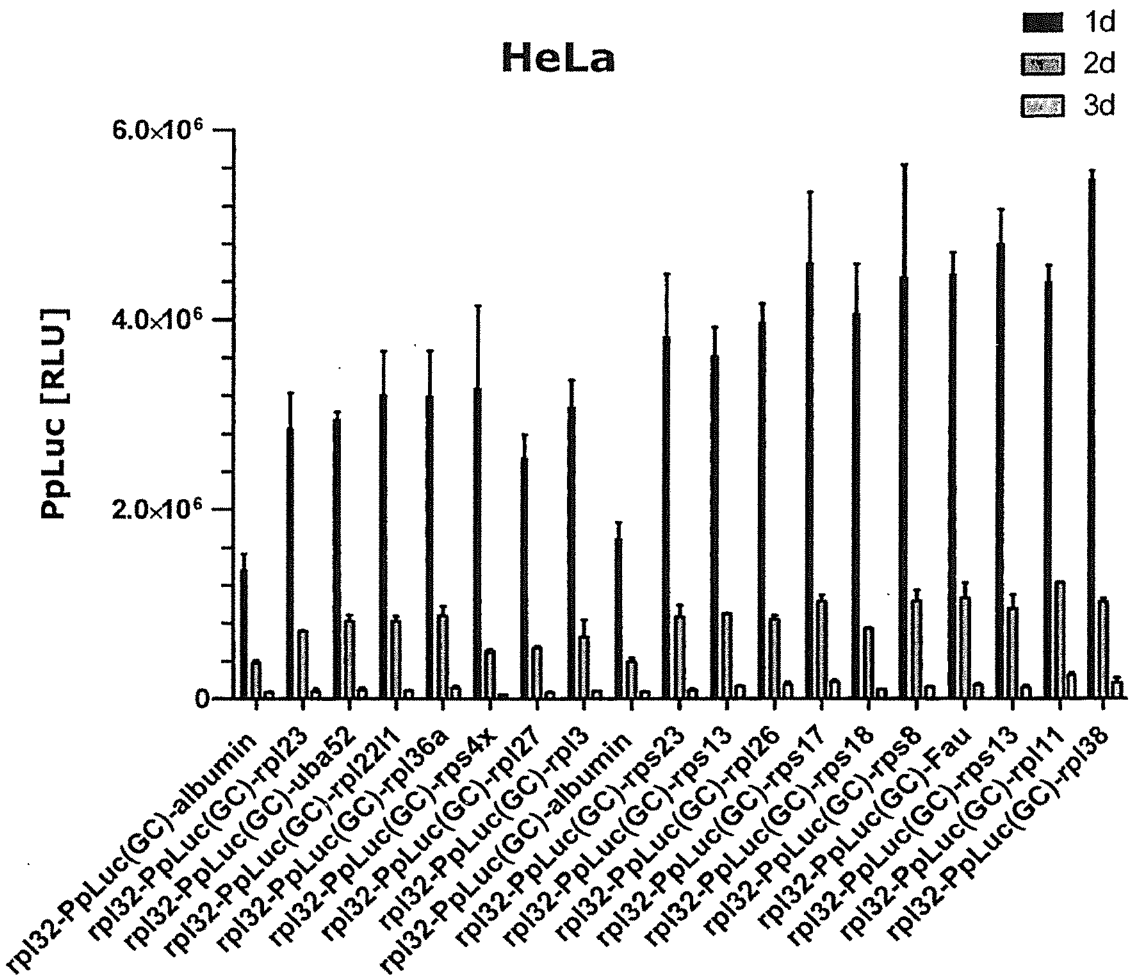
HeLa
1d
2d
3d
PpLuc [RLU]
6.0×10⁶
4.0×10⁶
2.0×10⁶
0
rpl32-PpLuc(GC)-albumin
rpl32-PpLuc(GC)-rpl23
rpl32-PpLuc(GC)-uba52
rpl32-PpLuc(GC)-rpl22l1
rpl32-PpLuc(GC)-rpl36a
rpl32-PpLuc(GC)-rps4x
rpl32-PpLuc(GC)-rpl27
rpl32-PpLuc(GC)-rpl3
rpl32-PpLuc(GC)-albumin
rpl32-PpLuc(GC)-rps23
rpl32-PpLuc(GC)-rps13
rpl32-PpLuc(GC)-rpl26
rpl32-PpLuc(GC)-rps17
rpl32-PpLuc(GC)-rps18
rpl32-PpLuc(GC)-rps8
rpl32-PpLuc(GC)-Fau
rpl32-PpLuc(GC)-rps13
rpl32-PpLuc(GC)-rpl11
rpl32-PpLuc(GC)-rpl38

Fig. 9

Europäisches Patentamt
European Patent Office
Office européen des brevets

# EUROPEAN SEARCH REPORT

Application Number
EP 20 20 2943

| Category | Citation of document with indication, where appropriate, of relevant passages | Relevant to claim | CLASSIFICATION OF THE APPLICATION (IPC) |
|---|---|---|---|
| X | WO 2013/102155 A1 (CELL MACHINES INC [US]; KELLY JAMES [US]) 4 July 2013 (2013-07-04)<br>* the whole document, in particular the claims and p. 47, 4th paragraph *<br>-& Gsn:Baq20674: "Human RPS10 gene, SEQ: 46",<br>,<br>4 July 2013 (2013-07-04), XP055795782,<br>Retrieved from the Internet:<br>URL:ibis.internal.epo.org/exam/dbfetch.jsp?id=GSN:BAQ20674<br>[retrieved on 2021-04-15]<br>----- | 1,2,4,5, 8-20 | INV.<br>C12N15/67<br>C12N15/85 |
| X | Em_std:Bc071946: "Homo sapiens ribosomal protein S10, mRNA",<br>,<br>16 June 2004 (2004-06-16), XP055795772,<br>Retrieved from the Internet:<br>URL:ibis.internal.epo.org/exam/dbfetch.jsp?id=EM_STD:BC071946<br>[retrieved on 2021-04-15]<br>* see sequence *<br>----- | 1,2,4-6, 8-16, 18-20 | |
| X | Em_est:Bq548819: "ik92a03.y1 Human insulinoma Homo sapiens cDNA clone IMAGE:6027916 5' similar to SW:RS10_HUMAN P46783 40S RIBOSOMAL PROTEIN S10. [1] ; mRNA sequence",<br>,<br>18 June 2002 (2002-06-18), XP055795769,<br>Retrieved from the Internet:<br>URL:ibis.internal.epo.org/exam/dbfetch.jsp?id=EM_EST:BQ548819<br>[retrieved on 2021-04-15]<br>* see sequence *<br>-----<br>-/-- | 1-6, 8-16, 18-20 | TECHNICAL FIELDS SEARCHED (IPC)<br>C12N |

1 The present search report has been drawn up for all claims

| Place of search | Date of completion of the search | Examiner |
|---|---|---|
| Munich | 18 May 2021 | Bassias, Ioannis |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 20 20 2943

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | Em_est:Cb126201: "K-Est0175092 L5hlk1s1 Homo Sapiens Cdna Clone L5hlk1s1-24-H07 5', Mrna Sequence", , 30 January 2003 (2003-01-30), XP055796325, Retrieved from the Internet: URL:http://ibis.internal.epo.org/exam/dbfetch.jsp?id=EM_EST:CB126201 [retrieved on 2021-04-16] * see sequence * ----- | 1-6, 8-16, 18-20 | |
| X | Em_std:Bc003853: "Mus musculus ribosomal protein S10, mRNA (cDNA clone MGC:6433 IMAGE:3601013), complete cds", , 17 March 2001 (2001-03-17), XP055796330, Retrieved from the Internet: URL:http://ibis.internal.epo.org/exam/dbfetch.jsp?id=EM_STD:BC003853 [retrieved on 2021-04-16] * see sequence * ----- | 1,2,4-6, 8-16, 18-20 | TECHNICAL FIELDS SEARCHED (IPC) |
| X | Em_est:Ck594582: "lai77d05.y2 Gastric Epithelial Progenitor 2 Mus musculus cDNA 5' similar to SW:RS10_HUMAN P46783 40S RIBOSOMAL PROTEIN S10. [1], mRNA sequence", , 21 January 2004 (2004-01-21), XP055795833, Retrieved from the Internet: URL:ibis.internal.epo.org/exam/dbfetch.jsp?id=EM_EST:CK594582 [retrieved on 2021-04-15] * see sequence * ----- -/-- | 1-6, 8-16, 18-20 | |

The present search report has been drawn up for all claims

1

| Place of search | Date of completion of the search | Examiner |
|---|---|---|
| Munich | 18 May 2021 | Bassias, Ioannis |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 20 20 2943

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | Em_est:Ck428771: "laj16c04.y1 Gastric Epithelial Progenitor 2 Mus musculus cDNA 5' similar to SW:RS10_HUMAN P46783 40S RIBOSOMAL PROTEIN S10. [1], mRNA sequence", , 7 January 2004 (2004-01-07), XP055795841, Retrieved from the Internet: URL:ibis.internal.epo.org/exam/dbfetch.jsp?id=EM_EST:CK428771 [retrieved on 2021-04-15] * see sequence * | 1-6, 8-16, 18-20 | |
| X | LEDDA M ET AL: "Effect of 3'UTR length on the translational regulation of 5'-terminal oligopyrimidine mRNAs", GENE, vol. 344, 3 January 2005 (2005-01-03), pages 213-220, XP027872598, ELSEVIER, AMSTERDAM, NL ISSN: 0378-1119 [retrieved on 2005-01-03] * the whole document * | 1-3,5-8, 11-16,20 | TECHNICAL FIELDS SEARCHED (IPC) |
| X | WO 2007/068265 A1 (KING FAISAL SPECIALIST HOSPITA [SA]; TERRAMARK MARKENCREATION GMBH [DE] 21 June 2007 (2007-06-21) * the whole document, in particular Example 1 and claims * | 1-3,5,8, 11-14, 16,18-20 | |
| X | DATABASE NUCLEOTIDE [Online] 30 May 1996 (1996-05-30), "Human ribosomal protein L9 mRNA, complete cds.", XP002729678, Database accession no. U09953.1 * the whole document * | 1,2,4-6, 8,11-13, 15,16,20 | |

-/--

The present search report has been drawn up for all claims

1

| Place of search | Date of completion of the search | Examiner |
|---|---|---|
| Munich | 18 May 2021 | Bassias, Ioannis |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 20 20 2943

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| | -& MAZURUK K ET AL: "Structural organization and chromosomal localization of the human ribosomal protein L9 gene.", BIOCHIMICA ET BIOPHYSICA ACTA, vol. 1305, no. 3, 1 March 1996 (1996-03-01), pages 151-162, XP002729679, ISSN: 0006-3002 * the whole document * ----- | | |
| A | WO 2013/143699 A1 (CUREVAC GMBH [DE]; THESS ANDREAS [DE]) 3 October 2013 (2013-10-03) ----- | 1-20 | |
| A | WO 2013/143700 A2 (CUREVAC GMBH [DE]; THES ANDREAS [DE]) 3 October 2013 (2013-10-03) ----- | 1-20 | |
| A | WO 2006/022712 A1 (PTC THERAPEUTICS INC [US]; CAO LIANGXIAN [US]; MEHTA ANURADHA [US]; NA) 2 March 2006 (2006-03-02) * the whole document * ----- | 1-20 | |

TECHNICAL FIELDS SEARCHED (IPC)

1 The present search report has been drawn up for all claims

| Place of search | Date of completion of the search | Examiner |
|---|---|---|
| Munich | 18 May 2021 | Bassias, Ioannis |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## ANNEX TO THE EUROPEAN SEARCH REPORT ON EUROPEAN PATENT APPLICATION NO. EP 20 20 2943

This annex lists the patent family members relating to the patent documents cited in the above-mentioned European search report.
The members are as contained in the European Patent Office EDP file on
The European Patent Office is in no way liable for these particulars which are merely given for the purpose of information.

18-05-2021

| Patent document cited in search report | Publication date | Patent family member(s) | Publication date |
|---|---|---|---|
| WO 2013102155 A1 | 04-07-2013 | NONE | |
| WO 2007068265 A1 | 21-06-2007 | AT 497009 T | 15-02-2011 |
| | | AU 2005339232 A1 | 21-06-2007 |
| | | CA 2632900 A1 | 21-06-2007 |
| | | EP 1974042 A1 | 01-10-2008 |
| | | US 2009181427 A1 | 16-07-2009 |
| | | WO 2007068265 A1 | 21-06-2007 |
| WO 2013143699 A1 | 03-10-2013 | AU 2013242404 A1 | 10-07-2014 |
| | | AU 2018264081 A1 | 06-12-2018 |
| | | BR 112014018210 A2 | 04-07-2017 |
| | | CA 2859452 A1 | 03-10-2013 |
| | | CN 104284979 A | 14-01-2015 |
| | | DK 2831241 T3 | 06-11-2017 |
| | | ES 2654205 T3 | 12-02-2018 |
| | | ES 2742473 T3 | 14-02-2020 |
| | | JP 6185553 B2 | 23-08-2017 |
| | | JP 6700227 B2 | 27-05-2020 |
| | | JP 2015512631 A | 30-04-2015 |
| | | JP 2018007682 A | 18-01-2018 |
| | | KR 20140142734 A | 12-12-2014 |
| | | MX 362981 B | 28-02-2019 |
| | | RU 2014142978 A | 20-05-2016 |
| | | RU 2018117941 A | 02-11-2018 |
| | | SG 10201607968W A | 29-12-2016 |
| | | SG 10201710631R A | 27-02-2018 |
| | | SG 11201403450V A | 26-09-2014 |
| | | US 2015218554 A1 | 06-08-2015 |
| | | US 2017247699 A1 | 31-08-2017 |
| | | US 2020332293 A1 | 22-10-2020 |
| | | WO 2013143699 A1 | 03-10-2013 |
| WO 2013143700 A2 | 03-10-2013 | AU 2013242405 A1 | 25-09-2014 |
| | | BR 112014023898 A2 | 11-07-2017 |
| | | CA 2866945 A1 | 03-10-2013 |
| | | CN 104321432 A | 28-01-2015 |
| | | CN 108929880 A | 04-12-2018 |
| | | ES 2660129 T3 | 20-03-2018 |
| | | JP 6301906 B2 | 28-03-2018 |
| | | JP 2015517803 A | 25-06-2015 |
| | | KR 20140139101 A | 04-12-2014 |
| | | MX 358706 B | 31-08-2018 |
| | | RU 2014142881 A | 20-05-2016 |
| | | SG 10201607966U A | 29-11-2016 |
| | | SG 11201405545X A | 27-11-2014 |

EPO FORM P0459

For more details about this annex : see Official Journal of the European Patent Office, No. 12/82

## ANNEX TO THE EUROPEAN SEARCH REPORT ON EUROPEAN PATENT APPLICATION NO. EP 20 20 2943

This annex lists the patent family members relating to the patent documents cited in the above-mentioned European search report.
The members are as contained in the European Patent Office EDP file on
The European Patent Office is in no way liable for these particulars which are merely given for the purpose of information.

18-05-2021

| Patent document cited in search report | Publication date | Patent family member(s) | Publication date |
|---|---|---|---|
| | | US 2015050302 A1 | 19-02-2015 |
| | | WO 2013143700 A2 | 03-10-2013 |
| WO 2006022712 A1 | 02-03-2006 | CA 2574076 A1 | 02-03-2006 |
| | | EP 1786933 A1 | 23-05-2007 |
| | | EP 2351859 A1 | 03-08-2011 |
| | | JP 5539180 B2 | 02-07-2014 |
| | | JP 5824407 B2 | 25-11-2015 |
| | | JP 6081412 B2 | 15-02-2017 |
| | | JP 2008507271 A | 13-03-2008 |
| | | JP 2011103886 A | 02-06-2011 |
| | | JP 2012187106 A | 04-10-2012 |
| | | JP 2015015950 A | 29-01-2015 |
| | | US 2005048549 A1 | 03-03-2005 |
| | | US 2015253304 A1 | 10-09-2015 |
| | | WO 2006022712 A1 | 02-03-2006 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 02098443 A
- WO 2013143700 A
- WO 2012019780 A
- US 4373071 A
- US 4401796 A
- US 4415732 A
- US 4458066 A
- US 4500707 A
- US 4668777 A
- US 4973679 A
- US 5047524 A
- US 5132418 A
- US 5153319 A
- US 5262530 A
- US 5700642 A
- WO 2010037539 A
- WO 2013143698 A


### Non-patent literature cited in the description


- **FRIEDEL et al.** Conserved principles of mammalian transcriptional regulation revealed by RNA half-life. *Nucleic Acid Research,* 2009, vol. 37 (17), 1-12
- **JOHNSON et al.** Newly synthesized RNA: Simultaneous measurement in intact cells of transcription rates and RNA stability of insulin-like growth factor I, actin, and albumin in growth hormone-stimulated hepatocytes. *Proc. Natl. Acad. Sci.,* 1991, vol. 88, 5287-5291
- **RODGERS et al.** Regulated α-globin mRNA decay is a cytoplasmic event proceeding through 3'-to-5' exosome-dependent decapping. *RNA,* 2002, vol. 8, 1526-1537
- **WANG et al.** An mRNA stability complex functions with poly(A)-binding protein to stabilize mRNA in vitro. *Molecular and Cellular biology,* July 1999, vol. 19 (7), 4552-4560
- **LEDDA et al.** Effect of the 3'-UTR length on the translational regulation of 5'-terminal oligopyrimidine mRNAs. *Gene,* 2005, vol. 344, 213-220
- **JANOVICK-GURETZKY et al.** Housekeeping Gene Expression in Bovine Liver is Affected by Physiological State, Feed Intake, and Dietary Treatment. *J. Dairy Sci.,* 2007, vol. 90, 2246-2252
- **AKASHI.** *Curr. Opin. Genet. Dev.,* 2001, vol. 11 (6), 660-666
- **CAKMAKCI ; LERNER ; WAGNER ; ZHENG ; WILLIAM F MARZLUFF.** *Mol. Cell. Biol.,* 2008, vol. 28 (3), 1182-94